(19) Europäisches Patentamt
European Patent Office
Office européen des brevets

(11) EP 4 310 931 A1

(12) EUROPEAN PATENT APPLICATION
published in accordance with Art. 153(4) EPC

(43) Date of publication:
**24.01.2024 Bulletin 2024/04**

(21) Application number: **22771494.6**

(22) Date of filing: **17.03.2022**

(51) International Patent Classification (IPC):
***H01L 51/50*** *(2006.01)* ***C07D 491/048*** *(2006.01)*
***C07D 495/04*** *(2006.01)* ***C09K 11/06*** *(2006.01)*

(52) Cooperative Patent Classification (CPC):
**C07D 491/048; C07D 495/04; C09K 11/06**

(86) International application number:
**PCT/JP2022/012157**

(87) International publication number:
**WO 2022/196749 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.03.2021 JP 2021045119**
**14.07.2021 JP 2021116642**

(71) Applicants:
- **Idemitsu Kosan Co., Ltd**
  **Tokyo 100-8321 (JP)**
- **Toray Industries, Inc.**
  **Tokyo 103-8666 (JP)**

(72) Inventors:
- **SHIOMI, Takushi**
  **Tokyo 100-8321 (JP)**
- **MATSUMOTO, Hisato**
  **Tokyo 100-8321 (JP)**
- **JINDE, Yukitoshi**
  **Tokyo 100-8321 (JP)**
- **HIGASHINO, Yuta**
  **Tokyo 100-8321 (JP)**
- **OGIWARA, Toshinari**
  **Tokyo 100-8321 (JP)**
- **HOSHINO, Hidetaka**
  **Otsu-shi, Shiga 520-8558 (JP)**
- **NAGAO, Kazumasa**
  **Otsu-shi, Shiga 520-8558 (JP)**

(74) Representative: **Gille Hrabal Partnerschaftsgesellschaft mbB**
**Patentanwälte**
**Brucknerstraße 20**
**40593 Düsseldorf (DE)**

# (54) ORGANIC ELECTROLUMINESCENT ELEMENT, COMPOUND, AND ELECTRONIC DEVICE

(57) An organic electroluminescence device (1) includes an anode (3), a cathode (4), and an emitting layer (5) provided between the anode (3) and the cathode (4), in which the emitting layer (5) contains a compound M3 represented by a formula (1) and a delayed fluorescent compound M2, the compound M3 and the compound M2 are different in structure, and a singlet energy $S_1$(M3) of the compound M3 and a singlet energy $S_1$(M2) of the compound M2 satisfy a relationship of a numerical formula (Numerical Formula 1) below. $S_1(M3) > S_1(M2)$...(Numerical Formula 1).

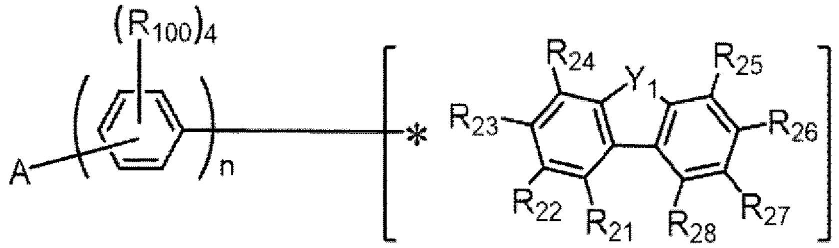

(1)

EP 4 310 931 A1

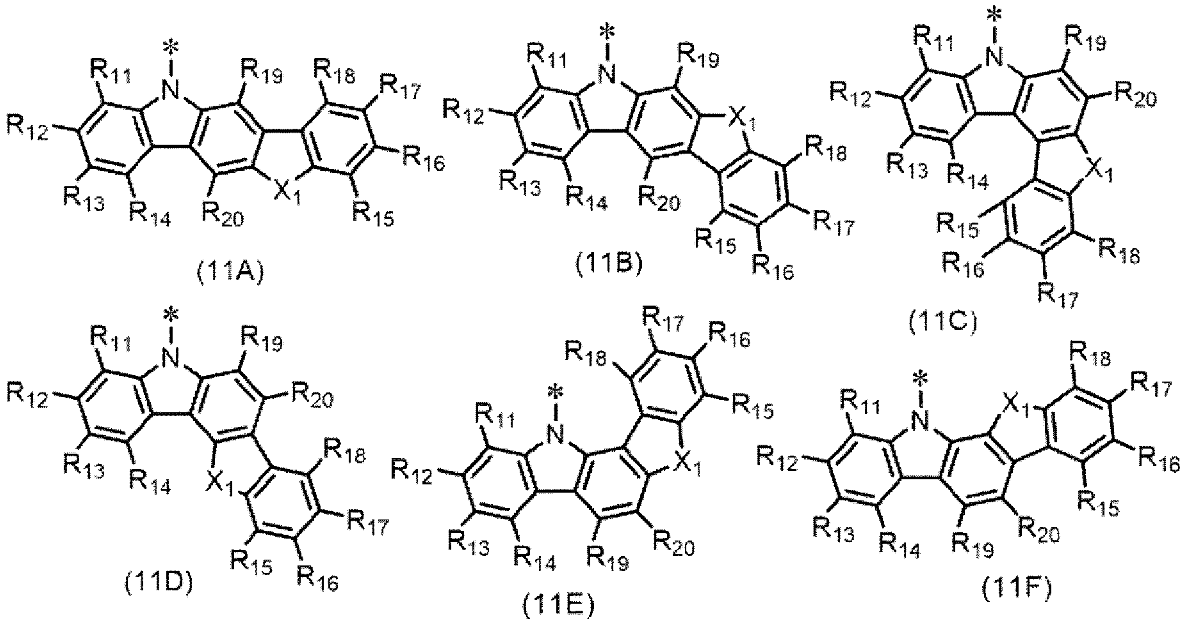

(11A) (11B) (11C) (11D) (11E) (11F)

In the formula (1), A is a group represented by any one of formulae (11A), (11B), (11C), (11D), (11E), and (11F) below, and $Y_1$ is an oxygen atom or a sulfur atom.

FIG.1

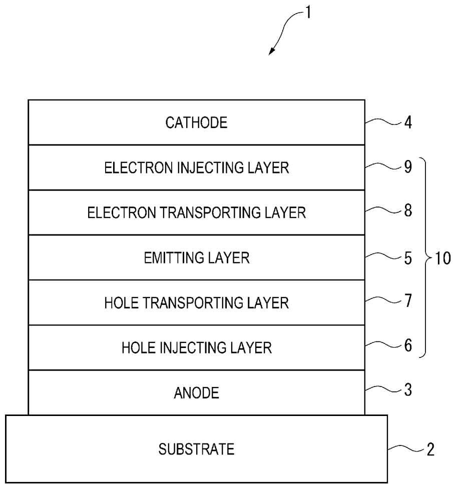

**Description**

TECHNICAL FIELD

The present invention relates to an organic electroluminescence device, a compound, and an electronic device.

BACKGROUND ART

When a voltage is applied to an organic electroluminescence device (hereinafter, occasionally referred to as "organic EL device"), holes are injected from an anode and electrons are injected from a cathode into an emitting layer. The injected electrons and holes are recombined in the emitting layer to form excitons. Specifically, according to the electron spin statistics theory, singlet excitons and triplet excitons are generated at a ratio of 25%:75%.
A fluorescent organic EL device using light emission from singlet excitons has been applied to a full-color display such as a mobile phone and a television set, but an internal quantum efficiency is said to be at a limit of 25%. Studies have thus been made to improve performance of the organic EL device.
For instance, the organic EL device is expected to emit light more efficiently using triplet excitons in addition to singlet excitons. In view of the above, a highly-efficient fluorescent organic EL device using thermally activated delayed fluorescence (hereinafter simply referred to as "delayed fluorescence" in some cases) has been proposed and studied.
For instance, TADF (Thermally Activated Delayed Fluorescence) mechanism has been studied. This TADF mechanism uses such a phenomenon in which inverse intersystem crossing from triplet excitons to singlet excitons thermally occurs when a material having a small energy difference ($\Delta ST$) between singlet energy level and triplet energy level is used. Thermally activated delayed fluorescence is explained in "Yuki Hando-tai no Debaisu Bussei (Device Physics of Organic Semiconductors)" (edited by ADACHI Chihaya, published by Kodansha, issued on April 1, 2012, on pages 261-268).
For instance, Patent Literatures 1 and 2 disclose a compound having a benzofurocarbazole ring or a benzothienocarbazole ring as a compound usable for an organic EL device in order to improve a performance of the organic EL device. Patent Literature 2 also discloses an organic EL device using the TADF mechanism.
The performance of the organic EL device is evaluable in terms of, for instance, luminance, emission wavelength, chromaticity, luminous efficiency, drive voltage, and lifetime.

CITATION LIST

PATENT LITERATURE(S)

Patent Literature 1: International Publication No. WO 2013/011891
Patent Literature 2: International Publication No. WO 2020/122118

SUMMARY OF THE INVENTION

PROBLEM(S) TO BE SOLVED BY THE INVENTION

A further improvement in performance has been required in an organic EL device using the TADF mechanism.
An object of the invention is to provide a high-performance organic electroluminescence device, a compound capable of producing the high-performance organic electroluminescence device, and an electronic device including the organic electroluminescence device.

MEANS FOR SOLVING THE PROBLEM(S)

According to an aspect of the invention, there is provided an organic electroluminescence device including: an anode; a cathode; an emitting layer provided between the anode and the cathode, in which

the emitting layer contains a compound M3 represented by a formula (1) below and a delayed fluorescent compound M2,
the compound M3 and the compound M2 are different in structure, and
a singlet energy $S_1(M3)$ of the compound M3 and a singlet energy $S_1(M2)$ of the compound M2 satisfy a relationship of a numerical formula (Numerical Formula 1) below.

$$S_1(M3) > S_1(M2) \quad \text{...(Numerical Formula 1)}$$

[Formula 1]

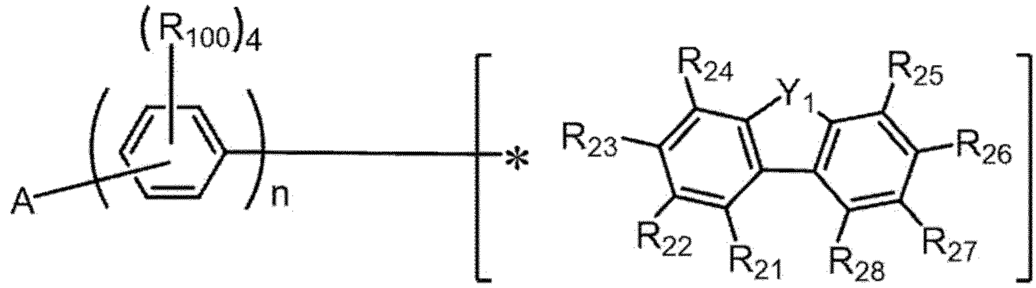

(1)

In the formula (1):

A is a group represented by any one of formulae (11A), (11B), (11C), (11D), (11E), and (11F) below;
$Y_1$ is an oxygen atom or a sulfur atom;
n is 0 or 1;
at least one combination of adjacent two or more of $R_{21}$ to $R_{28}$ are mutually bonded to form a substituted or unsubstituted monocyclic ring, mutually bonded to form a substituted or unsubstituted fused ring, or not mutually bonded;
$R_{21}$ to $R_{28}$ forming neither the substituted or unsubstituted monocyclic ring nor the substituted or unsubstituted fused ring, and $R_{100}$ are each independently a hydrogen atom, a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted cycloalkyl group having 3 to 30 ring carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, a group represented by $-N(Rz)_2$, a thiol group, a substituted or unsubstituted alkylthio group having 1 to 30 carbon atoms, a substituted or unsubstituted aralkyl group having 7 to 30 ring carbon atoms, a substituted germanium group, a substituted phosphine oxide group, a nitro group, a substituted boryl group, or a substituted or unsubstituted arylthio group having 6 to 30 ring carbon atoms;
Rz is a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms;
two Rz in $-N(Rz)_2$ are mutually the same or different;
a plurality of $R_{100}$ are mutually the same or different;
when n is 0 and $X_1$ in the formulae (11A), (11B), (11C), (11D), (11E), and (11F) is an oxygen atom, $R_{25}$ is not a substituted or unsubstituted dibenzofuranyl group; and
* in the formula (1) represents a bonding position to any one of carbon atoms of a six-membered ring to which $R_{21}$ to $R_{24}$ are bonded.

[Formula 2]

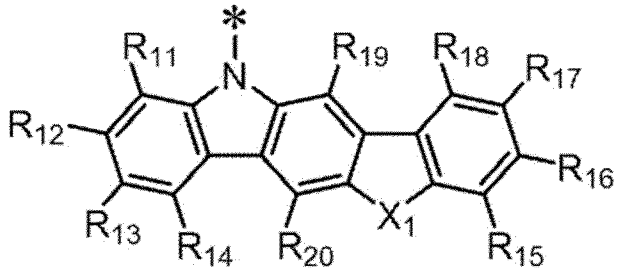

(11A)

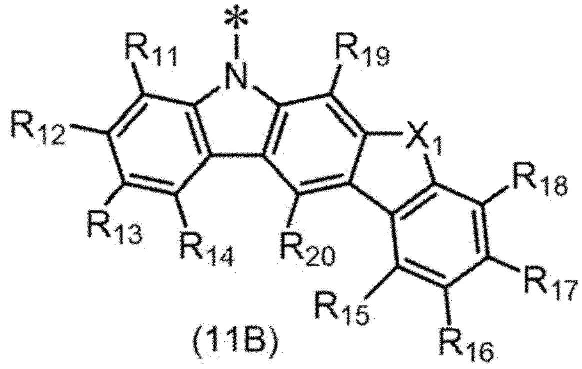

(11B)

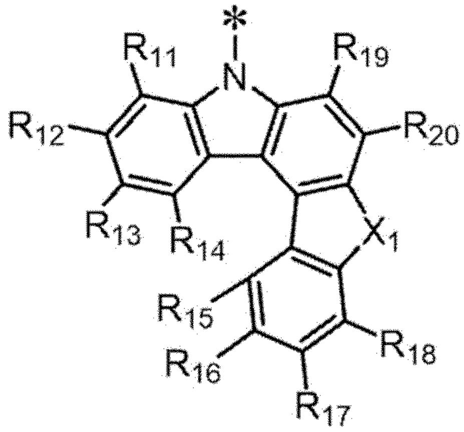

(11C)

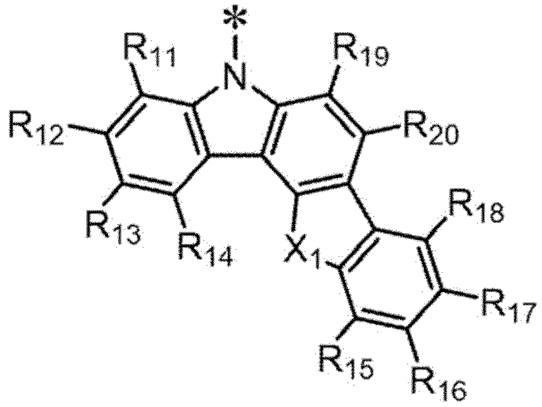

(11D)

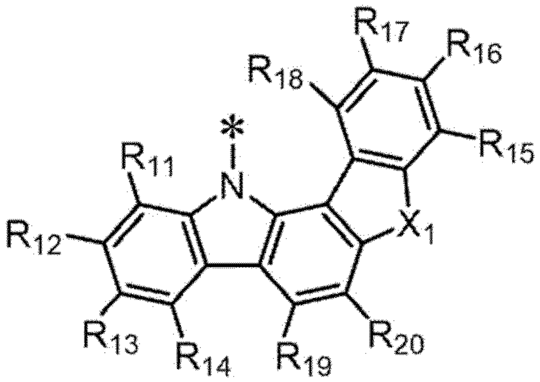

(11E)

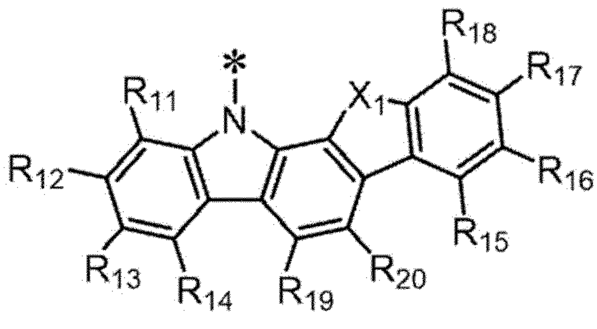

(11F)

In the formulae (11A), (11B), (11C), (11D), (11E), and (11F):

$X_1$ is an oxygen atom or a sulfur atom;
at least one combination of adjacent two or more of $R_{11}$ to $R_{14}$ are mutually bonded to form a substituted or unsubstituted monocyclic ring, mutually bonded to form a substituted or unsubstituted fused ring, or not mutually bonded;
at least one combination of adjacent two or more of $R_{15}$ to $R_{18}$ are mutually bonded to form a substituted or unsubstituted monocyclic ring, mutually bonded to form a substituted or unsubstituted fused ring, or not mutually bonded;
$R_{19}$ and $R_{20}$ are each a hydrogen atom;
$R_{11}$ to $R_{18}$ forming neither the substituted or unsubstituted monocyclic ring nor the substituted or unsubstituted fused ring each independently represent the same as $R_{21}$ to $R_{28}$ forming neither the substituted or unsubstituted monocyclic ring nor the substituted or unsubstituted fused ring in the formula (1);
when n is 0, * represents a bonding position to any one of carbon atoms of a six-membered ring to which $R_{21}$ to $R_{24}$ are bonded in the formula (1); and

when n is 1, * represents a bonding position to any one of carbon atoms of a benzene ring to which $R_{100}$ is bonded in the formula (1).

According to another aspect of the invention, there is provided an organic electroluminescence device including: an anode; a cathode; an emitting layer provided between the anode and the cathode, in which

the emitting layer contains a compound M3 represented by a formula (1) below and a delayed fluorescent compound M2,
the compound M3 and the compound M2 are different in structure, and
a singlet energy $S_1(M3)$ of the compound M3 and a singlet energy $S_1(M2)$ of the compound M2 satisfy a relationship of a numerical formula (Numerical Formula 1) below.

$$S_1(M3) > S_1(M2) \quad \text{...(Numerical Formula 1)}$$

[Formula 3]

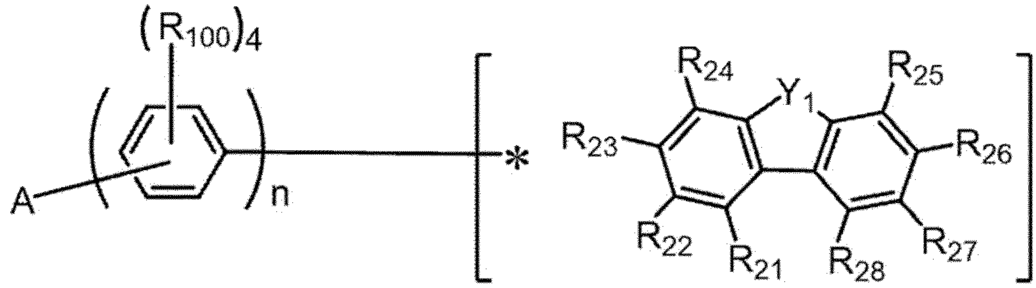
(1)

In the formula (1):

A is a group represented by any one of formulae (11A), (11B), (11C), (11D), (11E), and (11F) below;
$Y_1$ is an oxygen atom or a sulfur atom;
n is 0 or 1;
at least one combination of adjacent two or more of $R_{21}$ to $R_{28}$ are mutually bonded to form a substituted or unsubstituted monocyclic ring, mutually bonded to form a substituted or unsubstituted fused ring, or not mutually bonded;
$R_{21}$ to $R_{28}$ forming neither the substituted or unsubstituted monocyclic ring nor the substituted or unsubstituted fused ring, and $R_{100}$ are each independently a hydrogen atom, a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted cycloalkyl group having 3 to 30 ring carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, a group represented by $-N(Rz)_2$, a thiol group, a substituted or unsubstituted alkylthio group having 1 to 30 carbon atoms, a substituted or unsubstituted aralkyl group having 7 to 30 ring carbon atoms, a substituted germanium group, a substituted phosphine oxide group, a nitro group, a substituted boryl group, or a substituted or unsubstituted arylthio group having 6 to 30 ring carbon atoms;
Rz is a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms;
two Rz in $-N(Rz)_2$ are mutually the same or different;
a plurality of $R_{100}$ are mutually the same or different;
when n is 0 and $X_1$ in the formulae (11A), (11B), (11C), (11D), (11E), and (11F) is an oxygen atom, $R_{25}$ is not a substituted or unsubstituted dibenzofuranyl group; and
* in the formula (1) represents a bonding position to any one of carbon atoms of a six-membered ring to which $R_{21}$ to $R_{24}$ are bonded.

[Formula 4]

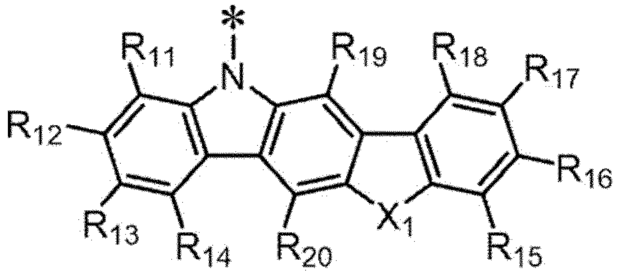

(11A)

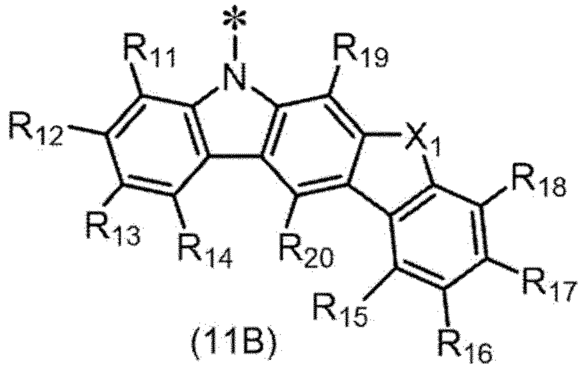

(11B)

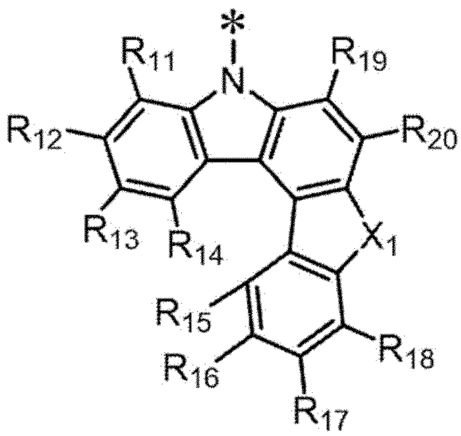

(11C)

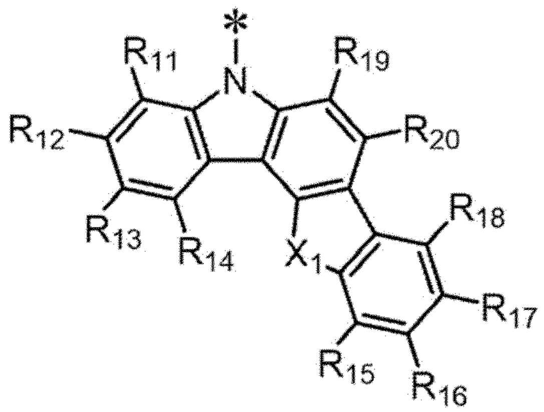

(11D)

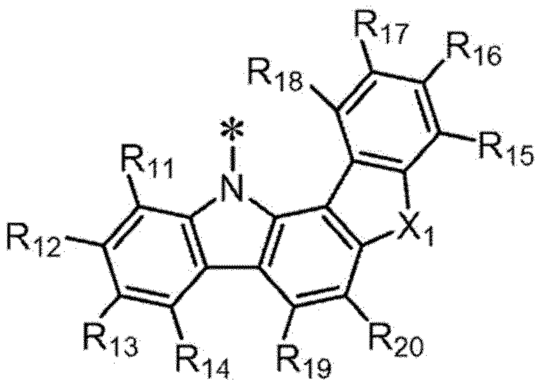

(11E)

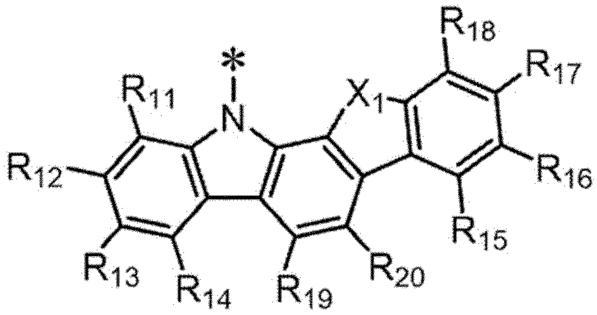

(11F)

In the formulae (11A), (11B), (11C), (11D), (11E), and (11F):

$X_1$ is an oxygen atom or a sulfur atom;

at least one combination of adjacent two or more of $R_{11}$ to $R_{20}$ are mutually bonded to form a substituted or unsubstituted monocyclic ring, mutually bonded to form a substituted or unsubstituted fused ring, or not mutually bonded;

$R_{11}$ to $R_{20}$ forming neither the substituted or unsubstituted monocyclic ring nor the substituted or unsubstituted fused ring each independently represent the same as $R_{21}$ to $R_{28}$ forming neither the substituted or unsubstituted monocyclic ring nor the substituted or unsubstituted fused ring in the formula (1);

when n is 0, * represents a bonding position to any one of carbon atoms of a six-membered ring to which $R_{21}$ to $R_{24}$ are bonded in the formula (1); and

when n is 1, * represents a bonding position to any one of carbon atoms of a benzene ring to which $R_{100}$ is bonded

in the formula (1).

According to still another aspect of the invention, there is provided an electronic device including the organic electroluminescence device according to the aspect of the invention.

According to a further aspect of the invention, there is provided a compound represented by a formula (100) below.

[Formula 5]

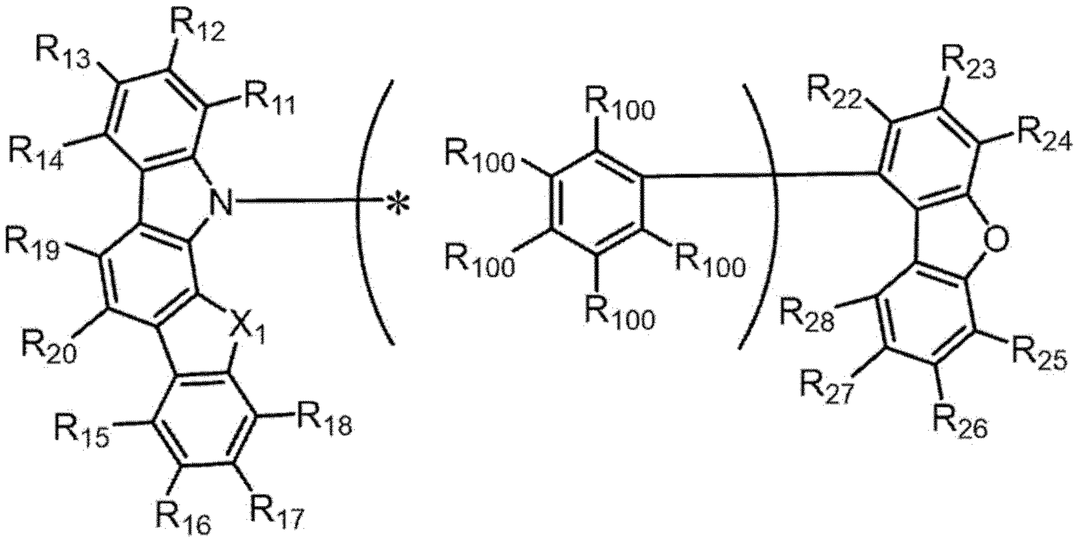

(100)

In the formula (100):

$X_1$ is an oxygen atom or a sulfur atom;

$R_{100}$, $R_{11}$ to $R_{20}$, and $R_{22}$ to $R_{28}$ are each independently a hydrogen atom, or a group represented by $-(L_{101})nx-R_{101}$;

nx is 0, 1, 2, or 3;

when a plurality of groups represented by $-(L_{101})nx-R_{101}$ are present, the plurality of groups represented by $-(L_{101})nx-R_{101}$ are mutually the same or different;

a plurality of $R_{100}$ are mutually the same or different;

$R_{101}$ is an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, an unsubstituted dibenzothienyl group, an unsubstituted (9-dibenzofuranyl)carbazolyl group, an unsubstituted (9-dibenzothienyl)carbazolyl group, a monovalent group derived from a compound represented by a formula (101) below, a monovalent group derived from a compound represented by a formula (102) below, a monovalent group derived from a compound represented by a formula (103) below, a monovalent group derived from a compound represented by a formula (104) below, a monovalent group derived from a compound represented by a formula (105) below, or a monovalent group derived from a compound represented by a formula (106) below;

$L_{101}$ is a substituted or unsubstituted alkylene group having 1 to 30 carbon atoms, a substituted or unsubstituted phenylene group, a substituted or unsubstituted dibenzofuranylene group, a substituted or unsubstituted dibenzothienylene group, a substituted or unsubstituted carbazolylene group, a substituted or unsubstituted (9-dibenzofuranyl)carbazolylene group, a substituted or unsubstituted (9-dibenzothienyl)carbazolylene group, a divalent group derived from a compound represented by the formula (101) below, a divalent group derived from a compound represented by the formula (102) below, a divalent group derived from a compound represented by the formula (103) below, a divalent group derived from a compound represented by the formula (104) below, a divalent group derived from a compound represented by the formula (105) below, or a divalent group derived from a compound represented by the formula (106) below;

when $L_{101}$ is a substituted alkylene group having 1 to 30 carbon atoms, a substituted phenylene group, a substituted dibenzofranylene group, a substituted dibenzothienylene group, a substituted carbazolylene group, a substituted (9-dibenzofuranyl)carbazolylene group, or a substituted (9-dibenzothienyl)carbazolylene group, a substituent for

$L_{101}$ is each independently an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;
when two or more $L_{101}$ are present, the two or more $L_{101}$ are mutually the same or different;
when two or more $R_{101}$ are present, the two or more $R_{101}$ are mutually the same or different; and
* represents a bonding position to any one of carbon atoms of a benzene ring to which $R_{100}$ is bonded.

[Formula 6]

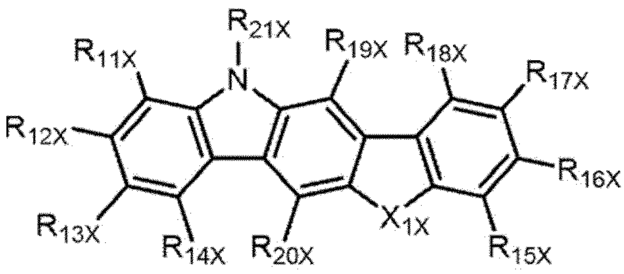

(101)

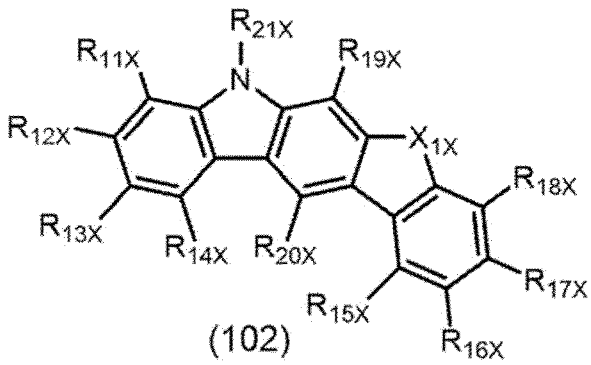

(102)

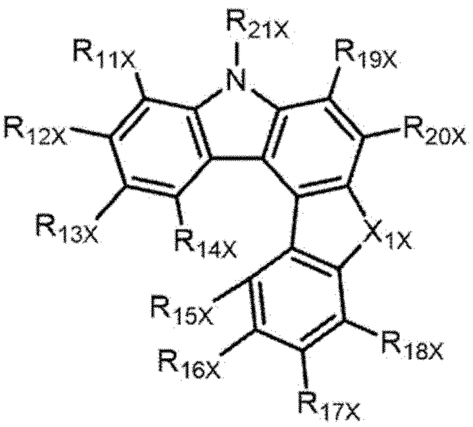

(103)

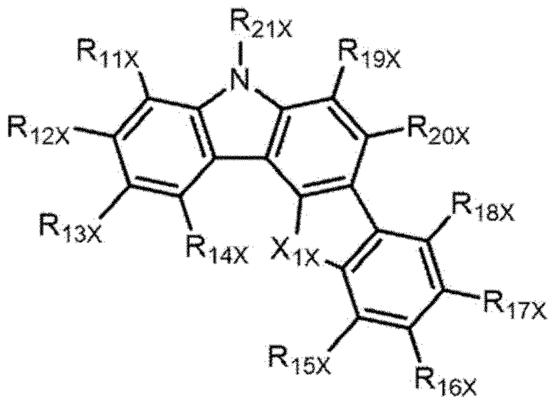

(104)

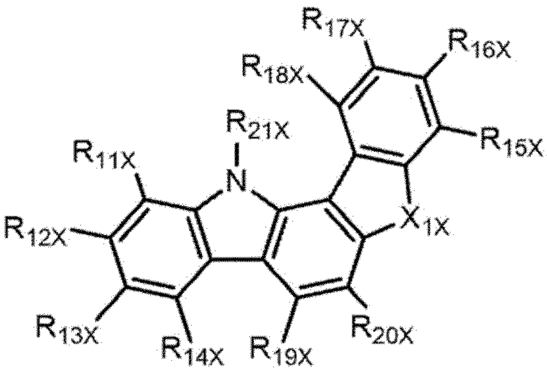

(105)

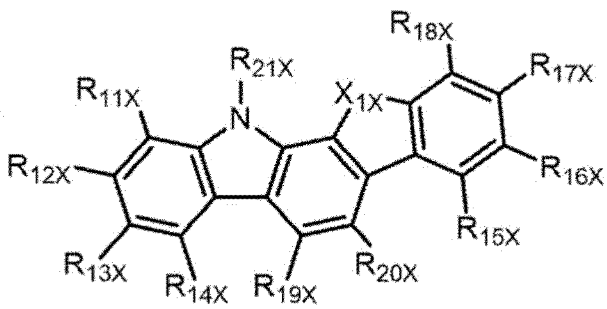

(106)

In the formulae (101) to (106):

$X_{1X}$ is an oxygen atom or a sulfur atom;

$R_{11X}$ to $R_{21X}$ are each independently a hydrogen atom, an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;

when $R_{101}$ is a monovalent group derived from a compound of any one of the formulae (101) to (106) and nx is 0, any one atom of: carbon atoms of a six-membered ring to which $R_{11X}$ to $R_{20X}$ are bonded; and a nitrogen atom bonded to $R_{21X}$, is bonded to any one of carbon atoms of a six-membered ring to which $R_{11}$ to $R_{20}$, $R_{22}$ to $R_{28}$, and $R_{100}$ are bonded,

when nx is 1, 2, or 3, any one atom of: carbon atoms of the six-membered ring to which $R_{11X}$ to $R_{20X}$ are bonded; and a nitrogen atom bonded to $R_{21X}$, is bonded to $L_{101}$;

when $L_{101}$ is a divalent group derived from a compound of any one of the formulae (101) to (106) and nx is 1, one of two atoms of: carbon atoms of a six-membered ring to which $R_{11X}$ to $R_{20X}$ are bonded; and a nitrogen atom bonded to $R_{21X}$, is bonded to $R_{101}$ and the other of the two atoms is bonded to any one of carbon atoms of a six-membered ring to which $R_{11}$ to $R_{20}$, $R_{22}$ to $R_{28}$, and $R_{100}$ are bonded; and

when $L_{101}$ is a divalent group derived from a compound of any one of the formulae (101) to (106) and nx is 2 or 3, one of two atoms of: carbon atoms of a six-membered ring to which $R_{11X}$ to $R_{20X}$ are bonded; and a nitrogen atom bonded to $R_{21X}$, is bonded to $R_{101}$ or $L_{101}$, and the other of the two atoms is bonded to $L_{101}$ or any one of carbon atoms of a six-membered ring to which $R_{11}$ to $R_{20}$, $R_{22}$ to $R_{28}$, and $R_{100}$ are bonded.

According to the above aspects of the invention, there can be provided a high-performance organic electroluminescence device, a compound capable of producing the high-performance organic electroluminescence device, and an electronic device including the organic electroluminescence device.

BRIEF DESCRIPTION OF DRAWINGS

Fig. 1 schematically illustrates an exemplary arrangement of an organic electroluminescence device according to a first exemplary embodiment of the invention.

Fig. 2 schematically depicts an apparatus for measuring transient PL.

Fig. 3 illustrates an example of decay curves of the transient PL.

Fig. 4 schematically illustrates a relationship in energy level and energy transfer between a compound M3 and a compound M2 in an emitting layer of an exemplary organic electroluminescence device according to the first exemplary embodiment of the invention.

Fig. 5 schematically illustrates a relationship in energy level and energy transfer between the compound M3, the compound M2 and a compound M1 in an emitting layer of an exemplary organic electroluminescence device according to a second exemplary embodiment of the invention.

DESCRIPTION OF EMBODIMENT(S)

Definitions

Herein, a hydrogen atom includes isotope having different specifically, protium, deuterium and tritium.

In chemical formulae herein, it is assumed that a hydrogen atom (i.e. protium, deuterium and tritium) is bonded to each of bondable positions that are not annexed with signs "R" or the like or "D" representing a deuterium.

Herein, the ring carbon atoms refer to the number of carbon atoms among atoms forming a ring of a compound (e.g., a monocyclic compound, fused-ring compound, crosslinking compound, carbon ring compound, and and heterocyclic compound) in which the atoms are bonded to each other to form the ring. When the ring is substituted by a substituent(s), carbon atom(s) contained in the substituent(s) is not counted in the ring carbon atoms. Unless specifically described, the same applies to the "ring carbon atoms" described later. For instance, a benzene ring has 6 ring carbon atoms, a naphthalene ring has 10 ring carbon atoms, a pyridine pyridine ring has 5 ring carbon atoms, and a furan ring 4 ring carbon atoms. For instance, a 9,9-diphenylfluorenyl group has 13 ring carbon atoms and 9,9'-spirobifluorenyl group has 25 ring carbon atoms.

When a benzene ring is substituted by a substituent (, e.g., an alkyl group), the number of carbon atoms of the alkyl group is not counted in the number of the ring carbon atoms. Accordingly, the benzene ring substituted by an alkyl group has 6 ring carbon atoms. When a naphthalene ring is substituted by a substituent in a form of, for instance, an alkyl group, the number of carbon atoms of the alkyl group is not counted in the number of the ring carbon atoms of the naphthalene ring. Accordingly, the naphthalene ring substituted by an alkyl group has 10 ring carbon atoms.

Herein, the ring atoms refer to the number of atoms forming a ring of a compound (e.g., a monocyclic compound,

fused-ring compound, cross-linking compound, carbon ring compound, and heterocyclic compound) in which the atoms are bonded to each other to form the ring (e.g., monocyclic ring, fused ring, and ring assembly). Atom(s) not forming the ring (e.g., hydrogen atom(s) for saturating the valence of the atom which forms the ring) and atom(s) in a substituent by which the ring is substituted are not counted as the ring atoms. Unless otherwise specified, the same applies to the "ring atoms" described later. For instance, a pyridine ring has 6 ring atoms, a quinazoline ring has 10 ring atoms, and a furan ring has 5 ring atoms. For instance, the number of hydrogen atom(s) bonded to a pyridine ring or the number of atoms forming a substituent is not counted as the pyridine ring atoms. Accordingly, a pyridine ring bonded to a hydrogen atom(s) or a substituent(s) has 6 ring atoms. For instance, the hydrogen atom(s) bonded to carbon atom(s) of a quinazoline ring or the atoms forming a substituent are not counted as the quinazoline ring atoms. Accordingly, a quinazoline ring bonded to hydrogen atom(s) or a substituent(s) has 10 ring atoms.

Herein, "XX to YY carbon atoms" in the description of "substituted or unsubstituted ZZ group having XX to YY carbon atoms" represent carbon atoms of an unsubstituted ZZ group and do not include carbon atoms of a substituent(s) of the substituted ZZ group. Herein, "YY" is larger than "XX," "XX" representing an integer of 1 or more and "YY" representing an integer of 2 or more.

Herein, "XX to YY atoms" in the description of "substituted or unsubstituted ZZ group having XX to YY atoms" represent atoms of an unsubstituted ZZ group and does not include atoms of a substituent(s) of the substituted ZZ group. Herein, "YY" is larger than "XX," "XX" representing an integer of 1 or more and "YY" representing an integer of 2 or more.

Herein, an unsubstituted ZZ group refers to an "unsubstituted ZZ group" in a "substituted or unsubstituted ZZ group," and a substituted ZZ group refers to a "substituted ZZ group" in a "substituted or unsubstituted ZZ group."

Herein, the term "unsubstituted" used in a "substituted or unsubstituted ZZ group" means that a hydrogen atom(s) in the ZZ group is not substituted with a substituent(s). The hydrogen atom(s) in the "unsubstituted ZZ group" is protium, deuterium, or tritium.

Herein, the term "substituted" used in a "substituted or unsubstituted ZZ group" means that at least one hydrogen atom in the ZZ group is substituted with a substituent. Similarly, the term "substituted" used in a "BB group substituted by AA group" means that at least one hydrogen atom in the BB group is substituted with the AA group.

## Substituents Mentioned Herein

Substituents mentioned herein will be described below.

An "unsubstituted aryl group" mentioned herein has, unless otherwise specified herein, 6 to 50, preferably 6 to 30, more preferably 6 to 18 ring carbon atoms.

An "unsubstituted heterocyclic group" mentioned herein has, unless otherwise specified herein, 5 to 50, preferably 5 to 30, more preferably 5 to 18 ring atoms.

An "unsubstituted alkyl group" mentioned herein has, unless otherwise specified herein, 1 to 50, preferably 1 to 20, more preferably 1 to 6 carbon atoms.

An "unsubstituted alkenyl group" mentioned herein has, unless otherwise specified herein, 2 to 50, preferably 2 to 20, more preferably 2 to 6 carbon atoms.

An "unsubstituted alkynyl group" mentioned herein has, unless otherwise specified herein, 2 to 50, preferably 2 to 20, more preferably 2 to 6 carbon atoms.

An "unsubstituted cycloalkyl group" mentioned herein has, unless otherwise specified herein, 3 to 50, preferably 3 to 20, more preferably 3 to 6 ring carbon atoms.

An "unsubstituted arylene group" mentioned herein has, unless otherwise specified herein, 6 to 50, preferably 6 to 30, more preferably 6 to 18 ring carbon atoms.

An "unsubstituted divalent heterocyclic group" mentioned herein has, unless otherwise specified herein, 5 to 50, preferably 5 to 30, more preferably 5 to 18 ring atoms.

An "unsubstituted alkylene group" mentioned herein has, unless otherwise specified herein, 1 to 50, preferably 1 to 20, more preferably 1 to 6 carbon atoms.

## Substituted or Unsubstituted Aryl Group

Specific examples (specific example group G1) of the "substituted or unsubstituted aryl group" mentioned herein include unsubstituted aryl groups (specific example group G1A) below and substituted aryl groups (specific example group G1B). Herein, an unsubstituted aryl group refers to an "unsubstituted aryl group" in a "substituted or unsubstituted aryl group", and a substituted aryl group refers to a "substituted aryl group" in a "substituted or unsubstituted aryl group. A simply termed "aryl group" herein includes both of an "unsubstituted aryl group" and a "substituted aryl group."

The "substituted aryl group" refers to a group derived by substituting at least one hydrogen atom in an "unsubstituted aryl group" with a substituent. Examples of the "substituted aryl group" include a group derived by substituting at least one hydrogen atom in the "unsubstituted aryl group" in the specific example group G1A below with a substituent,

and examples of the substituted aryl group in the specific example group G1B below. It should be noted that the examples of the "unsubstituted aryl group" and the "substituted aryl group" mentioned herein are merely exemplary, and the "substituted aryl group" mentioned herein includes a group derived by further substituting a hydrogen atom bonded to a carbon atom of a skeleton of a "substituted aryl group" in the specific example group G1B below, and a group derived by further substituting a hydrogen atom of a substituent of the "substituted aryl group" in the specific example group G1B below.

Unsubstituted Aryl Group (Specific Example Group G1A):
a phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, benzanthryl group, phenanthryl group, benzophenanthryl group, phenalenyl group, pyrenyl group, chrysenyl group, benzochrysenyl group, triphenylenyl group, benzotriphenylenyl group, tetracenyl group, pentacenyl group, fluorenyl group, 9,9'-spirobifluorenyl group, benzofluorenyl group, dibenzofluorenyl group, fluoranthenyl group, benzofluoranthenyl group, perylenyl group, and <<nret>> monovalent aryl group derived by removing one hydrogen atom from cyclic structures represented by formulae (TEMP-1) to (TEMP-15) below.

[Formula 7]

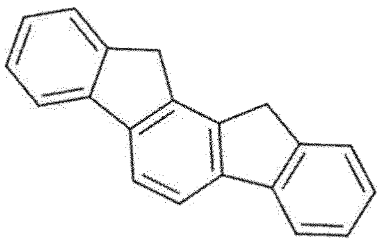
(TEMP-1)

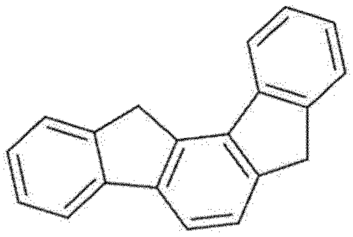
(TEMP-2)

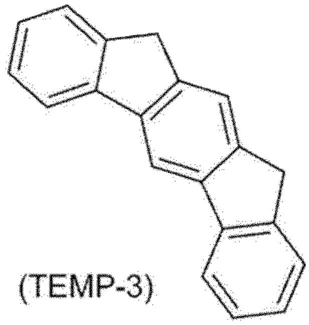
(TEMP-3)

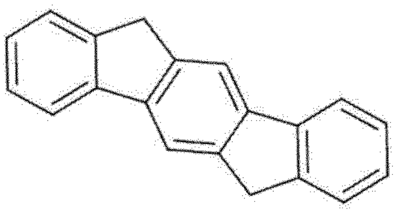
(TEMP-4)

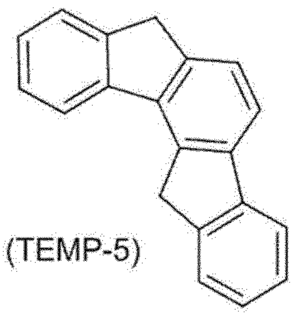
(TEMP-5)

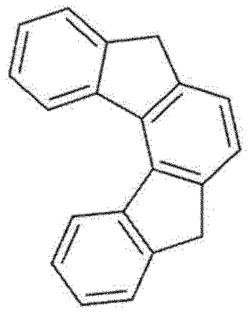
(TEMP-6)

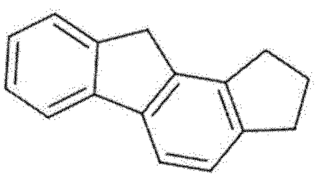
(TEMP-7)

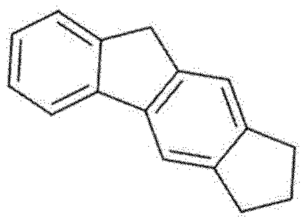
(TEMP-8)

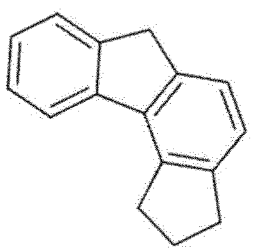
(TEMP-9)

[Formula 8]

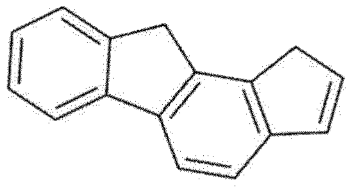

(TEMP-10)

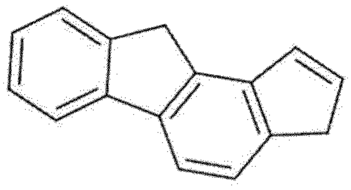

(TEMP-11)

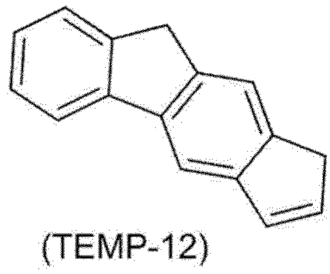

(TEMP-12)

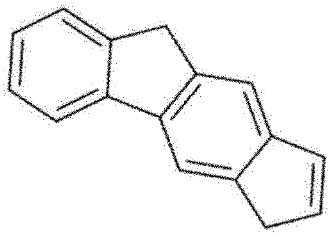

(TEMP-13)

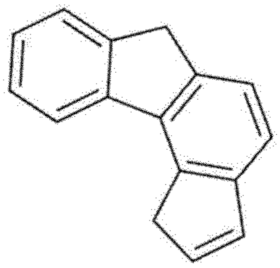

(TEMP-14)

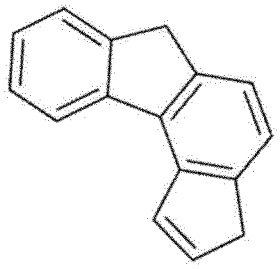

(TEMP-15)

Substituted Aryl Group (Specific Example Group G1B):
an o-tolyl group, m-tolyl group, p-tolyl group, para-xylyl group, meta-xylyl group, ortho-xylyl group, para-isopropylphenyl group, meta-isopropylphenyl group, ortho-isopropylphenyl group, para-t-butylphenyl group, meta-t-butylphenyl group, ortho-t-butylphenyl group, 3,4,5-trimethylphenyl group, 9,9-dimethylfluorenyl group, 9,9-diphenylfluorenyl group, 9,9-bis(4-methylphenyl)fluorenyl group, 9,9-bis(4-isopropylphenyl)fluorenyl group, 9,9-bis(4-t-butylphenyl)fluorenyl group, cyanophenyl group, triphenylsilylphenyl group, trimethylsilylphenyl group, phenylnaphthyl group, naphthylphenyl group, and <<nret>> group derived by substituting at least one hydrogen atom of a monovalent group derived from one of the cyclic structures represented by the formulae (TEMP-1) to (TEMP-15) with a substituent.

Substituted or Unsubstituted Heterocyclic Group

The "heterocyclic group" mentioned herein refers to a cyclic group having at least one hetero atom in the ring atoms. Specific examples of the hetero atom include a nitrogen atom, oxygen atom, sulfur atom, silicon atom, phosphorus atom, and boron atom.

The "heterocyclic group" mentioned herein is a monocyclic group or a fused-ring group.

The "heterocyclic group" mentioned herein is an aromatic heterocyclic group or a non-aromatic heterocyclic group.

Specific examples (specific example group G2) of the "substituted or unsubstituted heterocyclic group" mentioned herein include unsubstituted heterocyclic groups (specific example group G2A) and substituted heterocyclic groups (specific example group G2B). Herein, an unsubstituted heterocyclic group refers to an "unsubstituted heterocyclic group" in a "substituted or unsubstituted heterocyclic group," and a substituted heterocyclic group refers to a "substituted heterocyclic group" in a "substituted or unsubstituted heterocyclic group. A simply termed "heterocyclic group" herein includes both of an "unsubstituted heterocyclic group" and a "substituted heterocyclic group."

The "substituted heterocyclic group" refers to a group derived by substituting at least one hydrogen atom in an "unsubstituted heterocyclic group" with a substituent. Specific examples of the "substituted heterocyclic group" include a group derived by substituting at least one hydrogen atom in the "unsubstituted heterocyclic group" in the specific example group G2A below with a substituent, and examples of the substituted heterocyclic group in the specific example group G2B below. It should be noted that the examples of the "unsubstituted heterocyclic group" and the "substituted heterocyclic group" mentioned herein are merely exemplary, and the "substituted heterocyclic group" mentioned herein includes a group derived by further substituting a hydrogen atom bonded to a ring atom of a skeleton of a "substituted

heterocyclic group" in the specific example group G2B below, and a group derived by further substituting a hydrogen atom of a substituent of the "substituted heterocyclic group" in the specific example group G2B below.

The specific example group G2A includes, for instance, unsubstituted heterocyclic groups including a nitrogen atom (specific example group G2A1) below, unsubstituted heterocyclic groups including an oxygen atom (specific example group G2A2) below, unsubstituted heterocyclic groups including a sulfur atom (specific example group G2A3) below, and monovalent heterocyclic groups (specific example group G2A4) derived by removing a hydrogen atom from cyclic structures represented by formulae (TEMP-16) to (TEMP-33) below.

The specific example group G2B includes, for instance, substituted heterocyclic groups including a nitrogen atom (specific example group G2B1) below, substituted heterocyclic groups including an oxygen atom (specific example group G2B2) below, substituted heterocyclic groups including a sulfur atom (specific example group G2B3) below, and groups derived by substituting at least one hydrogen atom of the monovalent heterocyclic groups (specific example group G2B4) derived from the cyclic structures represented by formulae (TEMP-16) to (TEMP-33) below.

Unsubstituted Heterocyclic Groups Including Nitrogen Atom (Specific Example Group G2A1):

a pyrrolyl group, imidazolyl group, pyrazolyl group, triazolyl group, tetrazolyl group, oxazolyl group, isoxazolyl group, oxadiazolyl group, thiazolyl group, isothiazolyl group, thiadiazolyl group, pyridyl group, pyridazynyl group, pyrimidinyl group, pyrazinyl group, triazinyl group, indolyl group, isoindolyl group, indolizinyl group, quinolizinyl group, quinolyl group, isoquinolyl group, cinnolyl group, phthalazinyl group, quinazolinyl group, quinoxalinyl group, benzimidazolyl group, indazolyl group, phenanthrolinyl group, phenanthridinyl group, acridinyl group, phenazinyl group, carbazolyl group, benzocarbazolyl group, morpholino group, phenoxazinyl group, phenothiazinyl group, azacarbazolyl group, and diazacarbazolyl group.

Unsubstituted Heterocyclic Groups Including Oxygen Atom (Specific Example Group G2A2):

a furyl group, oxazolyl group, isoxazolyl group, oxadiazolyl group, xanthenyl group, benzofuranyl group, isobenzofuranyl group, dibenzofuranyl group, naphthobenzofuranyl group, benzoxazolyl group, benzisoxazolyl group, phenoxazinyl group, morpholino group, dinaphthofuranyl group, azadibenzofuranyl group, diazadibenzofuranyl group, azanaphthobenzofuranyl group, and diazanaphthobenzofuranyl group.

Unsubstituted Heterocyclic Groups Including Sulfur Atom (Specific Example Group G2A3):

a thienyl group, thiazolyl group, isothiazolyl group, thiadiazolyl group, benzothiophenyl group (benzothienyl group), isobenzothiophenyl group (isobenzothienyl group), dibenzothiophenyl group (dibenzothienyl group), naphthobenzothiophenyl group (nahthobenzothienyl group), benzothiazolyl group, benzisothiazolyl group, phenothiazinyl group, dinaphthothiophenyl group (dinaphthothienyl group), azadibenzothiophenyl group (azadibenzothienyl group), diazadibenzothiophenyl group (diazadibenzothienyl group), azanaphthobenzothiophenyl group (azanaphthobenzothienyl group), and diazanaphthobenzothiophenyl group (diazanaphthobenzothienyl group).

Monovalent Heterocyclic Groups Derived by Removing One Hydrogen Atom from Cyclic Structures Represented by Formulae (TEMP-16) to (TEMP-33) (Specific Example Group G2A4):

[Formula 9]

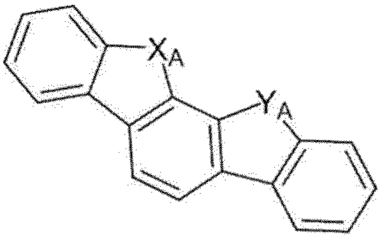
(TEMP-16)

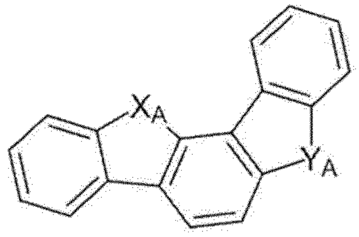
(TEMP-17)

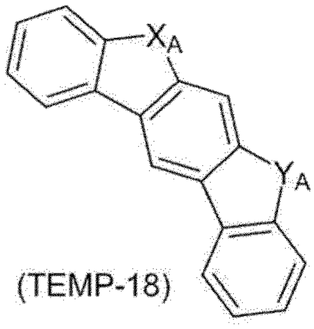
(TEMP-18)

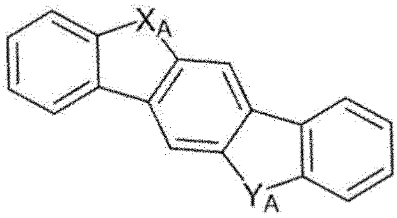
(TEMP-19)

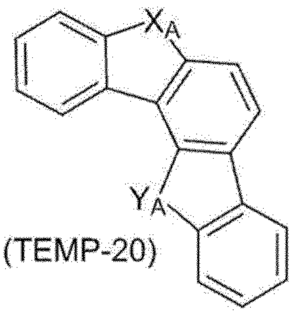
(TEMP-20)

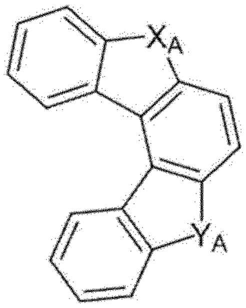
(TEMP-21)

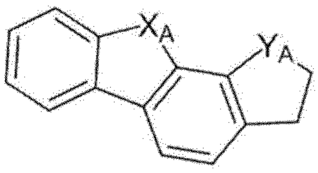
(TEMP-22)

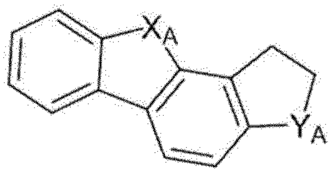
(TEMP-23)

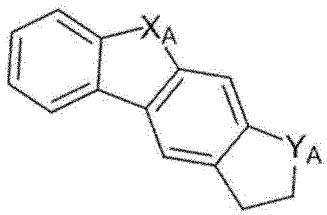
(TEMP-24)

[Formula 10]

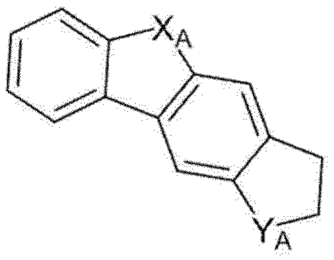
(TEMP-25)

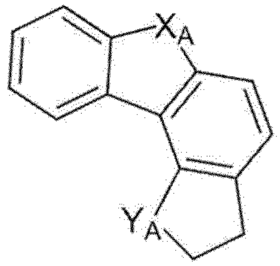
(TEMP-26)

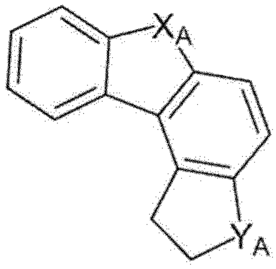
(TEMP-27)

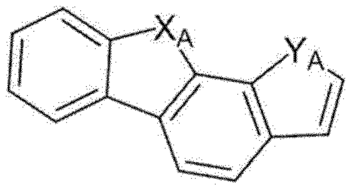
(TEMP-28)

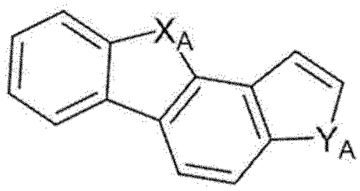
(TEMP-29)

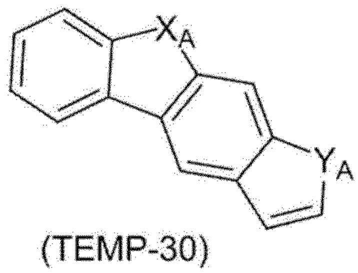
(TEMP-30)

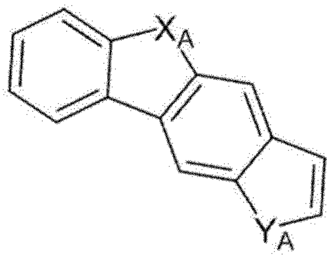
(TEMP-31)

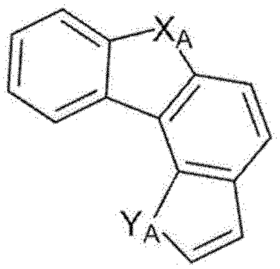
(TEMP-32)

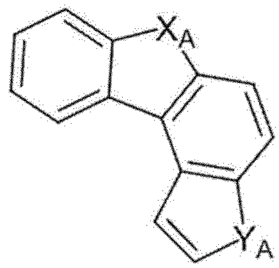
(TEMP-33)

In the formulae (TEMP-16) to (TEMP-33), $X_A$ and $Y_A$ are each independently an oxygen atom, a sulfur atom, NH or $CH_2$, with a proviso that at least one of $X_A$ or $Y_A$ is an oxygen atom, a sulfur atom, or NH.

When at least one of $X_A$ or $Y_A$ in the formulae (TEMP-16) to (TEMP-33) is NH or $CH_2$, the monovalent heterocyclic groups derived from the cyclic structures represented by the formulae (TEMP-16) to (TEMP-33) include a monovalent group derived by removing one hydrogen atom from NH or $CH_2$.

Substituted Heterocyclic Groups Including Nitrogen Atom (Specific Example Group G2B1):

a (9-phenyl)carbazolyl group, (9-biphenylyl)carbazolyl group, (9-phenyl)phenylcarbazolyl group, (9-naphthyl)carbazolyl group, diphenylcarbazole-9-yl group, phenylcarbazole-9-yl group, methylbenzimidazolyl group, ethylbenzimidazolyl group, phenyltriazinyl group, biphenylyltriazinyl group, diphenyltriazinyl group, phenylquinazolinyl group, and biphenylquinazolinyl group.

Substituted Heterocyclic Groups Including Oxygen Atom (Specific Example Group G2B2):

a phenyldibenzofuranyl group, methyldibenzofuranyl group, t-butyldibenzofuranyl group, and monovalent residue of spiro[9H-xanthene-9,9'-[9H]fluorene].

Substituted Heterocyclic Groups Including Sulfur Atom (Specific Example Group G2B3):

a phenyldibenzothiophenyl group, methyldibenzothiophenyl group, t-butyldibenzothiophenyl group, and monovalent residue of spiro[9H-thioxanthene-9,9'-[9H]fluorene].

Groups Obtained by Substituting at Least One Hydrogen Atom of Monovalent Heterocyclic Group Derived from Cyclic Structures Represented by Formulae (TEMP-16) to (TEMP-33) with Substituent (Specific Example Group G2B4):

The "at least one hydrogen atom of a monovalent heterocyclic group" means at least one hydrogen atom

selected from a hydrogen atom bonded to a ring carbon atom of the monovalent heterocyclic group, a hydrogen atom bonded to a nitrogen atom of at least one of $X_A$ or $Y_A$ in a form of NH, and a hydrogen atom of one of $X_A$ and $Y_A$ in a form of a methylene group ($CH_2$).

Substituted or Unsubstituted Alkyl Group

Specific examples (specific example group G3) of the "substituted or unsubstituted alkyl group" mentioned herein include unsubstituted alkyl groups (specific example group G3A) and substituted alkyl groups (specific example group G3B) below. Herein, an unsubstituted alkyl group refers to an "unsubstituted alkyl group" in a "substituted or unsubstituted alkyl group", and a substituted alkyl group refers to a "substituted alkyl group" in a "substituted or unsubstituted alkyl group. A simply termed "alkyl group" herein includes both of an "unsubstituted alkyl group" and a "substituted alkyl group."

The "substituted alkyl group" refers to a group derived by substituting at least one hydrogen atom in an "unsubstituted alkyl group" with a substituent. Specific examples of the "substituted alkyl group" include a group derived by substituting at least one hydrogen atom of an "unsubstituted alkyl group" (specific example group G3A) below with a substituent, and examples of the substituted alkyl group (specific example group G3B) below. Herein, the alkyl group for the "unsubstituted alkyl group" refers to a chain alkyl group. Accordingly, the "unsubstituted alkyl group" include linear "unsubstituted alkyl group" and branched "unsubstituted alkyl group." It should be noted that the examples of the "unsubstituted alkyl group" and the "substituted alkyl group" mentioned herein are merely exemplary, and the "substituted alkyl group" mentioned herein includes a group derived by further substituting a hydrogen atom of a skeleton of the "substituted alkyl group" in the specific example group G3B, and a group derived by further substituting a hydrogen atom of a substituent of the "substituted alkyl group" in the specific example group G3B.

Unsubstituted Alkyl Group (Specific Example Group G3A):
a methyl group, ethyl group, n-propyl group, isopropyl group, n-butyl group, isobutyl group, s-butyl group, and t-butyl group.
Substituted Alkyl Group (Specific Example Group G3B):
a heptafluoropropyl group (including isomer thereof), pentafluoroethyl group, 2,2,2-trifluoroethyl group, and trifluoromethyl group.

Substituted or Unsubstituted Alkenyl Group

Specific examples (specific example group G4) of the "substituted or unsubstituted alkenyl group" mentioned herein include unsubstituted alkenyl groups (specific example group G4A) and substituted alkenyl groups (specific example group G4B). Herein, an unsubstituted alkenyl group refers to an "unsubstituted alkenyl group" in a "substituted or unsubstituted alkenyl group", and a substituted alkenyl group refers to a "substituted alkenyl group" in a "substituted or unsubstituted alkenyl group. A simply termed "alkenyl group" herein includes both of an "unsubstituted alkenyl group" and a "substituted alkenyl group."

The "substituted alkenyl group" refers to a group derived by substituting at least one hydrogen atom in an "unsubstituted alkenyl group" with a substituent. Specific examples of the "substituted alkenyl group" include an "unsubstituted alkenyl group" (specific example group G4A) substituted by a substituent, and examples of the substituted alkenyl group (specific example group G4B) below. It should be noted that the examples of the "unsubstituted alkenyl group" and the "substituted alkenyl group" mentioned herein are merely exemplary, and the "substituted alkenyl group" mentioned herein includes a group derived by further substituting a hydrogen atom of a skeleton of the "substituted alkenyl group" in the specific example group G4B with a substituent, and a group derived by further substituting a hydrogen atom of a substituent of the "substituted alkenyl group" in the specific example group G4B with a substituent.

Unsubstituted Alkenyl Group (Specific Example Group G4A):
a vinyl group, allyl group, 1-butenyl group, 2-butenyl group, and 3-butenyl group.
Substituted Alkenyl Group (Specific Example Group G4B):
a 1,3-butanedienyl group, 1-methylvinyl group, 1-methylallyl group, 1,1-dimethylallyl group, 2-methylallyl group, and 1,2-dimethylallyl group.

Substituted or Unsubstituted Alkynyl Group

Specific examples (specific example group G5) of the "substituted or unsubstituted alkynyl group" mentioned herein include unsubstituted alkynyl groups (specific example group G5A) below. Herein, an unsubstituted alkynyl group refers to an "unsubstituted alkynyl group" in a "substituted or unsubstituted alkynyl group." A simply termed "alkynyl

group" herein includes both of an "unsubstituted alkynyl group" and a "substituted alkynyl group."

The "substituted alkynyl group" refers to a group derived by substituting at least one hydrogen atom in an "unsubstituted alkynyl group" with a substituent. Specific examples of the "substituted alkynyl group" include a group derived by substituting at least one hydrogen atom of the "unsubstituted alkynyl group" (specific example group G5A) below with a substituent.

Unsubstituted Alkynyl Group (Specific Example Group G5A):
an ethynyl group.

Substituted or Unsubstituted Cycloalkyl Group

Specific examples (specific example group G6) of the "substituted or unsubstituted cycloalkyl group" mentioned herein include unsubstituted cycloalkyl groups (specific example group G6A) and substituted cycloalkyl groups (specific example group G6B). Herein, an unsubstituted cycloalkyl group refers to an "unsubstituted cycloalkyl group" in a "substituted or unsubstituted cycloalkyl group", and a substituted cycloalkyl group refers to a "substituted cycloalkyl group" in a "substituted or unsubstituted cycloalkyl group. A simply termed "cycloalkyl group" herein includes both of an "unsubstituted cycloalkyl group" and a "substituted cycloalkyl group."

The "substituted cycloalkyl group" refers to a group derived by substituting at least one hydrogen atom of an "unsubstituted cycloalkyl group" with a substituent. Specific examples of the "substituted cycloalkyl group" include a group derived by substituting at least one hydrogen atom of the "unsubstituted cycloalkyl group" (specific example group G6A) below with a substituent, and examples of the substituted cycloalkyl group (specific example group G6B) below. It should be noted that the examples of the "unsubstituted cycloalkyl group" and the "substituted cycloalkyl group" mentioned herein are merely exemplary, and the "substituted cycloalkyl group" mentioned herein includes a group derived by substituting at least one hydrogen atom bonded to a carbon atom of a skeleton of the "substituted cycloalkyl group" in the specific example group G6B with a substituent, and a group derived by further substituting a hydrogen atom of a substituent of the "substituted cycloalkyl group" in the specific example group G6B with a substituent.

Unsubstituted Cycloalkyl Group (Specific Example Group G6A):
a cyclopropyl group, cyclobutyl group, cyclopentyl group, cyclohexyl group, 1-adamantyl group, 2-adamantyl group, 1-norbornyl group, and 2-norbornyl group.
Substituted Cycloalkyl Group (Specific Example Group G6B):
a 4-methylcyclohexyl group.

Group Represented by -Si($R_{901}$)($R_{902}$)($R_{903}$)

Specific examples (specific example group G7) of the group represented herein by -Si($R_{901}$)($R_{902}$)($R_{903}$) include:

-Si(G1)(G1)(G1); -Si(G1)(G2)(G2); -Si(G1)(G1)(G2); -Si(G2)(G2)(G2); - Si(G3)(G3)(G3); and -Si(G6)(G6)(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6;
a plurality of G1 in -Si(G1)(G1)(G1) are mutually the same or different;
a plurality of G2 in -Si(G1)(G2)(G2) are mutually the same or different;
a plurality of G1 in -Si(G1)(G1)(G2) are mutually the same or different;
a plurality of G2 in -Si(G2)(G2)(G2) are mutually the same or different;
a plurality of G3 in -Si(G3)(G3)(G3) are mutually the same or different; and
a plurality of G6 in -Si(G6)(G6)(G6) are mutually the same or different.

Group Represented by -O-($R_{904}$)

Specific examples (specific example group G8) of a group represented by - O-($R_{904}$) herein include: -O(G1); -O(G2); -O(G3); and -O(G6);
where:

G1 represents a "substituted or unsubstituted aryl group" in the specific example group G1;

G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

Group Represented by -S-($R_{905}$)

Specific examples (specific example group G9) of a group represented herein by -S-($R_{905}$) include: -S(G1); -S(G2); -S(G3); and -S(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

Group Represented by -N(R906)(R907)

Specific examples (specific example group G10) of a group represented herein by -N($R_{906}$)($R_{907}$) include: -N(G1)(G1); -N(G2)(G2); -N(G1)(G2); -N(G3)(G3); and -N(G6)(G6),
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

A plurality of G1 in -N(G1)(G1) are mutually the same or different.
A plurality of G2 in -N(G2)(G2) are mutually the same or different.
A plurality of G3 in -N(G3)(G3) are mutually the same or different.
A plurality of G6 in -N(G6)(G6) are mutually the same or different.

Halogen Atom

Specific examples (specific example group G11) of "halogen atom" mentioned herein include a fluorine atom, chlorine atom, bromine atom, and iodine atom.

Substituted or Unsubstituted Fluoroalkyl Group

The "substituted or unsubstituted fluoroalkyl group" mentioned herein refers to a group derived by substituting at least one hydrogen atom bonded to at least one of carbon atoms forming an alkyl group in the "substituted or unsubstituted alkyl group" with a fluorine atom, and also includes a group (perfluoro group) derived by substituting all of hydrogen atoms bonded to carbon atoms forming the alkyl group in the "substituted or unsubstituted alkyl group" with fluorine atoms. An "unsubstituted fluoroalkyl group" has, unless otherwise specified herein, 1 to 50, preferably 1 to 30, more preferably 1 to 18 carbon atoms. The "substituted fluoroalkyl group" refers to a group derived by substituting at least one hydrogen atom in a "fluoroalkyl group" with a substituent. It should be noted that the examples of the "substituted fluoroalkyl group" mentioned herein include a group derived by further substituting at least one hydrogen atom bonded to a carbon atom of an alkyl chain of a "substituted fluoroalkyl group" with a substituent, and a group derived by further substituting at least one hydrogen atom of a substituent of the "substituted fluoroalkyl group" with a substituent. Specific examples of the "substituted fluoroalkyl group" include a group derived by substituting at least one hydrogen atom of the "alkyl group" (specific example group G3) with a fluorine atom.

Substituted or Unsubstituted Haloalkyl Group

The "substituted or unsubstituted haloalkyl group" mentioned herein refers to a group derived by substituting at least one hydrogen atom bonded to carbon atoms forming the alkyl group in the "substituted or unsubstituted alkyl group" with a halogen atom, and also includes a group derived by substituting all hydrogen atoms bonded to carbon atoms forming the alkyl group in the "substituted or unsubstituted alkyl group" with halogen atoms. An "unsubstituted haloalkyl group" has, unless otherwise specified herein, 1 to 50, preferably 1 to 30, and more preferably 1 to 18 carbon

atoms. The "substituted haloalkyl group" refers to a group derived by substituting at least one hydrogen atom in a "haloalkyl group" with a substituent. It should be noted that the examples of the "substituted haloalkyl group" mentioned herein include a group derived by further substituting at least one hydrogen atom bonded to a carbon atom of an alkyl chain of a "substituted haloalkyl group" with a substituent, and a group derived by further substituting at least one hydrogen atom of a substituent of the "substituted haloalkyl group" with a substituent. Specific examples of the "substituted haloalkyl group" include a group derived by substituting at least one hydrogen atom of the "alkyl group" (specific example group G3) with a halogen atom. The haloalkyl group is sometimes referred to as a halogenated alkyl group.

Substituted or Unsubstituted Alkoxy Group

Specific examples of a "substituted or unsubstituted alkoxy group" mentioned herein include a group represented by -O(G3), G3 being the "substituted or unsubstituted alkyl group" in the specific example group G3. An "unsubstituted alkoxy group" has, unless otherwise specified herein, 1 to 50, preferably 1 to 30, more preferably 1 to 18 carbon atoms.

Substituted or Unsubstituted Alkylthio Group

Specific examples of a "substituted or unsubstituted alkylthio group" mentioned herein include a group represented by -S(G3), G3 being the "substituted or unsubstituted alkyl group" in the specific example group G3. An "unsubstituted alkylthio group" has, unless otherwise specified herein, 1 to 50, preferably 1 to 30, more preferably 1 to 18 carbon atoms.

Substituted or Unsubstituted Aryloxy Group

Specific examples of a "substituted or unsubstituted aryloxy group" mentioned herein include a group represented by -O(G1), G1 being the "substituted or unsubstituted aryl group" in the specific example group G1. An "unsubstituted aryloxy group" has, unless otherwise specified herein, 6 to 50, preferably 6 to 30, more preferably 6 to 18 ring carbon atoms.

Substituted or Unsubstituted Arylthio Group

Specific examples of a "substituted or unsubstituted arylthio group" mentioned herein include a group represented by -S(G1), G1 being the "substituted or unsubstituted aryl group" in the specific example group G1. An "unsubstituted arylthio group" has, unless otherwise specified herein, 6 to 50, preferably 6 to 30, more preferably 6 to 18 ring carbon atoms.

Substituted or Unsubstituted Trialkylsilyl Group

Specific examples of a "substituted or unsubstituted trialkylsilyl group" mentioned herein include a group represented by -Si(G3)(G3)(G3), G3 being the "substituted or unsubstituted alkyl group" in the specific example group G3. A plurality of G3 in -Si(G3)(G3)(G3) are mutually the same or different. Each of the alkyl groups in the "unsubstituted trialkylsilyl group" has, unless otherwise specified herein, 1 to 50, preferably 1 to 20, more preferably 1 to 6 carbon atoms.

Substituted or Unsubstituted Aralkyl Group

Specific examples of a "substituted or unsubstituted aralkyl group" mentioned herein include a group represented by -(G3)-(G1), G3 being the "substituted or unsubstituted alkyl group" in the specific example group G3, G1 being the "substituted or unsubstituted aryl group" in the specific example group G1. Accordingly, the "aralkyl group" is a group derived by substituting a hydrogen atom of the "alkyl group" with a substituent in a form of the "aryl group," which is an example of the "substituted alkyl group." An "unsubstituted aralkyl group," which is an "unsubstituted alkyl group" substituted by an "unsubstituted aryl group," has, unless otherwise specified herein, 7 to 50 carbon atoms, preferably 7 to 30 carbon atoms, more preferably 7 to 18 carbon atoms.

Specific examples of the "substituted or unsubstituted aralkyl group" include a benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, and 2-β-naphthylisopropyl group.

Preferable examples of the substituted or unsubstituted aryl group mentioned herein include, unless otherwise specified herein, a phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, phenanthryl group, pyrenyl group, chrysenyl group, triphenylenyl group, fluorenyl group, 9,9'-spirobifluorenyl group, 9,9-dimethylfluorenyl group, and 9,9-diphenylfluorenyl group.

Preferable examples of the substituted or unsubstituted heterocyclic group mentioned herein include, unless otherwise specified herein, a pyridyl group, pyrimidinyl group, triazinyl group, quinolyl group, isoquinolyl group, quinazolinyl group, benzimidazolyl group, phenanthrolinyl group, carbazolyl group (1-carbazolyl group, 2-carbazolyl group, 3-carbazolyl group, 4-carbazolyl group, or 9-carbazolyl group), benzocarbazolyl group, azacarbazolyl group, diazacarbazolyl group, dibenzofuranyl group, naphthobenzofuranyl group, azadibenzofuranyl group, diazadibenzofuranyl group, dibenzothiophenyl group, naphthobenzothiophenyl group, azadibenzothiophenyl group, diazadibenzothiophenyl group, (9-phenyl)carbazolyl group ((9-phenyl)carbazole-1-yl group, (9-phenyl)carbazole-2-yl group, (9-phenyl)carbazole-3-yl group, or (9-phenyl)carbazole-4-yl group), (9-biphenylyl)carbazolyl group, (9-phenyl)phenylcarbazolyl group, diphenylcarbazole-9-yl group, phenylcarbazole-9-yl group, phenyltriazinyl group, biphenylyltriazinyl group, diphenyltriazinyl group, phenyldibenzofuranyl group, and phenyldibenzothiophenyl group.

The carbazolyl group mentioned herein is, unless otherwise specified herein, specifically a group represented by one of formulae below.

[Formula 11]

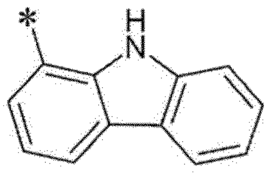

(TEMP-Cz1)

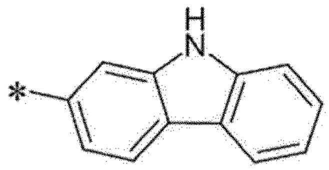

(TEMP-Cz2)

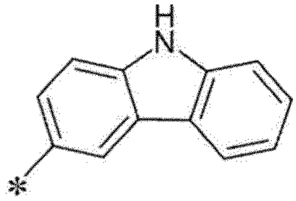

(TEMP-Cz3)

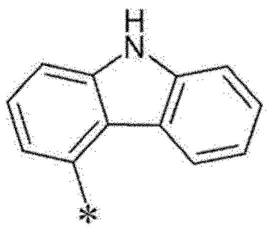

(TEMP-Cz4)

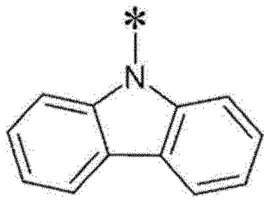

(TEMP-Cz5)

The (9-phenyl)carbazolyl group mentioned herein is, unless otherwise specified herein, specifically a group represented by one of formulae below.

[Formula 12]

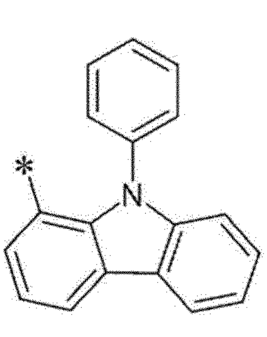

(TEMP-Cz6)

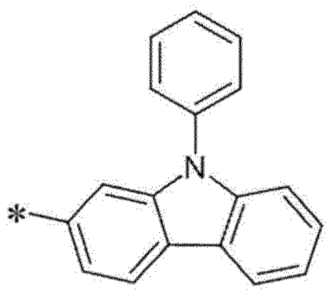

(TEMP-Cz7)

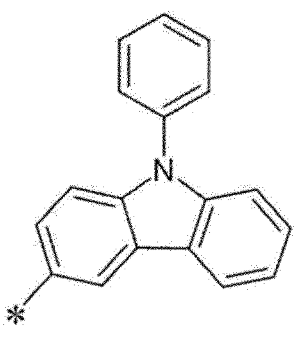

(TEMP-Cz8)

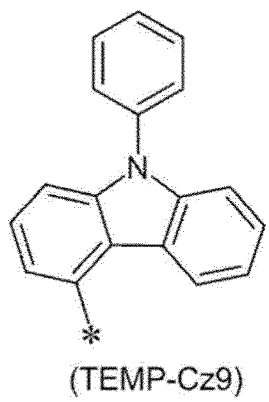

(TEMP-Cz9)

In the formulae (TEMP-Cz1) to (TEMP-Cz9), * represents a bonding position.
The dibenzofuranyl group and dibenzothiophenyl group mentioned herein are, unless otherwise specified herein, each specifically represented by one of formulae below.

[Formula 13]

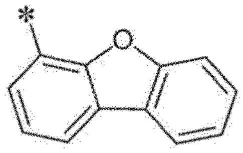
(TEMP-34)

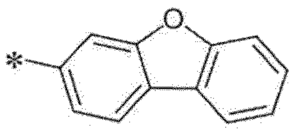
(TEMP-35)

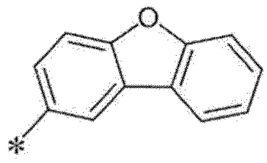
(TEMP-36)

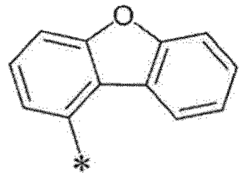
(TEMP-37)

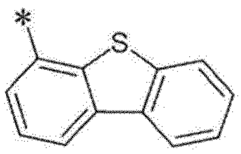
(TEMP-38)

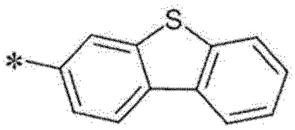
(TEMP-39)

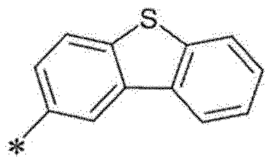
(TEMP-40)

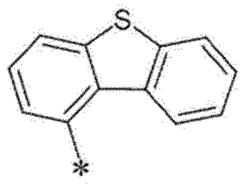
(TEMP-41)

In the formulae (TEMP-34) to (TEMP-41), * represents a bonding position.
Preferable examples of the substituted or unsubstituted alkyl group mentioned herein include, unless otherwise specified herein, a methyl group, ethyl group, propyl group, isopropyl group, n-butyl group, isobutyl group, and t-butyl group.

Substituted or Unsubstituted Arylene Group

The "substituted or unsubstituted arylene group" mentioned herein is, unless otherwise specified herein, a divalent group derived by removing one hydrogen atom on an aryl ring of the "substituted or unsubstituted aryl group." Specific examples of the "substituted or unsubstituted arylene group" (specific example group G12) include a divalent group derived by removing one hydrogen atom on an aryl ring of the "substituted or unsubstituted aryl group" in the specific example group G1.

Substituted or Unsubstituted Divalent Heterocyclic Group

The "substituted or unsubstituted divalent heterocyclic group" mentioned herein is, unless otherwise specified herein, a divalent group derived by removing one hydrogen atom on a heterocyclic ring of the "substituted or unsubstituted heterocyclic group." Specific examples of the "substituted or unsubstituted divalent heterocyclic group" (specific example group G13) include a divalent group derived by removing one hydrogen atom on a heterocyclic ring of the "substituted or unsubstituted heterocyclic group" in the specific example group G2.

Substituted or Unsubstituted Alkylene Group

The "substituted or unsubstituted alkylene group" mentioned herein is, unless otherwise specified herein, a divalent group derived by removing one hydrogen atom on an alkyl chain of the "substituted or unsubstituted alkyl group." Specific examples of the "substituted or unsubstituted alkylene group" (specific example group G14) include a divalent group derived by removing one hydrogen atom on an alkyl chain of the "substituted or unsubstituted alkyl group" in the specific example group G3.
The substituted or unsubstituted arylene group mentioned herein is, unless otherwise specified herein, preferably any one of groups represented by formulae (TEMP-42) to (TEMP-68) below.

[Formula 14]

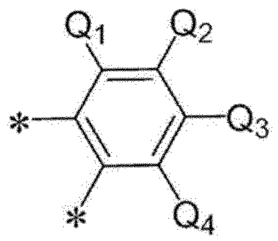

(TEMP-42)

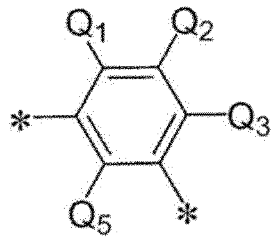

(TEMP-43)

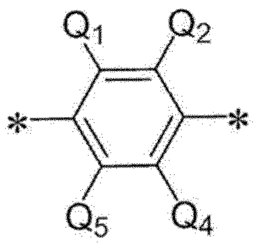

(TEMP-44)

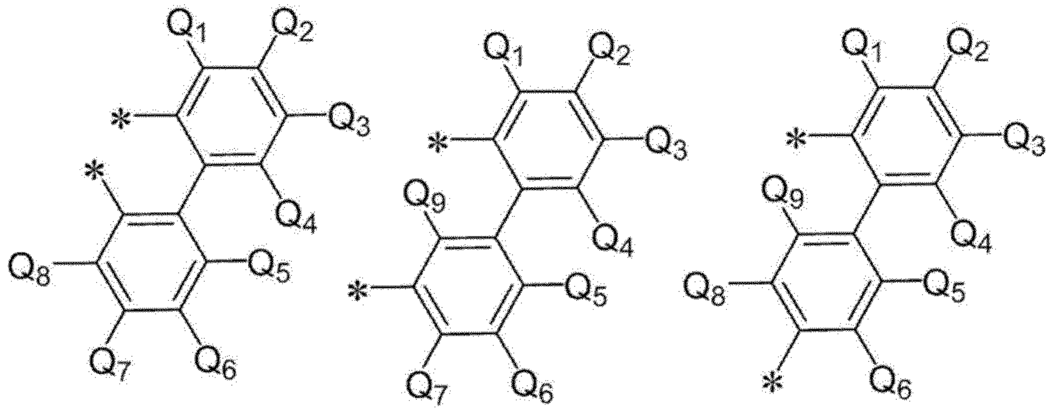

(TEMP-45) (TEMP-46) (TEMP-47)

[Formula 15]

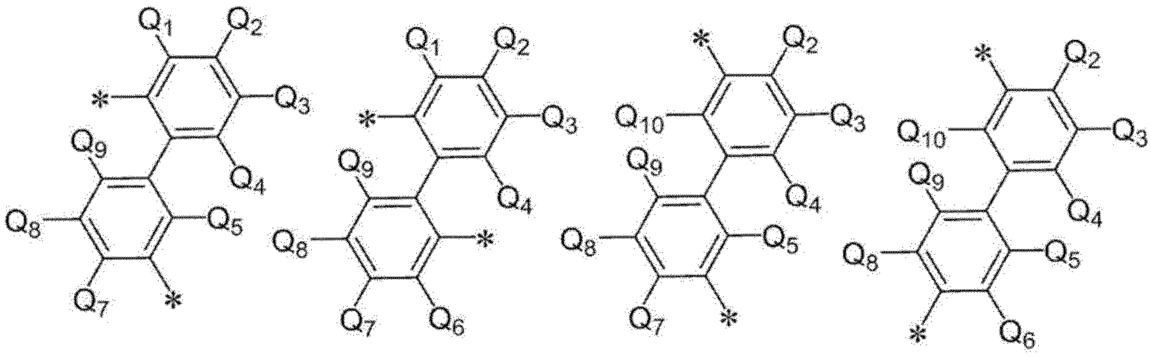

(TEMP-48) (TEMP-49) (TEMP-50) (TEMP-51)

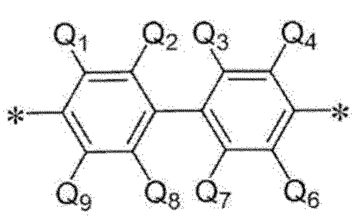

(TEMP-52)

In the formulae (TEMP-42) to (TEMP-52), $Q_1$ to $Q_{10}$ are each independently a hydrogen atom or a substituent.

In the formulae (TEMP-42) to (TEMP-52), * represents a bonding position.

[Formula 16]

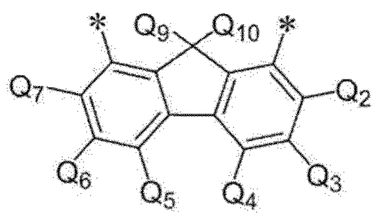

(TEMP-53)

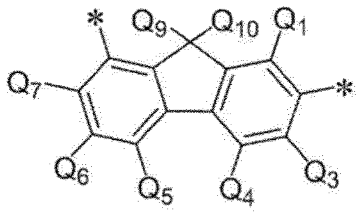

(TEMP-54)

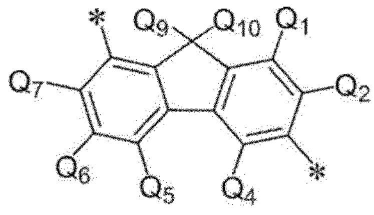

(TEMP-55)

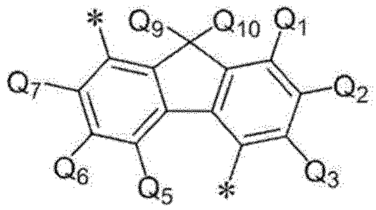

(TEMP-56)

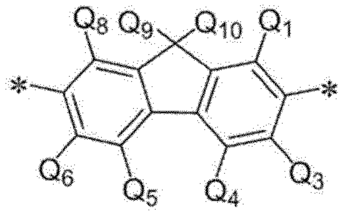

(TEMP-57)

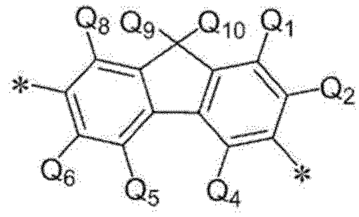

(TEMP-58)

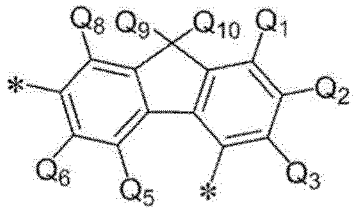

(TEMP-59)

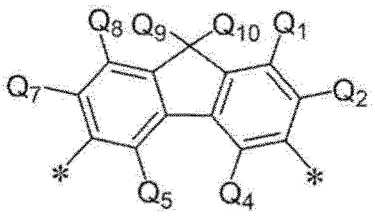

(TEMP-60)

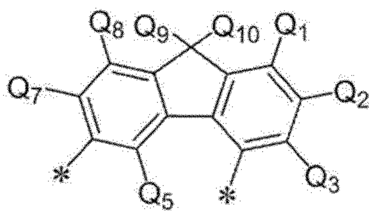

(TEMP-61)

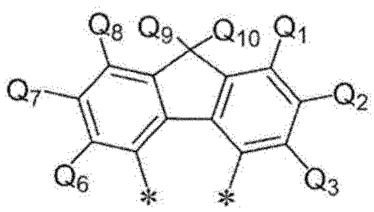

(TEMP-62)

In the formulae (TEMP-53) to (TEMP-62), $Q_1$ to $Q_{10}$ are each independently a hydrogen atom or a substituent.

In the formulae, $Q_9$ and $Q_{10}$ may be mutually bonded through a single bond to form a ring.

In the formulae (TEMP-53) to (TEMP-62), * represents a bonding position.

[Formula 17]

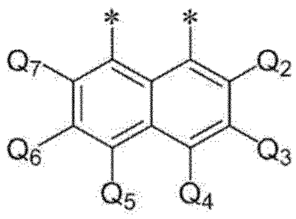
(TEMP-63)

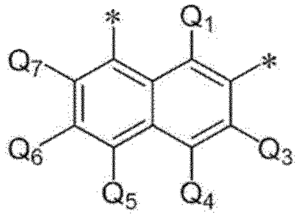
(TEMP-64)

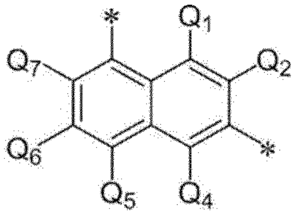
(TEMP-65)

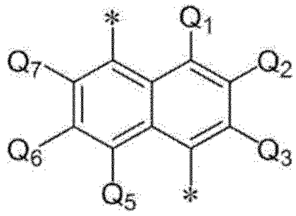
(TEMP-66)

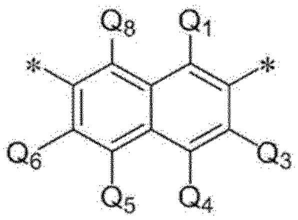
(TEMP-67)

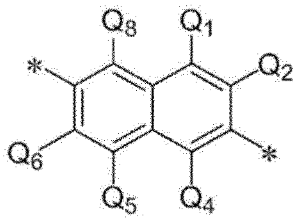
(TEMP-68)

In the formulae (TEMP-63) to (TEMP-68), $Q_1$ to $Q_8$ are each independently a hydrogen atom or a substituent.

In the formulae (TEMP-63) to (TEMP-68), * represents a bonding position.

The substituted or unsubstituted divalent heterocyclic group mentioned herein is, unless otherwise specified herein, preferably a group represented by any one of formulae (TEMP-69) to (TEMP-102) below.

[Formula 18]

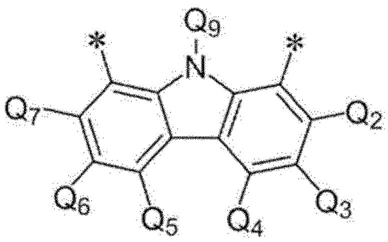
(TEMP-69)

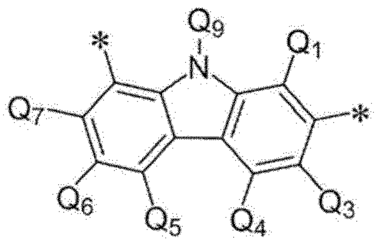
(TEMP-70)

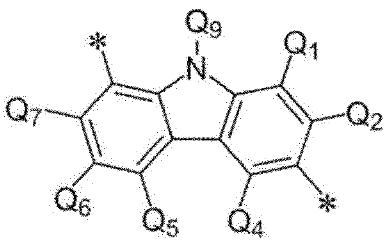
(TEMP-71)

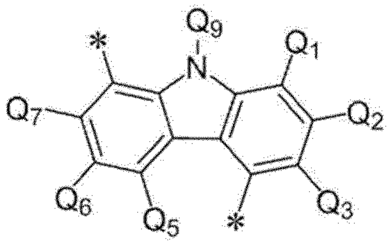
(TEMP-72)

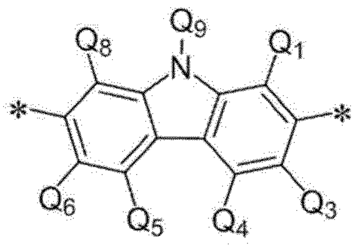
(TEMP-73)

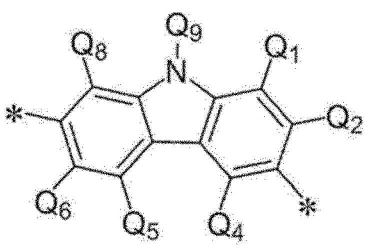
(TEMP-74)

[Formula 19]

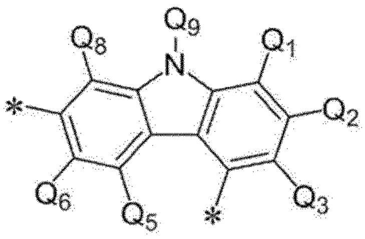

(TEMP-75)

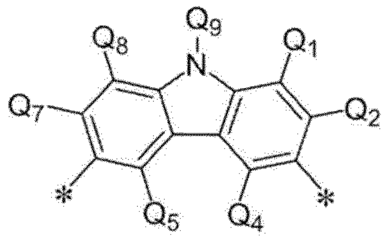

(TEMP-76)

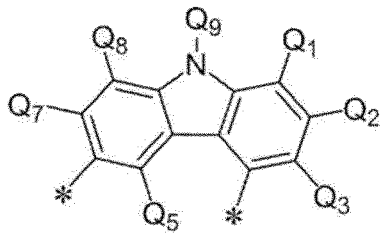

(TEMP-77)

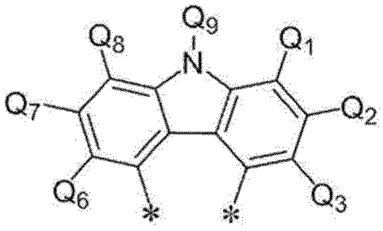

(TEMP-78)

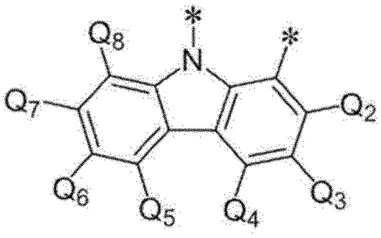

(TEMP-79)

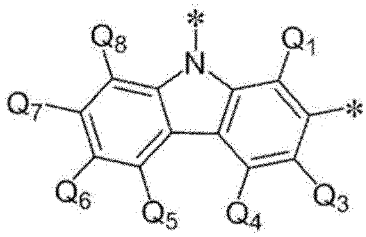

(TEMP-80)

[Formula 20]

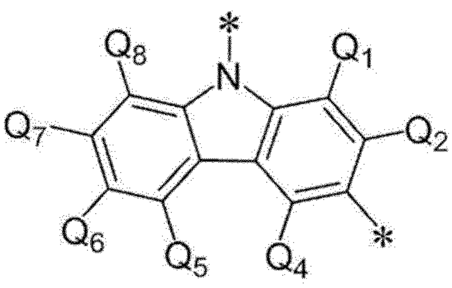

(TEMP-81)

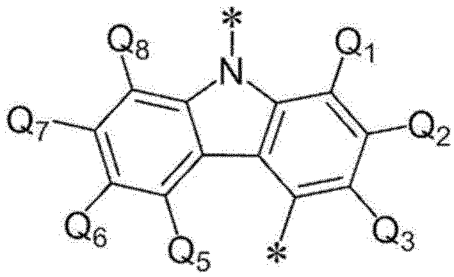

(TEMP-82)

In the formulae (TEMP-69) to (TEMP-82), $Q_1$ to $Q_9$ are each independently a hydrogen atom or a substituent. In the formulae (TEMP-69) to (TEMP-82), * represents a bonding position.

[Formula 21]

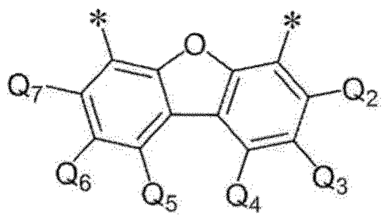

(TEMP-83)

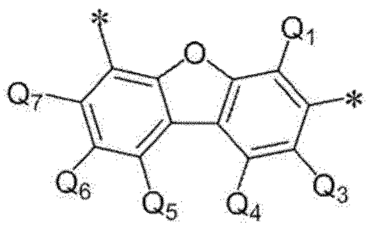

(TEMP-84)

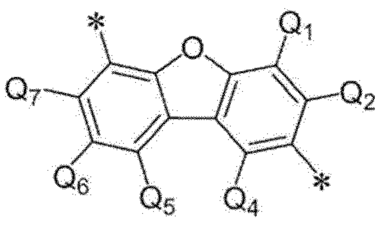

(TEMP-85)

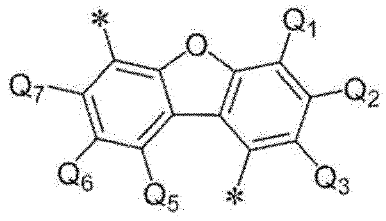

(TEMP-86)

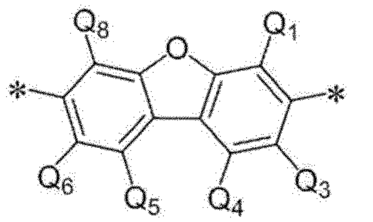

(TEMP-87)

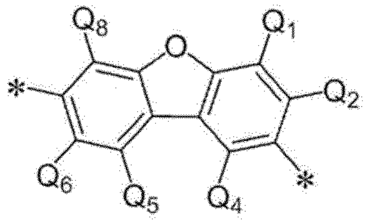

(TEMP-88)

[Formula 22]

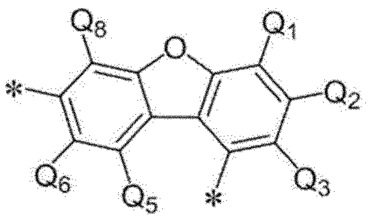

(TEMP-89)

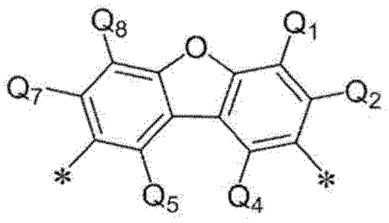

(TEMP-90)

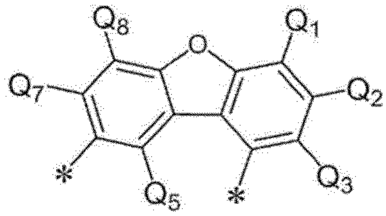

(TEMP-91)

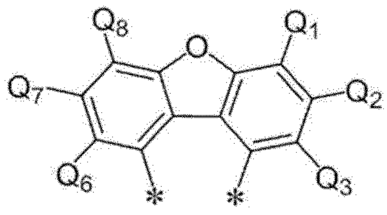

(TEMP-92)

[Formula 23]

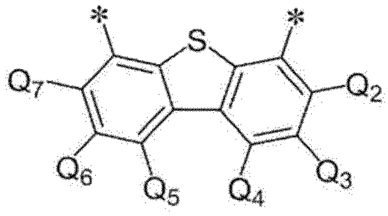
(TEMP-93)

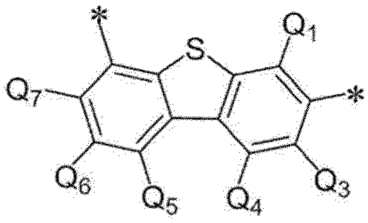
(TEMP-94)

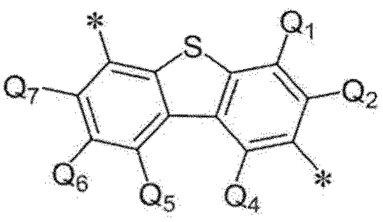
(TEMP-95)

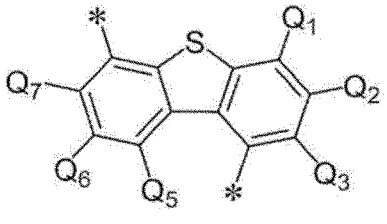
(TEMP-96)

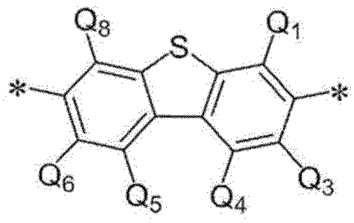
(TEMP-97)

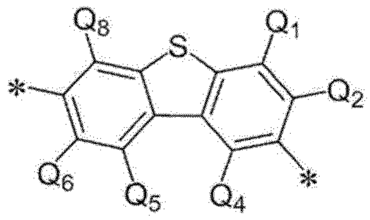
(TEMP-98)

[Formula 24]

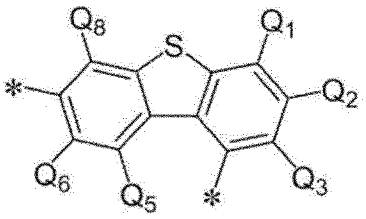
(TEMP-99)

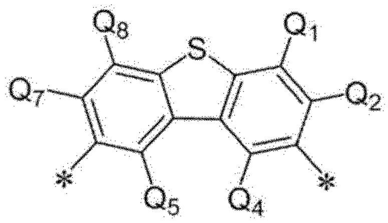
(TEMP-100)

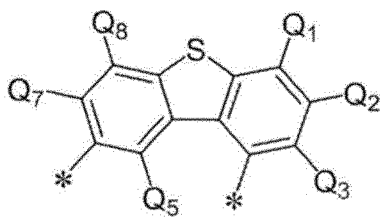
(TEMP-101)

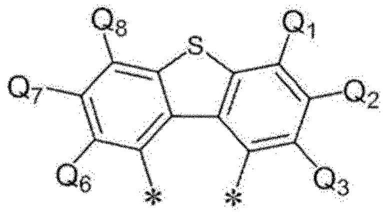
(TEMP-102)

In the formulae (TEMP-83) to (TEMP-102), $Q_1$ to $Q_8$ are each independently a hydrogen atom or a substituent. In the formulae (TEMP-83) to (TEMP-102), * represents a bonding position.

The substituent mentioned herein has been described above.

Instance of "Bonded to Form Ring"

Instances where "at least one combination of adjacent two or more (of...) are mutually bonded to form a substituted or unsubstituted monocyclic ring, mutually bonded to form a substituted or unsubstituted fused ring, or not mutually bonded" mentioned herein refer to instances where "at least one combination of adjacent two or more (of...) are mutually bonded to form a substituted or unsubstituted monocyclic ring, "at least one combination of adjacent two or more (of...) are mutually bonded to form a substituted or unsubstituted fused ring," and "at least one combination of adjacent two or more (of...) are not mutually bonded."

Instances where "at least one combination of adjacent two or more (of...) are mutually bonded to form a substituted or unsubstituted monocyclic ring" and "at least one combination of adjacent two or more (of...) are mutually bonded to form a substituted or unsubstituted fused ring" mentioned herein (these instances will be sometimes collectively referred to as an instance of "bonded to form a ring" hereinafter) will be described below. An anthracene compound having a basic skeleton in a form of an anthracene ring and represented by a formula (TEMP-103) below will be used as an example for the description.

[Formula 25]

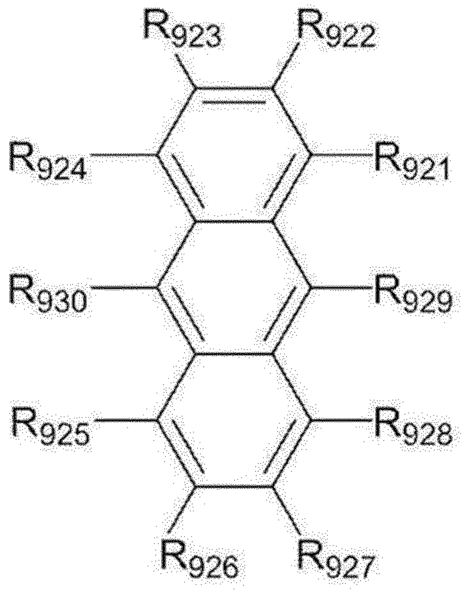

(TEMP-103)

For instance, when "at least one combination of adjacent two or more of $R_{921}$ to $R_{930}$ are mutually bonded to form a ring," the combination of adjacent ones of $R_{921}$ to $R_{930}$ (i.e. the combination at issue) is a combination of $R_{921}$ and $R_{922}$, a combination of $R_{922}$ and $R_{923}$, a combination of $R_{923}$ and $R_{924}$, a combination of $R_{924}$ and $R_{930}$, a combination of $R_{930}$ and $R_{925}$, a combination of $R_{925}$ and $R_{926}$, a combination of $R_{926}$ and $R_{927}$, a combination of $R_{927}$ and $R_{928}$, a combination of $R_{928}$ and $R_{929}$, or a combination of $R_{929}$ and $R_{921}$.

The term "at least one combination" means that two or more of the above combinations of adjacent two or more of $R_{921}$ to $R_{930}$ may simultaneously form rings. For instance, when $R_{921}$ and $R_{922}$ are mutually bonded to form a ring $Q_A$ and $R_{925}$ and $R_{926}$ are simultaneously mutually bonded to form a ring $Q_B$, the anthracene compound represented by the formula (TEMP-103) is represented by a formula (TEMP-104) below.

[Formula 26]

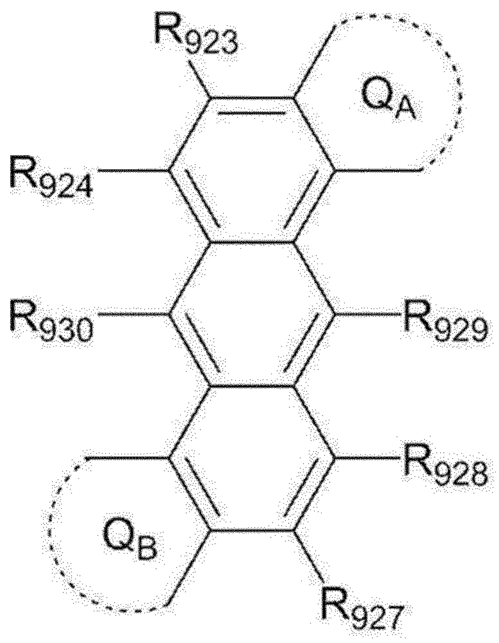

(TEMP-104)

The instance where the "combination of adjacent two or more" form a ring means not only an instance where

the "two" adjacent components are bonded but also an instance where adjacent "three or more" are bonded. For instance, $R_{921}$ and $R_{922}$ are mutually bonded to form a ring $Q_A$ and $R_{922}$ and $R_{923}$ are mutually bonded to form a ring Qc, and mutually adjacent three components ($R_{921}$, $R_{922}$ and $R_{923}$) are mutually bonded to form a ring fused to the anthracene basic skeleton. In this case, the anthracene compound represented by the formula (TEMP-103) is represented by a formula (TEMP-105) below. In the formula (TEMP-105) below, the ring $Q_A$ and the ring Qc share $R_{922}$.

[Formula 27]

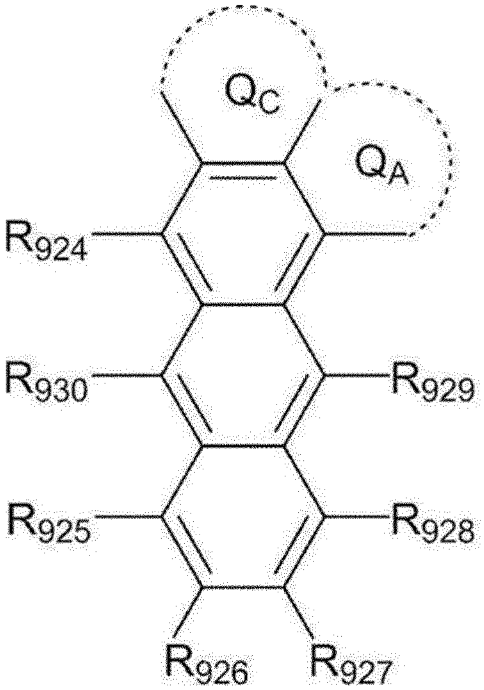

(TEMP-105)

The formed "monocyclic ring" or "fused ring" may be, in terms of the formed ring in itself, a saturated ring or an unsaturated ring. When the "combination of adjacent two" form a "monocyclic ring" or a "fused ring," the "monocyclic ring" or "fused ring" may be a saturated ring or an unsaturated ring. For instance, the ring $Q_A$ and the ring $Q_B$ formed in the formula (TEMP-104) are each independently a "monocyclic ring" or a "fused ring." Further, the ring $Q_A$ and the ring Qc formed in the formula (TEMP-105) are each a "fused ring." The ring $Q_A$ and the ring Qc in the formula (TEMP-105) are fused to form a fused ring. When the ring $Q_A$ in the formula (TEMP-104) is a benzene ring, the ring $Q_A$ is a monocyclic ring. When the ring $Q_A$ in the formula (TEMP-104) is a naphthalene ring, the ring $Q_A$ is a fused ring.

The "unsaturated ring" is at least one ring selected from the group consisting of an aromatic hydrocarbon ring, aromatic heterocyclic ring, aliphatic hydrocarbon ring having an unsaturated bond in a cyclic structure, and non-aromatic heterocyclic ring having an unsaturated bond in a cyclic structure. The unsaturated bond in the cyclic structure of the unsaturated ring is one or both of a double bond and a triple bond. The aliphatic hydrocarbon ring having the unsaturated bond in the cyclic structure is exemplified by cyclohexane and cyclohexadiene. The non-aromatic heterocyclic ring having the unsaturated bond in the cyclic structure is exemplified by dihydropyran, imidazoline, pyrazoline, quinolizine, indoline, and isoindoline.

The "saturated ring" is at least one ring selected from the group consisting of an aliphatic hydrocarbon ring having no unsaturated bond, and non-aromatic heterocyclic ring having no unsaturated bond. The saturated bond has none of a double bond and a triple bond in its cyclic structure.

Specific examples of the aromatic hydrocarbon ring include a ring formed by terminating a bond of a group in the specific examples of the specific example group G1 with a hydrogen atom.

Specific examples of the aromatic heterocyclic ring include a ring formed by terminating a bond of an aromatic heterocyclic group in the specific examples of the specific example group G2 with a hydrogen atom.

Specific examples of the aliphatic hydrocarbon ring include a ring formed by terminating a bond of a group in the specific examples of the specific example group G6 with a hydrogen atom.

The phrase "to form a ring" herein means that a ring is formed only by a plurality of atoms of a basic skeleton, or by a combination of a plurality of atoms of the basic skeleton and one or more optional atoms. For instance, a ring $Q_A$ formed by mutually bonding $R_{921}$ and $R_{922}$ shown in the formula (TEMP-104) is a ring formed by a carbon atom of an anthracene skeleton bonded to $R_{921}$, a carbon atom of an anthracene skeleton bonded to $R_{922}$, and one or more

optional atoms. Specifically, when the ring $Q_A$ is a monocyclic unsaturated ring formed by $R_{921}$ and $R_{922}$, the ring formed by a carbon atom of the anthracene skeleton bonded to $R_{921}$, a carbon atom of the anthracene skeleton bonded to $R_{922}$, and four carbon atoms is a benzene ring.

The "optional atom" is, unless otherwise specified herein, preferably at least one atom selected from the group consisting of a carbon atom, nitrogen atom, oxygen atom, and sulfur atom. A bond of the optional atom (e.g. a carbon atom and a nitrogen atom) not forming a ring may be terminated by a hydrogen atom or the like or may be substituted by an "optional substituent" described later. When the ring includes an optional atom other than a carbon atom, the resultant ring is a heterocyclic ring.

The number of "one or more optional atoms" forming the monocyclic ring or fused ring is, unless otherwise specified herein, preferably in a range from 2 to 15, more preferably in a range from 3 to 12, further preferably in a range from 3 to 5.

Unless otherwise specified herein, the ring, which may be a "monocyclic ring" or "fused ring," is preferably a "monocyclic ring."

Unless otherwise specified herein, the ring, which may be a "saturated ring" or "unsaturated ring," is preferably an "unsaturated ring."

Unless otherwise specified herein, the "monocyclic ring" is preferably a benzene ring.

Unless otherwise specified herein, the "unsaturated ring" is preferably a benzene ring.

When "at least one combination of adjacent two or more" (of...) are "mutually bonded to form a substituted or unsubstituted monocyclic ring" or "mutually bonded to form a substituted or unsubstituted fused ring," unless otherwise specified herein, at least one combination of adjacent two or more of components are preferably mutually bonded to form a substituted or unsubstituted "unsaturated ring" formed of a plurality of atoms of the basic skeleton, and 1 to 15 atoms of at least one atom selected from the group consisting of a carbon atom, a nitrogen atom, an oxygen atom, and a sulfur atom.

When the "monocyclic ring" or the "fused ring" has a substituent, the substituent is the substituent described in later-described "optional substituent." When the "monocyclic ring" or the "fused ring" has a substituent, specific examples of the substituent are the substituents described in the above under the subtitle "Substituent Mentioned Herein."

When the "saturated ring" or the "unsaturated ring" has a substituent, the substituent is the substituent described in later-described "optional substituent." When the "saturated ring" or the "unsaturated ring" has a substituent, specific examples of the substituent are the substituents described in the above under the subtitle "Substituent Mentioned Herein."

The above is the description for the instances where "at least one combination of adjacent two or more (of...) are mutually bonded to form a substituted or unsubstituted monocyclic ring" and "at least one combination of adjacent two or more (of...) are mutually bonded to form a substituted or unsubstituted fused ring" mentioned herein (sometimes referred to as an instance of "bonded to form a ring").

Substituent for Substituted or Unsubstituted Group

In an exemplary embodiment herein, the substituent for the substituted or unsubstituted group (sometimes referred to as an "optional substituent" hereinafter), is for instance, a group selected from the group consisting of an unsubstituted alkyl group having 1 to 50 carbon atoms, an unsubstituted alkenyl group having 2 to 50 carbon atoms, an unsubstituted alkynyl group having 2 to 50 carbon atoms, an unsubstituted cycloalkyl group having 3 to 50 ring carbon atoms, $-Si(R_{901})(R_{902})(R_{903})$, $-O-(R_{904})$, $-S-(R_{905})$, $-N(R_{906})(R_{907})$, a halogen atom, a cyano group, a nitro group, an unsubstituted aryl group having 6 to 50 ring carbon atoms, and an unsubstituted heterocyclic ring having 5 to 50 ring atoms.

$R_{901}$ to $R_{907}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when two or more $R_{901}$ are present, the two or more $R_{901}$ are mutually the same or different;
when two or more $R_{902}$ are present, the two or more $R_{902}$ are mutually the same or different;
when two or more $R_{903}$ are present, the two or more $R_{903}$ are mutually the same or different;
when two or more $R_{904}$ are present, the two or more $R_{904}$ are mutually the same or different;
when two or more $R_{905}$ are present, the two or more $R_{905}$ are mutually the same or different;
when two or more $R_{906}$ are present, the two or more $R_{906}$ are mutually the same or different; and
when two or more $R_{907}$ are present, the two or more $R_{907}$ are mutually the same or different.

In an exemplary embodiment, a substituent for "a substituted or unsubstituted" group is a group selected from the group consisting of an alkyl group having 1 to 50 carbon atoms, aryl group having 6 to 50 ring carbon atoms, and heterocyclic group having 5 to 50 ring atoms.

In an exemplary embodiment, a substituent for "a substituted or unsubstituted" group is a group selected from the group consisting of an alkyl group having 1 to 18 carbon atoms, aryl group having 6 to 18 ring carbon atoms, and heterocyclic group having 5 to 18 ring atoms.

Specific examples of the above optional substituent are the same as the specific examples of the substituent described in the above under the subtitle "Substituent Mentioned Herein."

Unless otherwise specified herein, adjacent ones of the optional substituents may form a "saturated ring" or an "unsaturated ring," preferably a substituted or unsubstituted saturated five-membered ring, a substituted or unsubstituted saturated six-membered ring, a substituted or unsubstituted unsaturated five-membered ring, or a substituted or unsubstituted unsaturated six-membered ring, more preferably a benzene ring.

Unless otherwise specified herein, the optional substituent may further include a substituent. Examples of the substituent for the optional substituent are the same as the examples of the optional substituent.

When a plurality of optional substituents are present, the plurality of optional substituents are mutually the same or different.

Herein, numerical ranges represented by "AA to BB" represent a range whose lower limit is the value (AA) recited before "to" and whose upper limit is the value (BB) recited after "to."

First Exemplary Embodiment

An arrangement of an organic EL device according to a first exemplary embodiment of the invention will be described below.

The organic EL device includes an anode, a cathode, and an organic layer between the anode and the cathode. The organic layer includes at least one layer formed from an organic compound(s). Alternatively, the organic layer includes a plurality of layers formed from an organic compound(s). The organic layer may further contain an inorganic compound(s). In the organic EL device of the exemplary embodiment, at least one layer of the organic layer is an emitting layer. For instance, the organic layer may be one emitting layer, or may further include a layer(s) usable in the organic EL device. Examples of the layer usable in the organic EL device, which are not particularly limited, include at least one selected from the group consisting of a hole injecting layer, a hole transporting layer, an electron injecting layer, an electron transporting layer, and a blocking layer.

The organic EL device of the exemplary embodiment includes the emitting layer between the anode and the cathode.

Fig. 1 schematically illustrates an exemplary arrangement of an organic EL device according to the exemplary embodiment.

An organic EL device 1 includes a light-transmissive substrate 2, an anode 3, a cathode 4, and organic layers 10 provided between the anode 3 and the cathode 4. The organic layers 10 are a hole injecting layer 6, a hole transporting layer 7, an emitting layer 5, an electron transporting layer 8, and an electron injecting layer 9 that are layered on the anode 3 in this order.

The emitting layer 5 may contain a metal complex.

The emitting layer 5 preferably does not contain a phosphorescent material (dopant material).

The emitting layer 5 preferably does not contain a heavy-metal complex and a phosphorescent rare earth metal complex. Examples of the heavy-metal complex herein include iridium complex, osmium complex, and platinum complex.

The emitting layer 5 also preferably does not contain a metal complex.

In the organic EL device 1 according to the exemplary embodiment, the emitting layer 5 contains a delayed fluorescent compound M2 and a compound M3 represented by a formula (1).

In this arrangement, the compound M2 is preferably a dopant material (also referred to as a guest material, emitter or luminescent material), and the compound M3 is preferably a host material (also referred to as a matrix material).

The compound M3 may be a delayed fluorescent compound or a compound exhibiting no delayed fluorescence.

Patent Literature 2 discloses a compound in which benzofuranocarbazole or benzothienocarbazole is bonded to dibenzofuran or dibenzothiophene via biphenylene with a long conjugation length (hereinafter also referred to as a compound of Patent Literature 2), and an organic EL device in which the compound of Patent Literature 2 and a delayed fluorescent compound are contained in an emitting layer. However, since the compound of Patent Literature 2 has a low triplet energy and therefore a triplet energy of the delayed fluorescent compound cannot be sufficiently trapped, the resultant device cannot be sufficiently improved in efficiency.

The inventors have found that a high-performance organic EL device is achievable by containing the compound M3 represented by the formula (1) (the compound M3 of the exemplary embodiment) and the delayed fluorescent compound M2 in the emitting layer.

The compound M3 of the exemplary embodiment is a compound in which benzofuranocarbazole or benzothienocarbazole, which supplies an adequate amount of holes to the emitting layer, is bonded to dibenzofuran or dibenzothiophene, which is highly durable, via phenylene with a short conjugation length or via a single bond. Since the

compound M3 of the exemplary embodiment exhibits a high triplet energy, a triplet energy of the delayed fluorescent compound can be sufficiently trapped.

Therefore, the organic EL device according to the exemplary embodiment can provide a high-performance organic EL device, particularly, an organic EL device that emits light at a high efficiency.

An arrangement of an organic EL device according to the exemplary embodiment will be described in details below. It should be noted that the description of reference numerals are omitted below.

Emitting Layer

Compound M3

The emitting layer of the exemplary embodiment includes the compound M3 represented by the formula (1) below.

The compound M3 of the exemplary embodiment may be a thermally activated delayed fluorescent compound or a compound exhibiting no thermally activated delayed fluorescence. However, the compound M3 is preferably a compound exhibiting no thermally activated delayed fluorescence.

[Formula 28]

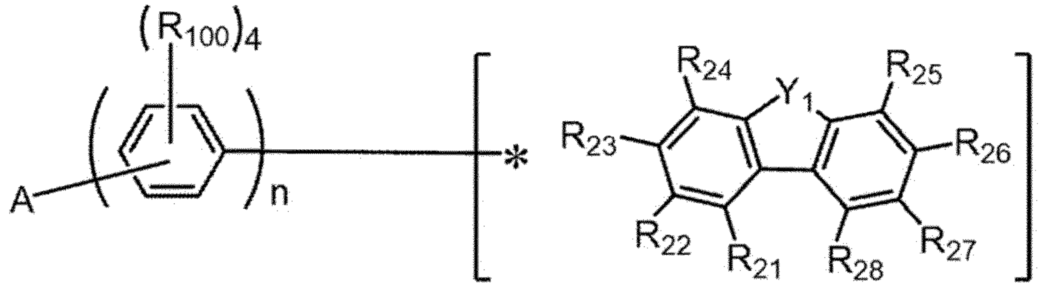

(1)

In the formula (1):

A is a group represented by any one of formulae (11A), (11B), (11C), (11D), (11E), and (11F) below;
$Y_1$ is an oxygen atom or a sulfur atom;
n is 0 or 1;
at least one combination of adjacent two or more of $R_{21}$ to $R_{28}$ are mutually bonded to form a substituted or unsubstituted monocyclic ring, mutually bonded to form a substituted or unsubstituted fused ring, or not mutually bonded;
$R_{21}$ to $R_{28}$ forming neither the substituted or unsubstituted monocyclic ring nor the substituted or unsubstituted fused ring, and $R_{100}$ are each independently a hydrogen atom, a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted cycloalkyl group having 3 to 30 ring carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, a group represented by $-N(Rz)_2$, a thiol group, a substituted or unsubstituted alkylthio group having 1 to 30 carbon atoms, a substituted or unsubstituted aralkyl group having 7 to 30 ring carbon atoms, a substituted germanium group, a substituted phosphine oxide group, a nitro group, a substituted boryl group, or a substituted or unsubstituted arylthio group having 6 to 30 ring carbon atoms;
Rz is a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms;
two Rz in $-N(Rz)_2$ are mutually the same or different;
a plurality of $R_{100}$ are mutually the same or different;
when n is 0 and $X_1$ in the formulae (11A), (11B), (11C), (11D), (11E), and (11F) is an oxygen atom, $R_{25}$ is not a substituted or unsubstituted dibenzofuranyl group; and
* in the formula (1) represents a bonding position to any one of carbon atoms of a six-membered ring to which $R_{21}$ to $R_{24}$ are bonded.

[Formula 29]

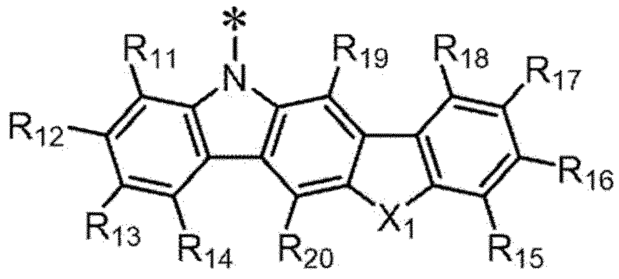

(11A)

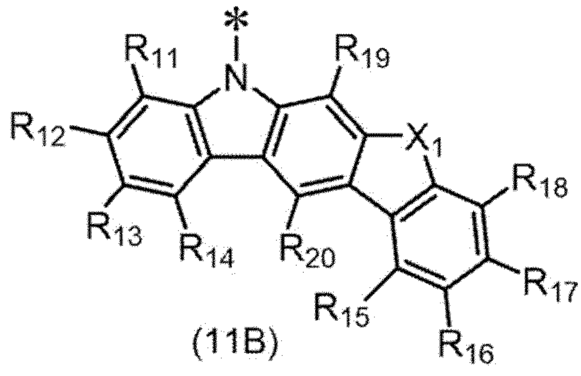

(11B)

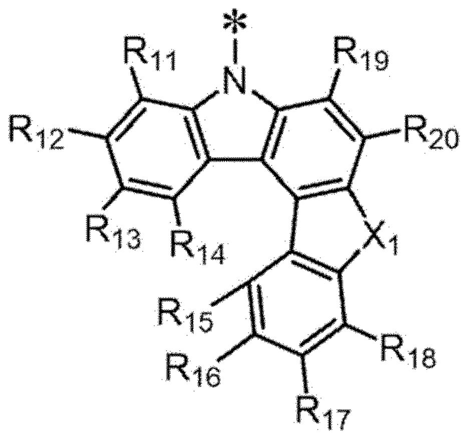

(11C)

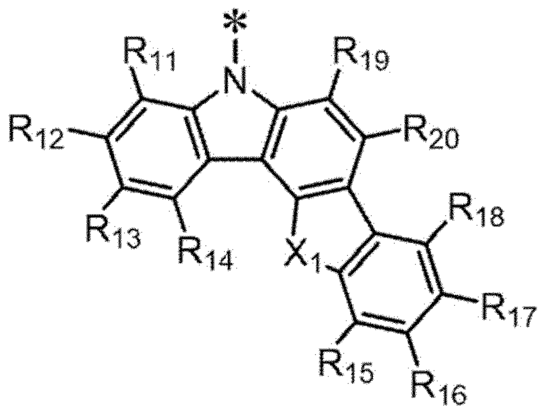

(11D)

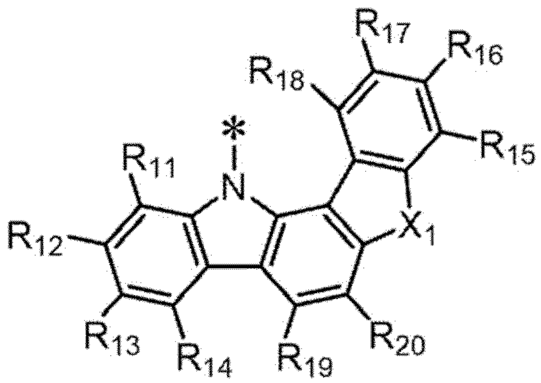

(11E)

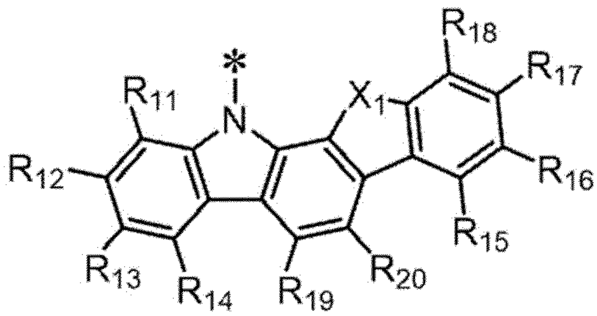

(11F)

In an example of each of the formulae (11A), (11B), (11C), (11D), (11E), and (11F):

$X_1$ is an oxygen atom or a sulfur atom;
at least one combination of adjacent two or more of $R_{11}$ to $R_{20}$ are mutually bonded to form a substituted or unsubstituted monocyclic ring, mutually bonded to form a substituted or unsubstituted fused ring, or not mutually bonded;
$R_{11}$ to $R_{20}$ forming neither the substituted or unsubstituted monocyclic ring nor the substituted or unsubstituted fused ring each independently represent the same as $R_{21}$ to $R_{28}$ forming neither the substituted or unsubstituted monocyclic ring nor the substituted or unsubstituted fused ring in the formula (1);
when n is 0, * represents a bonding position to any one of carbon atoms of a six-membered ring to which $R_{21}$ to $R_{24}$ are bonded in the formula (1); and

when n is 1, * represents a bonding position to any one of carbon atoms of a benzene ring to which $R_{100}$ is bonded in the formula (1).

In an example of each of the formulae (11A), (11B), (11C), (11D), (11E), and (11F):

$X_1$ is an oxygen atom or a sulfur atom;
at least one combination of adjacent two or more of $R_{11}$ to $R_{14}$ are mutually bonded to form a substituted or unsubstituted monocyclic ring, mutually bonded to form a substituted or unsubstituted fused ring, or not mutually bonded;
at least one combination of adjacent two or more of $R_{15}$ to $R_{18}$ are mutually bonded to form a substituted or unsubstituted monocyclic ring, mutually bonded to form a substituted or unsubstituted fused ring, or not mutually bonded;
$R_{19}$ and $R_{20}$ are each a hydrogen atom;
$R_{11}$ to $R_{18}$ forming neither the substituted or unsubstituted monocyclic ring nor the substituted or unsubstituted fused ring each independently represent the same as $R_{21}$ to $R_{28}$ forming neither the substituted or unsubstituted monocyclic ring nor the substituted or unsubstituted fused ring in the formula (1);
when n is 0, * represents a bonding position to any one of carbon atoms of a six-membered ring to which $R_{21}$ to $R_{24}$ are bonded in the formula (1); and
when n is 1, * represents a bonding position to any one of carbon atoms of a benzene ring to which $R_{100}$ is bonded in the formula (1).

The formulae (11A), (11B), (11C), (11D), (11E), and (11F) are exemplified by formulae (111A), (111B), (111C), (111D), (111E), and (111F) below, respectively.

[Formula 30]

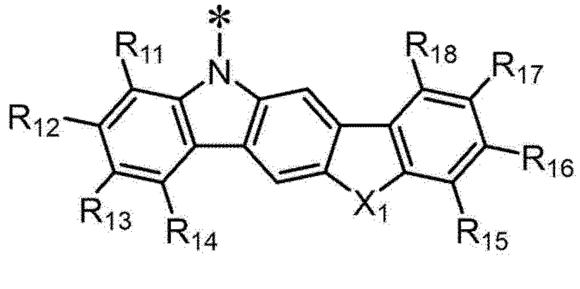

(111A)

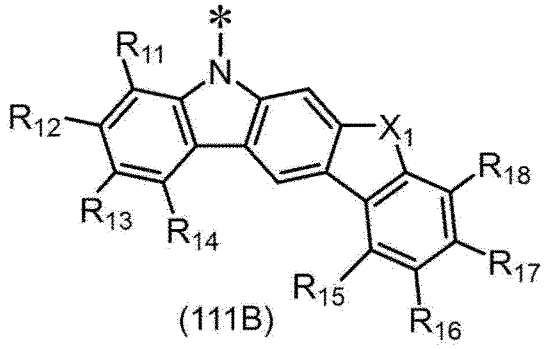

(111B)

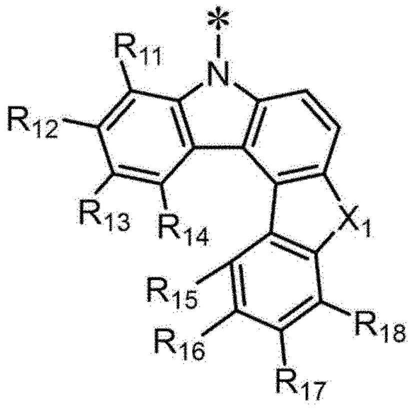

(111C)

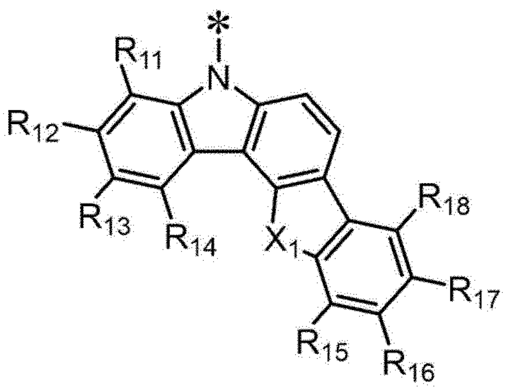

(111D)

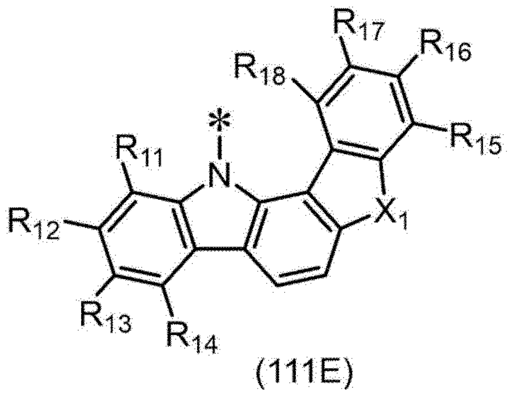

(111E)

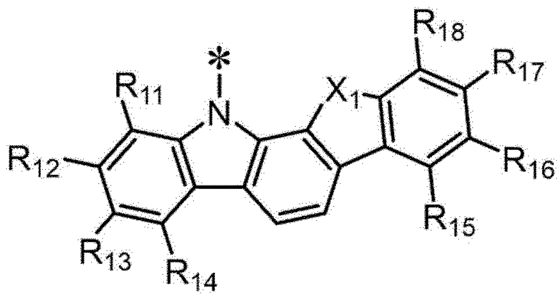

(111F)

In the formulae (111A), (111B), (111C), (111D), (111E), and (111F): $X_1$ represents the same as $X_1$ in the formula (11A); $R_{11}$ to $R_{18}$ each independently represent the same as $R_{21}$ to $R_{28}$ forming neither the substituted or unsubstituted monocyclic ring nor the substituted or unsubstituted fused ring in the formula (1); and * each represents a bonding position.

The compound M3 represented by the formula (1) also can be represented by a formula (1-1), (1-2), (1-3), or (1-4) below.

The compound of the exemplary embodiment (the compound M3 represented by the formula (1)) is a compound represented by the formula (1-1), (1-2), (1-3), or (1-4) below.

[Formula 31]

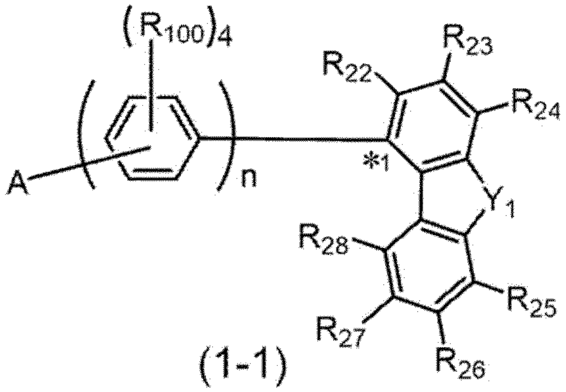

(1-1)

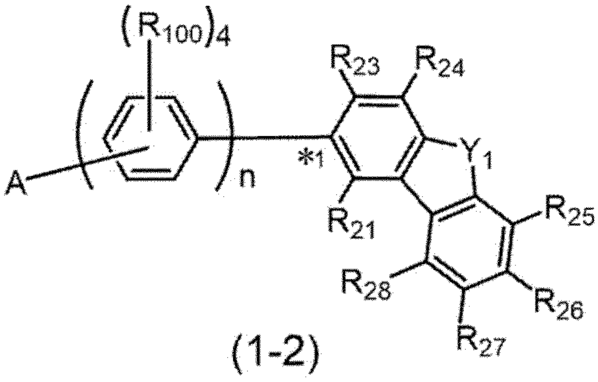

(1-2)

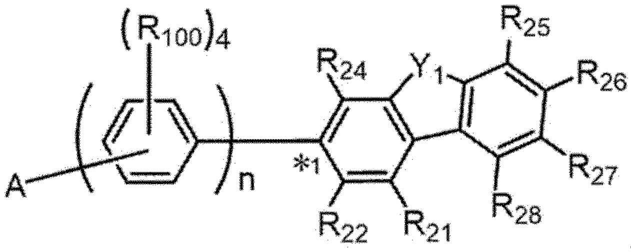

(1-3)

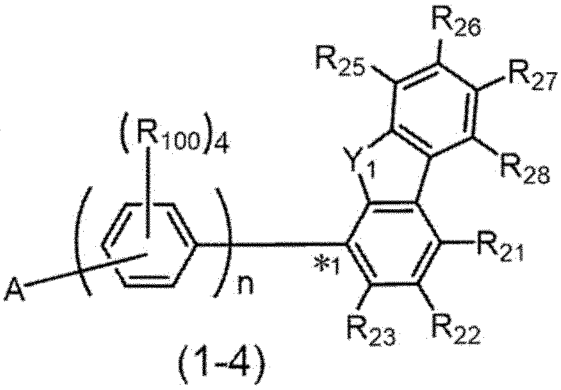

(1-4)

In the formulae (1-1), (1-2), (1-3), and (1-4):
A, $R_{100}$, n, $Y_1$, and $R_{21}$ to $R_{28}$ respectively independently represent the same as A, $R_{100}$, n, $Y_1$, and $R_{21}$ to $R_{28}$ in the formula (1), a plurality of $R_{100}$ being mutually the same or different.

When n is 0, each * in the formulae (11A), (11B), (11C), (11D), (11E), and (11F) represents a bonding position to *1.

When n is 1, each * in the formulae (11A), (11B), (11C), (11D), (11E), and (11F) represents a bonding position to any one of carbon atoms of a benzene ring to which $R_{100}$ is bonded.

In the compound M3 according to an exemplary embodiment, n is 1.

The compound M3 of the exemplary embodiment is a compound represented by a formula (12A) below.

In a case where the compound represented by the formula (12A) (compound M3) and a compound represented by a formula (2A) described later (compound M1) are used in combination, external quantum efficiency is more improvable than in a case where a compound represented by a formula (12C) below (compound M3) and a compound represented by a formula (2A) described later (compound M1) are used in combination. Especially, by selecting a compound represented by the formula (12A) in which $X_1$ in A is a sulfur atom as the compound M3, the external quantum efficiency and a lifetime are more improvable.

[Formula 32]

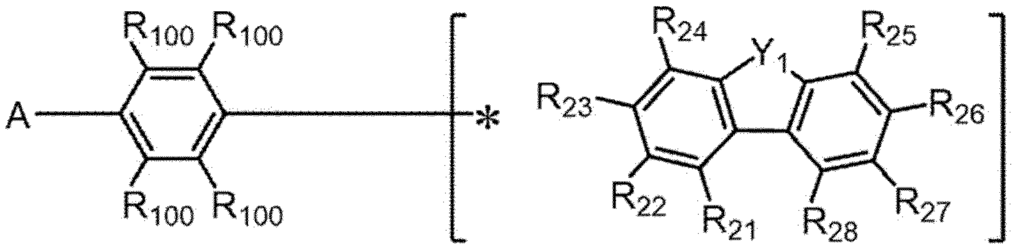

(12A)

In the formula (12A): A, $R_{100}$, $Y_1$, and $R_{21}$ to $R_{28}$ in the formula (12A) respectively independently represent the

same as A, $R_{100}$, $Y_1$, and $R_{21}$ to $R_{28}$ in the formula (1), a plurality of $R_{100}$ being mutually the same or different; and * represents a bonding position to any one of carbon atoms of a six-membered ring to which $R_{21}$ to $R_{24}$ are bonded.

In an exemplary embodiment, the compound M3 is a compound represented by a formula (12B) below.

[Formula 33]

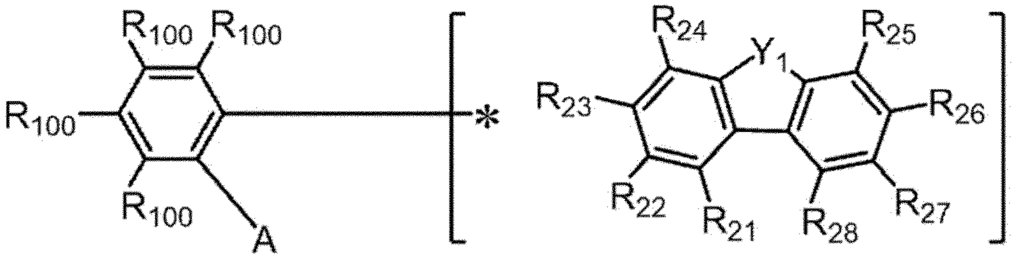

(12B)

In the formula (12B): A, $R_{100}$, $Y_1$, and $R_{21}$ to $R_{28}$ in the formula (12B) respectively independently represent the same as A, $R_{100}$, $Y_1$, and $R_{21}$ to $R_{28}$ in the formula (1), a plurality of $R_{100}$ being mutually the same or different; and * represents a bonding position to any one of carbon atoms of a six-membered ring to which $R_{21}$ to $R_{24}$ are bonded.

In an exemplary embodiment, the compound M3 is a compound represented by a formula (12C) below.

[Formula 34]

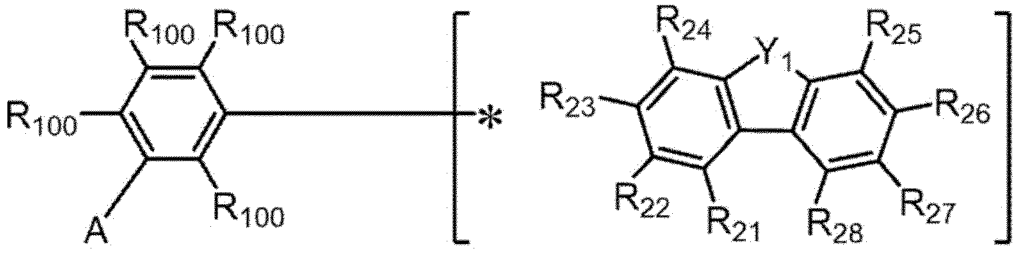

(12C)

In the formula (12C): A, $R_{100}$, $Y_1$, and $R_{21}$ to $R_{28}$ in the formula (12C) respectively independently represent the same as A, $R_{100}$, $Y_1$, and $R_{21}$ to $R_{28}$ in the formula (1), a plurality of $R_{100}$ being mutually the same or different; and * represents a bonding position to any one of carbon atoms of a six-membered ring to which $R_{21}$ to $R_{24}$ are bonded.

In the compound M3 according to an exemplary embodiment, n is 0, or n is 1 and $R_{100}$ is a hydrogen atom.

In the compound M3 according to an exemplary embodiment, n is 0.

In the compound M3 according to an exemplary embodiment, A is a group represented by the formula (11A), (11B), (11C), (11E) or (11F).

In the compound M3 according to an exemplary embodiment, A is a group represented by the formula (11E) or (11F).

In the compound M3 according to an exemplary embodiment, A is a group represented by the formula (11F).

In the compound M3 according to an exemplary embodiment, $X_1$ is an oxygen atom.

In the compound M3 according to an exemplary embodiment, $X_1$ is a sulfur atom.

In the compound M3 according to an exemplary embodiment, $Y_1$ is an oxygen atom.

In the compound M3 according to an exemplary embodiment, $X_1$ and $Y_1$ are each an oxygen atom.

In the compound M3 according to an exemplary embodiment, when n is 0, $R_{25}$ is neither a substituted or unsubstituted dibenzofuranyl group nor a substituted or unsubstituted dibenzothienyl group.

In the compound M3 according to an exemplary embodiment, when n is 0, $R_{21}$ to $R_{28}$ are neither a substituted or unsubstituted dibenzofuranyl group nor a substituted or unsubstituted dibenzothienyl group.

In the compound M3 according to an exemplary embodiment, when n is 0, in the formula (1), a carbon atom of a six-membered ring to which $R_{22}$ is bonded is not bonded to *.

In the compound M3 according to an exemplary embodiment, $R_{19}$ and $R_{20}$ are each a hydrogen atom in the formulae (11A), (11B), (11C), (11D), (11E), and (11F).

In the compound M3 according to an exemplary embodiment, $R_{11}$ to $R_{20}$ are each a hydrogen atom in the formulae (11A), (11B), (11C), (11D), (11E), and (11F).

In an exemplary embodiment, the compound M3 is a compound represented by a formula (12A-1) below.

[Formula 35]

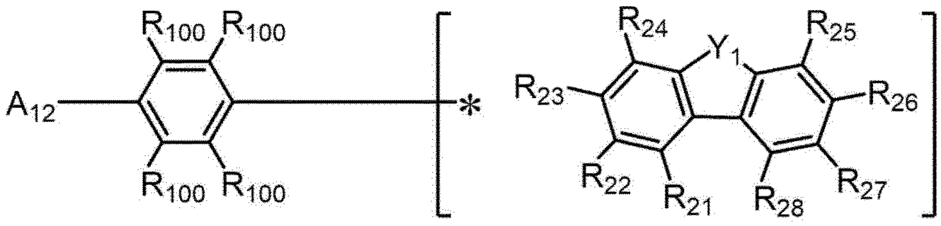

(12A-1)

In the formula (12A-1):

$A_{12}$ is a group represented by any one of formulae (11A-1), (11B-1), (11C-1), (11D-1), (11E-1), and (11F-1) below; $R_{100}$, $Y_1$, and $R_{21}$ to $R_{28}$ respectively independently represent the same as $R_{100}$, $Y_1$, and $R_{21}$ to $R_{28}$ in the formula (1), a plurality of $R_{100}$ being mutually the same or different; and
* represents a bonding position to any one of carbon atoms of a six-membered ring to which $R_{21}$ to $R_{24}$ are bonded.

[Formula 36]

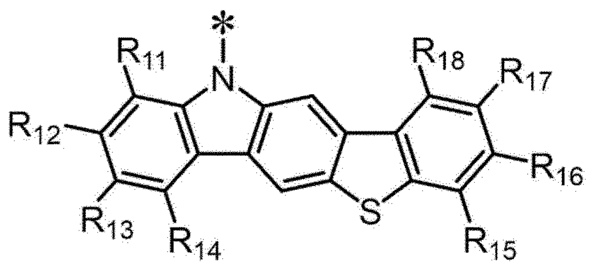

(11A-1)

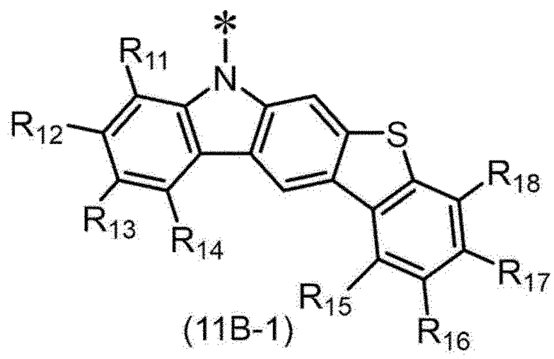

(11B-1)

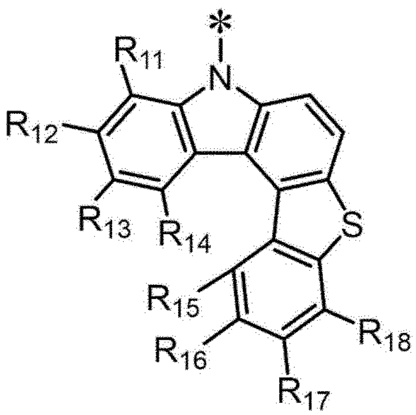

(11C-1)

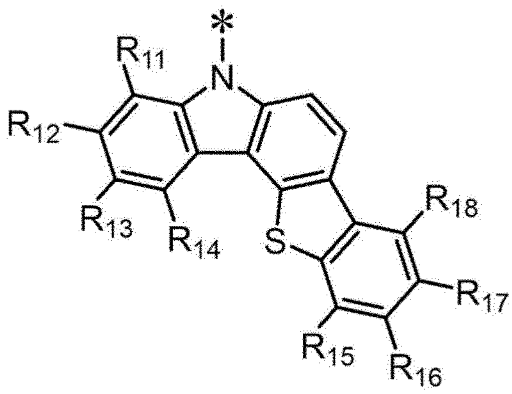

(11D-1)

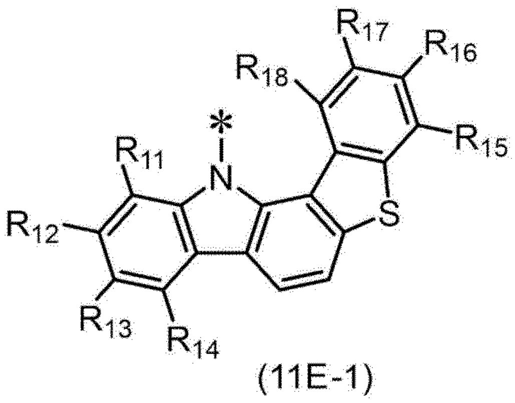

(11E-1)

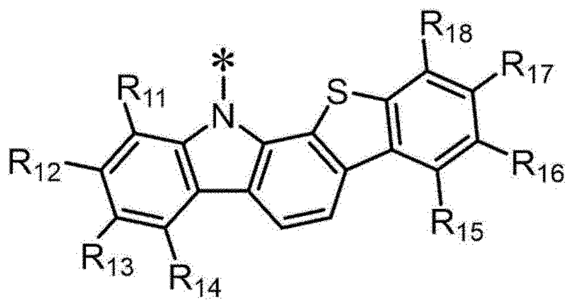

(11F-1)

In the formulae (11A-1), (11B-1), (11C-1), (11D-1), (11E-1), and (11F-1), $R_{11}$ to $R_{18}$ each independently represent the same as $R_{11}$ to $R_{18}$ in the formula (1); and each* represents a bonding position.

In the compound M3 according to an exemplary embodiment, $R_{11}$ to $R_{18}$ are each a hydrogen atom in the formulae (11A-1), (11B-1), (11C-1), (11D-1), (11E-1), and (11F-1).

In the emitting layer according to an exemplary embodiment, only the compound M3 has a larger singlet energy $S_1$ than a singlet energy $S_1$(M2) of the delayed fluorescent compound M2.

In the compound M3 according to an exemplary embodiment, when n is 1, $R_{100}$ is not a substituted or unsubstituted dibenzofuranyl group.

In an exemplary embodiment, the compound M3 is a compound represented by a formula (100) below.

[Formula 37]

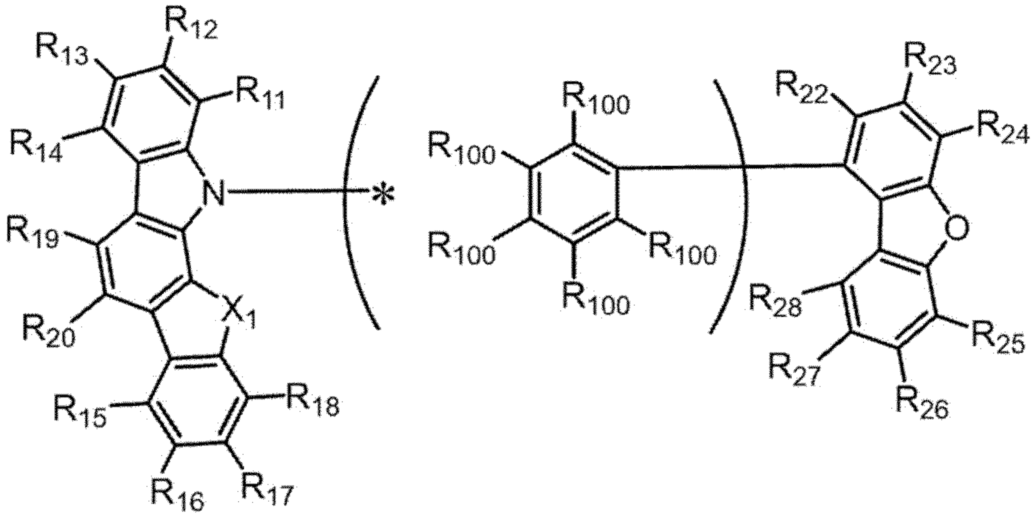

(100)

In the formula (100):

$X_1$ is an oxygen atom or a sulfur atom;

$R_{100}$, $R_{11}$ to $R_{20}$, and $R_{22}$ to $R_{28}$ are each independently a hydrogen atom, a group represented by $-(L_{101})nx-R_{101}$;

nx is 0, 1, 2, or 3;

when a plurality of groups represented by $-(L_{101})nx-R_{101}$ are present, the plurality of groups represented by $-(L_{101})nx-R_{101}$ are mutually the same or different;

a plurality of $R_{100}$ are mutually the same or different;

$R_{101}$ is an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, an unsubstituted dibenzothienyl group, an unsubstituted (9-dibenzofuranyl)carbazolyl group, an unsubstituted (9-dibenzothienyl)carbazolyl group, a monovalent group derived from a compound represented by a formula (101) below, a monovalent group derived from a compound represented by a formula (102) below, a monovalent group derived from a compound represented by a formula (103) below, a monovalent group derived from a compound represented by a formula (104) below, a monovalent group derived from a compound represented by a formula (105) below, or a monovalent group derived from a compound represented by a formula (106) below;

$L_{101}$ is a substituted or unsubstituted alkylene group having 1 to 30 carbon atoms, a substituted or unsubstituted phenylene group, a substituted or unsubstituted dibenzofuranylene group, a substituted or unsubstituted dibenzothienylene group, a substituted or unsubstituted carbazolylene group, a substituted or unsubstituted (9-dibenzofuranyl)carbazolylene group, a substituted or unsubstituted (9-dibenzothienyl)carbazolylene group, a divalent group derived from a compound represented by the formula (101) below, a divalent group derived from a compound represented by the formula (102) below, a divalent group derived from a compound represented by the formula (103) below, a divalent group derived from a compound represented by the formula (104) below, a divalent group derived from a compound represented by the formula (105) below, or a divalent group derived from a compound represented by the formula (106) below;

when $L_{101}$ is a substituted alkylene group having 1 to 30 carbon atoms, a substituted phenylene group, a substituted dibenzofranylene group, a substituted dibenzothienylene group, a substituted carbazolylene group, a substituted (9-dibenzofuranyl)carbazolylene group, or a substituted (9-dibenzothienyl)carbazolylene group, a substituent for $L_{101}$ is each independently an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;

when two or more $L_{101}$ are present, the two or more $L_{101}$ are mutually the same or different;

when two or more $R_{101}$ are present, the two or more $R_{101}$ are mutually the same or different; and

* represents a bonding position to any one of carbon atoms of a benzene ring to which $R_{100}$ is bonded.

[Formula 38]

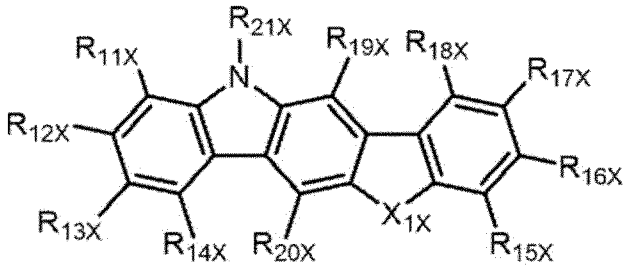

(101)

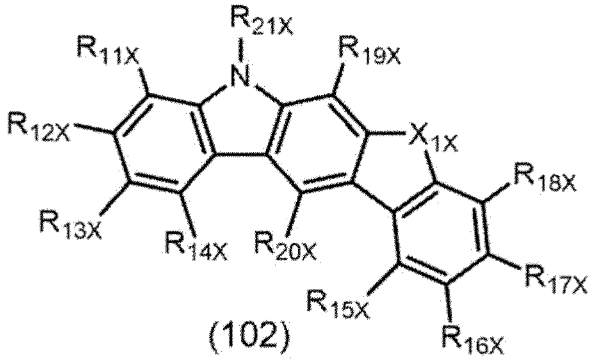

(102)

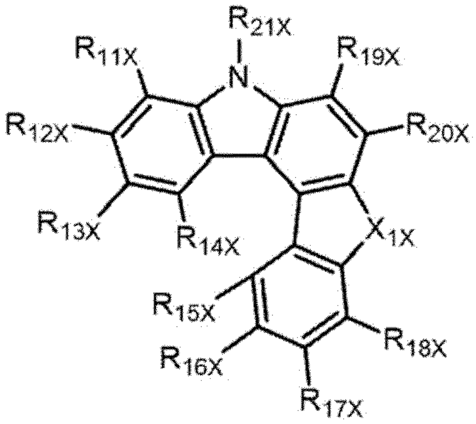

(103)

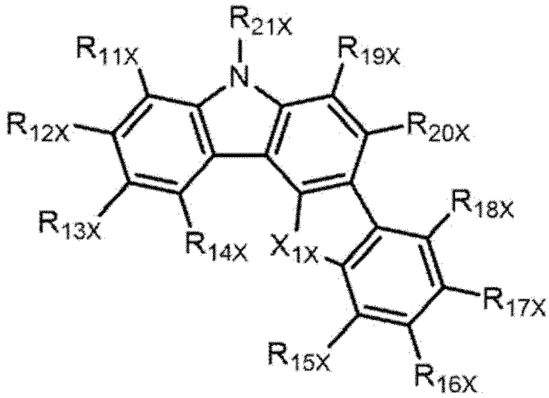

(104)

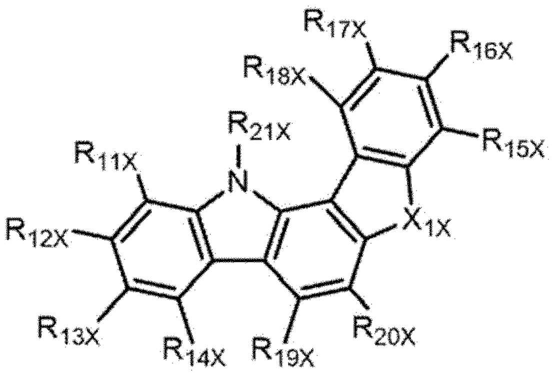

(105)

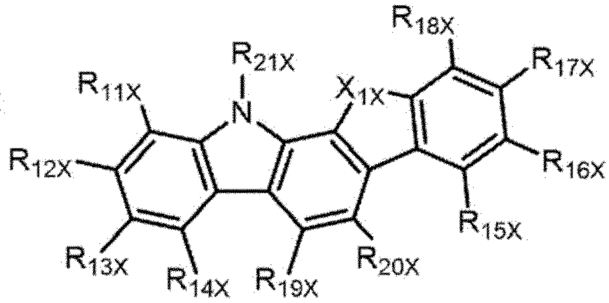

(106)

In the formulae (101) to (106):

$X_{1X}$ is an oxygen atom or a sulfur atom;
$R_{11X}$ to $R_{21X}$ are each independently a hydrogen atom, an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;
when $R_{101}$ is a monovalent group derived from a compound of any one of the formulae (101) to (106) and nx is 0, any one atom of: carbon atoms of a six-membered ring to which $R_{11X}$ to $R_{20X}$ are bonded; and a nitrogen atom bonded to $R_{21X}$, is bonded to any one of carbon atoms of a six-membered ring to which $R_{11}$ to $R_{20}$, $R_{22}$ to $R_{28}$, and $R_{100}$ are bonded,
when nx is 1, 2, or 3, any one atom of: carbon atoms of the six-membered ring to which $R_{11X}$ to $R_{20X}$ are bonded; and a nitrogen atom bonded to $R_{21X}$, is bonded to $L_{101}$;

when $L_{101}$ is a divalent group derived from a compound of any one of the formulae (101) to (106) and nx is 1, one of two atoms of: carbon atoms of a six-membered ring to which $R_{11X}$ to $R_{20X}$ are bonded; and a nitrogen atom bonded to $R_{21X}$, is bonded to $R_{101}$ and the other of the two atoms is bonded to any one of carbon atoms of a six-membered ring to which $R_{11}$ to $R_{20}$, $R_{22}$ to $R_{28}$, and $R_{100}$ are bonded; and

when $L_{101}$ is a divalent group derived from a compound of any one of the formulae (101) to (106) and nx is 2 or 3, one of two atoms of: carbon atoms of a six-membered ring to which $R_{11X}$ to $R_{20X}$ are bonded; and a nitrogen atom bonded to $R_{21X}$, is bonded to $R_{101}$ or $L_{101}$, and the other of the two atoms is bonded to $L_{101}$ or any one of carbon atoms of a six-membered ring to which $R_{11}$ to $R_{20}$, $R_{22}$ to $R_{28}$, and $R_{100}$ are bonded.

In the compound M3 according to an exemplary embodiment, $R_{19}$ and $R_{20}$ are each a hydrogen atom in the formula (100).

Producing Method of Compound M3 in Embodiment

The compound M3 of the exemplary embodiment can be produced, for instance, by a method described in Example described later. The compound M3 of the exemplary embodiment can be produced by application of known substitution reactions and materials depending on a target compound according to reactions described in Example described later.

Specific examples of the compound M3 in the exemplary embodiment include compounds below. However, the invention is by no means limited to the specifically listed compounds.

[Formula 39]

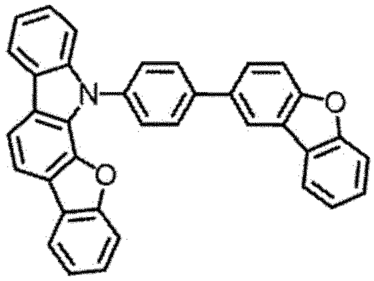

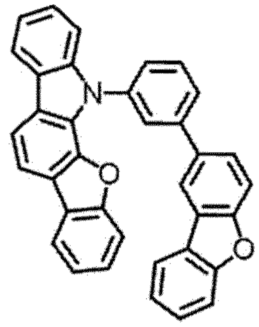

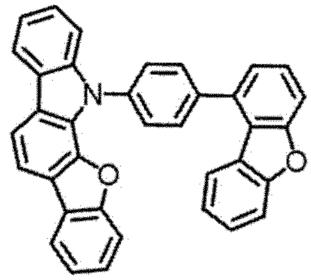

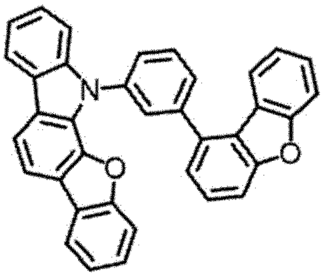

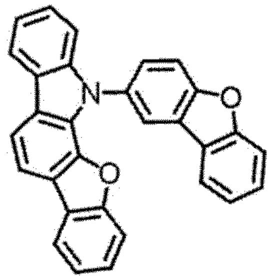

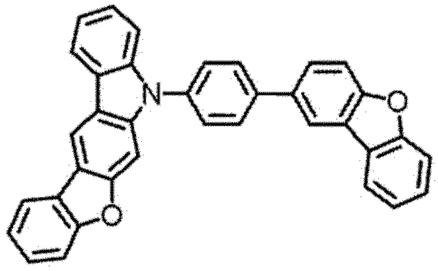

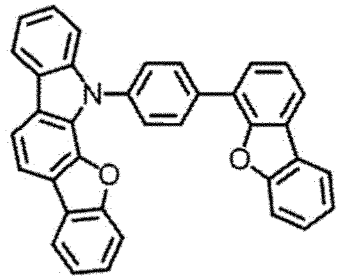

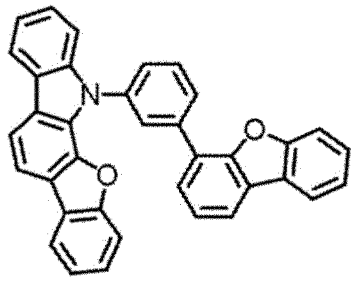

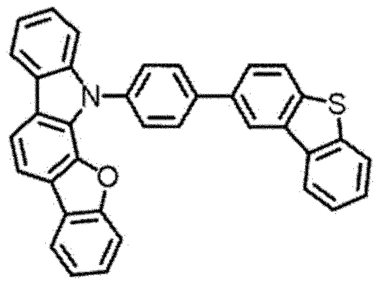

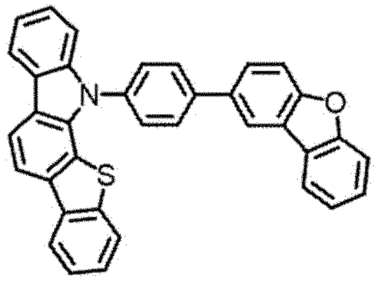

[Formula 40]

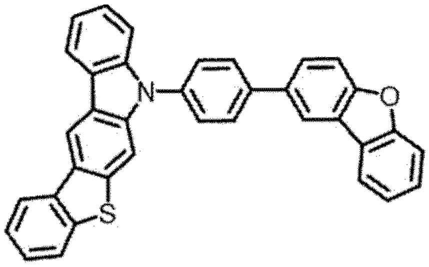
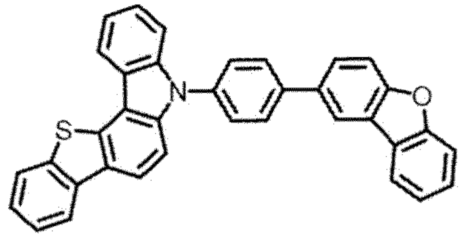
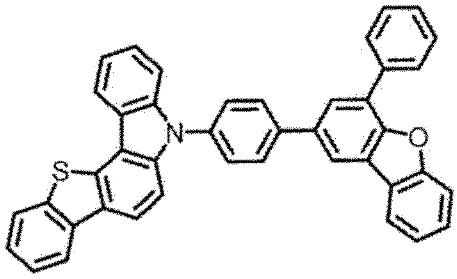
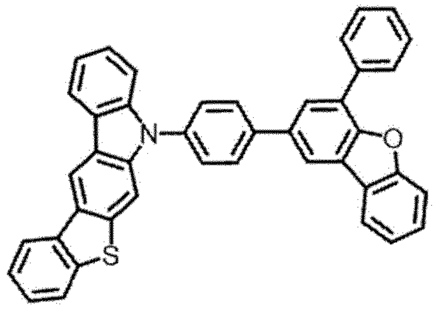
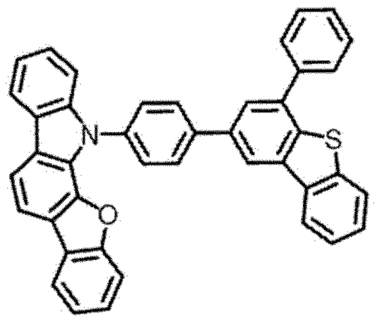
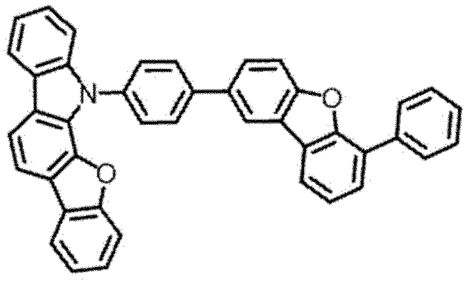
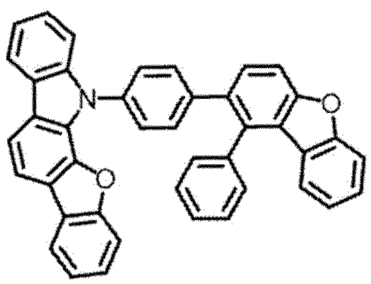
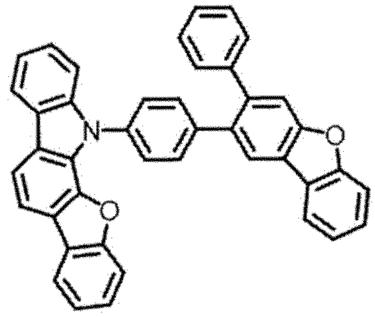
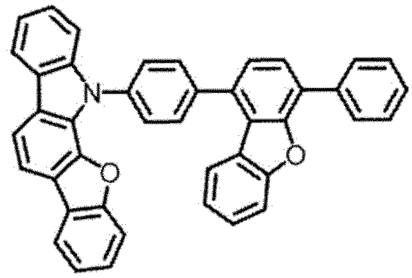
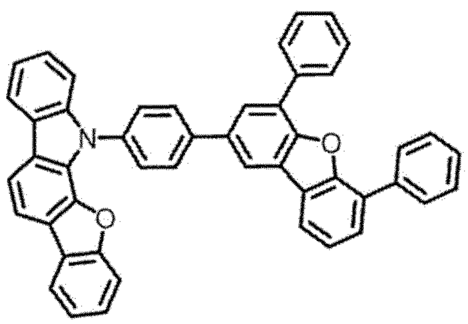
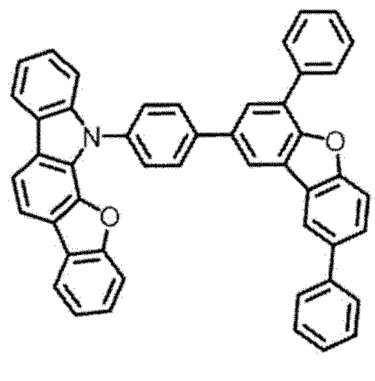
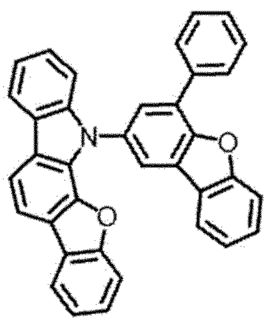

[Formula 41]

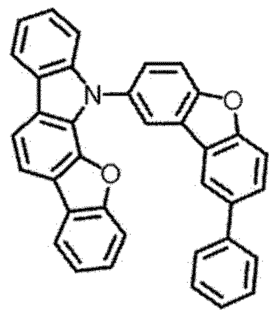

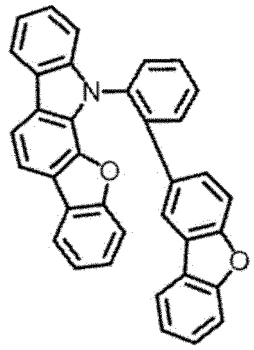

[Formula 42]

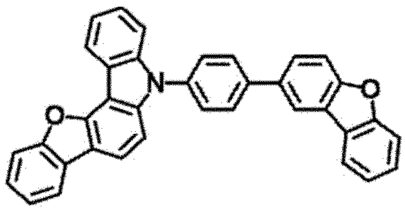

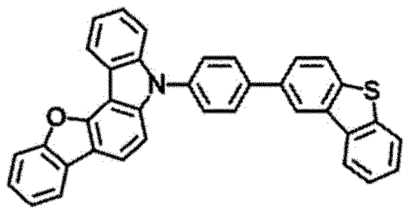

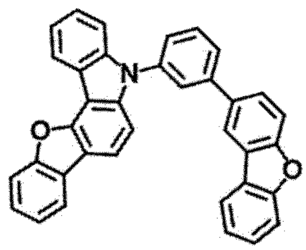

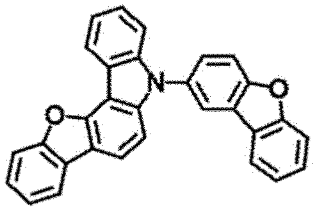

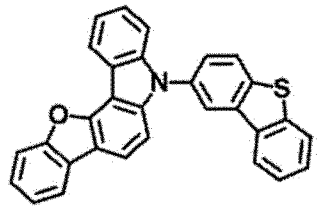

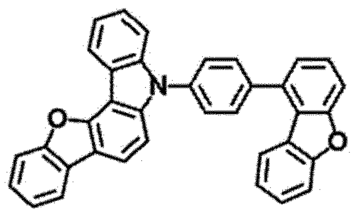

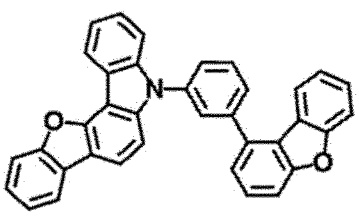

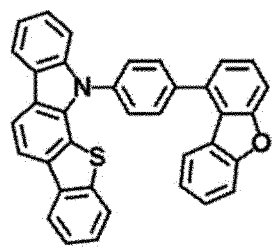

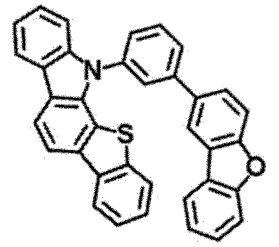

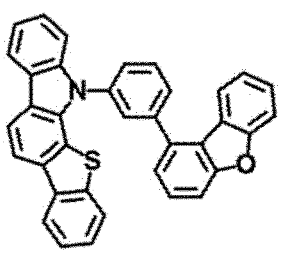

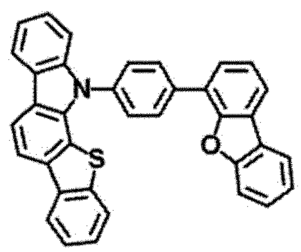

[Formula 43]

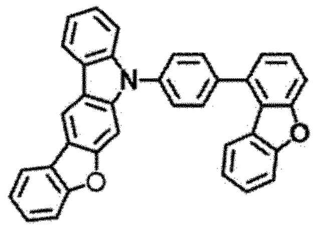
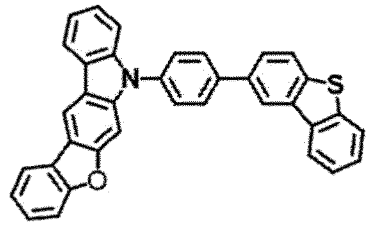
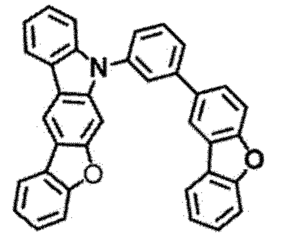
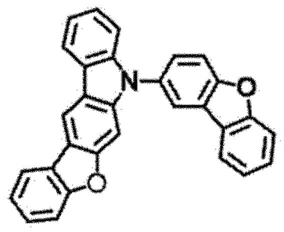
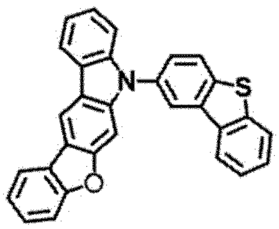
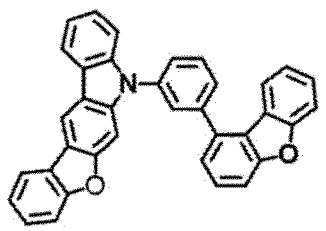
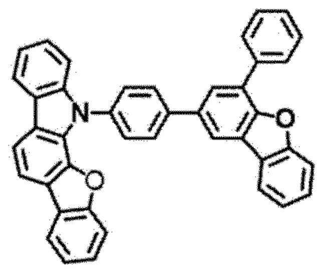
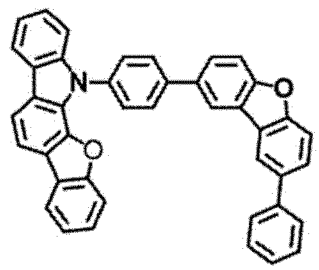
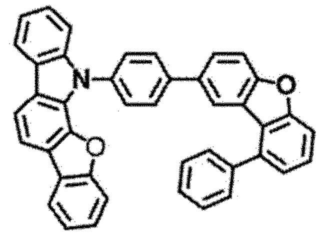
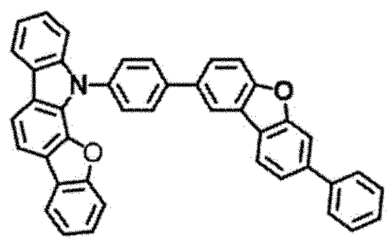
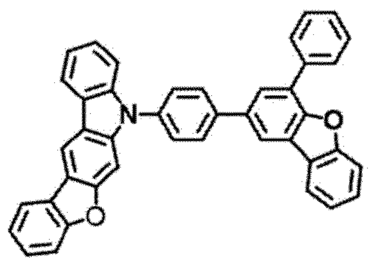
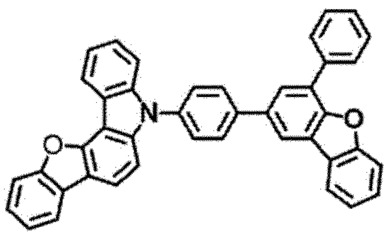
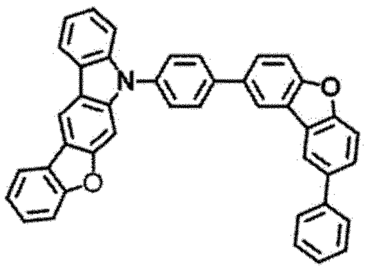
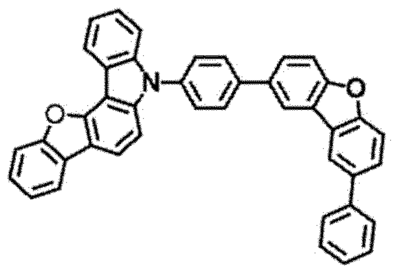

[Formula 44]

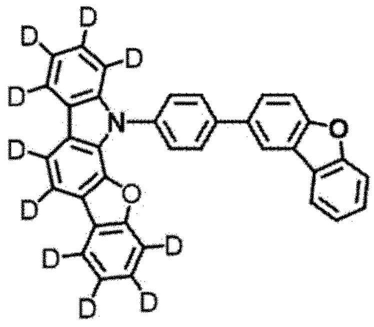
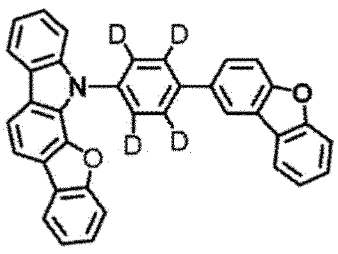
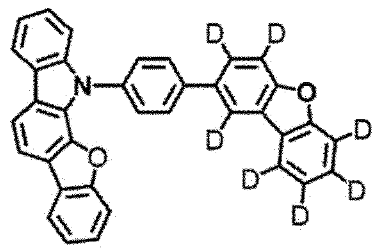
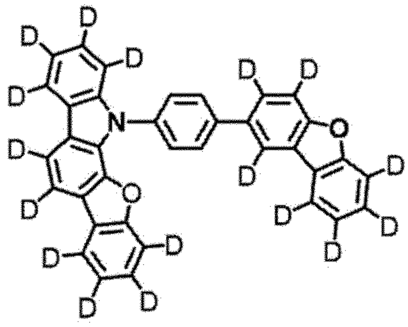
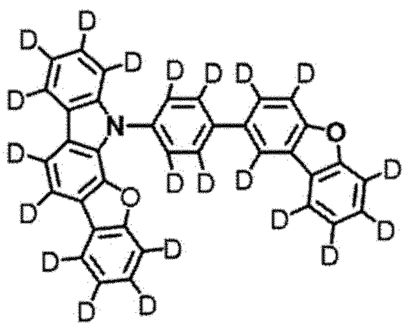
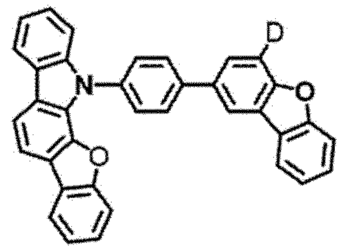
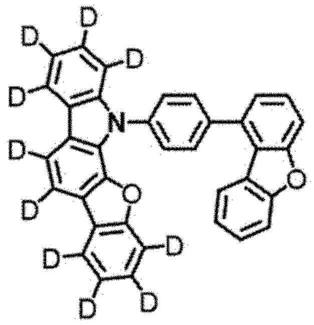
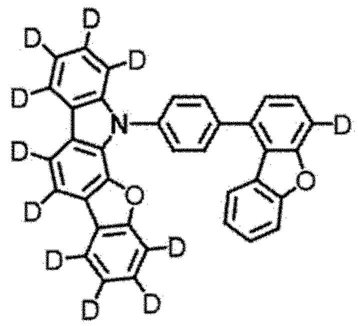
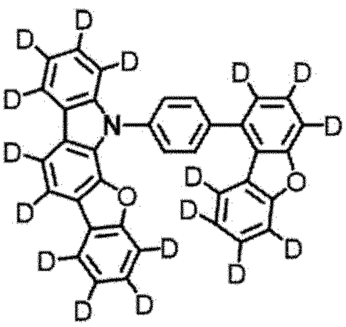
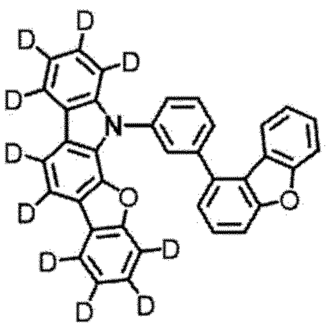
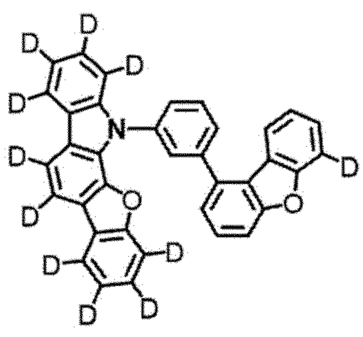
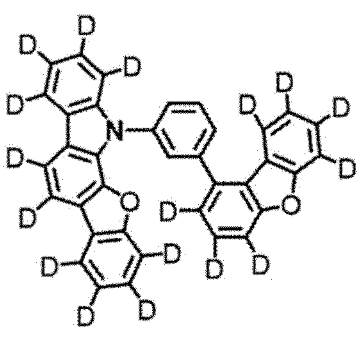

[Formula 45]

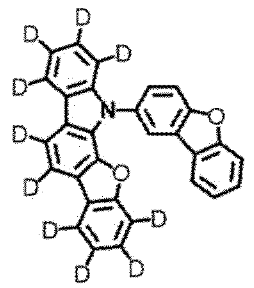
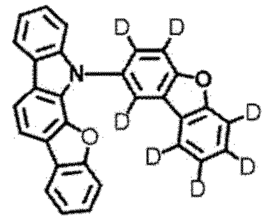
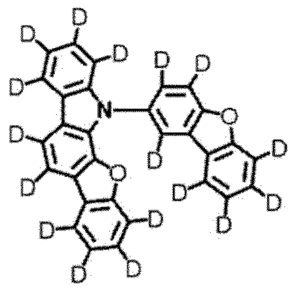

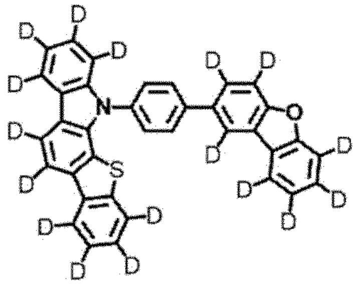
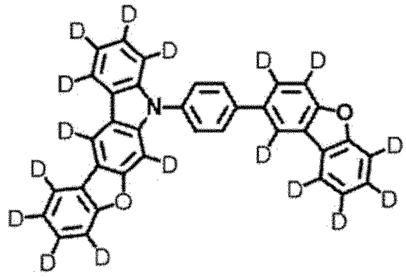
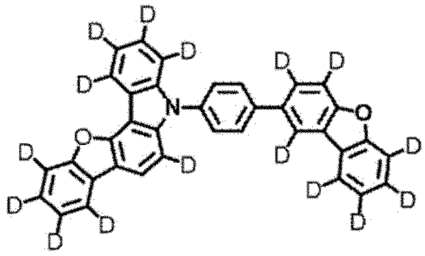

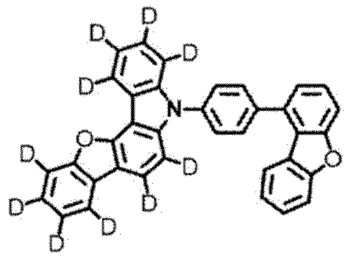
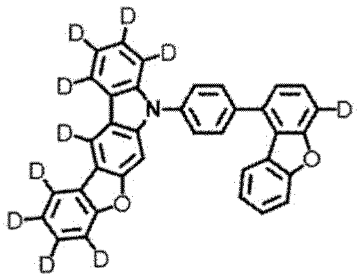
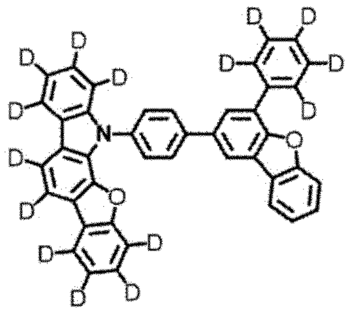

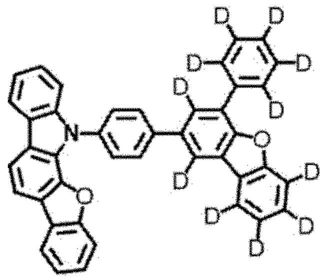
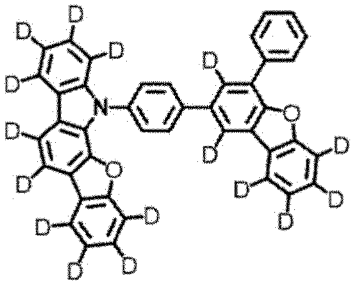
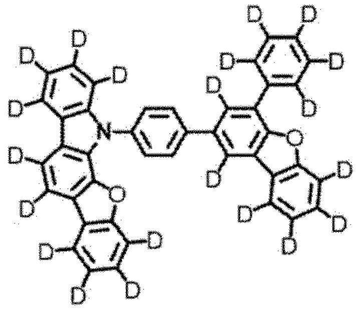

[Formula 46]

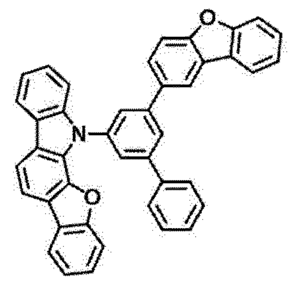

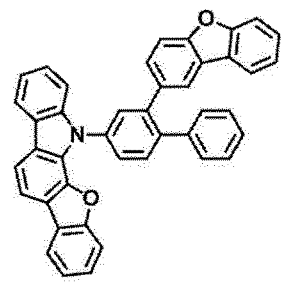

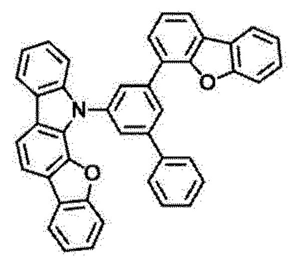

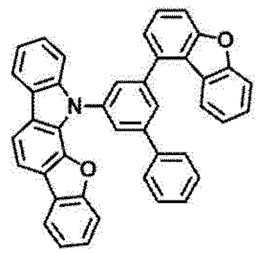

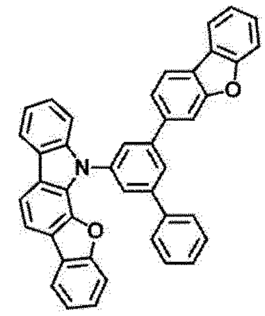

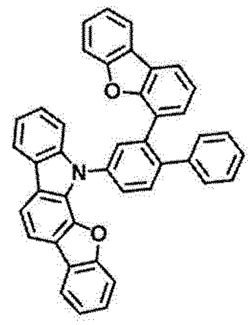

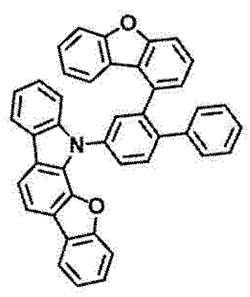

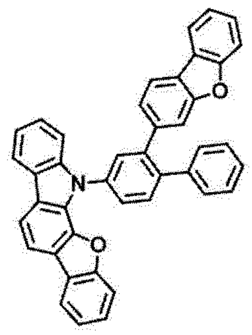

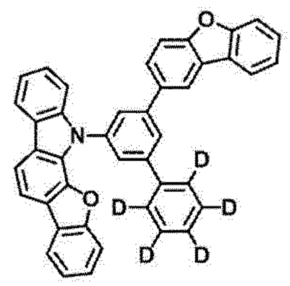

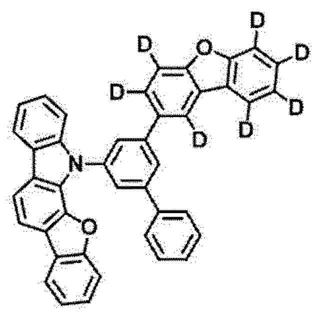

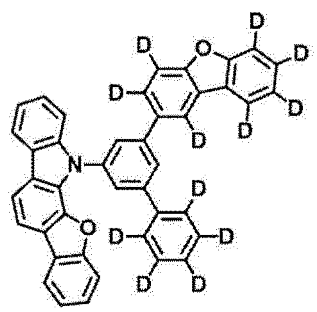

[Formula 47]

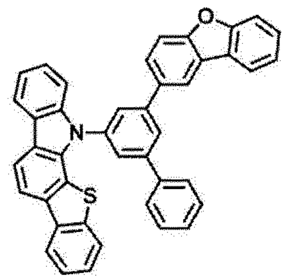
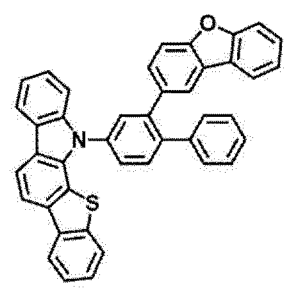

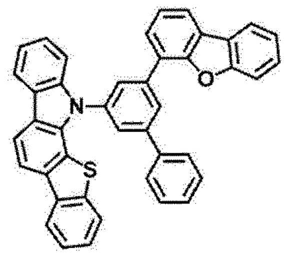
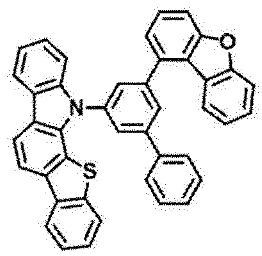
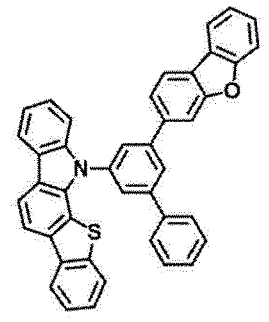

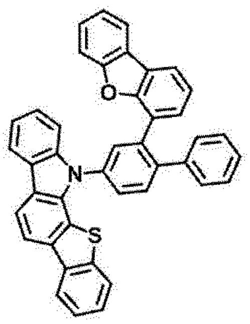
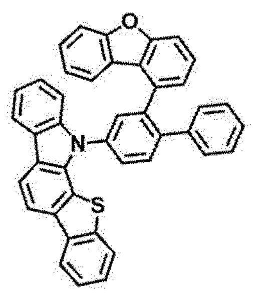
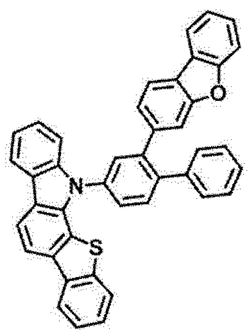

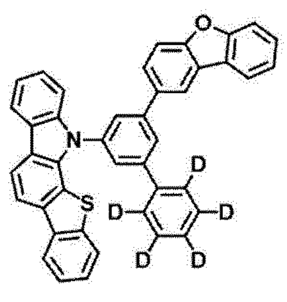
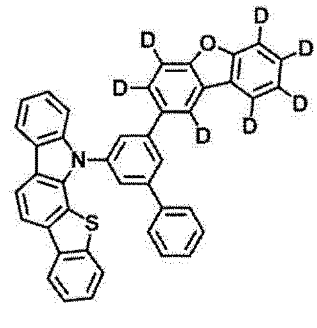
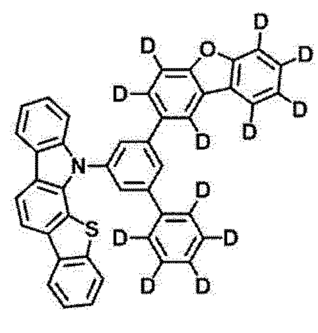

[Formula 48]

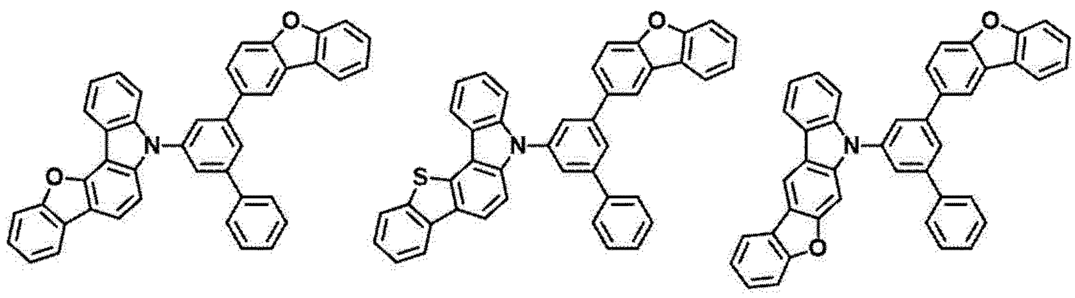

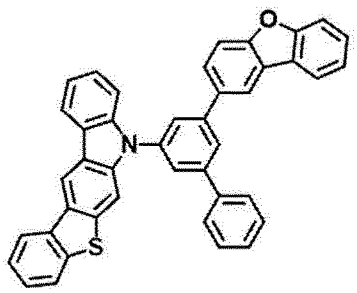

[Formula 49]

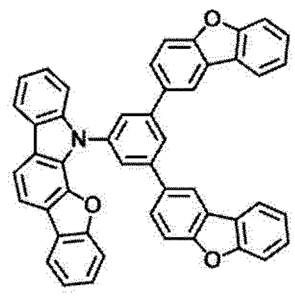
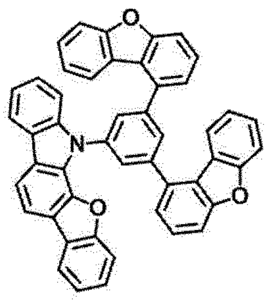
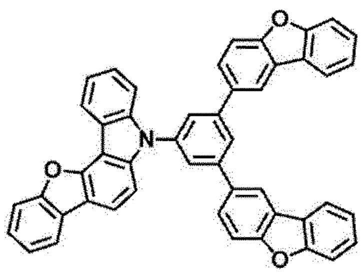
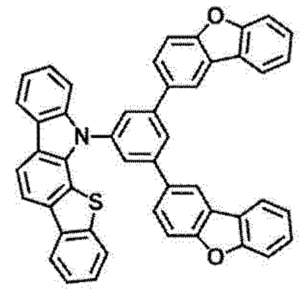
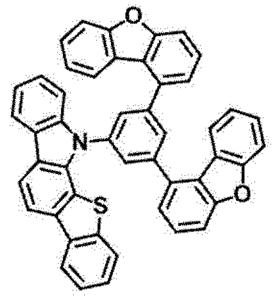
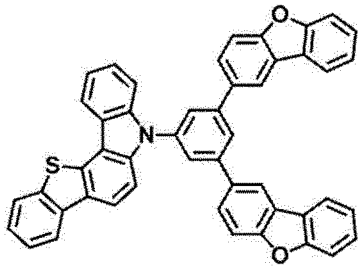
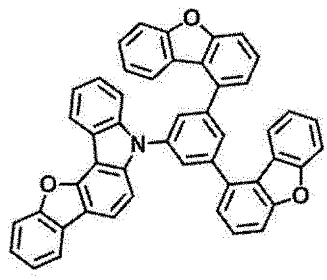
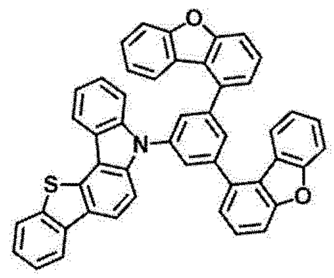
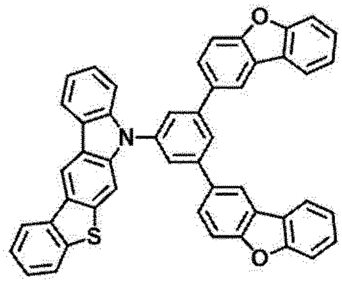
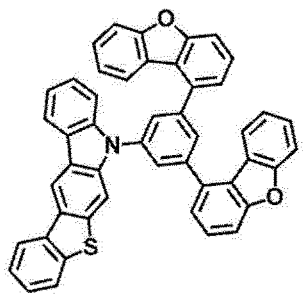

Compound M2

The emitting layer of the exemplary embodiment contains a delayed fluorescent compound M2.

Delayed Fluorescence

Delayed fluorescence is explained in "Yuki Hando-tai no Debaisu Bussei (Device Physics of Organic Semiconductors)" (edited by ADACHI, Chihaya, published by Kodansha, on pages 261-268). This document describes that, if an energy difference $\Delta E_{13}$ of a fluorescent material between a singlet state and a triplet state is reducible, a reverse energy transfer from the triplet state to the singlet state, which usually occurs at a low transition probability, would occur at a high efficiency to express thermally activated delayed fluorescence (TADF). Further, a generation mechanism of delayed fluorescence is explained in Fig. 10.38 in the document. The compound M2 of the exemplary embodiment is preferably a compound exhibiting thermally activated delayed fluorescence generated by such a mechanism.

In general, emission of delayed fluorescence can be confirmed by measuring the transient PL (Photo Luminescence).

The behavior of delayed fluorescence can also be analyzed based on the decay curve obtained from the transient PL measurement. The transient PL measurement is a method of irradiating a sample with a pulse laser to excite the sample, and measuring the decay behavior (transient characteristics) of PL emission after the irradiation is stopped. PL emission in TADF materials is classified into a light emission component from a singlet exciton generated by the first PL excitation and a light emission component from a singlet exciton generated via a triplet exciton. The lifetime of the singlet exciton generated by the first PL excitation is on the order of nanoseconds and is very short. Therefore, light emission from the singlet exciton rapidly attenuates after irradiation with the pulse laser.

On the other hand, the delayed fluorescence is gradually attenuated due to light emission from a singlet exciton generated via a triplet exciton having a long lifetime. As described above, there is a large temporal difference between the light emission from the singlet exciton generated by the first PL excitation and the light emission from the singlet exciton generated via the triplet exciton. Therefore, the luminous intensity derived from delayed fluorescence can be determined.

Fig. 2 is a schematic diagram of an exemplary apparatus for measuring the transient PL. An example of a method of measuring a transient PL as illustrated in Fig. 2 and an example of behavior analysis of delayed fluorescence will be described.

A transient PL measuring apparatus 100 in Fig. 2 includes: a pulse laser 101 capable of radiating a light having a predetermined wavelength; a sample chamber 102 configured to house a measurement sample; a spectrometer 103 configured to divide a light radiated from the measurement sample; a streak camera 104 configured to provide a two-dimensional image; and a personal computer 105 configured to import and analyze the two-dimensional image. An apparatus for measuring the transient PL is not limited to the apparatus illustrated in Fig. 2

The sample housed in the sample chamber 102 is obtained by forming a thin film, in which a matrix material is doped with a doping material at a concentration of 12 mass%, on the quartz substrate.

The thin film sample housed in the sample chamber 102 is irradiated with the pulse laser from the pulse laser 101 to excite the doping material. Emission is extracted in a direction of 90 degrees with respect to a radiation direction of the excited light. The extracted emission is divided by the spectrometer 103 to form a two-dimensional image in the streak camera 104. As a result, the two-dimensional image is obtainable in which the ordinate axis represents a time, the abscissa axis represents a wavelength, and a bright spot represents a luminous intensity. When this two-dimensional image is taken out at a predetermined time axis, an emission spectrum in which the ordinate axis represents the luminous intensity and the abscissa axis represents the wavelength is obtainable. Moreover, when this two-dimensional image is taken out at the wavelength axis, a decay curve (transient PL) in which the ordinate axis represents a logarithm of the luminous intensity and the abscissa axis represents the time is obtainable.

For instance, a thin film sample A was prepared as described above from a reference compound H1 as the matrix material and a reference compound D1 as the doping material and was measured in terms of the transient PL.

[Formula 50]

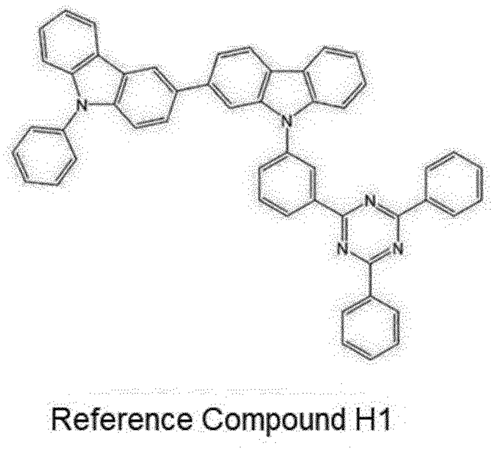

Reference Compound H1

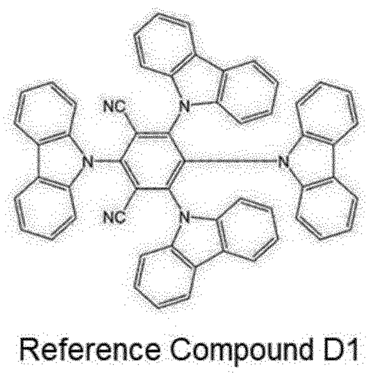

Reference Compound D1

The decay curve was analyzed with respect to the above thin film sample A and a thin film sample B. The thin film sample B was produced in the same manner as described above from a reference compound H2 as the matrix material and the reference compound D1 as the doping material.

Fig. 3 illustrated decay curves obtained from transient PL obtained by measuring the thin film samples A and B.

[Formula 51]

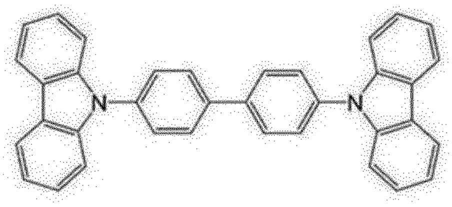

Reference Compound H2

As described above, an emission decay curve in which the ordinate axis represents the luminous intensity and the abscissa axis represents the time can be obtained by the transient PL measurement. Based on the emission decay curve, a fluorescence intensity ratio between fluorescence emitted from a singlet state generated by photo-excitation and delayed fluorescence emitted from a singlet state generated by reverse energy transfer via a triplet state can be estimated. In a delayed fluorescent material, a ratio of the intensity of the slowly decaying delayed fluorescence to the intensity of the promptly decaying fluorescence is relatively large.

Specifically, Prompt emission and Delay emission are present as emission from the delayed fluorescent material. Prompt emission is observed promptly when the excited state is achieved by exciting the compound of the exemplary embodiment with a pulse beam (i.e., a beam emitted from a pulse laser) having a wavelength absorbable by the delayed fluorescent material. Delay emission is observed not promptly when the excited state is achieved but after the excited state is achieved.

An amount of Prompt emission, an amount of Delay emission and a ratio between the amounts thereof can be obtained according to the method as described in "Nature 492, 234-238, 2012" (Reference Document 1). The amount of Prompt emission and the amount of Delay emission may be calculated using an apparatus different from one described in Reference Document 1 or the apparatus illustrated in Fig. 2.

Herein, a sample produced by the following method is used for measuring delayed fluorescence of the compound M2. For instance, the compound M2 is dissolved in toluene to prepare a dilute solution with an absorbance of 0.05 or less at the excitation wavelength to eliminate the contribution of self-absorption. In order to prevent quenching due to oxygen, the sample solution is frozen and degassed and then sealed in a cell with a lid under an argon atmosphere to obtain an oxygen-free sample solution saturated with argon.

The fluorescence spectrum of the sample solution is measured with a spectrofluorometer FP-8600 (produced by JASCO Corporation), and the fluorescence spectrum of a 9,10-diphenylanthracene ethanol solution is measured under the same conditions. Using the fluorescence area intensities of both spectra, the total fluorescence quantum yield is calculated by an equation (1) in Morris et al. J. Phys. Chem. 80 (1976) 969.

An amount of Prompt emission, an amount of Delay emission and a ratio between the amounts thereof can be obtained according to the method as described in "Nature 492, 234-238, 2012" (Reference Document 1). The amount of Prompt emission and the amount of Delay emission may be calculated using an apparatus different from one described in Reference Document 1 or the apparatus illustrated in Fig. 2.

In the exemplary embodiment, provided that an amount of Prompt emission of a measurement target compound (compound M2) is denoted by $X_P$ and an amount of Delay emission is denoted by $X_D$, a value of $X_D/X_P$ is preferably 0.05 or more.

The amounts of Prompt emission and Delay emission and a ratio of the amounts thereof in compounds other than the compound M2 herein are measured in the same manner as those of the compound M2.

Producing Method of Compound M2 in Embodiment

The compound M2 of the exemplary embodiment can be produced by a known method.

Specific examples of the compound M2 in the exemplary embodiment include compounds below. However, the invention is by no means limited to the specifically listed compounds.

[Formula 52]

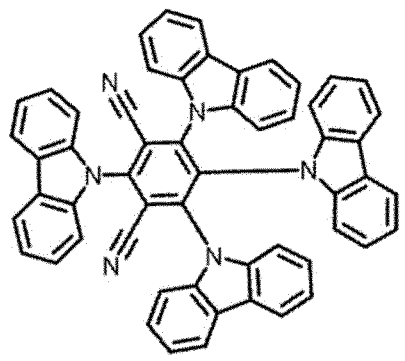

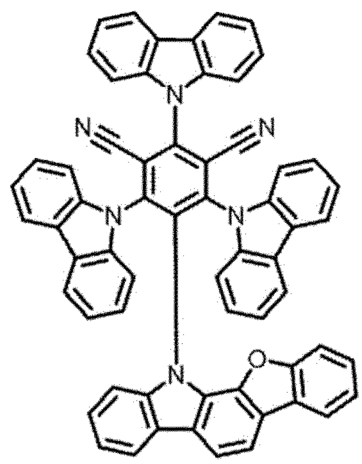

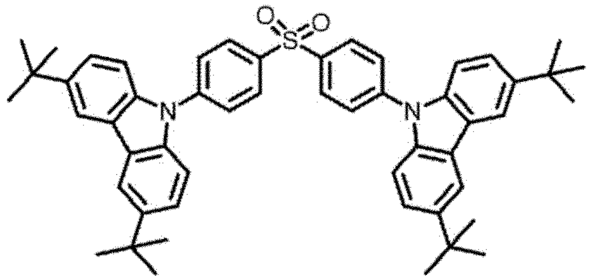

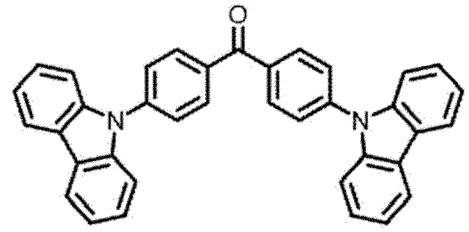

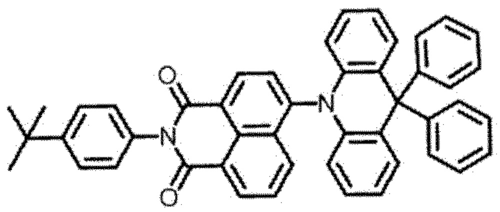

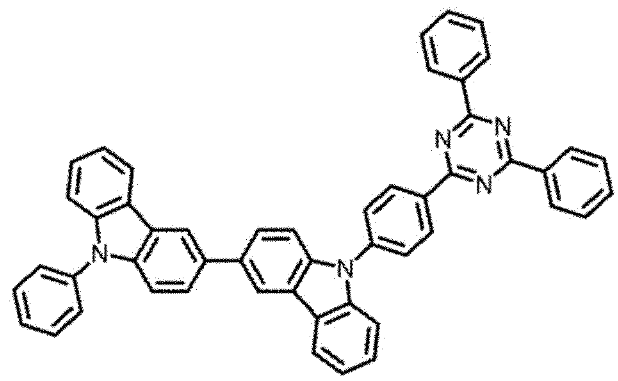

[Formula 53]

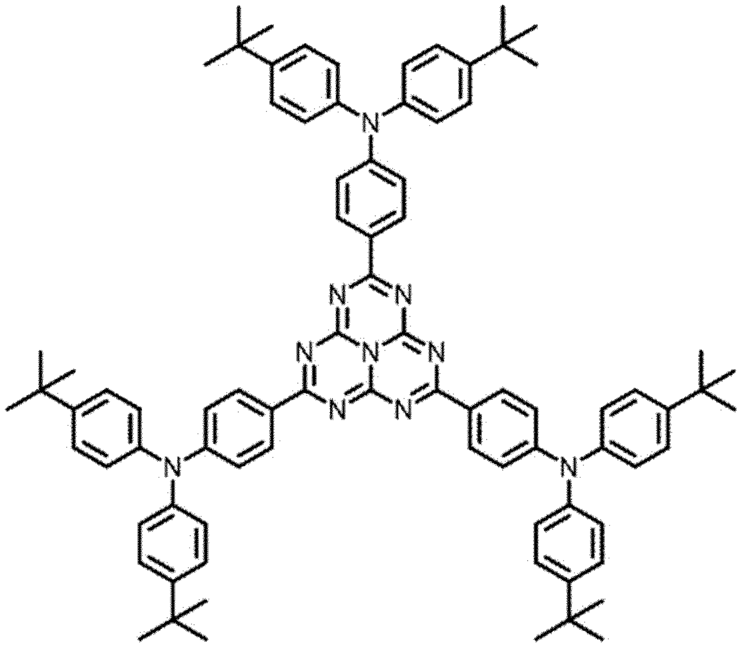

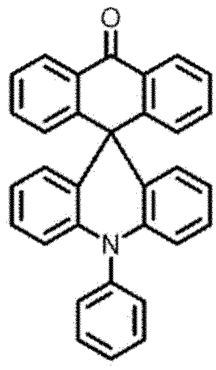

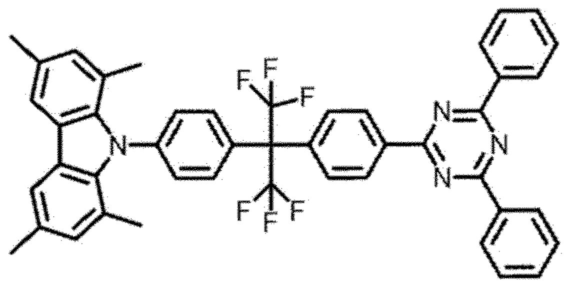

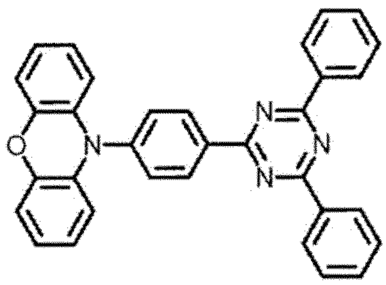

[Formula 54]

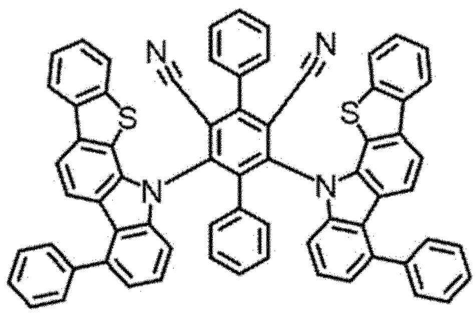

Relationship between Compound M3 and Compound M2 in Emitting Layer

In the organic EL device according to the exemplary embodiment, a singlet energy $S_1(M2)$ of the compound M2 and a singlet energy $S_1(M3)$ of the compound M3 satisfy a relationship of a numerical formula (Numerical Formula 1) below.

$$S_1(M3) > S_1(M2) \quad \text{...(Numerical Formula 1)}$$

An energy gap $T_{77K}(M3)$ at 77K of the compound M3 is preferably larger than an energy gap $T_{77K}(M2)$ at 77K of the compound M2. In other words, a relationship of a numerical formula (Numerical Formula 11) below is preferably satisfied.

$$T_{77K}(M3) > T_{77K}(M2) \quad \text{...(Numerical Formula 11)}$$

When the organic EL device according to the exemplary embodiment emits light, it is preferable that the compound M3 does not mainly emit light in the emitting layer.

Relationship between Triplet Energy and Energy Gap at 77K

Here, a relationship between a triplet energy and an energy gap at 77K will be described. In the exemplary embodiment, the energy gap at 77K is different from a typically defined triplet energy in some aspects.

The triplet energy is measured as follows. First, a solution in which a compound (measurement target) is dissolved in an appropriate solvent is encapsulated in a quartz glass tube to prepare a sample. A phosphorescence spectrum (ordinate axis: phosphorescent luminous intensity, abscissa axis: wavelength) of the sample is measured at a low temperature (77K). A tangent is drawn to the rise of the phosphorescence spectrum close to the short-wavelength region. The triplet energy is calculated by a predetermined conversion equation based on a wavelength value at an intersection of the tangent and the abscissa axis.

Herein, among the compounds of the exemplary embodiment, the thermally activated delayed fluorescent compound is preferably a compound having a small $\Delta ST$. When $\Delta ST$ is small, intersystem crossing and inverse intersystem crossing are likely to occur even at a low temperature (77K), so that the singlet state and the triplet state coexist. As a result, the spectrum to be measured in the same manner as the above includes emission from both the singlet state and the triplet state. Although it is difficult to distinguish from which state, the singlet state or the triplet state, light is emitted, the value of the triplet energy is basically considered dominant.

Accordingly, in the exemplary embodiment, the triplet energy is measured by the same method as a typical triplet energy T, but a value measured in the following manner is referred to as an energy gap T77K in order to differentiate the measured energy from the typical triplet energy in a strict meaning. The measurement target compound is dissolved in EPA (diethylether:isopentane:ethanol = 5:5:2 in volume ratio) at a concentration of 10 μmol/L, and the obtained solution is put in a quartz cell to provide a measurement sample. A phosphorescence spectrum (ordinate axis: phosphorescent luminous intensity, abscissa axis: wavelength) of the sample is measured at a low temperature (77K). A tangent is drawn to the rise of the phosphorescence spectrum close to the short-wavelength region. An energy amount is calculated by a conversion equation (F1) below based on a wavelength value $\lambda_{edge}$ [nm] at an intersection of the tangent and the abscissa axis and is defined as an energy gap $T_{77K}$ at 77K.

$$\text{Conversion Equation (F1): } T_{77K}\,[\text{eV}] = 1239.85/\lambda_{edge}$$

The tangent to the rise of the phosphorescence spectrum close to the short-wavelength region is drawn as follows. While moving on a curve of the phosphorescence spectrum from the short-wavelength region to the local maximum value closest to the short-wavelength region among the local maximum values of the phosphorescence spectrum, a tangent is checked at each point on the curve toward the long-wavelength of the phosphorescence spectrum. An inclination of the tangent is increased along the rise of the curve (i.e., a value of the ordinate axis is increased). A tangent drawn at a point of the local maximum inclination (i.e., a tangent at an inflection point) is defined as the tangent to the rise of the phosphorescence spectrum close to the short-wavelength region.

A local maximum point where a peak intensity is 15% or less of the maximum peak intensity of the spectrum is not counted as the above-mentioned local maximum peak intensity closest to the short-wavelength region. The tangent drawn at a point that is closest to the local maximum peak intensity closest to the short-wavelength region and where the inclination of the curve is the local maximum is defined as a tangent to the rise of the phosphorescence spectrum close to the short-wavelength region.

For phosphorescence measurement, a spectrophotofluorometer body F-4500 (manufactured by Hitachi High-Technologies Corporation) is usable. Any apparatus for phosphorescence measurement is usable. A combination of a cooling unit, a low temperature container, an excitation light source and a light-receiving unit may be used for phosphorescence measurement.

Singlet Energy $S_1$

A method of measuring a singlet energy $S_1$ with use of a solution (occasionally referred to as a solution method) is exemplified by a method below.

A toluene solution of a measurement target compound at a concentration of 10 μmol/L is prepared and put in a quartz cell. An absorption spectrum (ordinate axis: absorption intensity, abscissa axis: wavelength) of the thus-obtained

sample is measured at a normal temperature (300K). A tangent is drawn to the fall of the absorption spectrum close to the long-wavelength region, and a wavelength value $\lambda_{edge}$ (nm) at an intersection of the tangent and the abscissa axis is assigned to a conversion equation (F2) below to calculate singlet energy.

$$\text{Conversion Equation (F2): } S_1 \text{ [eV]} = 1239.85/\lambda_{edge}$$

Any apparatus for measuring absorption spectrum is usable. For instance, a spectrophotometer (U3310 manufactured by Hitachi, Ltd.) is usable.

The tangent to the fall of the absorption spectrum close to the long-wavelength region is drawn as follows. While moving on a curve of the absorption spectrum from the local maximum value closest to the long-wavelength region, among the local maximum values of the absorption spectrum, in a long-wavelength direction, a tangent at each point on the curve is checked. An inclination of the tangent is decreased and increased in a repeated manner as the curve falls (i.e., a value of the ordinate axis is decreased). A tangent drawn at a point where the inclination of the curve is the local minimum closest to the long-wavelength region (except when absorbance is 0.1 or less) is defined as the tangent to the fall of the absorption spectrum close to the long-wavelength region.

The local maximum absorbance of 0.2 or less is not counted as the above-mentioned local maximum absorbance closest to the long-wavelength region.

In the exemplary embodiment, a difference ($S_1$ - $T_{77K}$) between the singlet energy $S_1$ and the energy gap $T_{77K}$ at 77K is defined as ΔST.

In the exemplary embodiment, a difference ΔST(M2) between the singlet energy $S_1$(M2) of the compound M2 and the energy gap $T_{77K}$(M2) at 77K of the compound M2 is preferably less than 0.3 eV, more preferably less than 0.2 eV, still more preferably less than 0.1 eV, and still further more preferably less than 0.01 eV. In other words, ΔST(M2) preferably satisfies a relationship of one of numerical formulae (Numerical Formula 1A) to (Numerical Formula 1D) below.

$$\Delta ST(M2) = S_1(M2) - T_{77K}(M2) < 0.3 \text{ eV} \quad \text{...(Numerical Formula 1A)}$$

$$\Delta ST(M2) = S_1(M2) - T_{77K}(M2) < 0.2 \text{ eV} \quad \text{...(Numerical Formula 1B)}$$

$$\Delta ST(M2) = S_1(M2) - T_{77K}(M2) < 0.1 \text{ eV} \quad \text{...(Numerical Formula 1C)}$$

$$\Delta ST(M2) = S_1(M2) - T_{77K}(M2) < 0.01 \text{ eV} \quad \text{...(Numerical Formula 1D)}$$

Film Thickness of Emitting Layer

The film thickness of the emitting layer of the organic EL device in the exemplary embodiment is preferably in a range from 5 nm to 50 nm, more preferably in a range from 7 nm to 50 nm, most preferably in a range from 10 nm to 50 nm. When the film thickness of the emitting layer is 5 nm or more, the formation of the emitting layer and the adjustment of the chromaticity are easy. When the film thickness of the emitting layer is 50 nm or less, an increase in the drive voltage is likely to be reduced.

Content Ratios of Compounds in Emitting Layer

For instance, content ratios of the compound M2 and the compound M3 in the emitting layer preferably fall within ranges shown below.

The content ratio of the compound M2 is preferably in a range from 10 mass% to 80 mass%, more preferably in a range from 10 mass% to 60 mass%, and still more preferably in a range from 20 mass% to 60 mass%.

The content ratio of the compound M3 is preferably in a range from 20 mass% to 90 mass%, more preferably in a range from 40 mass% to 90 mass%, and still more preferably in a range from 40 mass% to 80 mass%.

It should be noted that the emitting layer of the exemplary embodiment may contain a material other than the compound M2 and the compound M3.

The emitting layer may contain a single type of the compound M2 or may contain two or more types of the compound M2. The emitting layer may contain a single type of the compound M3 or may contain two or more types of the compound M3.

Fig. 4 shows an example of a relationship between energy levels of the compound M3 and the compound M2 in the emitting layer. In Fig. 4, S0 represents a ground state. S1 (M2) represents the lowest singlet state of the compound M2. T1 (M2) represents the lowest triplet state of the compound M2. S1(M3) represents the lowest singlet state of the compound M3. T1 (M3) represents the lowest triplet state of the compound M3. As shown in Fig. 4, when a compound having a small ΔST(M2) is used as the compound M2, inverse intersystem crossing from the lowest triplet state T1 to the lowest singlet state S1 can be caused by heat energy.

The inverse intersystem crossing caused in the compound M2 enables light emission from the lowest singlet state S1 (M2) of the compound M2 to be observed when the emitting layer does not contain a fluorescent dopant with the lowest singlet state S1 smaller than the lowest singlet state S1(M2) of the compound M2. It is inferred that the internal quantum efficiency can be theoretically raised up to 100% also by using delayed fluorescence by the TADF mechanism.

In the organic EL device according to the exemplary embodiment, a high-performance organic EL device is achieved by containing the delayed fluorescent compound M2 and the compound M3 (compound M3 represented by the formula (1)) having a larger singlet energy than the compound M2 in the emitting layer.

The organic EL device according to the exemplary embodiment is usable in an electronic device such as a display device and a light-emitting unit.

An arrangement of the organic EL device will be further described below.

Substrate

The substrate is used as a support for the organic EL device. For instance, glass, quartz, plastics and the like are usable for the substrate. A flexible substrate is also usable. The flexible substrate is a bendable substrate, which is exemplified by a plastic substrate. Examples of the material for the plastic substrate include polycarbonate, polyarylate, polyethersulfone, polypropylene, polyester, polyvinyl fluoride, polyvinyl chloride, polyimide, and polyethylene naphthalate. Moreover, an inorganic vapor deposition film is also usable.

Anode

Metal, an alloy, an electrically conductive compound, a mixture thereof, or the like having a large work function (specifically, 4.0 eV or more) is preferably used as the anode formed on the substrate. Specific examples of the material include ITO (Indium Tin Oxide), indium oxide-tin oxide containing silicon or silicon oxide, indium oxide-zinc oxide, indium oxide containing tungsten oxide and zinc oxide, and graphene. In addition, gold (Au), platinum (Pt), nickel (Ni), tungsten (W), chrome (Cr), molybdenum (Mo), iron (Fe), cobalt (Co), copper (Cu), palladium (Pd), titanium (Ti), and nitrides of a metal material (e.g., titanium nitride) are usable.

The material is typically formed into a film by a sputtering method. For instance, the indium oxide-zinc oxide can be formed into a film by the sputtering method using a target in which zinc oxide in a range from 1 mass% to 10 mass% is added to indium oxide. Moreover, for instance, the indium oxide containing tungsten oxide and zinc oxide can be formed by the sputtering method using a target in which tungsten oxide in a range from 0.5 mass% to 5 mass% and zinc oxide in a range from 0.1 mass% to 1 mass% are added to indium oxide. In addition, the anode may be formed by a vacuum deposition method, a coating method, an inkjet method, a spin coating method or the like.

Among the organic layers formed on the anode, since the hole injecting layer adjacent to the anode is formed of a composite material into which holes are easily injectable irrespective of the work function of the anode, a material usable as an electrode material (e.g., metal, an alloy, an electroconductive compound, a mixture thereof, and the elements belonging to the group 1 or 2 of the periodic table) is also usable for the anode.

The elements belonging to the group 1 or 2 of the periodic table, which are a material having a small work function, specifically, an alkali metal such as lithium (Li) and cesium (Cs), an alkaline earth metal such as magnesium (Mg), calcium (Ca) and strontium (Sr), an alloy containing the alkali metal and the alkaline earth metal (e.g., MgAg, AlLi), a rare earth metal such as europium (Eu) and ytterbium (Yb), and an alloy containing the rare earth metal are usable for the anode. It should be noted that the vacuum deposition method and the sputtering method are usable for forming the anode using the alkali metal, alkaline earth metal and the alloy thereof. Further, when a silver paste is used for the anode, the coating method and the inkjet method are usable.

Cathode

It is preferable to use metal, an alloy, an electroconductive compound, a mixture thereof, or the like having a small work function (specifically, 3.8 eV or less) for the cathode. Examples of materials for the cathode include elements belonging to the group 1 or 2 of the periodic table, specifically, an alkali metal such as lithium (Li) and cesium (Cs), an alkaline earth metal such as magnesium (Mg), calcium (Ca) and strontium (Sr), an alloy containing the alkali metal and the alkaline earth metal (e.g., MgAg, AlLi), a rare earth metal such as europium (Eu) and ytterbium (Yb), and an alloy

containing the rare earth metal.

It should be noted that the vacuum deposition method and the sputtering method are usable for forming the cathode using the alkali metal, alkaline earth metal and the alloy thereof. Further, when a silver paste is used for the cathode, the coating method and the inkjet method are usable.

By providing the electron injecting layer, various conductive materials such as Al, Ag, ITO, graphene, and indium oxide-tin oxide containing silicon or silicon oxide may be used for forming the cathode regardless of the work function. The conductive materials can be formed into a film using the sputtering method, inkjet method, spin coating method and the like.

Hole Injecting Layer

The hole injecting layer is a layer containing a substance exhibiting a high hole injectability. Examples of the substance exhibiting a high hole injectability include molybdenum oxide, titanium oxide, vanadium oxide, rhenium oxide, ruthenium oxide, chrome oxide, zirconium oxide, hafnium oxide, tantalum oxide, silver oxide, tungsten oxide, and manganese oxide.

In addition, the examples of the highly hole-injectable substance include: an aromatic amine compound, which is a low-molecule organic compound, such that 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl(abbreviation: DPAB), 4,4'-bis(N-{4-[N'-(3-methylphenyl)-N'-phenylamino]phenyl} -N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1); and dipyrazino[2,3-f:20,30-h]quinoxaline-2,3,6,7,10,11-hexacarbonitrile (HAT-CN).

In addition, a high polymer compound (e.g., oligomer, dendrimer and polymer) is usable as the substance exhibiting a high hole injectability. Examples of the high-molecule compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). Moreover, an acid-added high polymer compound such as poly(3,4-ethylenedioxythiophene)/poly(styrene sulfonic acid) (PEDOT/PSS) and polyaniline/poly(styrene sulfonic acid) (PAni/PSS) is also usable.

Hole Transporting Layer

The hole transporting layer is a layer containing a highly hole-transporting substance. An aromatic amine compound, carbazole derivative, anthracene derivative and the like are usable for the hole transporting layer. Specific examples of a material for the hole transporting layer include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The above-described substances mostly have a hole mobility of $10^{-6}$ $cm^2/(V•s)$ or more.

For the hole transporting layer, a carbazole derivative such as CBP, 9-[4-(N-carbazolyl)]phenyl-10-phenylanthracene (CzPA), and 9-phenyl-3-[4-(10-phenyl-9-anthryl)phenyl]-9H-carbazole (PCzPA) and an anthracene derivative such as t-BuDNA, DNA, and DPAnth may be used. A high polymer compound such as poly(N-vinylcarbazole) (abbreviation: PVK) and poly(4-vinyltriphenylamine) (abbreviation: PVTPA) is also usable.

However, in addition to the above substances, any substance exhibiting a higher hole transportability than an electron transportability may be used. It should be noted that the layer containing the substance exhibiting a high hole transportability may be not only a single layer but also a laminate of two or more layers formed of the above substance(s).

When the hole transporting layer includes two or more layers, one of the layers with a larger energy gap is preferably provided closer to the emitting layer. Such a material is exemplified by HT-2 used in Examples described later.

Electron Transporting Layer

The electron transporting layer is a layer containing a highly electron-transporting substance. For the electron transporting layer, 1) a metal complex such as an aluminum complex, beryllium complex, and zinc complex, 2) a hetero aromatic compound such as an imidazole derivative, benzimidazole derivative, azine derivative, carbazole derivative, and phenanthroline derivative, and 3) a high polymer compound are usable. Specifically, as a low-molecule organic

compound, a metal complex such as Alq, tris(4-methyl-8-quinolinato)aluminum (abbreviation: $Almq_3$), bis(10-hydroxybenzo[h]quinolinato)beryllium (abbreviation: $BeBq_2$), BAlq, Znq, ZnPBO and ZnBTZ is usable. In addition to the metal complex, a heteroaromatic compound such as 2-(4-biphenylyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) is usable. In the exemplary embodiment, a benzimidazole compound is preferably usable. The above-described substances mostly have an electron mobility of $10^{-6}$ $cm^2/(V•s)$ or more. It should be noted that any substance other than the above substance may be used for the electron transporting layer as long as the substance exhibits a higher electron transportability than the hole transportability. The electron transporting layer may be provided in the form of a single layer or a laminate of two or more layers of the above substance(s).

Further, a high polymer compound is usable for the electron transporting layer. For instance, poly[(9,9-dihexylfluorene-2,7-diyl)-co-(pyridine-3,5-diyl)] (abbreviation: PF-Py), poly[(9,9-dioctylfluorene-2,7-diyl)-co-(2,2'-bipyridine-6,6'-diyl)] (abbreviation: PF-BPy) and the like are usable.

Electron Injecting Layer

The electron injecting layer is a layer containing a highly electron-injectable substance. Examples of a material for the electron injecting layer include an alkali metal, alkaline earth metal and a compound thereof, examples of which include lithium (Li), cesium (Cs), calcium (Ca), lithium fluoride (LiF), cesium fluoride (CsF), calcium fluoride ($CaF_2$), and lithium oxide (LiOx). In addition, the alkali metal, alkaline earth metal or the compound thereof may be added to the substance exhibiting the electron transportability in use. Specifically, for instance, magnesium (Mg) added to Alq may be used. In this case, the electrons can be more efficiently injected from the cathode.

Alternatively, the electron injecting layer may be provided by a composite material in a form of a mixture of the organic compound and the electron donor. Such a composite material exhibits excellent electron injectability and electron transportability since electrons are generated in the organic compound by the electron donor. In this case, the organic compound is preferably a material excellent in transporting the generated electrons. Specifically, the above examples (e.g., the metal complex and the hetero aromatic compound) of the substance forming the electron transporting layer are usable. As the electron donor, any substance exhibiting electron donating property to the organic compound is usable. Specifically, the electron donor is preferably alkali metal, alkaline earth metal and rare earth metal such as lithium, cesium, magnesium, calcium, erbium and ytterbium. The electron donor is also preferably alkali metal oxide and alkaline earth metal oxide such as lithium oxide, calcium oxide, and barium oxide. Moreover, a Lewis base such as magnesium oxide is usable. Further, the organic compound such as tetrathiafulvalene (abbreviation: TTF) is usable.

Layer Formation Method

A method for forming each layer of the organic EL device in the exemplary embodiment is subject to no limitation except for the above particular description. However, known methods of dry film-forming such as vacuum deposition, sputtering, plasma or ion plating and wet film-forming such as spin coating, dipping, flow coating or ink-jet are applicable.

Film Thickness

A thickness of each of the organic layers in the organic EL device according to the exemplary embodiment is not limited except for the above particular description. In general, the thickness preferably ranges from several nanometers to 1 μm because excessively small film thickness is likely to cause defects (e.g. pin holes) and excessively large thickness leads to the necessity of applying high voltage and consequent reduction in efficiency.

Second Exemplary Embodiment

An arrangement of an organic EL device according to a second exemplary embodiment of the invention will be described below. In the description of the second exemplary embodiment, the same components as those in the first exemplary embodiment are denoted by the same reference signs and names to simplify or omit an explanation of the components. In the second exemplary embodiment, the same materials and compounds as described in the first exemplary embodiment are usable, unless otherwise specified.

The organic EL device according to the second exemplary embodiment is different from the organic EL device according to the first exemplary embodiment in that the emitting layer further includes a fluorescent compound M1. The second exemplary embodiment is the same as the first exemplary embodiment in other respects.

Specifically, in the second exemplary embodiment, the emitting layer contains the compound M3 represented by the formula (1), the delayed fluorescent compound M2, and a fluorescent compound M1.

In this arrangement, it is preferable that the compound M1 is a dopant material, the compound M2 is a host material, and the compound M3 is a host material. Occasionally, one of the compound M2 and the compound M3 is referred as a first host material and the other thereof is referred to as a second host material.

Compound M1

The emitting layer of the exemplary embodiment contains the fluorescent compound M1.

The compound M1 of the exemplary embodiment is not a phosphorescent metal complex. The compound M1 of the exemplary embodiment is preferably not a heavy-metal complex. The compound M1 of the exemplary embodiment is preferably not a metal complex.

The compound M1 of the exemplary embodiment is preferably a compound not exhibiting thermally activated delayed fluorescence.

A fluorescent material is usable as the compound M1 of the exemplary embodiment. Specific examples of the fluorescent material include a bisarylaminonaphthalene derivative, aryl-substituted naphthalene derivative, bisarylaminoanthracene derivative, aryl-substituted anthracene derivative, bisarylaminopyrene derivative, aryl-substituted pyrene derivative, bisarylamino chrysene derivative, aryl-substituted chrysene derivative, bisarylaminofluoranthene derivative, aryl-substituted fluoranthene derivative, indenoperylene derivative, acenaphthofluoranthene derivative, compound including a boron atom, pyromethene boron complex compound, compound having a pyromethene skeleton, metal complex of the compound having a pyrromethene skeleton, diketopyrrolopyrrole derivative, perylene derivative, and naphthacene derivative.

When the compound M1 is a fluorescent compound, the compound M1 preferably emits light having a main peak wavelength in a range from 400 nm to 700 nm.

Herein, the maximum peak wavelength means a peak wavelength of a fluorescence spectrum exhibiting a maximum luminous intensity among fluorescence spectra measured in a toluene solution in which a measurement target compound is dissolved at a concentration ranging from $10^{-6}$ mol/l to $10^{-5}$ mol/l. A spectrophotofluorometer (F-7000 manufactured by Hitachi High-Tech Science Corporation) is used as a measurement device.

The compound M1 preferably exhibits red or green light emission.

Herein, the red light emission refers to light emission whose maximum peak wavelength of fluorescence spectrum is in a range from 600 nm to 660 nm.

When the compound M1 is a red fluorescent compound, the maximum peak wavelength of the compound M1 is preferably in a range from 600 nm to 660 nm, more preferably in a range from 600 nm to 640 nm, and still more preferably in a range from 610 nm to 630 nm.

Herein, the green light emission refers to light emission whose maximum peak wavelength of fluorescence spectrum is in a range from 500 nm to 560 nm.

When the compound M1 is a green fluorescent compound, the maximum peak wavelength of the compound M1 is preferably in a range from 500 nm to 560 nm, more preferably in a range from 500 nm to 540 nm, and still more preferably in a range from 510 nm to 540 nm.

Herein, the blue light emission refers to light emission whose maximum peak wavelength of fluorescence spectrum is in a range from 430 nm to 480 nm.

When the compound M1 is a blue fluorescent compound, the maximum peak wavelength of the compound M1 is preferably in a range from 430 nm to 480 nm, more preferably in a range from 440 nm to 480 nm.

The maximum peak wavelength of the light emitted from the organic EL device is measured as follows.

Voltage is applied on the organic EL devices such that a current density becomes 10 mA/cm$^2$, where spectral radiance spectrum is measured by a spectroradiometer CS-2000 (manufactured by Konica Minolta, Inc.).

A peak wavelength of an emission spectrum, at which the luminous intensity of the resultant spectral radiance spectrum is at the maximum, is measured and defined as the maximum peak wavelength (unit: nm).

Compound Represented by Formula (2A)

The compound M1 of the exemplary embodiment is preferably a compound represented by a formula (2A) below. The compound M1 preferably emits light having a maximum peak wavelength in a range from 500 nm to 560 nm.

In a case where a compound represented by the formula (2A) below is used as the compound M1, a lifetime is remarkably improvable as compared with a case where a compound having a pyrromethene skeleton is used as the compound M1.

[Formula 55]

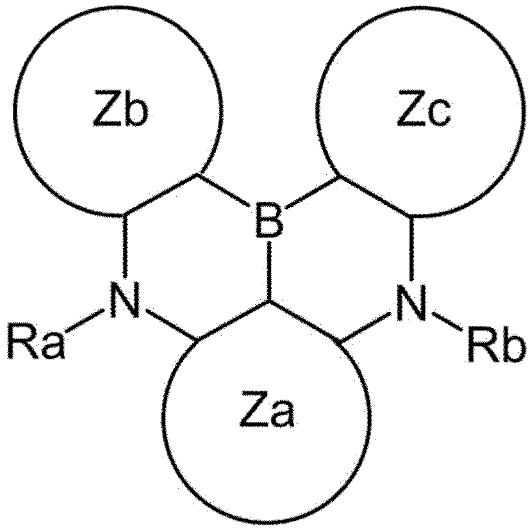

(2A)

In the formula (2A):

a Za ring, a Zb ring, and a Zc ring are each independently a substituted or unsubstituted aromatic hydrocarbon ring having 6 to 50 ring carbon atoms, or a substituted or unsubstituted heterocyclic ring having 5 to 50 ring atoms;
Ra is bonded to the Za ring or the Zb ring to form a substituted or unsubstituted heterocyclic ring, or not bonded thereto to form no substituted or unsubstituted heterocyclic ring;
Rb is bonded to the Za ring or the Zc ring to form a substituted or unsubstituted heterocyclic ring, or not bonded thereto to form no substituted or unsubstituted heterocyclic ring; and
Ra and Rb not forming the substituted or unsubstituted heterocycling ring are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

Compound Represented by Formula (D11)

The compound M1 of the exemplary embodiment is also preferably a compound represented by a formula (D11) below. A compound represented by the formula (2A) is also preferably a compound represented by a formula (D11) below.
In a case where a compound represented by the formula (D11) is used as the compound M1, external quantum efficiency is improvable as compared with in a case where a compound represented by a formula (16) described later is used as the compound M1.
In a case where a compound represented by the formula (D11) is used as the compound M1, the external quantum efficiency and a lifetime are improvable as compared with a case where a compound having a pyrromethene skeleton is used as the compound M1.

[Formula 56]

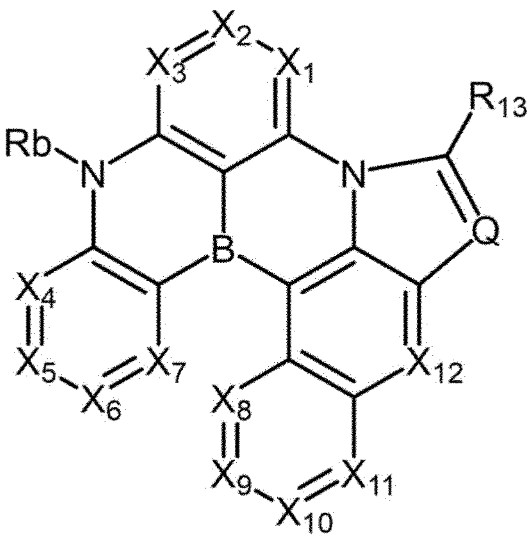

(D11)

In the formula (D11):

Rb represents the same as Rb in the formula (2A);
$X_1$ is $CR_1$ or a nitrogen atom;
$X_2$ is $CR_2$ or a nitrogen atom;
$X_3$ is $CR_3$ or a nitrogen atom;
$X_4$ is $CR_4$ or a nitrogen atom;
$X_5$ is $CR_5$ or a nitrogen atom;
$X_6$ is $CR_6$ or a nitrogen atom;
$X_7$ is $CR_7$, a nitrogen atom, or a carbon atom bonded to $X_8$ with a single bond;
$X_8$ is $CR_8$, a nitrogen atom, or a carbon atom bonded to $X_7$ with a single bond;
$X_9$ is $CR_9$ or a nitrogen atom;
$X_{10}$ is $CR_{10}$ or a nitrogen atom;
$X_{11}$ is $CR_{11}$ or a nitrogen atom;
$X_{12}$ is $CR_{12}$ or a nitrogen atom;
Q is $CR_Q$ or a nitrogen atom;
at least one combination of adjacent two or more of $R_1$ to $R_6$ and $R_9$ to $R_{11}$ are mutually bonded to form a substituted or unsubstituted monocyclic ring, mutually bonded to form a substituted or unsubstituted fused ring, or not mutually bonded;
at least one combination of adjacent two or more of $R_3$, $R_4$, and Rb are mutually bonded to form a substituted or unsubstituted monocyclic ring, mutually bonded to form a substituted or unsubstituted fused ring, or not mutually bonded;
at least one hydrogen atom in a monocyclic ring or a fused ring formed by mutually bonding at least one combination of adjacent two or more of $R_3$, $R_4$, and Rb is substituted or not substituted by at least one substituent selected from the group consisting of an alkyl group having 1 to 50 carbon atoms, an aryl group having 6 to 50 ring carbon atoms, a heterocyclic group having 5 to 50 ring atoms, a group represented by $-O-(R_{920})$, and a group represented by $-N(R_{921})(R_{922})$;
at least one hydrogen atom of the substituent is substituted or not substituted by an aryl group having 6 to 50 ring carbon atoms or an alkyl group having 1 to 50 carbon atoms;
$R_1$ to $R_{11}$ forming neither the substituted or unsubstituted monocyclic ring nor the substituted or unsubstituted fused ring, $R_{12}$ to $R_{13}$, and $R_Q$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{911})(R_{912})(R_{913})$, a group represented by $-O-(R_{914})$, a group represented by $-S-(R_{915})$, a group represented by $-N(R_{916})(R_{917})$, a substituted or unsubstituted aralkyl group having

7 to 50 carbon atoms, a group represented by - C(=O)$R_{918}$, a group represented by -COO$R_{919}$, a halogen atom, a cyano group, a nitro group, a substituted or unsubstituted aryl group having 6 to 50 ring carbon atoms, or a substituted or unsubstituted heterocyclic group having 5 to 50 ring atoms;

Rb forming neither the substituted or unsubstituted monocyclic ring nor the substituted or unsubstituted fused ring is a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

$R_{911}$ to $R_{922}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{911}$ are present, the plurality of $R_{911}$ are mutually the same or different;
when a plurality of $R_{912}$ are present, the plurality of $R_{912}$ are mutually the same or different;
when a plurality of $R_{913}$ are present, the plurality of $R_{913}$ are mutually the same or different;
when a plurality of $R_{914}$ are present, the plurality of $R_{914}$ are mutually the same or different;
when a plurality of $R_{915}$ are present, the plurality of $R_{915}$ are mutually the same or different;
when a plurality of $R_{916}$ are present, the plurality of $R_{916}$ are mutually the same or different;
when a plurality of $R_{917}$ are present, the plurality of $R_{917}$ are mutually the same or different;
when a plurality of $R_{918}$ are present, the plurality of $R_{918}$ are mutually the same or different;
when a plurality of $R_{919}$ are present, the plurality of $R_{919}$ are mutually the same or different;
when a plurality of $R_{920}$ are present, the plurality of $R_{920}$ are mutually the same or different;
when a plurality of $R_{921}$ are present, the plurality of $R_{921}$ are mutually the same or different; and
when a plurality of $R_{922}$ are present, the plurality of $R_{922}$ are mutually the same or different.

The compound represented by the formula (D11) is also preferably represented by a formula (D13) below.

[Formula 57]

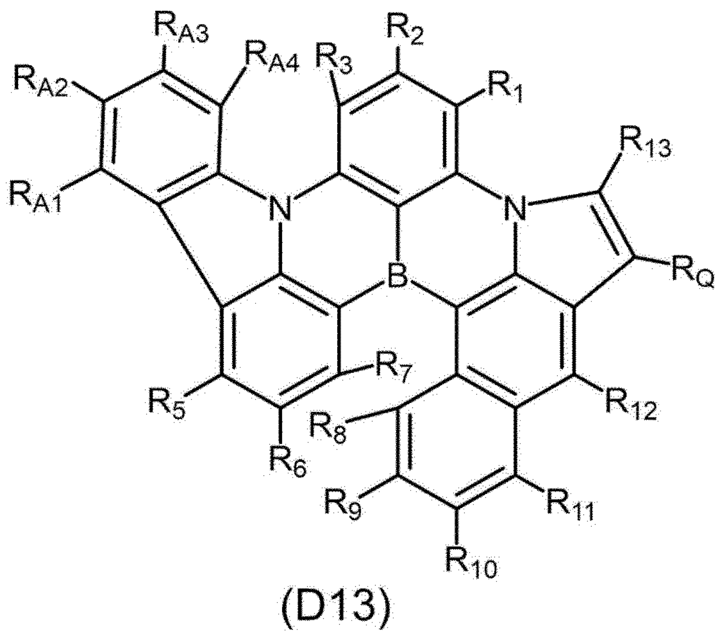

(D13)

In the formula (D13):

$R_1$ to $R_3$, $R_5$ to $R_{13}$, and $R_Q$ respectively represent the same as $R_1$ to $R_3$, $R_5$ to $R_{13}$, and $R_Q$ in the formula (D11);

at least one combination of adjacent two or more of $R_{A1}$ to $R_{A4}$ are mutually bonded to form a substituted or unsubstituted monocyclic ring, mutually bonded to form a substituted or unsubstituted fused ring, or not mutually bonded;

$R_{A1}$ to $R_{A4}$ forming neither the substituted or unsubstituted monocyclic ring nor the substituted or unsubstituted

fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aralkyl group having 7 to 50 carbon atoms, a group represented by -Si($R_{931}$)($R_{932}$)($R_{933}$), a group represented by -O-($R_{934}$), a group represented by -S-($R_{935}$), a group represented by -N($R_{936}$)($R_{937}$), a substituted or unsubstituted aralkyl group having 7 to 50 carbon atoms, a group represented by -C(=O)$R_{938}$, a group represented by -COO$R_{939}$, a halogen atom, a cyano group, a nitro group, a substituted or unsubstituted aryl group having 6 to 50 ring carbon atoms, or a substituted or unsubstituted heterocyclic group having 5 to 50 ring atoms;

$R_{931}$ to $R_{939}$ are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms;

when a plurality of $R_{931}$ are present, the plurality of $R_{931}$ are mutually the same or different;

when a plurality of $R_{932}$ are present, the plurality of $R_{932}$ are mutually the same or different;

when a plurality of $R_{933}$ are present, the plurality of $R_{933}$ are mutually the same or different;

when a plurality of $R_{934}$ are present, the plurality of $R_{934}$ are mutually the same or different;

when a plurality of $R_{935}$ are present, the plurality of $R_{935}$ are mutually the same or different;

when a plurality of $R_{936}$ are present, the plurality of $R_{936}$ are mutually the same or different;

when a plurality of $R_{937}$ are present, the plurality of $R_{937}$ are mutually the same or different;

when a plurality of $R_{938}$ are present, the plurality of $R_{938}$ are mutually the same or different; and

when a plurality of $R_{939}$ are present, the plurality of $R_{939}$ are mutually the same or different.

The compound represented by the formula (D11) is also preferably represented by a formula (D13A) below.

[Formula 58]

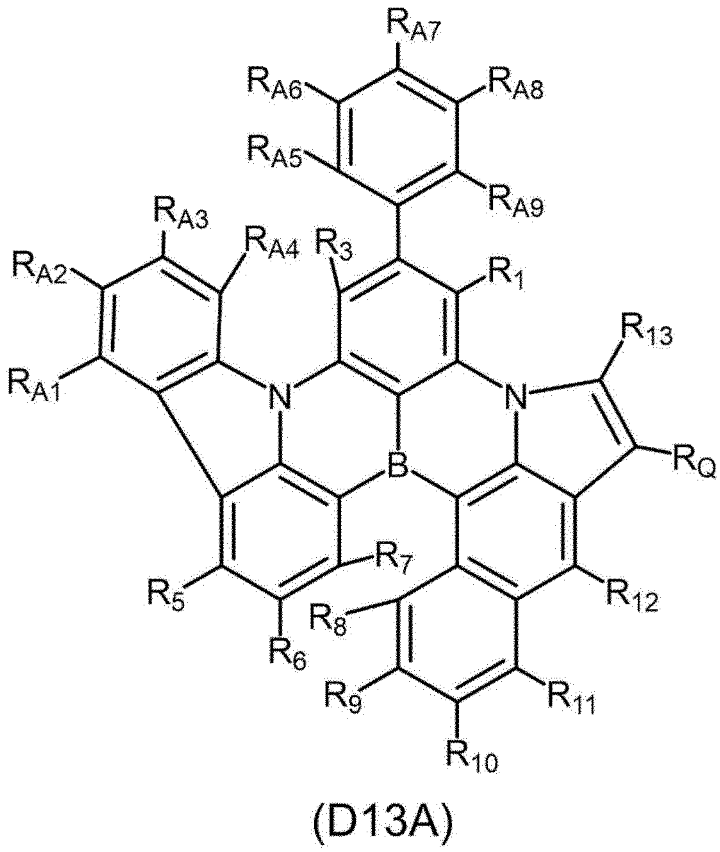

(D13A)

In the formula (D13A): $R_1$, $R_3$, $R_5$ to $R_{13}$, $R_Q$, and $R_{A1}$ to $R_{A4}$ each independently represent the same as $R_1$, $R_3$, $R_5$ to $R_{13}$, $R_Q$, and $R_{A1}$ to $R_{A4}$ in the formula (D13);

at least one combination of adjacent two or more of $R_{A5}$ to $R_{A9}$ are mutually bonded to form a substituted or

unsubstituted monocyclic ring, mutually bonded to form a substituted or unsubstituted fused ring, or not mutually bonded; and

$R_{A5}$ to $R_{A9}$ forming neither the substituted or unsubstituted monocyclic ring nor the substituted or unsubstituted fused ring each independently represent the same as $R_{A1}$ to $R_{A4}$ forming neither the substituted or unsubstituted monocyclic ring nor the substituted or unsubstituted fused ring.

In the formulae (D13) and (D13A), for instance, a combination of $R_5$ and $R_6$ are mutually bonded to form a substituted or unsubstituted monocyclic ring, mutually bonded to form a substituted or unsubstituted fused ring, or not bonded.

It is also preferable that $R_1$ to $R_{13}$ and $R_Q$ in the compound represented by the formula (D11) are each independently a hydrogen atom, a substituted or unsubstituted alkyl group having 1 to 50 carbon atoms, a substituted or unsubstituted aryl group having 6 to 50 ring carbon atoms, or a substituted or unsubstituted heteroaryl group having 5 to 50 ring atoms.

It is also preferable that $R_1$ to $R_{13}$ and $R_Q$ in the compound represented by the formula (D11) are each independently a hydrogen atom, a substituted or unsubstituted alkyl group having 1 to 25 carbon atoms, a substituted or unsubstituted aryl group having 6 to 25 ring carbon atoms, or a substituted or unsubstituted heteroaryl group having 5 to 25 ring atoms.

It is also preferable that $R_1$ to $R_3$, $R_5$ to $R_{13}$, $R_Q$, and $R_{A1}$ to $R_{A9}$ in a compound represented by each of the formulae (D13) and (D13A) are each independently a hydrogen atom, a substituted or unsubstituted alkyl group having 1 to 50 carbon atoms, a substituted or unsubstituted aryl group having 6 to 50 ring carbon atoms, or a substituted or unsubstituted heteroaryl group having 5 to 50 ring atoms.

It is also preferable that $R_1$ to $R_3$, $R_5$ to $R_{13}$, $R_Q$, and $R_{A1}$ to $R_{A9}$ in a compound represented by each of the formulae (D13) and (D13A) are each independently a hydrogen atom, a substituted or unsubstituted alkyl group having 1 to 25 carbon atoms, a substituted or unsubstituted aryl group having 6 to 25 ring carbon atoms, or a substituted or unsubstituted heteroaryl group having 5 to 25 ring atoms.

The compound represented by the formula (D11) is also preferably represented by a formula (D14) below.

[Formula 59]

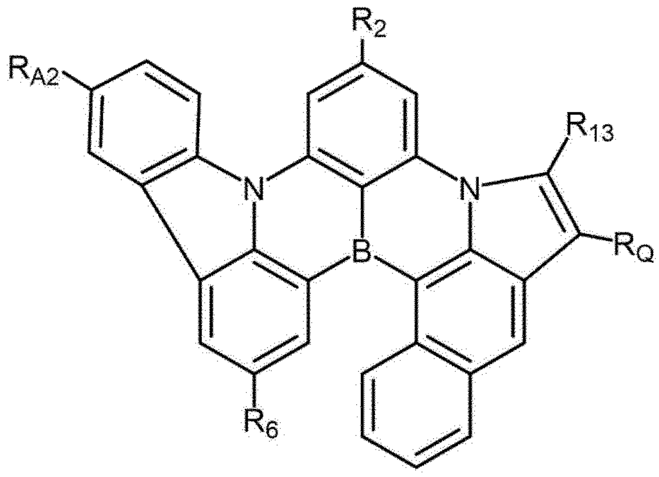

(D14)

In a compound represented by the formula (D14), $R_2$, $R_6$, $R_{13}$, $R_Q$, and $R_{A2}$ are each independently a hydrogen atom, a substituted or unsubstituted alkyl group having 1 to 10 carbon atoms, a substituted or unsubstituted aryl group having 6 to 12 ring carbon atoms, or a substituted or unsubstituted heteroaryl group having 5 to 18 ring atoms.

It is preferable that $R_{13}$ and $R_Q$ in the compound represented by the formula (D14) are each independently a substituted or unsubstituted alkyl group having 1 to 10 carbon atoms, a substituted or unsubstituted phenyl group, a substituted or unsubstituted naphthyl group, or a substituted or unsubstituted dibenzofuranyl group.

In a compound represented by the formula (D14), $R_6$ and $R_{A2}$ are preferably each independently a hydrogen atom or a substituted or unsubstituted alkyl group having 1 to 10 atoms.

Compound Represented by Formula (16)

The compound M1 of the exemplary embodiment is also preferably a compound represented by a formula (16) below. A compound represented by the formula (2A) is also preferably a compound represented by the formula (16) below.
In a case where a compound represented by the formula (16) is used as the compound M1, a lifetime is improvable as compared with in a case where a compound represented by the formula (D11) is used as the compound M1.
In a case where a compound represented by the formula (16) is used as the compound M1, a lifetime is remarkably improvable as compared with a case where a compound having a pyrromethene skeleton is used as the compound M1.

[Formula 60]

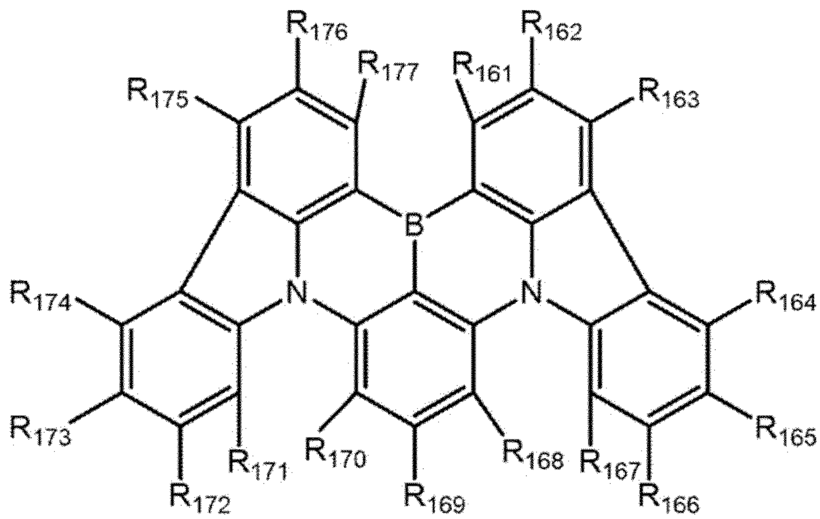

(16)

In a compound represented by the formula (16):

at least one combination of adjacent two or more of $R_{161}$ to $R_{177}$ are mutually bonded to form a substituted or unsubstituted monocyclic ring, mutually bonded to form a substituted or unsubstituted fused ring, or not mutually bonded;
$R_{161}$ to $R_{177}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aralkyl group having 7 to 50 carbon atoms, a group represented by -Si($R_{961}$)($R_{962}$)($R_{963}$), a group represented by -O-($R_{964}$), a group represented by -S-($R_{965}$), a group represented by -N($R_{966}$)($R_{967}$), a group represented by -C(=O)$R_{968}$, a group represented by -COO$R_{969}$, a halogen atom, a cyano group, a nitro group, a substituted or unsubstituted aryl group having 6 to 50 ring carbon atoms, or a substituted or unsubstituted heterocyclic group having 5 to 50 ring atoms;
$R_{961}$ to $R_{969}$ are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms;
when a plurality of $R_{961}$ are present, the plurality of $R_{961}$ are mutually the same or different;
when a plurality of $R_{962}$ are present, the plurality of $R_{962}$ are mutually the same or different;
when a plurality of $R_{963}$ are present, the plurality of $R_{963}$ are mutually the same or different;
when a plurality of $R_{964}$ are present, the plurality of $R_{964}$ are mutually the same or different;
when a plurality of $R_{965}$ are present, the plurality of $R_{965}$ are mutually the same or different;
when a plurality of $R_{966}$ are present, the plurality of $R_{966}$ are mutually the same or different;
when a plurality of $R_{967}$ are present, the plurality of $R_{967}$ are mutually the same or different;
when a plurality of $R_{968}$ are present, the plurality of $R_{968}$ are mutually the same or different; and

when a plurality of $R_{969}$ are present, the plurality of $R_{969}$ are mutually the same or different.

It is preferable that $R_{161}$ to $R_{177}$ in a compound represented by the formula (16) are each independently a hydrogen atom, a substituted or unsubstituted aryl group having 6 to 30 ring carbon atoms, a substituted or unsubstituted heterocyclic group having 5 to 30 ring atoms, or a substituted or unsubstituted alkyl group having 1 to 30.

In a compound represented by the formula (16), at least one of $R_{168}$ to $R_{170}$ is preferably a substituted or unsubstituted aryl group having 6 to 50 ring carbon atoms, or a substituted or unsubstituted heterocyclic group having 5 to 50 ring atoms.

It is preferable that $R_{161}$ to $R_{177}$ in a compound represented by the formula (16) are each independently a hydrogen atom, or a substituted or unsubstituted aryl group having 6 to 30 ring carbon atoms.

In a compound represented by the formula (16), $R_{161}$ to $R_{177}$ are also preferably each a hydrogen atom.

In a compound represented by the formula (16), it is also preferable that at least one combination of adjacent two or more of $R_{161}$ to $R_{177}$ are mutually bonded to form a ring represented by a formula (16A) below.

[Formula 61]

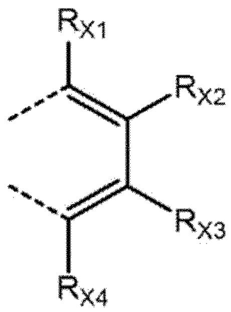

(16A)

In the formula (16A): a dotted line represents a bonding position; and

$R_{X1}$ to $R_{X4}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aralkyl group having 7 to 50 carbon atoms, a group represented by $-Si(R_{961})(R_{962})(R_{963})$, a group represented by $-O-(R_{964})$, a group represented by $-S-(R_{965})$, a group represented by $-N(R_{966})(R_{967})$, a group represented by $-C(=O)R_{968}$, a group represented by $-COOR_{969}$, a halogen atom, a cyano group, a nitro group, a substituted or unsubstituted aryl group having 6 to 50 ring carbon atoms, or a substituted or unsubstituted heterocyclic group having 5 to 50 ring atoms.

When a plurality of $R_{X1}$ are present, the plurality of $R_{X1}$ are mutually the same or different.

When a plurality of $R_{X2}$ are present, the plurality of $R_{X2}$ are mutually the same or different.

When a plurality of $R_{X3}$ are present, the plurality of $R_{X3}$ are mutually the same or different.

When a plurality of $R_{X4}$ are present, the plurality of $R_{X4}$ are mutually the same or different.

In the formula (16), a combination of $R_{161}$ and $R_{162}$, a combination of $R_{165}$ and Rise, a combination of $R_{172}$ and $R_{173}$, and a combination of $R_{176}$ and $R_{177}$ are mutually bonded to form a ring represented by the formula (16A).

In the formula (16), it is preferable that a combination of $R_{161}$ and $R_{162}$ and a combination of $R_{176}$ and $R_{177}$ do not form a ring represented by the formula (16A) concurrently.

In the formula (16), it is also preferable that a combination of $R_{165}$ and $R_{166}$ are mutually bonded to form a ring represented by the formula (16A) and a combination of $R_{172}$ and $R_{173}$ are mutually bonded to form a ring represented by the formula (16A). In this arrangement, the compound M1 is represented by a formula (161) below.

A compound represented by the formula (16) is also preferably a compound represented by a formula (161) below.

[Formula 62]

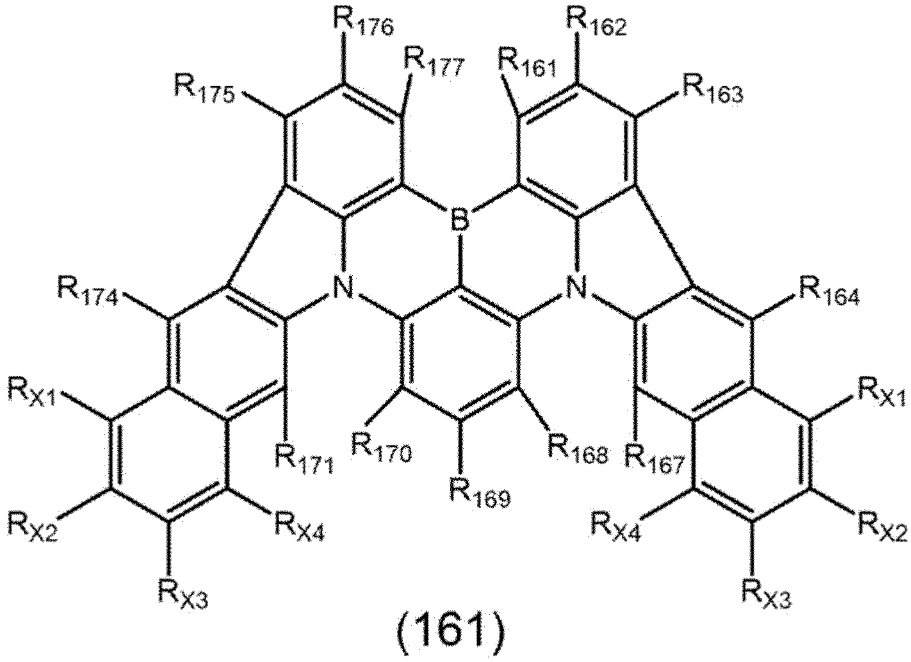

(161)

In the formula (161), $R_{161}$ to $R_{164}$, $R_{167}$ to $R_{171}$, $R_{174}$ to $R_{177}$, and $R_{X1}$ to $R_{X4}$ each independently represent the same as $R_{161}$ to $R_{164}$, $R_{167}$ to $R_{171}$, and $R_{174}$ to $R_{177}$ in the formula (16) and $R_{X1}$ to $R_{X4}$ in the formula (16A).

A compound represented by the formula (16) is also preferably a compound represented by a formula (162) below.

[Formula 63]

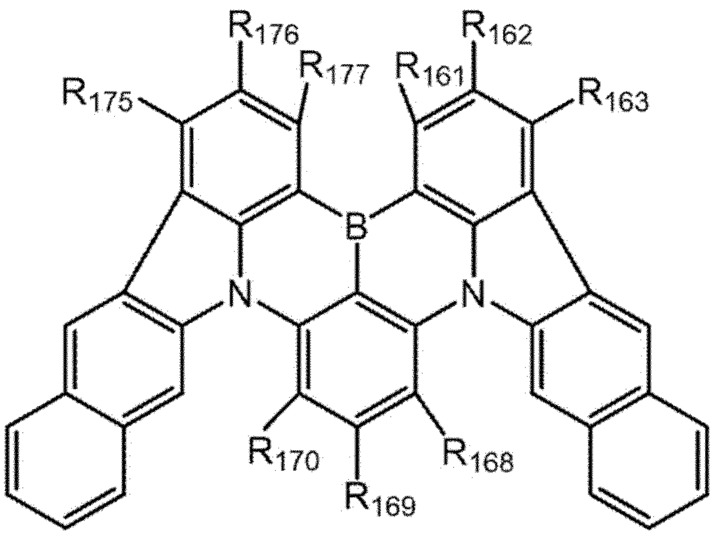

(162)

In the formula (162), $R_{161}$ to $R_{163}$, $R_{168}$ to $R_{170}$, and $R_{175}$ to $R_{177}$ each independently represent the same as $R_{161}$ to $R_{163}$, $R_{168}$ to $R_{170}$, and $R_{175}$ to $R_{177}$ in the formula (16).

A compound represented by the formula (16) is also preferably a compound represented by a formula (163) below.

[Formula 64]

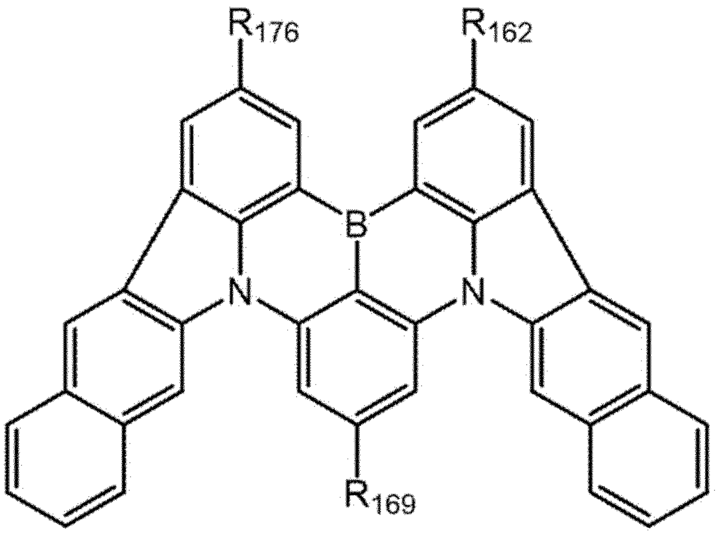

(163)

In the formula (163), $R_{162}$, $R_{169}$, and $R_{176}$ each independently represent the same as $R_{162}$, $R_{169}$, and $R_{176}$ in the formula (16).

In the compound M1, the groups specified to be "substituted or unsubstituted" are each also preferably an "unsubstituted" group.

Producing Method of Compound M1

The compound M1 can be produced by a known method.

Specific examples of the compound M1 in the exemplary embodiment are shown below. However, the invention is by no means limited to the specifically listed compounds.

A coordinate bond between a boron atom and a nitrogen atom in a pyrromethene skeleton is shown by various means such as a solid line, a broken line, an arrow, and omission. Herein, the coordinate bond is shown by a solid line or a broken line, or the description of the coordinate bond is omitted.

[Formula 65]

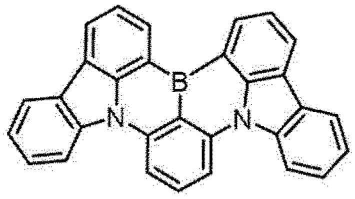

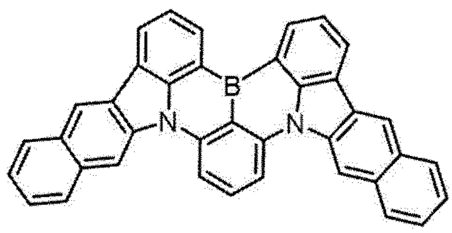

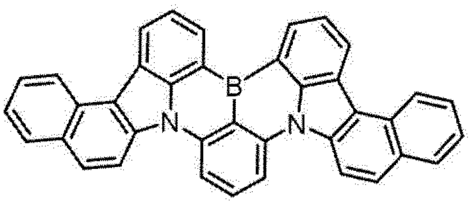

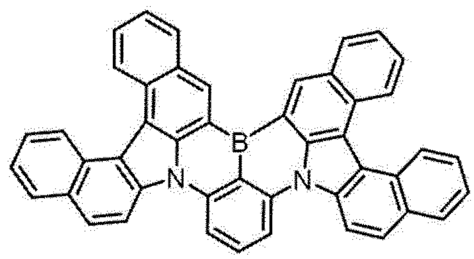

[Formula 66]

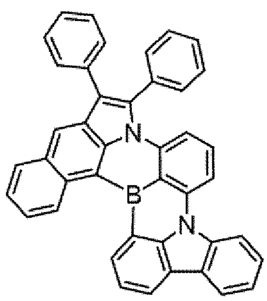
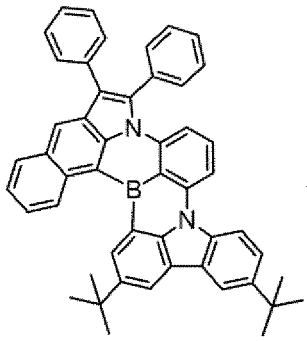
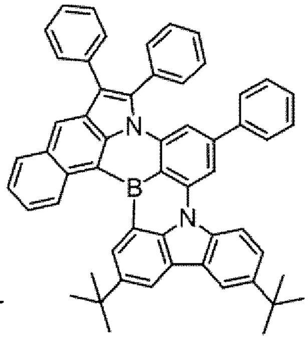
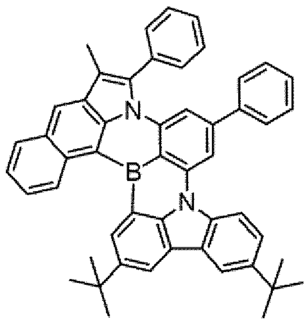
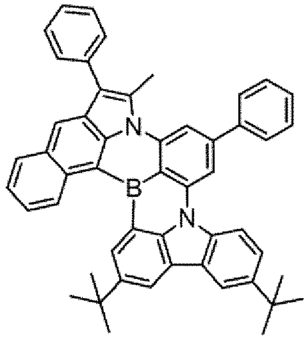
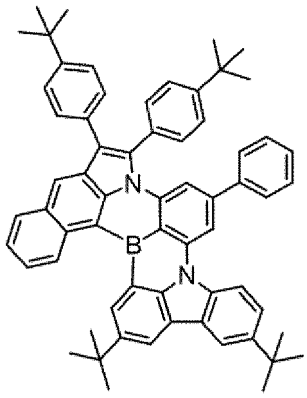
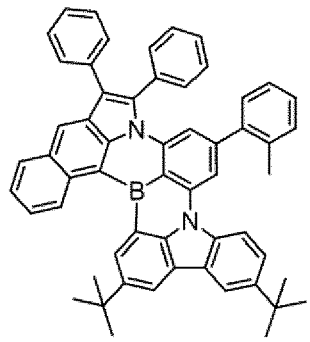
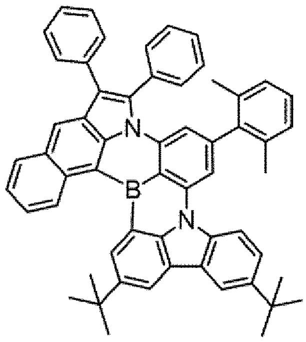
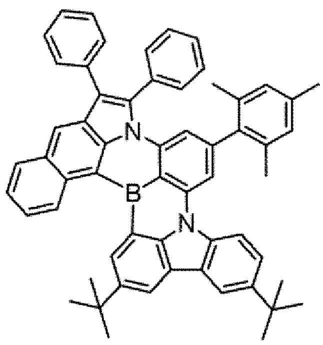
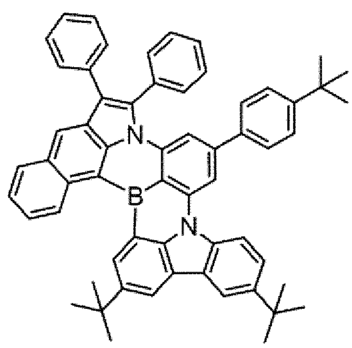

[Formula 67]

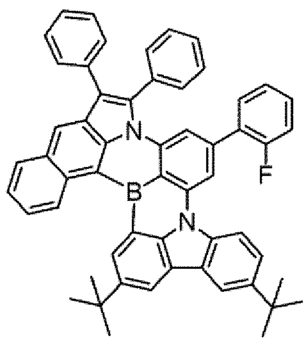
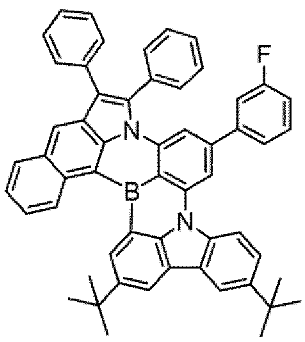
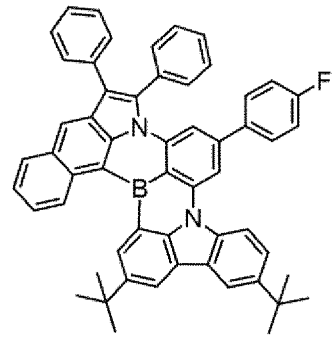

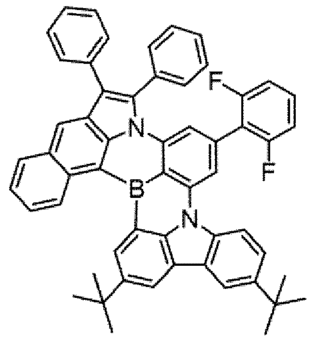
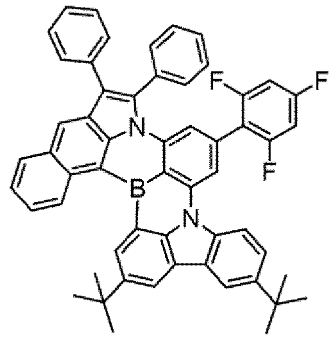

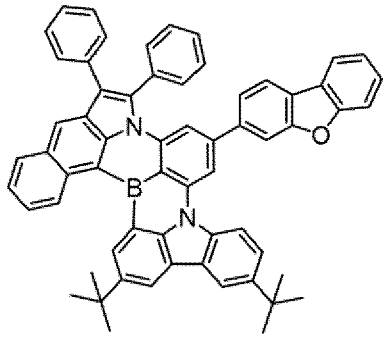
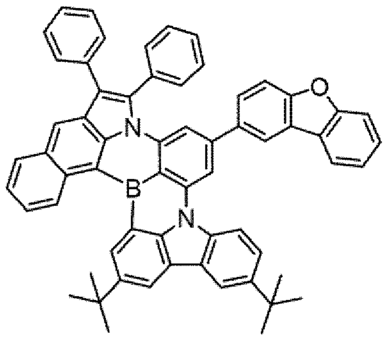

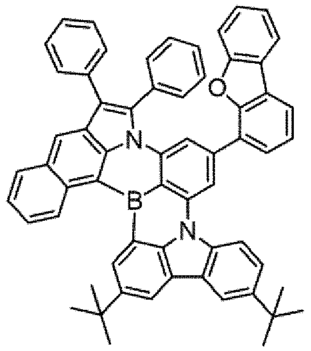
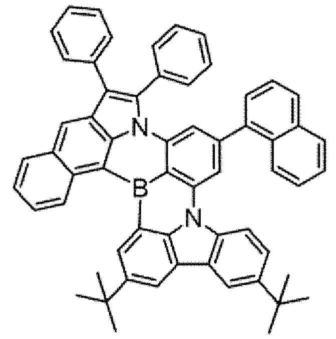
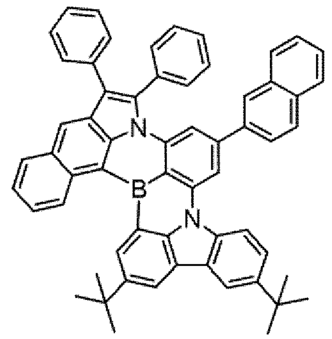

[Formula 68]

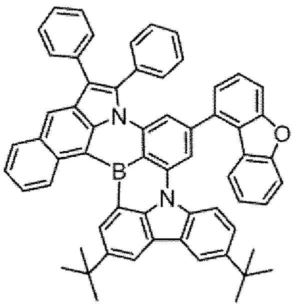

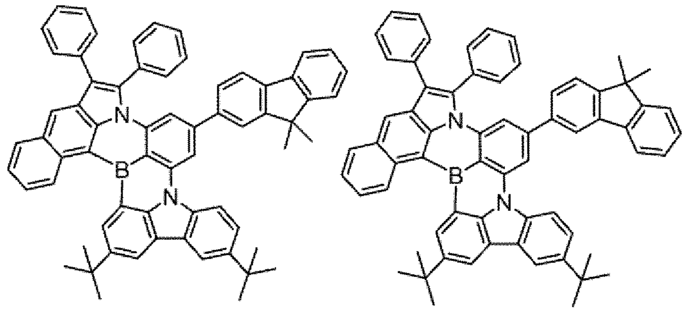

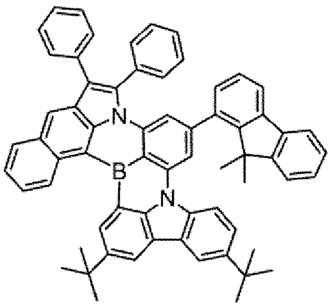

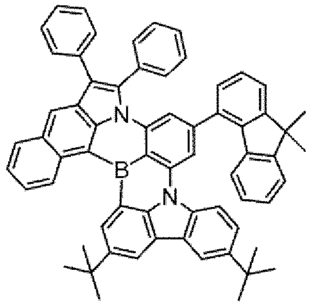

[Formula 69]

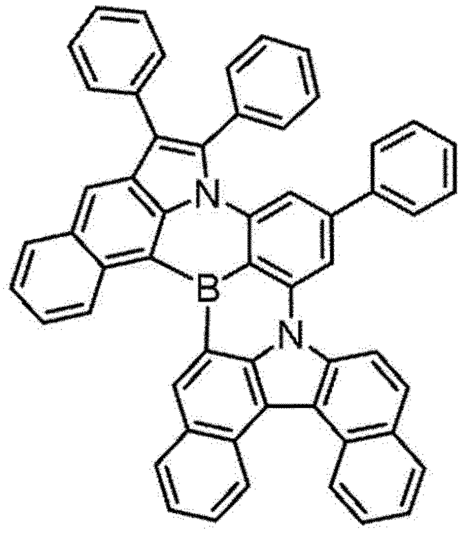

[Formula 70]

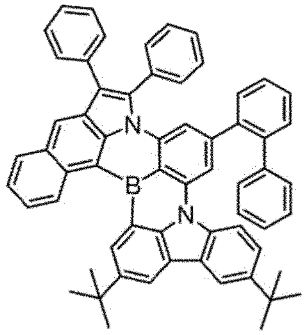
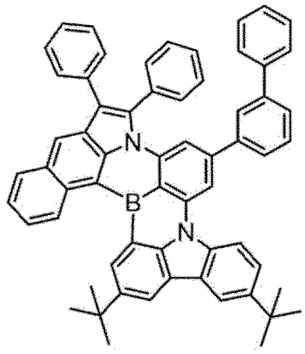
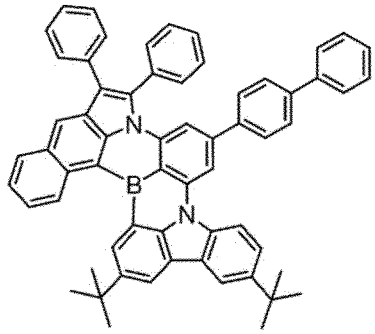

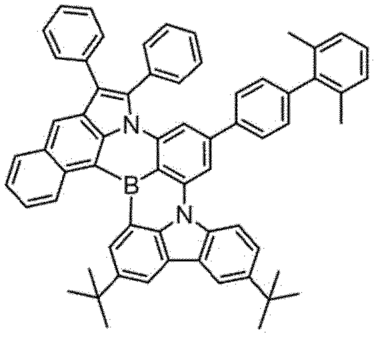
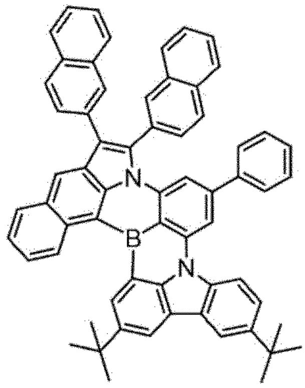

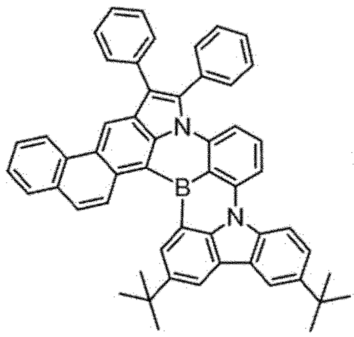
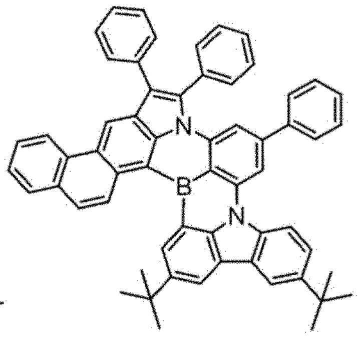
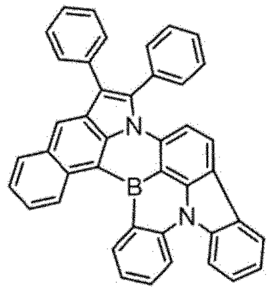

[Formula 71]

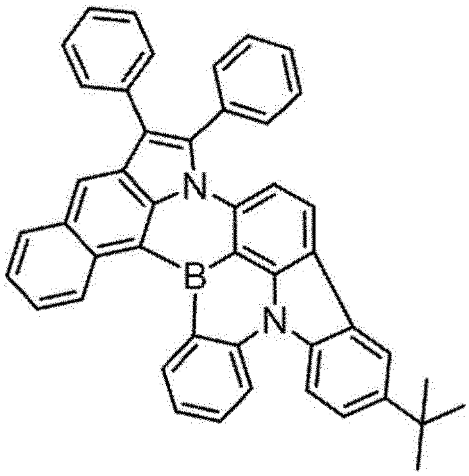

[Formula 72]

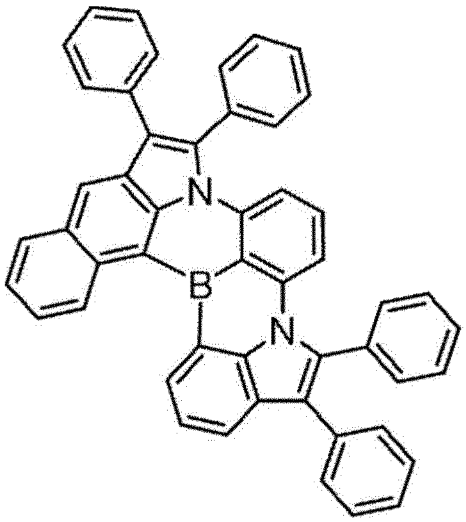

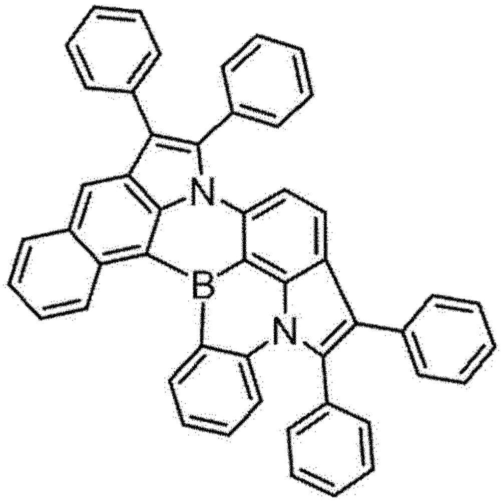

[Formula 73]

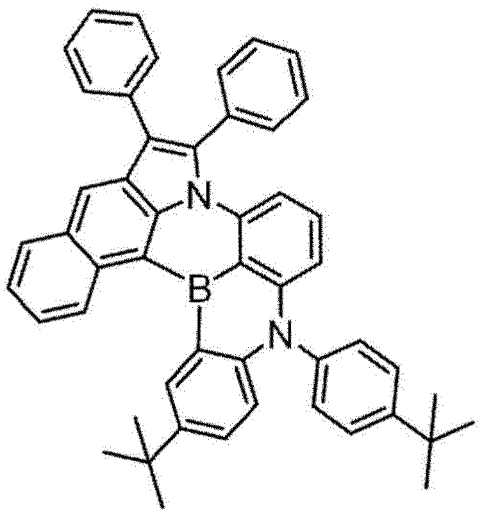

[Formula 74]

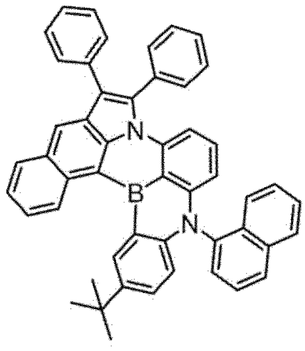

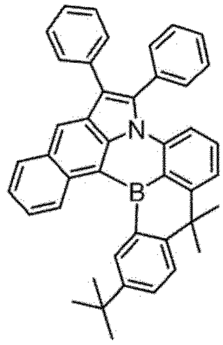

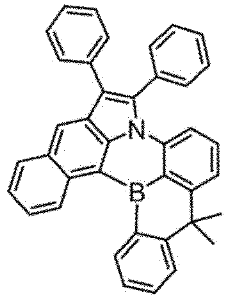

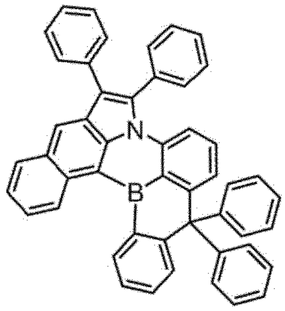

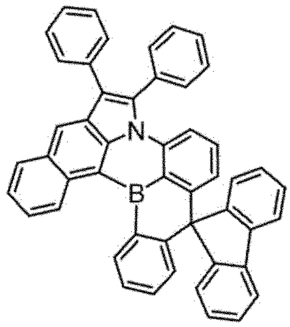

[Formula 75]

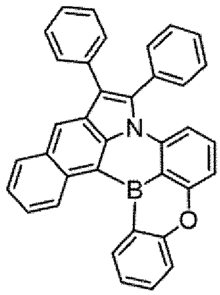

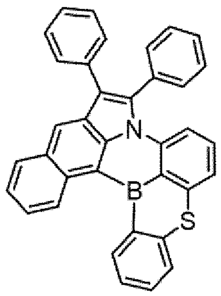

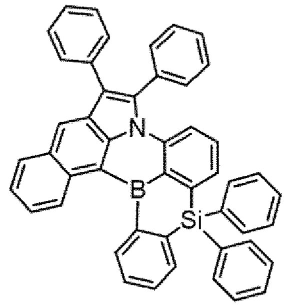

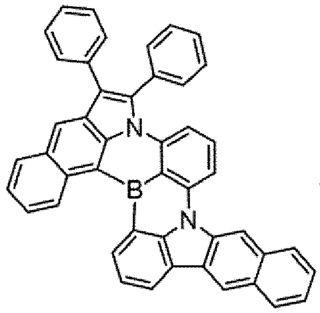

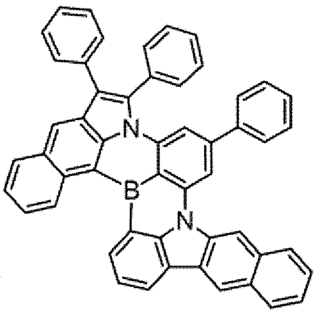

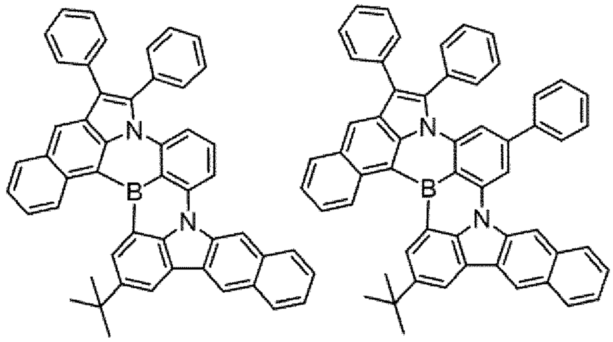

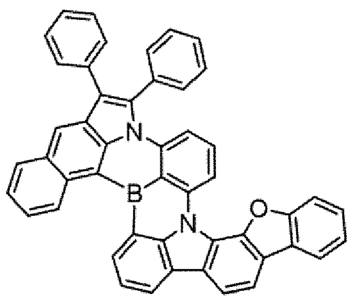

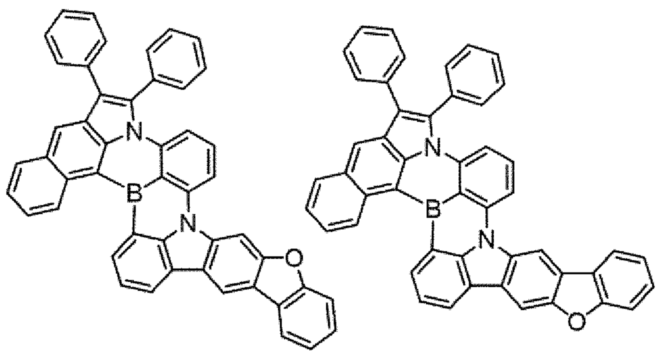

[Formula 76]

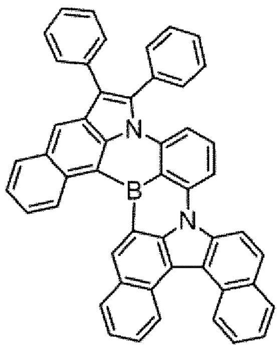
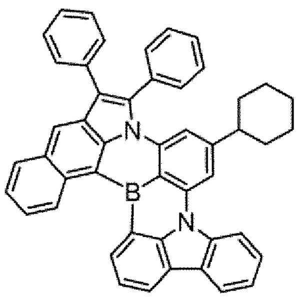
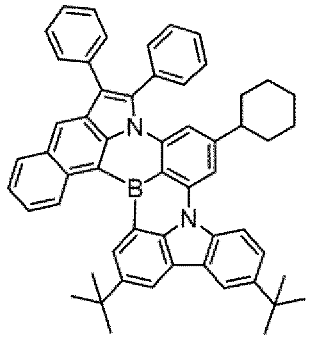
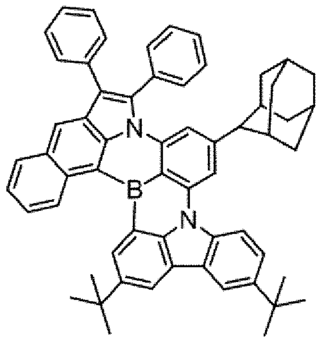
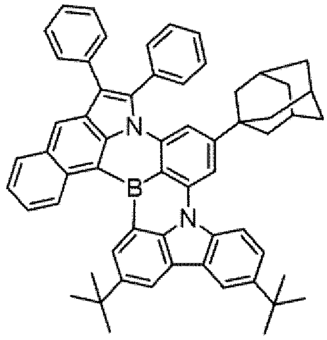
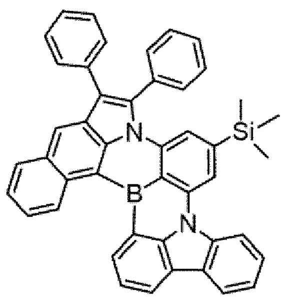
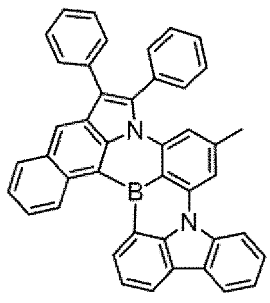
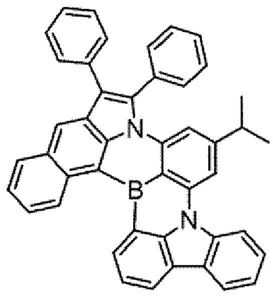
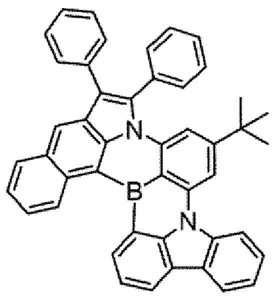
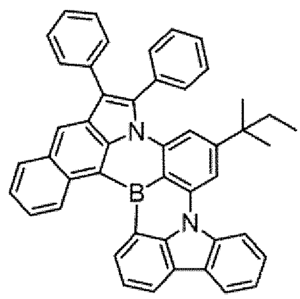

[Formula 77]

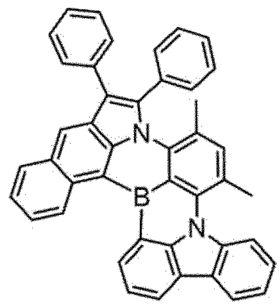

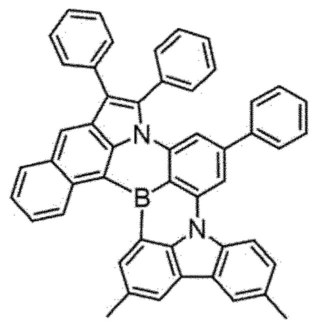

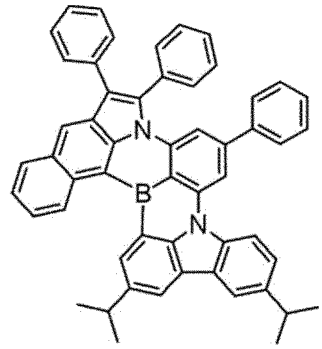

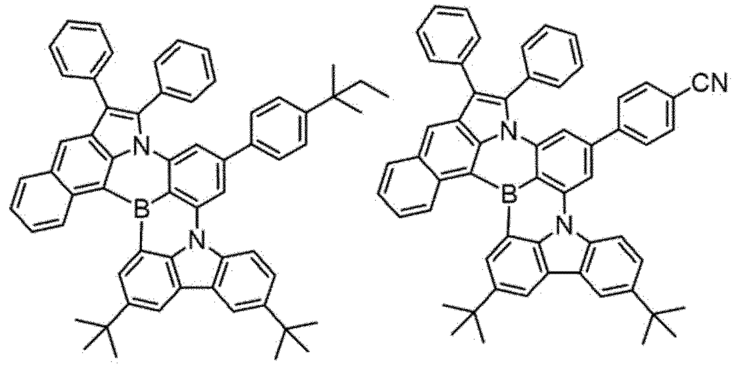

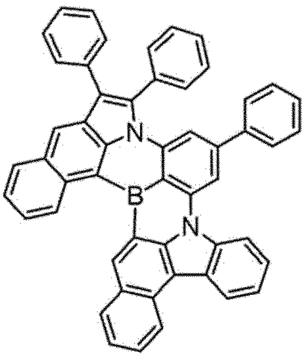

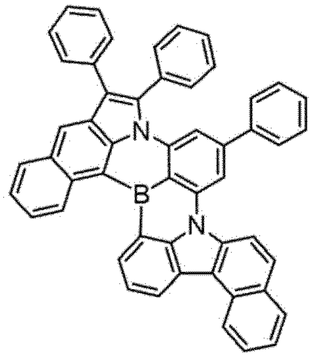

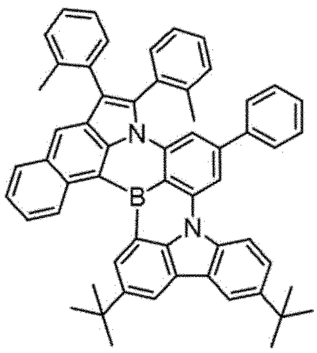

[Formula 78]

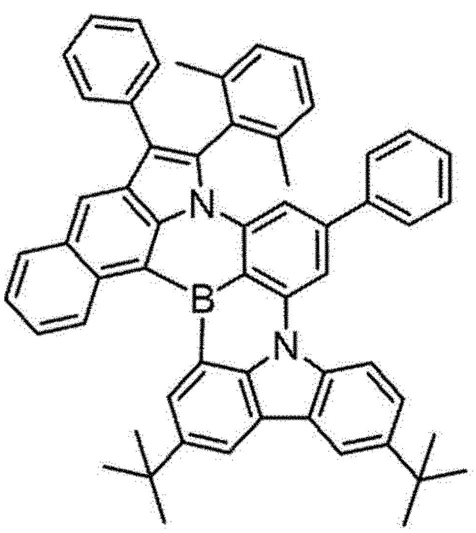

[Formula 79]

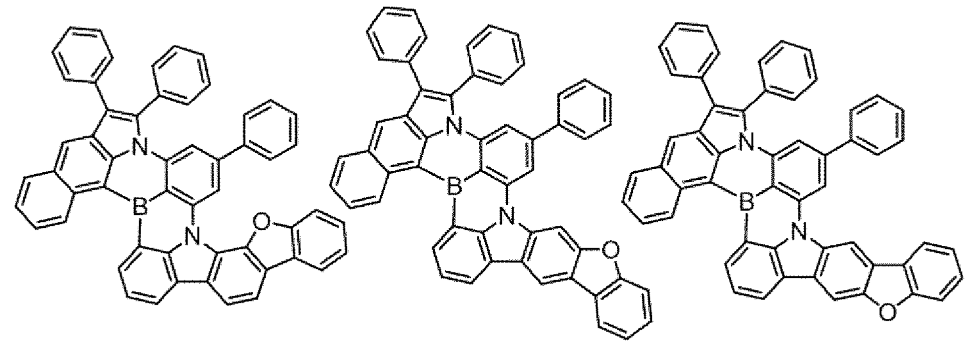

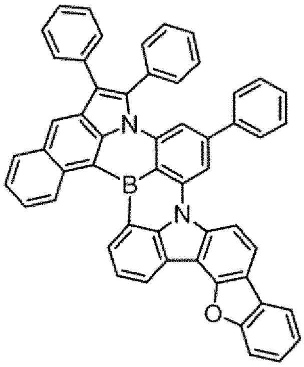

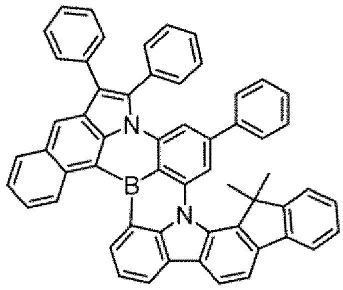

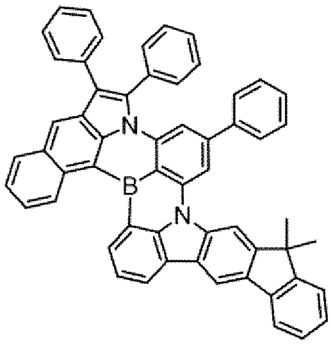

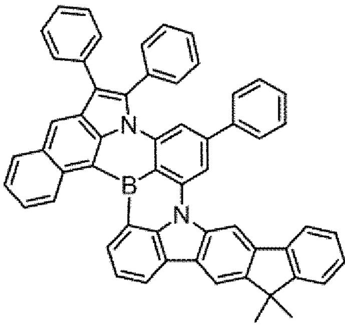

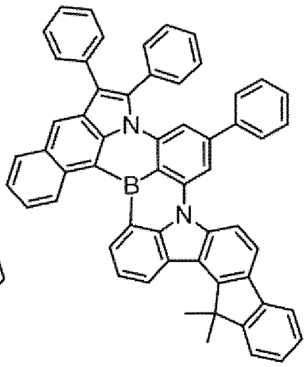

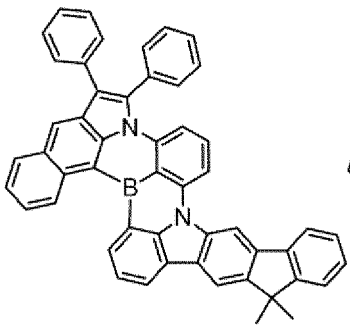

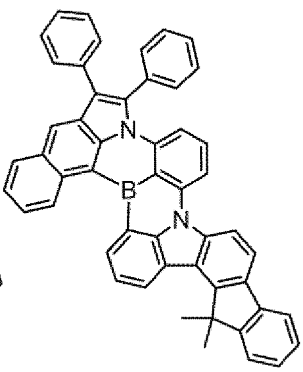

[Formula 80]

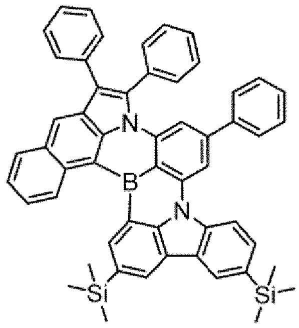

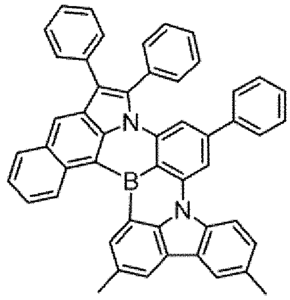

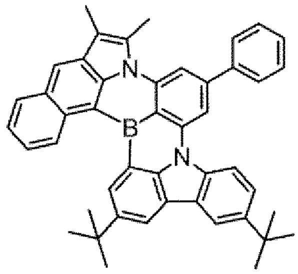

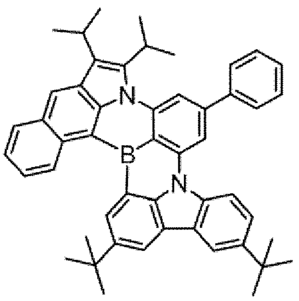

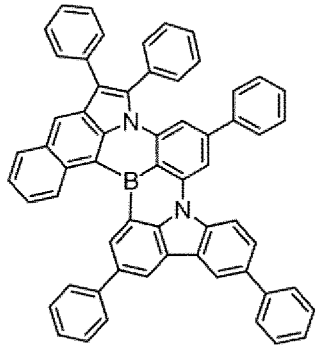

[Formula 81]

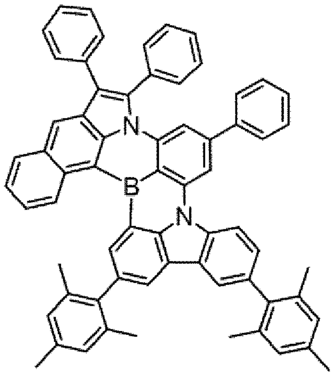

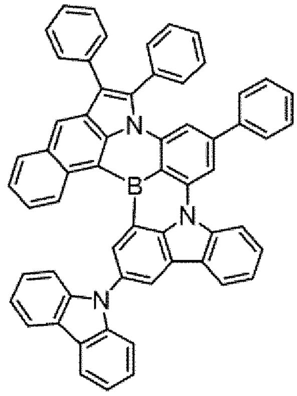

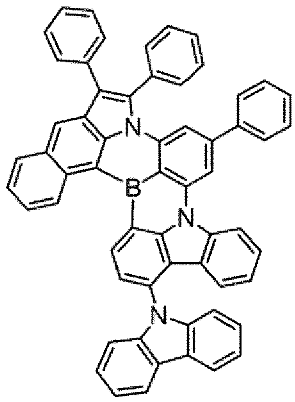

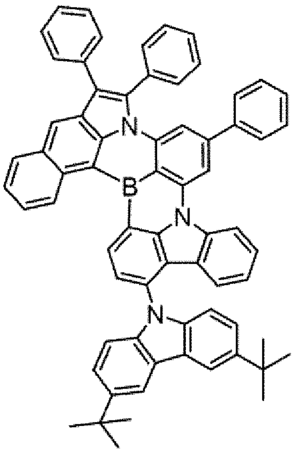

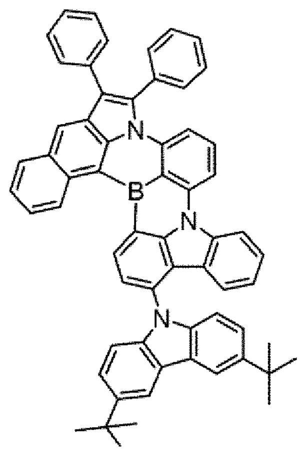

[Formula 82]

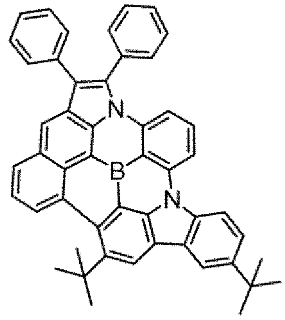

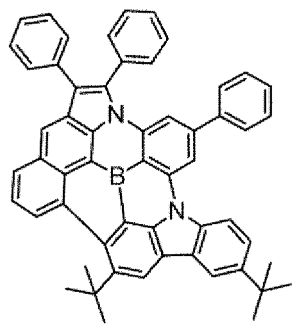

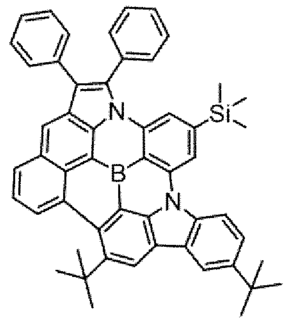

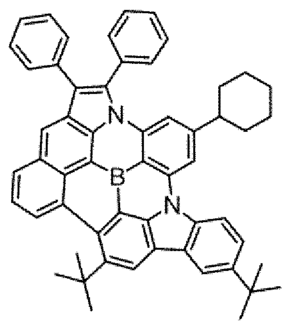

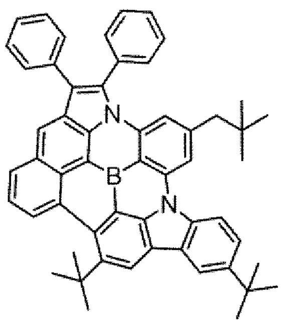

[Formula 83]

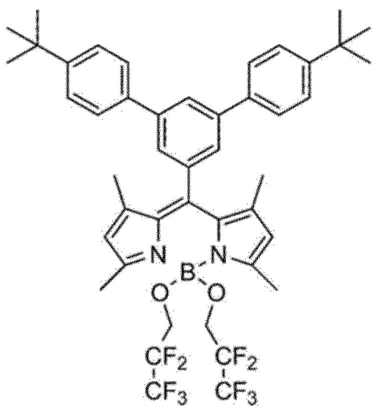

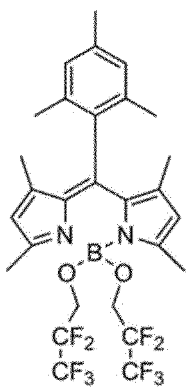

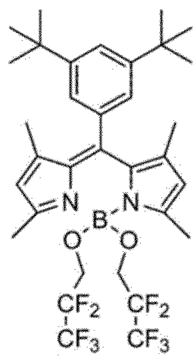

[Formula 84]

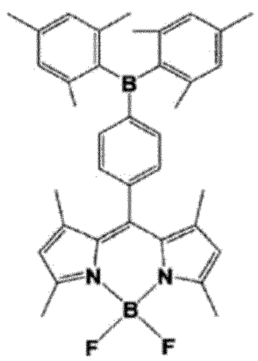

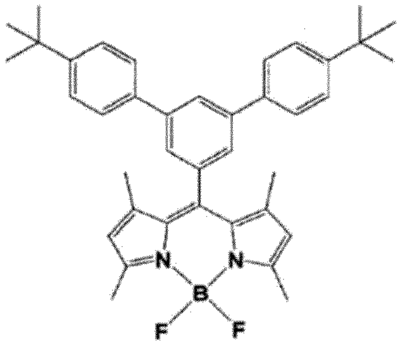

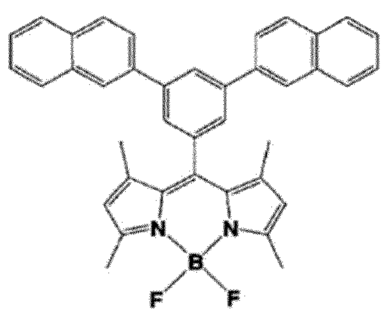

Relationship between Compound M3, Compound M2 and Compound M1 in Emitting Layer

In the organic EL device according to the exemplary embodiment, a singlet energy $S_1$(M1) of the compound M1 and a singlet energy $S_1$(M2) of the compound M2 satisfy a relationship of a numerical formula (Numerical Formula

2) below.

$$S_1(M2) > S_1(M1) \quad ...(\text{Numerical Formula 2})$$

Moreover, the singlet energy S1 (M3) of the compound M3 is preferably larger than the singlet energy S1(M1) of the compound M1.

The singlet energy $S_1$(M3) of the compound M3, the singlet energy $S_1$(M2) of the compound M2, and the singlet energy $S_1$(M1) of the compound M1 preferably satisfy a relationship of a numerical formula (Numerical Formula 2A) below.

$$S_1(M3) > S_1(M2) > S_1(M1) \quad ...(\text{Numerical Formula 2A})$$

It is preferable that mainly the fluorescent compound M1 emits light in the emitting layer when the organic EL device of the exemplary embodiment emits light.

The organic EL device according to the exemplary embodiment preferably emits red light or green light.

Content Ratios of Compounds in Emitting Layer

Content ratios of the compound M3, the compound M2, and the compound M1 in the emitting layer preferably fall, for instance, within a range below.

The content ratio of the compound M3 is preferably in a range from 10 mass% to 80 mass%.

The content ratio of the compound M2 is preferably in a range from 10 mass% to 80 mass%, more preferably in a range from 10 mass% to 60 mass%, and still more preferably in a range from 20 mass% to 60 mass%.

The content ratio of the compound M1 is preferably in a range from 0.01 mass% to 10 mass%, more preferably in a range from 0.01 mass% to 5 mass%, and still more preferably in a range from 0.01 mass% to 1 mass%.

The upper limit of a total of the content ratios of the compound M3, the compound M2, and the compound M1 in the emitting layer is 100 mass%. It should be noted that the emitting layer of the exemplary embodiment may further contain material(s) other than the compounds M3, M2 and M1.

The emitting layer may contain a single type of the compound M3 or may contain two or more types of the compound M3. The emitting layer may contain a single type of the compound M2 or may contain two or more types of the compound M2. The emitting layer may contain a single type of the compound M1 or may contain two or more types of the compound M1.

Fig. 5 illustrates an example of a relationship between energy levels of the compound M3, the compound M2, and the compound M1 in the emitting layer. In Fig. 5, S0 represents a ground state. S1(M1) represents the lowest singlet state of the compound M1. T1(M1) represents the lowest triplet state of the compound M1. S1(M2) represents the lowest singlet state of the compound M2. T1(M2) represents the lowest triplet state of the compound M2. S1(M3) represents the lowest singlet state of the compound M3. T1(M3) represents the lowest triplet state of the compound M3. A dashed arrow directed from S1(M2) to S1(M1) in Fig. 5 represents Förster energy transfer from the lowest singlet state of the compound M2 to the lowest singlet state of the compound M1.

As shown in Fig. 5, when a compound having a small $\Delta$ST(M2) is used as the compound M2, inverse intersystem crossing from the lowest triplet state T1(M2) to the lowest singlet state S1(M2) can be caused by a heat energy. Subsequently, Förster energy transfer from the lowest singlet state S1(M2) of the compound M2 to the compound M1 occurs to generate the lowest singlet state S1(M1). Consequently, fluorescence from the lowest singlet state S1(M1) of the compound M1 can be observed. It is inferred that the internal quantum efficiency can be theoretically raised up to 100% also by using delayed fluorescence by the TADF mechanism.

The organic EL device according to the second exemplary embodiment contains the delayed fluorescent compound M2, the compound M3 (compound M3 represented by the formula (1)) having the singlet energy larger than that of the delayed fluorescent compound M2, and the compound M1 having the singlet energy smaller than that of the delayed fluorescent compound M2 in the emitting layer.

According to the second exemplary embodiment, a high-performance organic EL device is achievable.

The organic EL device according to the second exemplary embodiment is usable in an electronic device such as a display device and a light-emitting unit.

Third Exemplary Embodiment

Electronic Device

An electronic device according to a sixth exemplary embodiment is installed with any one of the organic EL devices according to the above exemplary embodiments. Examples of the electronic device include a display device and a light-emitting unit. Examples of the display device include a display component (e.g., an organic EL panel module), TV, mobile phone, tablet and personal computer. Examples of the light-emitting unit include an illuminator and a vehicle light.

Fourth Exemplary Embodiment

Compound

The compound according to the fourth exemplary embodiment is a compound represented by the formula (100) described in the first exemplary embodiment. According to the compound according to the fourth exemplary embodiment, a high-performance organic EL device is achievable.

Organic EL Device

An organic EL device, which is an example of the fourth exemplary embodiment, contains the compound of the fourth exemplary embodiment (compound represented by the formula (100)) in any layer of the organic layers provided between the anode and the cathode.

The compound according to the fourth exemplary embodiment enables an organic EL device to have a high performance. Accordingly, the organic EL device, which is an example of the fourth exemplary embodiment, also has a high performance.

Fifth Exemplary Embodiment

Organic-EL-Device Material

An organic-EL-device material of a fifth exemplary embodiment contains the compound of the fourth exemplary embodiment.

According to the organic-EL-device material of the six exemplary embodiment, high-performance organic EL device and electronic device are achievable.

The organic-EL-device material of the sixth exemplary embodiment may further contain an additional compound. When the organic-EL-device material of the sixth exemplary embodiment further contains an additional compound, the additional compound may be solid or liquid.

Modification of Embodiment(s)

The scope of the invention is not limited by the above-described exemplary embodiments but includes any modification and improvement as long as such modification and improvement are compatible with the invention.

For instance, the emitting layer is not limited to a single layer, but may be provided by laminating a plurality of emitting layers. When the organic EL device has the plurality of emitting layers, it is only required that at least one of the emitting layers satisfies the conditions described in the above exemplary embodiments. For instance, the rest of the emitting layers may be a fluorescent emitting layer or a phosphorescent emitting layer with use of emission caused by electron transfer from the triplet excited state directly to the ground state.

When the organic EL device includes a plurality of emitting layers, these emitting layers may be mutually adjacently provided, or may form a so-called tandem organic EL device, in which a plurality of emitting units are layered via an intermediate layer.

For instance, a blocking layer may be provided adjacent to at least one of a side of the emitting layer close to the anode or a side of the emitting layer close to the cathode. The blocking layer is preferably provided in contact with the emitting layer to block at least any of holes, electrons, or excitons.

For instance, when the blocking layer is provided in contact with the side of the emitting layer close to the cathode, the blocking layer permits transport of electrons, and blocks holes from reaching a layer provided closer to the cathode (e.g., the electron transporting layer) beyond the blocking layer. When the organic EL device includes the electron transporting layer, the blocking layer is preferably interposed between the emitting layer and the electron

transporting layer.

When the blocking layer is provided in contact with the side of the emitting layer close to the anode, the blocking layer permits transport of holes and blocks electrons from reaching a layer provided closer to the anode (e.g., the hole transporting layer) beyond the blocking layer. When the organic EL device includes the hole transporting layer, the blocking layer is preferably interposed between the emitting layer and the hole transporting layer.

Alternatively, the blocking layer may be provided adjacent to the emitting layer so that the excitation energy does not leak out from the emitting layer toward neighboring layer(s). The blocking layer blocks excitons generated in the emitting layer from being transferred to a layer(s) (e.g., the electron transporting layer and the hole transporting layer) closer to the electrode(s) beyond the blocking layer.

The emitting layer is preferably bonded with the blocking layer.

Specific structure, shape and the like of the components in the invention may be designed in any manner as long as an object of the invention can be achieved. Examples

The invention will be described in more detail below with reference to Examples. However, the invention is by no means limited to these Examples.

Compounds

The compound M3 and the delayed fluorescent compound M2 used for producing organic EL devices in Examples 1 to to 19 are shown below.

[Formula 85]

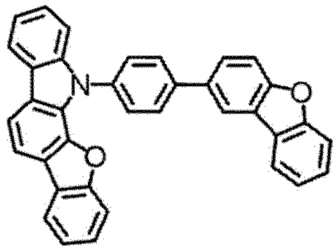

M3-1

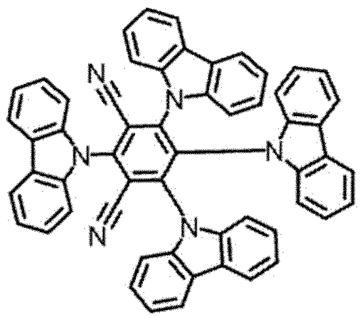

TADF-1

[Formula 86]

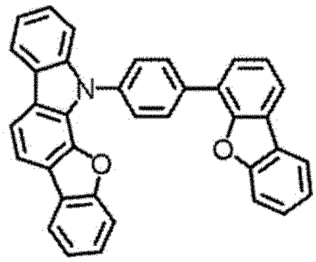

M3-6

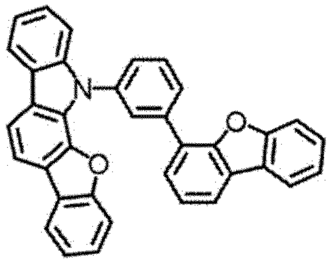

M3-7

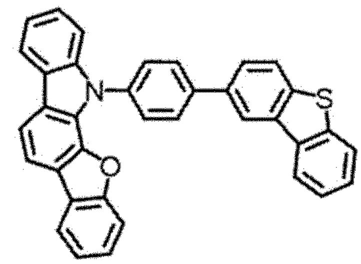

M3-8

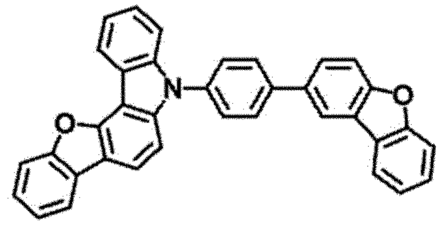

M3-9

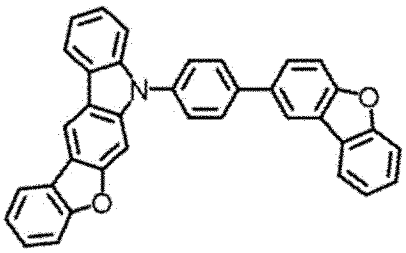

M3-10

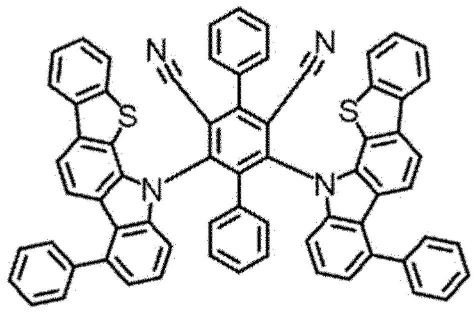

TADF-2

[Formula 87]

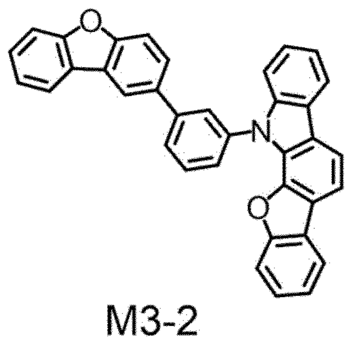

M3-2

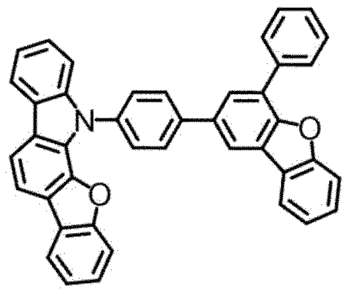

M3-11

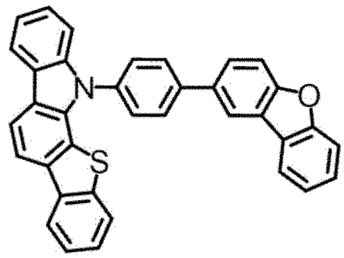

M3-12

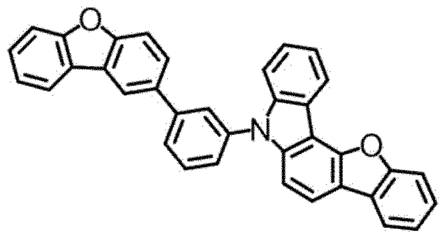

M3-13

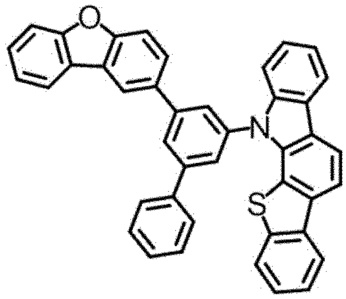

M3-15

A structure of a compound used for producing an organic EL device in Comparative 1 is shown below.

[Formula 88]

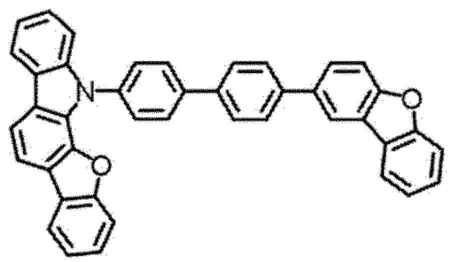

Ref-1

Structures of other compounds used for producing organic EL devices in Examples 1 to 19 and Comparative 1 are shown below.

[Formula 89]

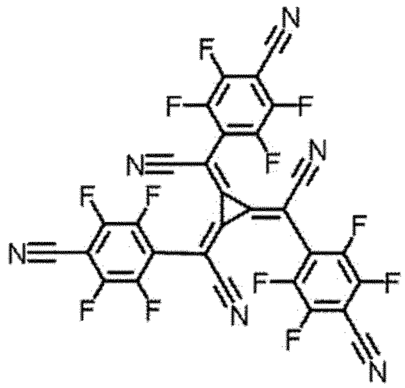

HA

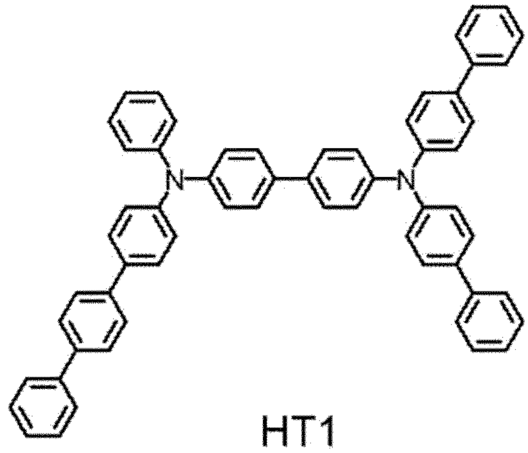

HT1

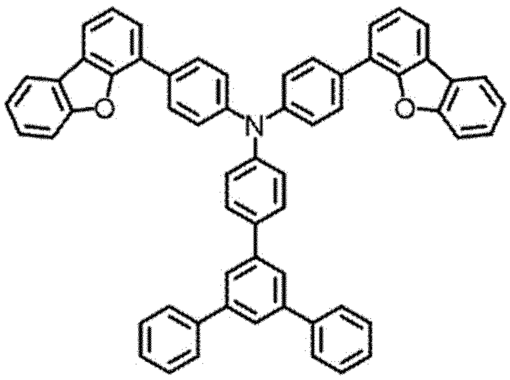

HT2

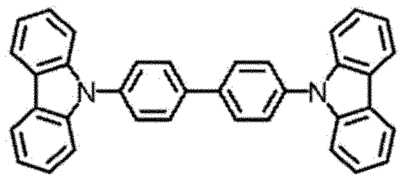

HT3

[Formula 90]

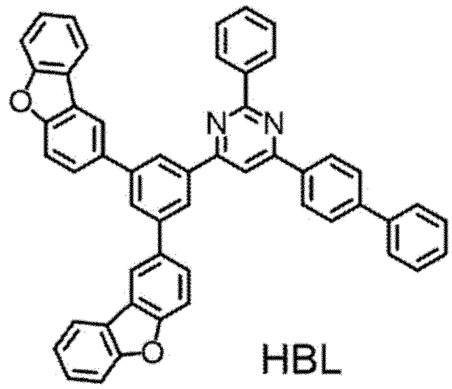

HBL

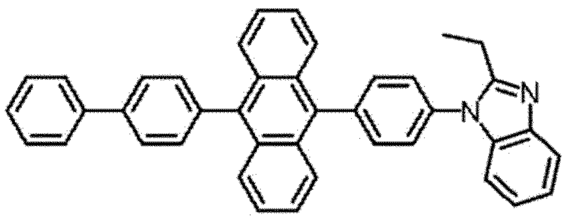

ET

[Formula 91]

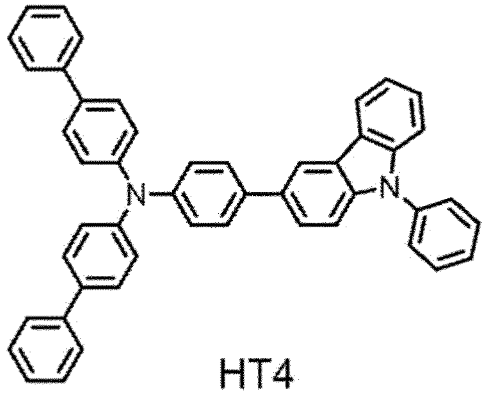

HT4

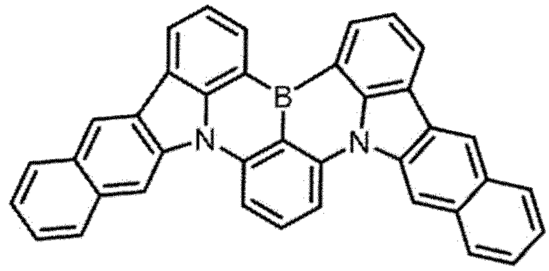

FD

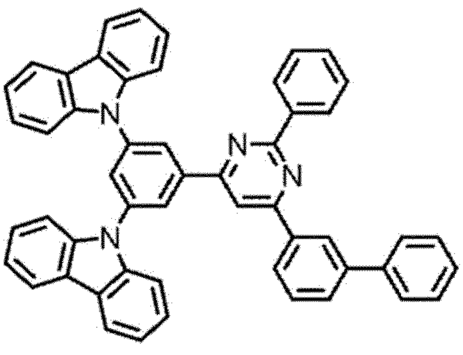

HBL2

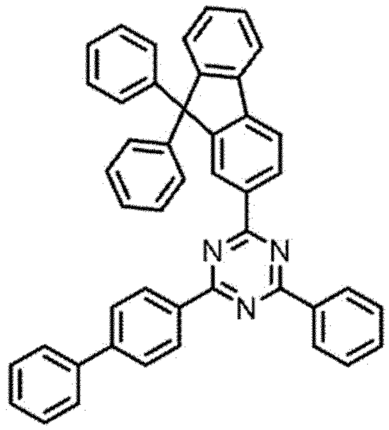

ET2

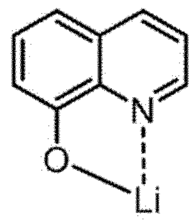

Liq

[Formula 92]

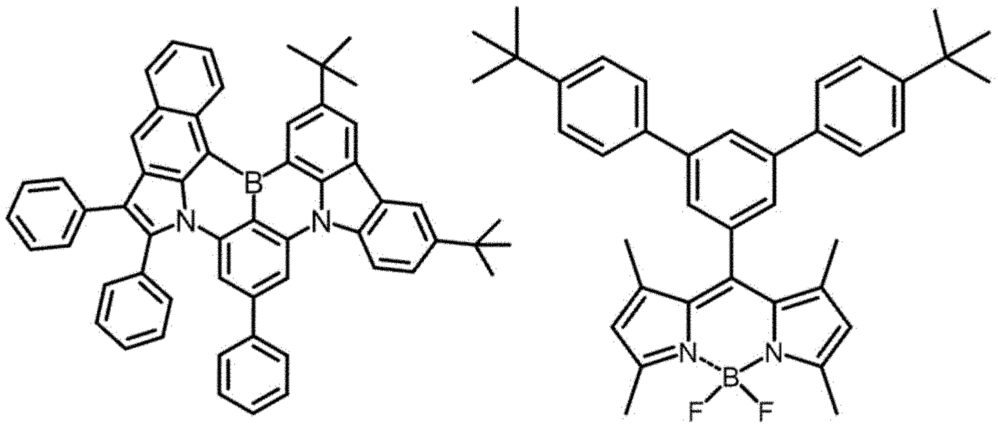

FD-2

FD-3

Production 1 of Organic EL Device

The organic EL devices were produced and evaluated as follows.

Example 1

A glass substrate (size: 25 mm × 75 mm × 1.1 mm thick, produced by Geomatec Co., Ltd.) having an ITO transparent electrode (anode) was ultrasonic-cleaned in isopropyl alcohol for five minutes, and then UV-ozone-cleaned

for one minute. The film thickness of ITO was 130 nm.

After the glass substrate having the transparent electrode line was cleaned, the glass substrate was mounted on a substrate holder of a vacuum deposition apparatus. Firstly, a compound HT1 and a compound HA were co-deposited on a surface of the glass substrate where the transparent electrode line was provided in a manner to cover the transparent electrode, thereby forming a 10-nm-thick hole injecting layer. Concentrations of the compound HT1 and the compound HA in the hole injecting layer were 97 mass% and 3 mass%, respectively.

Next, the compound HT1 was vapor-deposited on the hole injecting layer to form a 110-nm-thick first hole transporting layer.

A compound HT2 was then vapor-deposited on the first hole transporting layer to form a 5-nm-thick second hole transporting layer.

A compound HT3 was then vapor-deposited on the second hole transporting layer to form a 5-nm-thick electron blocking layer.

Next, a compound M3-1 as the compound M3, and a compound TADF-1 as the delayed fluorescent compound M2 were co-deposited on the electron blocking layer to form a 25-nm-thick emitting layer. Concentrations of the compound M3-1 and the compound TADF-1 in the emitting layer were 75 mass% and 25 mass%, respectively.

Next, a compound HBL was vapor-deposited on the emitting layer to form a 5-nm-thick hole blocking layer.

A compound ET was then vapor-deposited on the hole blocking layer to form a 50-nm-thick electron transporting layer.

Next, lithium fluoride (LiF) was vapor-deposited on the electron transporting layer to form a 1-nm-thick electron injecting electrode (cathode).

Subsequently, metal aluminum (Al) was vapor-deposited on the electron injectable electrode to form an 80-nm-thick metal Al cathode.

A device arrangement of the organic EL device in Example 1 is roughly shown as follows.

ITO(130) / HT1:HA(10,97%:3%) / HT1(110) / HT2(5) / HT3(5) / M3-1 :TADF-1 (25,75%:25%) / HBL(5) / ET(50) / LiF(1) / Al(80)

Numerals in parentheses represent a film thickness (unit: nm).

The numerals (97%:3%) represented by percentage in the same parentheses indicate a ratio (mass%) between the compound HT1 and the compound HA in the hole injecting layer. The numerals (75%:25%) represented by percentage in the same parentheses indicate a ratio (mass%) between the the compound M3 and the compound M2 in the emitting layer.

Evaluation 1 of Organic EL Device

It was confirmed that the organic EL device produced in Example 1 emitted light when voltage was applied on the organic EL device so that a current density was 10 $mA/cm^2$.

Production 2 of Organic EL Device

The organic EL devices were produced and evaluated as follows.

Example 2

A glass substrate (size: 25 mm × 75 mm × 1.1 mm thick, produced by Geomatec Co., Ltd.) having an ITO transparent electrode (anode) was ultrasonic-cleaned in isopropyl alcohol for five minutes, and then UV-ozone-cleaned for one minute. The film thickness of ITO was 130 nm.

After the glass substrate having the transparent electrode line was cleaned, the glass substrate was mounted on a substrate holder of a vacuum deposition apparatus. Firstly, a compound HT4 and a compound HA were co-deposited on a surface of the glass substrate where the transparent electrode line was provided in a manner to cover the transparent electrode, thereby forming a 10-nm-thick hole injecting layer. Concentrations of the compound HT4 and the compound HA in the hole injecting layer were 97 mass% and 3 mass%, respectively.

Next, the compound HT4 was vapor-deposited on the hole injecting layer to form a 110-nm-thick first hole transporting layer.

A compound HT2 was then vapor-deposited on the first hole transporting layer to form a 5-nm-thick second hole transporting layer.

A compound HT3 was then vapor-deposited on the second hole transporting layer to form a 5-nm-thick electron blocking layer.

Next, a compound M3-1 as the compound M3, a compound TADF-2 as the delayed fluorescent compound M2, and a compound FD as the compound M1 were co-deposited on the electron blocking layer to form a 25-nm-thick emitting

layer. Concentrations of the compound M3-1, the compound TADF-2, and the compound FD in the emitting layer were 79.2 mass%, 20 mass%, and 0.8 mass%, respectively.

Next, a compound HBL2 was vapor-deposited on the emitting layer to form a 5-nm-thick hole blocking layer.

A compound ET2 and a compound Liq were then co-deposited on the hole blocking layer to form a 50-nm-thick electron transporting layer. Concentrations of the compound ET2 and the compound Liq in the electron transporting layer were 50 mass% and 50 mass%, respectively.

Next, Yb was vapor-deposited on the electron transporting layer to form a 1-nm-thick electron injecting electrode (cathode).

Subsequently, metal aluminum (Al) was vapor-deposited on the electron injectable electrode to form an 80-nm-thick metal Al cathode.

A device arrangement of the organic EL device in Example 2 is roughly shown as follows.

ITO(130) / HT4:HA(10,97%:3%) / HT4(110) / HT2(5) / HT3(5) / M3-1:TADF-2:FD(25,79.2%:20%:0.8%) / HBL2(5) / ET2:Liq(50,50%:50%) / Yb(1) / Al(80)

Numerals in parentheses represent a film thickness (unit: nm).

The numerals (97%:3%) represented by percentage in the same parentheses indicate a ratio (mass%) between the compound HT4 and the compound HA in the hole injecting layer. The numerals (79.2%: 20%:0.8%) represented by percentage in the same parentheses indicate a ratio (mass%) between the compound M3, the compound M2, and the compound M1 in the emitting layer. The numerals (50%:50%) represented by percentage in the same parentheses indicate a ratio (mass%) between the compound ET2 and the compound Liq in the electron transporting layer.

Examples 3 to 7 and Comparative 1

The organic EL devices in Examples 3 to 7 and Comparative 1 were produced in the same manner as in Example 2 except that the compound M3-1 used in Example 2 was replaced by compounds shown in Table 1.

Examples 8 and 9

The organic EL devices in Examples 8 and 9 were produced in the same manner as in Example 2 except that the compound M3-1 used in Example 2 was replaced by compounds shown in Table 2.

Examples 10 to 19

The organic EL devices in Examples 10 to 19 were produced in the same manner as in Example 2 except that the compound M3-1 used in Example 2 was replaced by compounds shown in Table 2 and the compound FD used in Example 2 was replaced by compounds shown in Table 2.

Evaluation 2 of Organic EL Device

The organic EL devices produced were evaluated as follows. Tables 1 and 2 show evaluation results. Evaluation results of Comparative 1 are shown in both of Tables 1 and 2. In Table 2, "-" represents that no measurement was made.

Maximum Peak Wavelength λp

Voltage was applied to the organic EL devices such that a current density was 10 mA/$cm^2$, where spectral radiance spectrum was measured with a spectroradiometer CS-2000 (produced by Konica Minolta, Inc.). The maximum peak wavelength λp (unit: nm) was calculated based on the obtained spectral-radiance spectra.

External Quantum Efficiency EQE

Voltage was applied to the organic EL devices such that a current density was 10 mA/$cm^2$, where spectral radiance spectrum was measured with a spectroradiometer CS-2000 (produced by Konica Minolta, Inc.). The external quantum efficiency EQE (unit: %) was calculated based on the obtained spectral radiance spectra, assuming that the spectra was provided under a Lambertian radiation.

Lifetime LT95

Voltage was applied to the organic EL device produced in each Example so that a current density was 50 mA/$cm^2$, where a time (LT95 (unit: hr)) elapsed before a luminance intensity was reduced to 95% of the initial luminance

intensity was measured. The luminance intensity was measured with a spectroradiometer CS-2000 (manufactured by Konica Minolta, Inc.).

Table 1

| | Com pound M 3 | | | Compound M2 | | | Compound M1 | | | Device Evaluation Results | |
|---|---|---|---|---|---|---|---|---|---|---|---|
| | Name | $S_1$ [eV] | $T_{77K}$ [eV] | Name | $S_1$ [eV] | ΔST [eV] | Name | $S_1$ [eV] | A [nm] | λp [nm] | EQE [%] |
| Ex. 2 | M3-1 | 3.43 | 2.89 | TADF-2 | 2.61 | <0.01 | FD | 2.45 | 500 | 513 | 13.5 |
| Ex. 3 | M3-6 | 3.43 | 2.89 | TADF2 | 2.61 | <0.01 | FD | 2.45 | 500 | 512 | 13.4 |
| Ex. 4 | M3-7 | 3.46 | 2.89 | TADF2 | 2.61 | <0.01 | FD | 2.45 | 500 | 511 | 13.6 |
| Ex. 5 | M3-8 | 3.43 | 2.89 | TADF-2 | 2.61 | <0.01 | FD | 2.45 | 500 | 512 | 14.9 |
| Ex. 6 | M3-9 | 3.43 | 2.99 | TADF2 | 2.61 | <0.01 | FD | 2.45 | 500 | 514 | 13.7 |
| Ex. 7 | M3-10 | 3.38 | 2.84 | TADF-2 | 2.61 | <0.01 | FD | 2.45 | 500 | 513 | 13.3 |
| Com p. 1 | Ref-1 | 3.41 | 2.72 | TADF-2 | 2.61 | <0.01 | FD | 2.45 | 500 | 513 | 11.7 |

The organic EL devices of Examples 2 to 7 containing the compound M3 represented by the formula (1) and the delayed fluorescent compound M2 emitted light at a higher efficiency than the organic EL device of Comparative 1 having the compound Ref-1 in place of the compound M3.

Table 2

| | Compound M3 | | | Compound M2 | | | Compound M1 | | | Device Evaluation Results | | |
|---|---|---|---|---|---|---|---|---|---|---|---|---|
| | Name | $S_1$ [eV] | $T_{77K}$ [eV] | Name | $S_1$ [eV] | ΔST [eV] | Name | $S_1$ [eV] | λ [nm] | λp [nm] | EQE [%] | LT95 [hr] |
| Ex. 8 | M3-11 | 3.43 | 2.89 | TADF-2 | 2.61 | <0.01 | FD | 2.45 | 500 | 513 | 12.6 | 43 |
| Ex. 9 | M3-12 | 3.34 | 2.85 | TADF-2 | 2.61 | <0.01 | FD | 2.45 | 500 | 513 | 13.2 | 49 |
| Ex. 10 | M3-11 | 3.43 | 2.89 | TADF-2 | 2.61 | <0.01 | FD-2 | 2.39 | 512 | 519 | 18.9 | 34 |
| Ex. 11 | M3-12 | 3.34 | 2.85 | TADF-2 | 2.61 | <0.01 | FD-2 | 2.39 | 512 | 519 | 19.2 | 40 |
| Ex. 12 | M3-13 | 3.45 | 2.99 | TADF-2 | 2.61 | <0.01 | FD-2 | 2.39 | 512 | 520 | 18.6 | 29 |
| Ex. 13 | M3-2 | 3.45 | 2.90 | TADF-2 | 2.61 | <0.01 | FD-2 | 2.39 | 512 | 519 | 17.7 | 36 |
| Ex. 14 | M3-15 | 3.36 | 2.84 | TADF-2 | 2.61 | <0.01 | FD-2 | 2.39 | 512 | 519 | 18.1 | 18 |
| Ex. 15 | M3-11 | 3.43 | 2.89 | TADF-2 | 2.61 | <0.01 | FD-3 | 2.39 | 517 | 523 | 13.6 | 5 |
| Ex. 16 | M3-12 | 3.34 | 2.85 | TADF-2 | 2.61 | <0.01 | FD-3 | 2.39 | 517 | 523 | 12.9 | 6 |
| Ex. 17 | M3-13 | 3.45 | 2.99 | TADF-2 | 2.61 | <0.01 | FD-3 | 2.39 | 517 | 523 | 13.8 | 6 |

(continued)

| | Compound M3 | | | Compound M2 | | | Compound M1 | | | Device Evaluation Results | | |
|---|---|---|---|---|---|---|---|---|---|---|---|---|
| | Name | $S_1$ [eV] | $T_{77K}$ [eV] | Name | $S_1$ [eV] | ΔST [eV] | Name | $S_1$ [eV] | λ [nm] | λp [nm] | EQE [%] | LT95 [hr] |
| Ex. 18 | M3-2 | 3.45 | 2.90 | TADF-2 | 2.61 | <0.01 | FD-3 | 2.39 | 517 | 523 | 14.6 | 6 |
| Ex. 19 | M3-15 | 3.36 | 2.84 | TADF-2 | 2.61 | <0.01 | FD-3 | 2.39 | 517 | 524 | 13.4 | 5 |
| Comp. 1 | Ref-1 | 3.41 | 2.72 | TADF-2 | 2.61 | <0.01 | FD | 2.45 | 500 | 513 | 11.7 | - |

The organic EL devices of Examples 8 to 19 containing the compound M3 represented by the formula (1), the delayed fluorescent compound M2, and the fluorescent compound M1 in the emitting layer emitted light at a higher efficiency than the organic EL device of Comparative 1.

In comparison between the organic EL devices of Examples 8 to 14 and the organic EL devices of Examples 15 to 19, the organic EL devices of Examples 8 to 14, in which a compound (FD or FD-2) represented by the formula (2A) was used as the fluorescent compound M1, had a remarkably longer lifetime than the organic EL devices of Examples 15 to 19, in which the compound (FD-3) having a pyrromethene skeleton was used as the fluorescent compound M1.

In comparison between the organic EL devices of Examples 8 and 9 and the organic EL devices of Examples 10 and 11, the organic EL devices of Examples 8 and 9, in which the compound (FD) represented by the formula (16) was used as the fluorescent compound M1, had a longer lifetime than the organic EL devices of Examples 10 and 11, in which the compound (FD-2) represented by the formula (D11) was used as the fluorescent compound M1. However, the organic EL devices of Examples 10 and 11, in which the compound (FD-2) represented by the formula (D11) was used as the fluorescent compound M1, emitted light at a higher efficiency than the organic EL devices of Examples 8 and 9, in which the compound (FD) represented by the formula (16) was used as the fluorescent compound M1.

In comparison between the organic EL devices of Examples 10 to 14, the organic EL devices of Examples 10 and 11, in each of which the compound (M3-11 or M3-12) represented by the formula (12A) was used as the compound M3, emitted light at a higher efficiency than the organic EL devices in Examples 12 to 14, in each of which the compounds (M3-13, M3-2, or M3-15) represented by the formula (12C) was used as the compound M3. Among the organic EL devices of Examples 10 to 14, the organic EL device in Example 11, in which the compound (M3-12) represented by the formula (12A) and having a sulfur atom for $X_1$ in A, exhibited more remarkable improvement in external quantum efficiency and lifetime.

Evaluation of Compounds

Physical property values of compounds were measured according to the following methods. Tables 1 to 3 shows measurement results.

Thermally Activated Delayed Fluorescence (Delayed Fluorescence of Compound TADF-1)

Delayed fluorescence was checked by measuring transient PL using an apparatus illustrated in Fig. 2. The compound TADF-1 was dissolved in toluene to prepare a dilute solution with an absorbance of 0.05 or less at the excitation wavelength to eliminate contribution of self-absorption. In order to prevent quenching due to oxygen, the sample solution was frozen and degassed and then sealed in a cell with a lid under an argon atmosphere to obtain an oxygen-free sample solution saturated with argon.

The fluorescence spectrum of the sample solution was measured with a spectrofluorometer FP-8600 (produced by JASCO Corporation), and the fluorescence spectrum of a 9,10-diphenylanthracene ethanol solution was measured under the same conditions. Using the fluorescence area intensities of both spectra, the total fluorescence quantum yield was calculated by an equation (1) in Morris et al. J. Phys. Chem. 80 (1976) 969.

Prompt emission was observed immediately when the excited state was achieved by exciting the compound TADF-1 with a pulse beam (i.e., a beam emitted from a pulse laser) having a wavelength to be absorbed by the compound TADF-1, and Delay emission was observed not immediately when the excited state was achieved but after the excited state was achieved. The delayed fluorescence in Examples means that an amount of Delay emission is 5% or more with respect to an amount of Prompt emission. Specifically, provided that the amount of Prompt emission is denoted by

$X_P$ and the amount of Delay emission is denoted by $X_D$, the delayed fluorescence means that a value of $X_D/X_P$ is 0.05 or more.

An amount of Prompt emission, an amount of Delay emission and a ratio between the amounts thereof can be obtained according to the method as described in "Nature 492, 234-238, 2012" (Reference Document 1). The amount of Prompt emission and the amount of Delay emission may be calculated using an apparatus different from one described in Reference Document 1 or the apparatus illustrated in Fig. 2.

It was confirmed that the amount of Delay emission was 5% or more with respect to the amount of Prompt emission in the compound TADF-1.

Specifically, the value of $X_D/X_P$ was 0.05 or more with respect to the compound TADF-1.

Delayed Fluorescence of Compound TADF-2

Delayed fluorescence of the compound TADF-2 was checked in the same manner as that of the compound TADF-1 except for that the compound TADF-2 was used in place of the compound TADF-1.

A value of $X_D/X_P$ was 0.05 or more with respect to the compound TADF-2.

Singlet Energy $S_1$

A singlet energy $S_1$ of each of the compounds M3-1, M3-6 to M3-15, TADF-1, TADF-2, FD, FD-2, FD-3, and Ref-1 was measured according to the above-described solution method.

Energy Gap $T_{77K}$ at 77K

An energy gap $T_{77K}$ of each of the compounds M3-1, M3-6 to M3-15, TADF-1, TADF-2, and Ref-1 was measured according to the measurement method of the energy gap $T_{77K}$ described in the above "Relationship between Triplet Energy and Energy Gap at 77K." $\Delta$ST was checked from the measurement results of $T_{77K}$ and the above values of the singlet energy $S_1$.

Maximum Peak Wavelength $\lambda$ of Compounds

A maximum peak wavelength $\lambda$ of each of the compounds TADF-1, FD, FD-2, and FD-3 was measured according to the following method.

A toluene solution of each measurement target compound at a concentration of 5 $\mu$mol/L was prepared and put in a quartz cell. An emission spectrum (ordinate axis: luminous intensity, abscissa axis: wavelength) of the thus-obtained sample was measured at a normal temperature (300K). In Examples, the emission spectrum was measured using a spectrophotometer manufactured by Hitachi, Ltd. (device name: F-7000). It should be noted that the machine for measuring the emission spectrum is not limited to the machine used herein. A peak wavelength of the emission spectrum exhibiting the maximum luminous intensity was defined as the maximum peak wavelength $\lambda$.

Table 3

| Compound M3 | | | Compound M2 | | | |
|---|---|---|---|---|---|---|
| Name | $S_1$[eV] | $T_{77K}$ [eV] | Name | $S_1$[eV] | $\Delta$ST[eV] | $\lambda$[nm] |
| M3 -1 | 3.43 | 2.89 | TADF-1 | 2.62 | <0.01 | 499 |

Synthesis of Compounds

Compounds M3-1 to M3-10, which were the compound M3, were synthesized.

Synthesis Example 1: Synthesis of Compound M3-1

(1-1) Synthesis of Compound M3-1

[Formula 93]

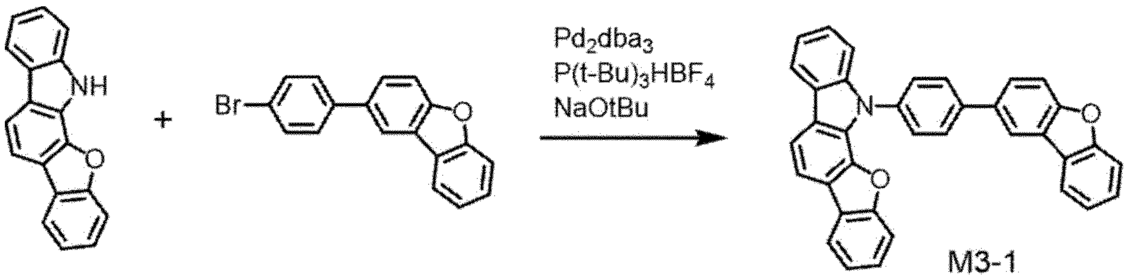


M3-1

Under a nitrogen atmosphere, xylene (200 mL) was added to a mixture of 12H-benzofuro[2,3-a]carbazole (10.3 g, 40.0 mmol), 2-(4-bromophenyl)dibenzo[b,d]furan (12.9 g, 40.0 mmol), tris(dibenzylideneacetone)dipalladium (0.549 g, 0.600 mmol), tri-tert-butylphosphonium tetrafluoroborate (0.696 g, 2.40 mmol), and sodium tert-butoxide (11.5 g, 120 mmol). The obtained mixture was stirred at 140 degrees C for five hours. After the reaction, water was added to the reaction mixture and a solid was filtrated and recrystallized with toluene to obtain the compound M3-1 (9.37 g, a yield of 46%). The obtained compound was identified as the compound M3-1 by analysis according to LC-MS (Liquid Chromatography-Mass spectrometry).

Synthesis Example 2: Synthesis of Compound M3-2

(2-1) Synthesis of Compound M3-2

[Formula 94]

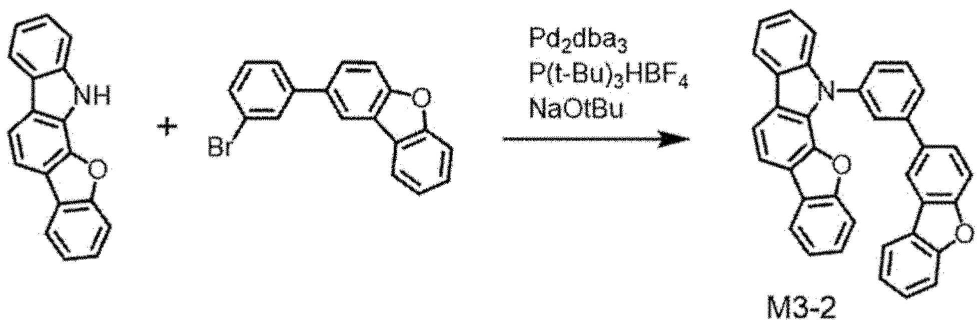


M3-2

The compound M3-2 was obtained in the same manner as in Synthesis Example 1 (1-1) except for using 2-(3-bromophenyl)dibenzo[b,d]furan in place of 2-(4-bromophenyl)dibenzo[b,d]furan. A yield was 71%. The obtained compound was identified as the compound M3-2 by analysis according to LC-MS.

Synthesis Example 3: Synthesis of Compound M3-3

(3-1) Synthesis of Compound M3-3

[Formula 95]

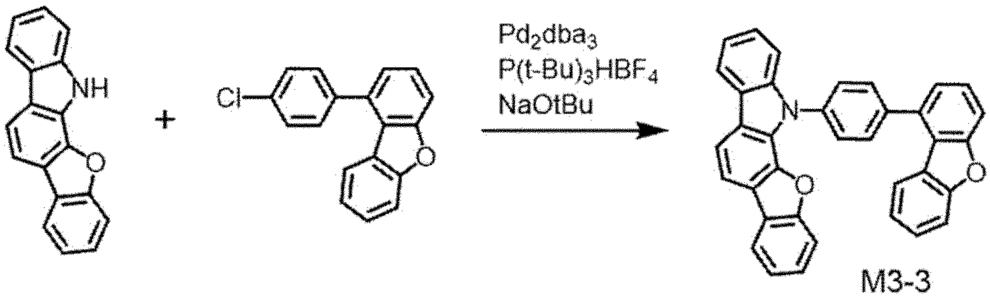


M3-3

The compound M3-3 was obtained in the same manner as in Synthesis Example 1 (1-1) except for using 1-(4-chlorophenyl)dibenzo[b,d]furan in place of 2-(4-bromophenyl)dibenzo[b,d]furan. A yield was 57%. The obtained compound was identified as the compound M3-3 by analysis according to LC-MS.

Synthesis Example 4: Synthesis of Compound M3-4

(4-1) Synthesis of 1-(3-chlorophenyl)dibenzo[b,d]furan

[Formula 96]

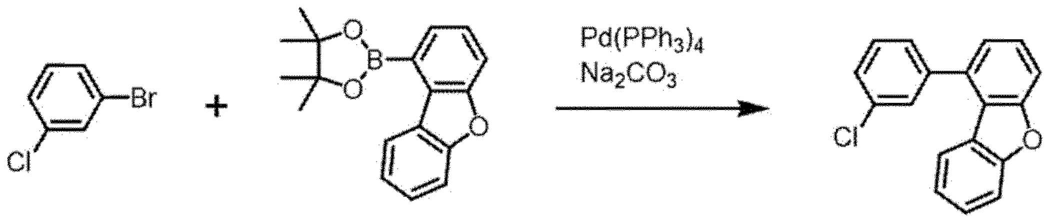


Under a nitrogen atmosphere, 1 ,2-dimethoxyethane (267 mL) and water (133 mL) were added to a mixture of 1-bromo-3-chlorobenzene (7.66 g, 40.0 mmol), 2-(dibenzo[b,d]furan-1-yl)-4,4,5,5-tetramethyl-1,3,2-dioxaborolane (11.8 g, 40.0 mmol), tetrakis(triphenylphosphine)palladium (2.3 g, 2.00 mmol), and sodium carbonate (12.7 g, 120 mmol). The obtained mixture was stirred at 80 degrees C for three hours. After the reaction, an organic layer was extracted with ethyl acetate and a solvent was distilled away. The residue was purified by silica gel column chromatography to obtain 1-(3-chlorophenyl)dibenzo[b,d]furan (10.8 g, a yield 95%).

(4-2) Synthesis of Compound M3-4

[Formula 97]

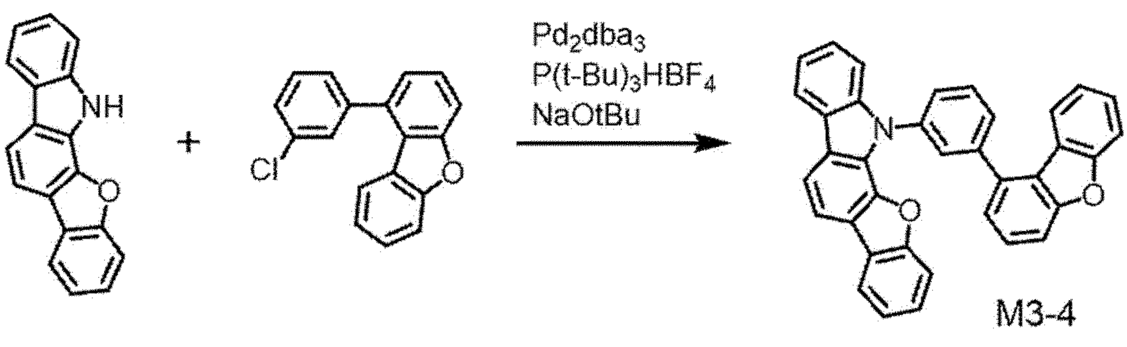


M3-4

The compound M3-4 was obtained in the same manner as in Synthesis Example 1 (1-1) except for using 1-(3-chlorophenyl)dibenzo[b,d]furan in place of 2-(4-bromophenyl)dibenzo[b,d]furan. A yield was 57%. The obtained compound was identified as the compound M3-4 by analysis according to LC-MS.

Synthesis Example 5: Synthesis of Compound M3-5

(5-1) Synthesis of Compound M3-5

[Formula 98]

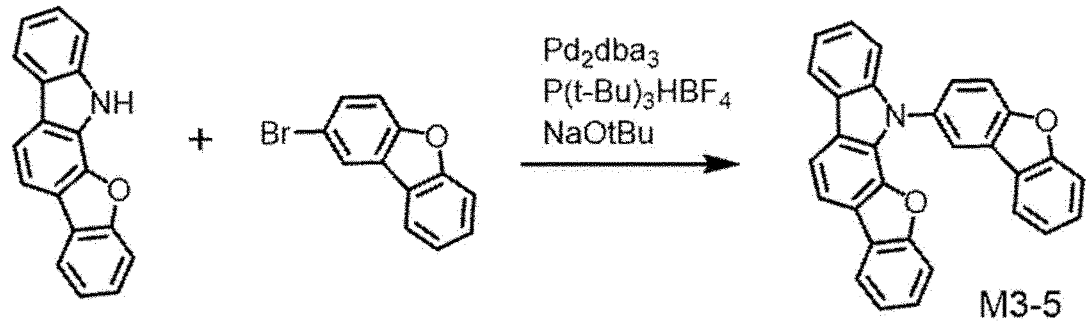


M3-5

The compound M3-5 was obtained in the same manner as in Synthesis Example 1 (1-1) except for using 2-bromodibenzo[b,d]furan in place of 2-(4-bromophenyl)dibenzo[b,d]furan. A yield was 61%. The obtained compound was identified as the compound M3-5 by analysis according to LC-MS.

Synthesis Example 6: Synthesis of Compound M3-6

(6-1) Synthesis of Compound M3-6

[Formula 99]

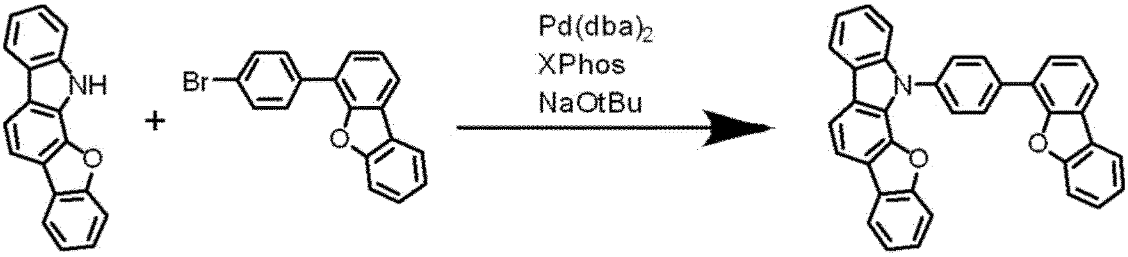


M3-6

Under a nitrogen atmosphere, toluene (30 mL) was added to a mixture of 12H-benzofuro[2,3-a]carbazole (1.70 g, 6.61 mmol), 4-(4-bromophenyl)dibenzo[b,d]furan (1.94 g, 6.01 mmol), dibenzylideneacetone palladium (0.104 g, 0.18 mmol), 2-dicyclohexylphosphino-2',4',6'-triisopropylbiphenyl (0.172 g, 0.36 mmol), and sodium tert-butoxide (0.866 g, 9.02 mmol). The obtained mixture was stirred at 110 degrees C for two hours. After the reaction, a solid was filtrated, washed with methanol, and recrystallized with toluene to obtain the compound M3-6 (2.43 g, a yield of 81%). The obtained compound was identified as the compound M3-6 by analysis according to LC-MS.

Synthesis Example 7: Synthesis of Compound M3-7

(7-1) Synthesis of Compound M3-7

[Formula 100]

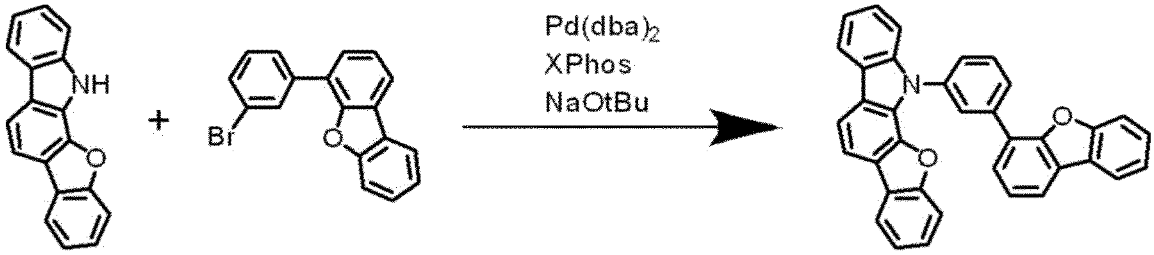


M3-7

The compound M3-7 was obtained in the same manner as in Synthesis Example 6 (6-1) except for using 4-(3-bromophenyl)dibenzo[b,d]furan in place of 4-(4-bromophenyl)dibenzo[b,d]furan. A yield was 80%. The obtained compound was identified as the compound M3-7 by analysis according to LC-MS.

Synthesis Example 8: Synthesis of Compound M3-8

(8-1) Synthesis of Compound M3-8

[Formula 101]

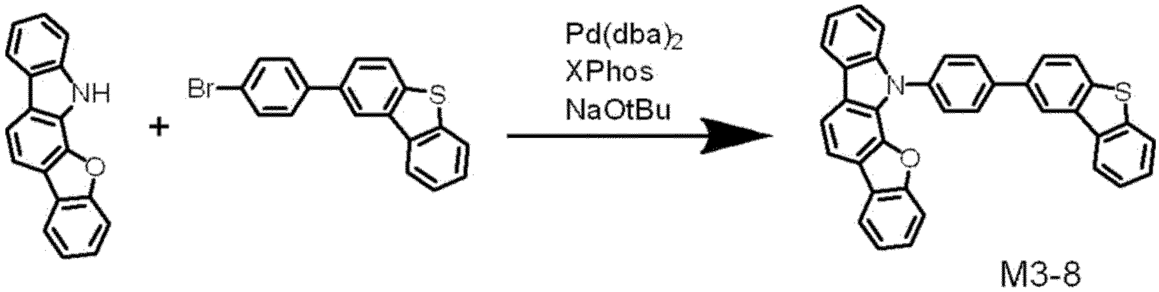


M3-8

The compound M3-8 was obtained in the same manner as in Synthesis Example 6 (6-1) except for using 2-(4-bromophenyl)dibenzo[b,d]thiophene in place of 4-(4-bromophenyl)dibenzo[b,d]furan. A yield was 61%. The obtained compound was identified as the compound M3-8 by analysis according to LC-MS.

Synthesis Example 9: Synthesis of Compound M3-9

(9-1) Synthesis of Compound M3-9

[Formula 102]

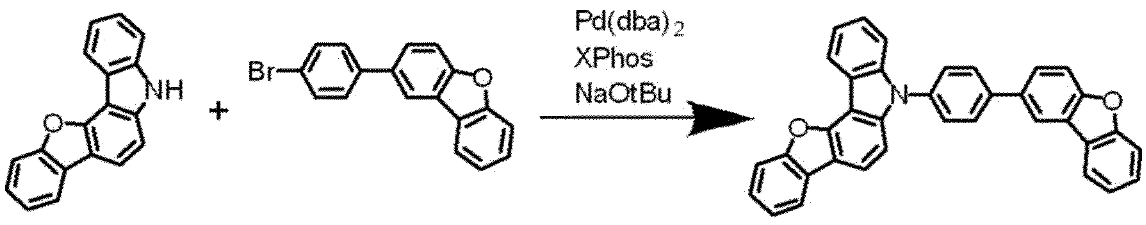


M3-9

The compound M3-9 was obtained in the same manner as in Synthesis Example 6 (6-1) except for using 5H-benzofuro[3,2-c]carbazole in place of 12H-benzofuro[2,3-a]carbazole and using 2-(4-bromophenyl)dibenzo[b,d]furan in place of 4-(4-bromophenyl)dibenzo[b,d]furan. A yield was 67%. The obtained compound was identified as the compound M3-9 by analysis according to LC-MS.

Synthesis Example 10: Synthesis of Compound M3-10

(10-1) Synthesis of Compound M3-10

[Formula 103]

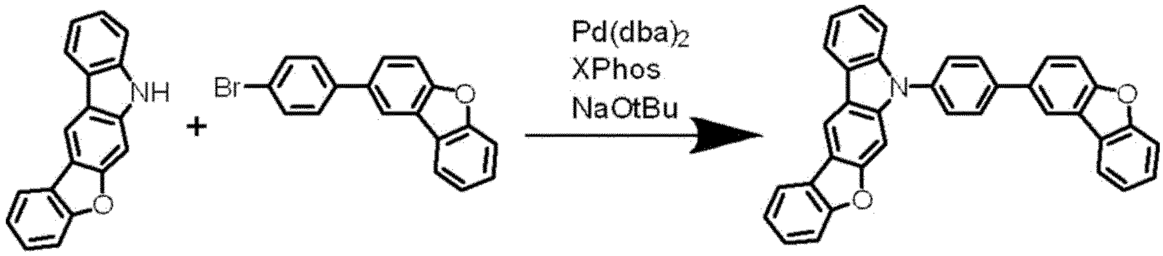


M3-10

Under a nitrogen atmosphere, toluene (35 mL) was added to a mixture of 7H-benzofuro[2,3-b]carbazole (1.98 g, 7.71 mmol), 2-(4-bromophenyl)dibenzo[b,d]furan (2.26 g, 7.01 mmol), dibenzylideneacetone palladium (0.08 g, 0.14 mmol), 2-dicyclohexylphosphino-2',4',6'-triisopropylbiphenyl (0.13 g, 0.36 mmol), and sodium tert-butoxide (1.01 g, 10.5 mmol). The obtained mixture was stirred at 110 degrees C for 3.5 hours. After the reaction, a solid was filtrated, washed with methanol, and recrystallized with toluene to obtain the compound M3-10 (1.44 g, a yield of 41%). The obtained compound was identified as the compound M3-10 by analysis according to LC-MS.

Synthesis Example 11: Synthesis of Compound M3-11

(11-1) Synthesis of Compound M3-11

[Formula 104]

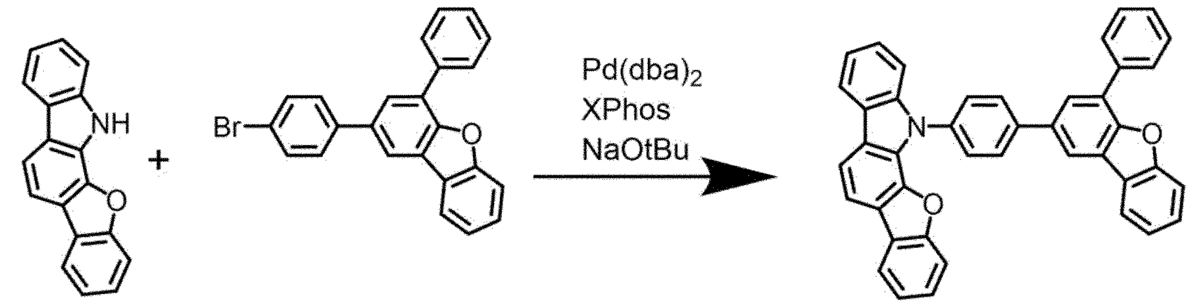


M3-11

The compound M3-11 was obtained in the same manner as in Synthesis Example 10 (10-1) except for using 12H-benzofuro[2,3-a]carbazole in place of 7H-benzofuro[2,3-b]carbazole and using 2-(4-bromophenyl)-4-phenyldibenzo[b,d]furan in place of 2-(4-bromophenyl)dibenzo[b,d]furan. A yield was 46%. The obtained compound was identified as the compound M3-11 by analysis according to LC-MS.

Synthesis Example 12: Synthesis of Compound M3-12

(12-1) Synthesis of Compound M3-12

[Formula 105]

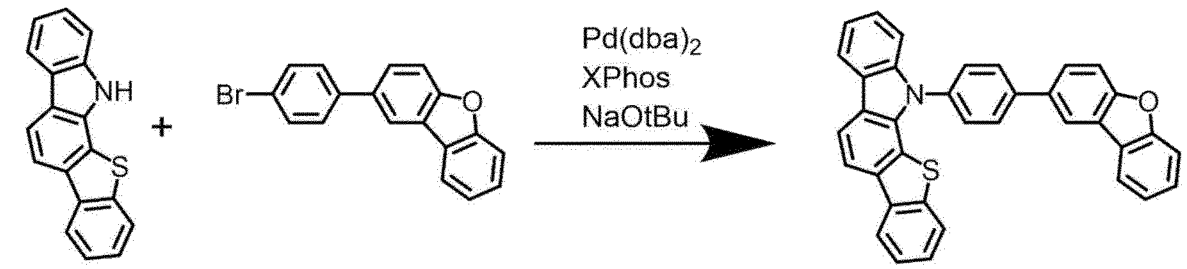


M3-12

The compound M3-12 was obtained in the same manner as in Synthesis Example 10 (10-1) except for using 12H-benzo[4,5]thieno[2,3-a]carbazole in place of 7H-benzofuro[2,3-b]carbazole. A yield was 53%. The obtained compound was identified as the compound M3-12 by analysis according to LC-MS.

Synthesis Example 13: Synthesis of Compound M3-13

(13-1) Synthesis of Compound M3-13

[Formula 106]

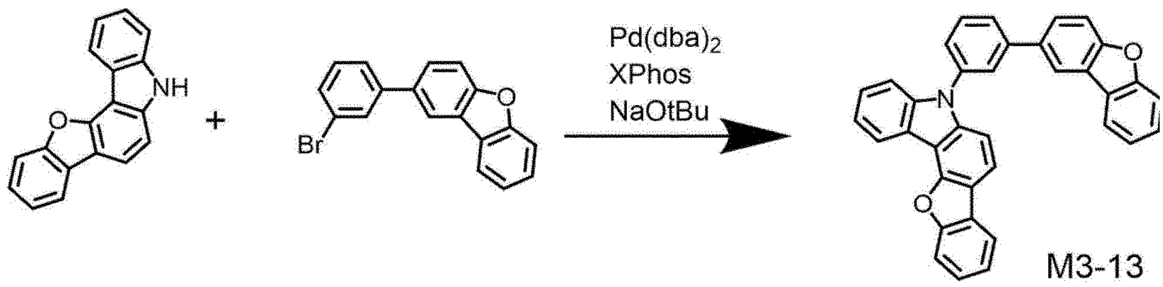


M3-13

The compound M3-13 was obtained in the same manner as in Synthesis Example 10 (10-1) except for using 5H-benzofuro[3,2-c]carbazole in place of 7H-benzofuro[2,3-b]carbazole and using 2-(3-bromophenyl)dibenzo[b,d]furan in place of 2-(4-bromophenyl)dibenzo[b,d]furan. A yield was 53%. The obtained compound was identified as the compound M3-12 by analysis according to LC-MS.

Synthesis Example 15: Synthesis of Compound M3-15

(13-1) Synthesis of Compound M3-15

[Formula 107]

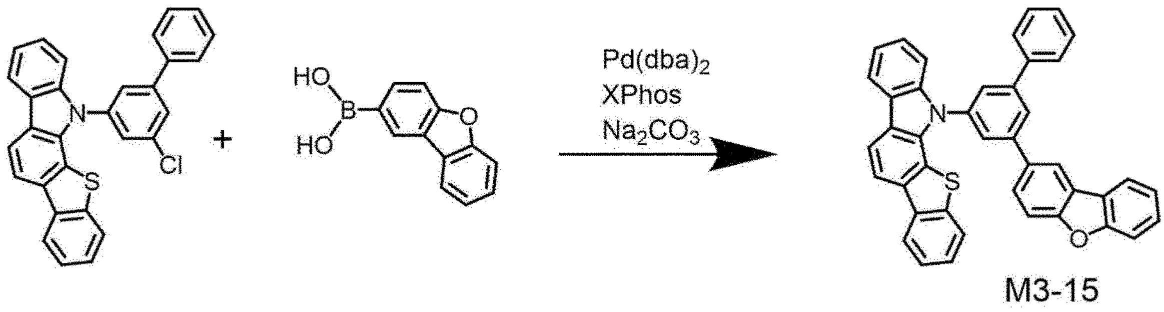


M3-15

Under a nitrogen atmosphere, dimethoxyethane (30 mL) and water (6 mL) were added to a mixture of 12-(5-chloro-[1,1'-biphenyl]-3-yl)-12H-benzo[4,5]thieno[2,3-a]carbazole (2.34 g, 5.09 mmol), dibenzo[b, d]furan-2-ylboronic acid (1.08 g, 5.09 mmol), dibenzylideneacetone palladium (0.09 g, 0.15 mmol), 2-dicyclohexylphosphino-2',4',6'-triisopropylbiphenyl (0.15 g, 0.31 mmol), and sodium carbonate (0.81 g, 7.6 mmol). The obtained mixture was stirred at 80 degrees C for seven hours. After the reaction, a solid was filtrated, washed with methanol, and recrystallized with a mixed solvent of toluene and methanol to obtain the compound M3-15 (1.37 g, a yield of 46%). The obtained compound was identified as the compound M3-15 by analysis according to LC-MS.

EXPLANATION OF CODES

1...organic EL device, 2...substrate, 3...anode, 4...cathode, 5...emitting layer, 6...hole injecting layer, 7...hole transporting layer, 8...electron transporting layer, 9...electron injecting layer

## Claims

**1.** An organic electroluminescence device comprising:

an anode;
a cathode;

an emitting layer provided between the anode and the cathode, wherein

the emitting layer comprises a compound M3 represented by a formula (1) below and a delayed fluorescent compound M2,

the compound M3 and the compound M2 are different in structure, and

a singlet energy $S_1(M3)$ of the compound M3 and a singlet energy $S_1(M2)$ of the compound M2 satisfy a relationship of a numerical formula (Numerical Formula 1) below,

$$S_1(M3) > S_1(M2) \quad \text{...(Numerical Formula 1)}$$

[Formula 1]

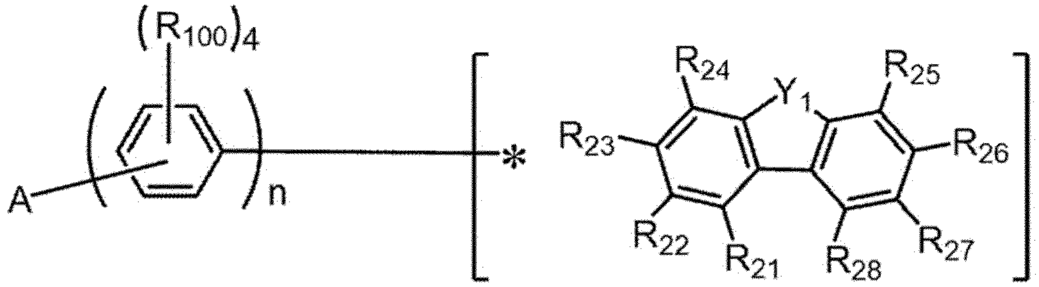

(1)

where, in the formula (1):

A is a group represented by any one of formulae (11A), (11B), (11C), (11D), (11E), and (11F) below;

$Y_1$ is an oxygen atom or a sulfur atom;

n is 0 or 1;

at least one combination of adjacent two or more of $R_{21}$ to $R_{28}$ are mutually bonded to form a substituted or unsubstituted monocyclic ring, mutually bonded to form a substituted or unsubstituted fused ring, or not mutually bonded;

$R_{21}$ to $R_{28}$ forming neither the substituted or unsubstituted monocyclic ring nor the substituted or unsubstituted fused ring, and $R_{100}$ are each independently a hydrogen atom, a halogen atom, a cyano group, a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted alkyl halide group having 1 to 30 carbon atoms, a substituted or unsubstituted cycloalkyl group having 3 to 30 ring carbon atoms, a substituted or unsubstituted alkenyl group having 2 to 30 carbon atoms, a substituted or unsubstituted alkynyl group having 2 to 30 carbon atoms, a substituted or unsubstituted alkylsilyl group having 3 to 30 carbon atoms, a substituted or unsubstituted arylsilyl group having 6 to 60 ring carbon atoms, a substituted or unsubstituted arylphosphoryl group having 6 to 60 ring carbon atoms, a hydroxy group, a substituted or unsubstituted alkoxy group having 1 to 30 carbon atoms, a substituted or unsubstituted aryloxy group having 6 to 30 ring carbon atoms, a group represented by $-N(Rz)_2$, a thiol group, a substituted or unsubstituted alkylthio group having 1 to 30 carbon atoms, a substituted or unsubstituted aralkyl group having 7 to 30 ring carbon atoms, a substituted germanium group, a substituted phosphine oxide group, a nitro group, a substituted boryl group, or a substituted or unsubstituted arylthio group having 6 to 30 ring carbon atoms;

Rz is a substituted or unsubstituted aryl group having 6 to 30 ring carbon atoms, a substituted or unsubstituted heterocyclic group having 5 to 30 ring atoms, or a substituted or unsubstituted alkyl group having 1 to 30 carbon atoms;

two Rz in $-N(Rz)_2$ are mutually the same or different;

a plurality of $R_{100}$ are mutually the same or different;

when n is 0 and $X_1$ in the formulae (11A), (11B), (11C), (11D), (11E), and (11F) is an oxygen atom, $R_{25}$ is not a substituted or unsubstituted dibenzofuranyl group; and

* in the formula (1) represents a bonding position to any one of carbon atoms of a six-membered ring to which $R_{21}$ to $R_{24}$ are bonded,

[Formula 2]

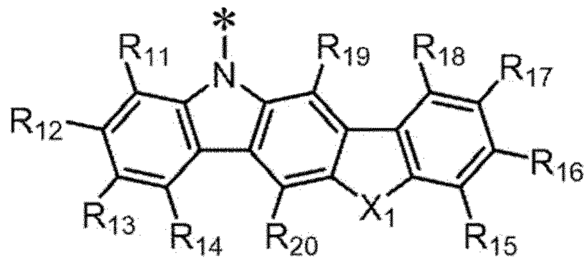

(11A)

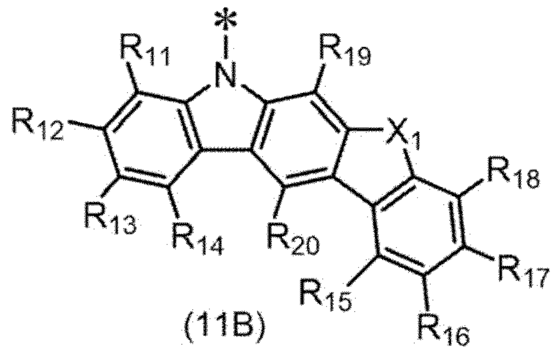

(11B)

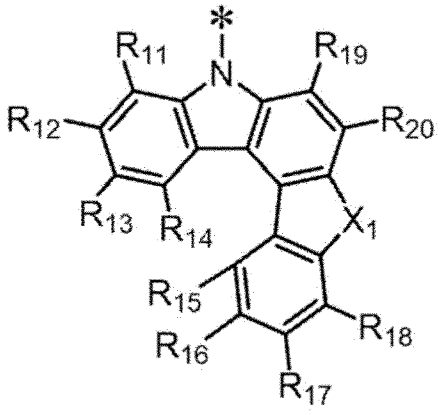

(11C)

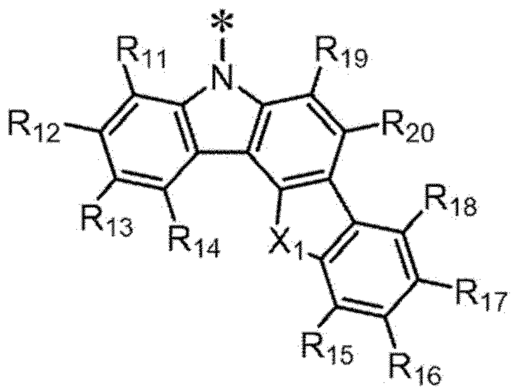

(11D)

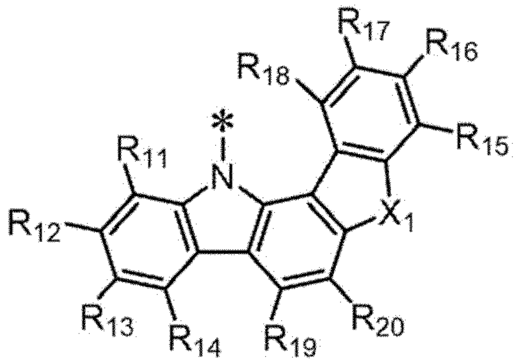

(11E)

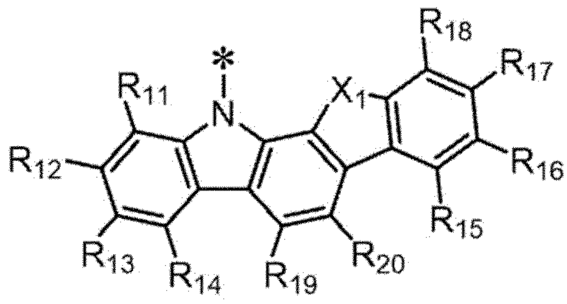

(11F)

where, in the formulae (11A), (11B), (11C), (11D), (11E), and (11F):

$X_1$ is an oxygen atom or a sulfur atom;
at least one combination of adjacent two or more of $R_{11}$ to $R_{14}$ are mutually bonded to form a substituted or unsubstituted monocyclic ring, mutually bonded to form a substituted or unsubstituted fused ring, or not mutually bonded;
at least one combination of adjacent two or more of $R_{15}$ to $R_{18}$ are mutually bonded to form a substituted or unsubstituted monocyclic ring, mutually bonded to form a substituted or unsubstituted fused ring, or not mutually bonded;
$R_{19}$ and $R_{20}$ are each a hydrogen atom;
$R_{11}$ to $R_{18}$ forming neither the substituted or unsubstituted monocyclic ring nor the substituted or unsubstituted fused ring each independently represent the same as $R_{21}$ to $R_{28}$ forming neither the substituted or unsubstituted monocyclic ring nor the substituted or unsubstituted fused ring in the formula (1);
when n is 0, * represents a bonding position to any one of carbon atoms of a six-membered ring to which $R_{21}$ to $R_{24}$ are bonded in the formula (1); and
when n is 1, * represents a bonding position to any one of carbon atoms of a benzene ring to which

$R_{100}$ is bonded in the formula (1).

**2.** The organic electroluminescence device according to claim 1, wherein n is 1.

**3.** The organic electroluminescence device according to claim 1 or 2, wherein

the compound M3 is a compound represented by a formula (12A) below,

[Formula 3]

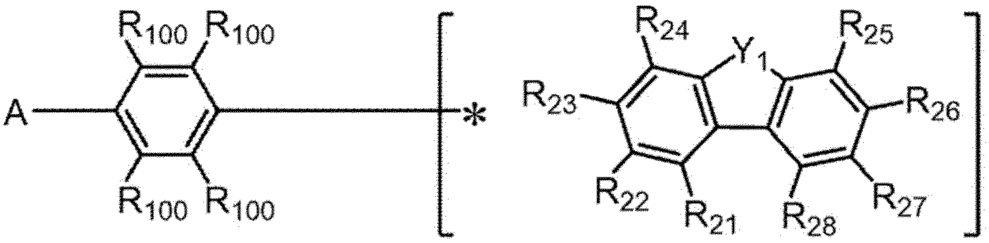

(12A)

where: A, $R_{100}$, $Y_1$, and $R_{21}$ to $R_{28}$ in the formula (12A) respectively independently represent the same as A, $R_{100}$, $Y_1$, and $R_{21}$ to $R_{28}$ in the formula (1), a plurality of $R_{100}$ being mutually the same or different; and
* represents a bonding position to any one of carbon atoms of a six-membered ring to which $R_{21}$ to $R_{24}$ are bonded.

**4.** The organic electroluminescence device according to claim 1 or 2, wherein

the compound M3 is a compound represented by a formula (12B) below,

[Formula 4]

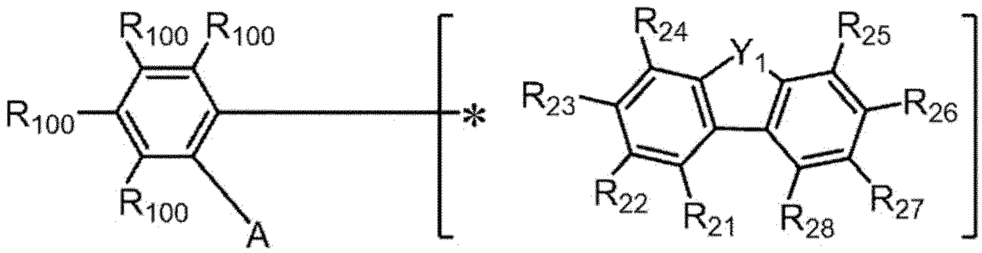

(12B)

where: A, $R_{100}$, $Y_1$, and $R_{21}$ to $R_{28}$ in the formula (12B) respectively independently represent the same as A, $R_{100}$, $Y_1$, and $R_{21}$ to $R_{28}$ in the formula (1), a plurality of $R_{100}$ being mutually the same or different; and
* represents a bonding position to any one of carbon atoms of a six-membered ring to which $R_{21}$ to $R_{24}$ are bonded.

**5.** The organic electroluminescence device according to claim 1 or 2, wherein

the compound M3 is a compound represented by a formula (12C) below,

[Formula 5]

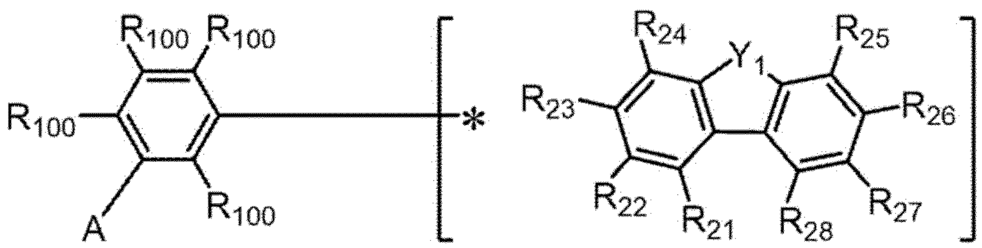

(12C)

where: A, $R_{100}$, $Y_1$, and $R_{21}$ to $R_{28}$ in the formula (12C) respectively independently represent the same as A, $R_{100}$, $Y_1$, and $R_{21}$ to $R_{28}$ in the formula (1), a plurality of $R_{100}$ being mutually the same or different; and
* represents a bonding position to any one of carbon atoms of a six-membered ring to which $R_{21}$ to $R_{24}$ are bonded.

**6.** The organic electroluminescence device according to claim 1, wherein

n is 0, or
n is 1 and $R_{100}$ is a hydrogen atom.

**7.** The organic electroluminescence device according to claim 6, wherein n is 0.

**8.** The organic electroluminescence device according to any one of claims 1 to 7, wherein A is a group represented by the formula (11A), (11B), (11C), (11E) or (11F).

**9.** The organic electroluminescence device according to any one of claims 1 to 7, wherein A is a group represented by the formula (11E) or (11F).

**10.** The organic electroluminescence device according to claim 9, wherein A is a group represented by the formula (11F).

**11.** The organic electroluminescence device according to any one of claims 1 to 10, wherein $X_1$ is an oxygen atom.

**12.** The organic electroluminescence device according to any one of claims 1 to 10, wherein $X_1$ and $Y_1$ are each an oxygen atom.

**13.** The organic electroluminescence device according to any one of claims 1 to 10, wherein $X_1$ is a sulfur atom.

**14.** The organic electroluminescence device according to claim 13, wherein
the compound M3 is a compound represented by a formula (12A-1) below,

[Formula 6]

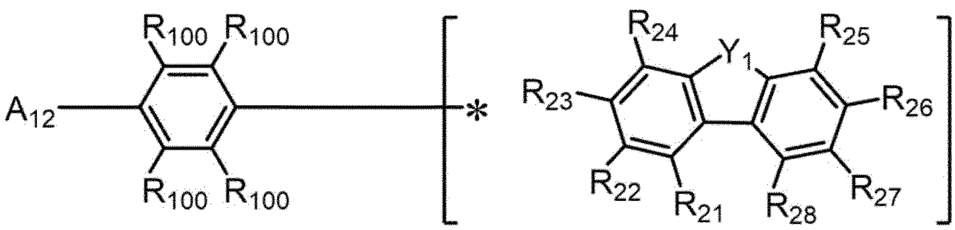

(12A-1)

where: in the formula (12A-1):

$A_{12}$ is a group represented by any one of formulae (11A-1), (11B-1), (11C-1), (11D-1), (11E-1), and (11F-1) below;
$R_{100}$, $Y_1$, and $R_{21}$ to $R_{28}$ respectively independently represent the same as $R_{100}$, $Y_1$, and $R_{21}$ to $R_{28}$ in the formula (1), a plurality of $R_{100}$ being mutually the same or different; and
* represents a bonding position to any one of carbon atoms of a six-membered ring to which $R_{21}$ to $R_{24}$ are bonded,

[Formula 7]

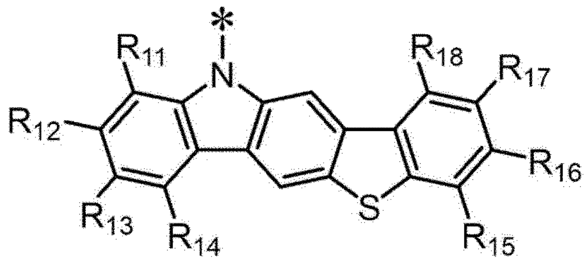

(11A-1)

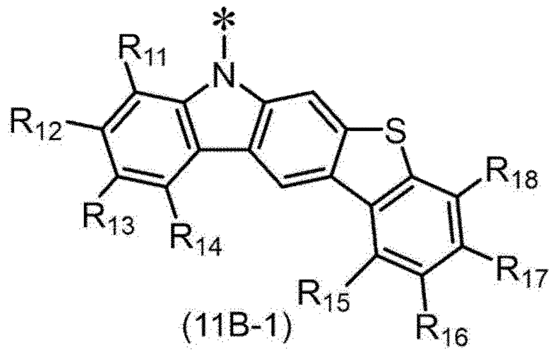

(11B-1)

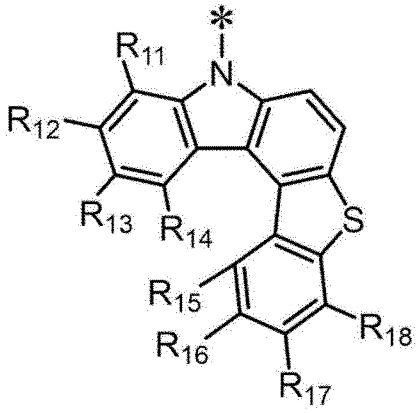

(11C-1)

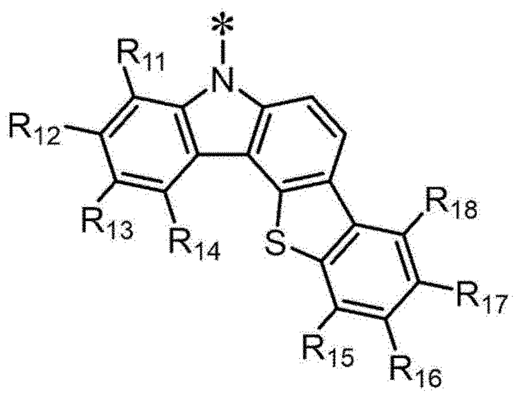

(11D-1)

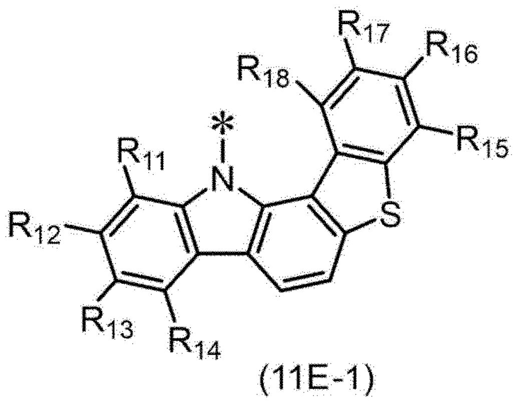

(11E-1)

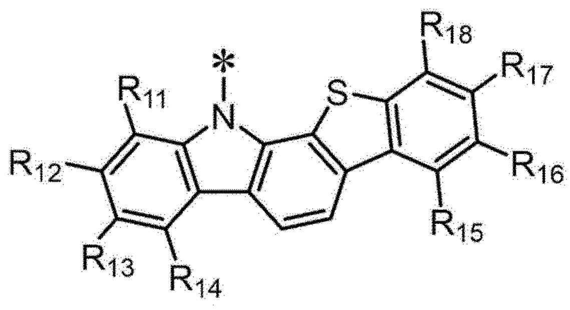

(11F-1)

where, in the formulae (11A-1), (11B-1), (11C-1), (11D-1), (11E-1), and (11F-1), $R_{11}$ to $R_{18}$ each independently represent the same as $R_{11}$ to $R_{18}$ in the formula (1); and each* represents a bonding position.

**15.** The organic electroluminescence device according to any one of claims 1 to 14, wherein $Y_1$ is an oxygen atom.

**16.** The organic electroluminescence device according to any one of claims 1 to 5, wherein the compound M3 is a compound represented by a formula (100) below,

[Formula 8]

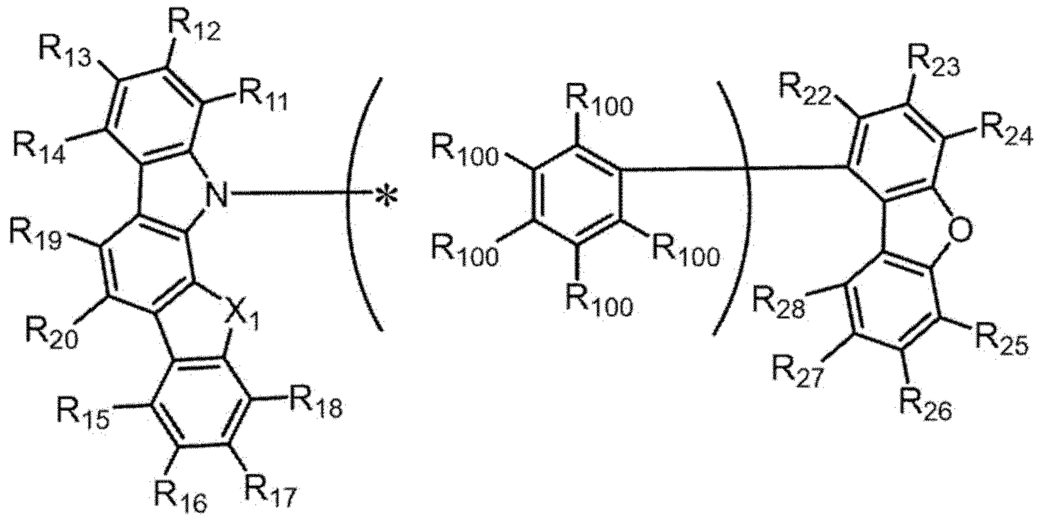

(100)

where, in the formula (100):

$X_1$ is an oxygen atom or a sulfur atom;

$R_{19}$ and $R_{20}$ are each a hydrogen atom;

$R_{100}$, $R_{11}$ to $R_{18}$, and $R_{22}$ to $R_{28}$ are each independently a hydrogen atom, a group represented by $-(L_{101})nx-R_{101}$;

nx is 0, 1, 2, or 3;

when a plurality of groups represented by $-(L_{101})nx-R_{101}$ are present, the plurality of groups represented by $-(L_{101})nx-R_{101}$ are mutually the same or different;

a plurality of $R_{100}$ are mutually the same or different;

$R_{101}$ is an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, an unsubstituted dibenzothienyl group, an unsubstituted (9-dibenzofuranyl)carbazolyl group, an unsubstituted (9-dibenzothienyl)carbazolyl group, a monovalent group derived from a compound represented by a formula (101) below, a monovalent group derived from a compound represented by a formula (102) below, a monovalent group derived from a compound represented by a formula (103) below, a monovalent group derived from a compound represented by a formula (104) below, a monovalent group derived from a compound represented by a formula (105) below, or a monovalent group derived from a compound represented by a formula (106) below;

$L_{101}$ is a substituted or unsubstituted alkylene group having 1 to 30 carbon atoms, a substituted or unsubstituted phenylene group, a substituted or unsubstituted dibenzofuranylene group, a substituted or unsubstituted dibenzothienylene group, a substituted or unsubstituted carbazolylene group, a substituted or unsubstituted (9-dibenzofuranyl)carbazolylene group, a substituted or unsubstituted (9-dibenzothienyl)carbazolylene group, a divalent group derived from a compound represented by the formula (101) below, a divalent group derived from a compound represented by the formula (102) below, a divalent group derived from a compound represented by the formula (103) below, a divalent group derived from a compound represented by the formula (104) below, a divalent group derived from a compound represented by the formula (105) below, or a divalent group derived from a compound represented by the formula (106) below;

when $L_{101}$ is a substituted alkylene group having 1 to 30 carbon atoms, a substituted phenylene group, a substituted dibenzofranylene group, a substituted dibenzothienylene group, a substituted carbazolylene group, a substituted (9-dibenzofuranyl)carbazolylene group, or a substituted (9-dibenzothienyl)carbazolylene group, a substituent for $L_{101}$ is each independently an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;

when two or more $L_{101}$ are present, the two or more $L_{101}$ are mutually the same or different;

when two or more $R_{101}$ are present, the two or more $R_{101}$ are mutually the same or different; and

* represents a bonding position to any one of carbon atoms of a benzene ring to which $R_{100}$ is bonded,

[Formula 9]

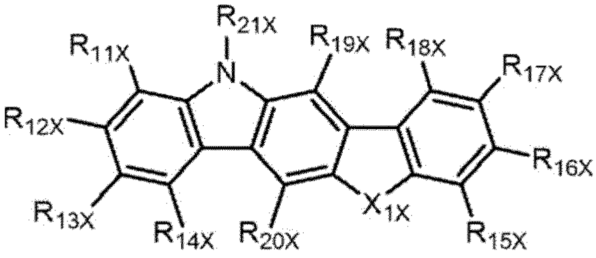

(101)

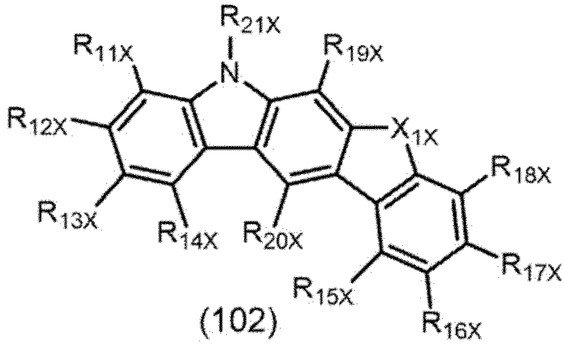

(102)

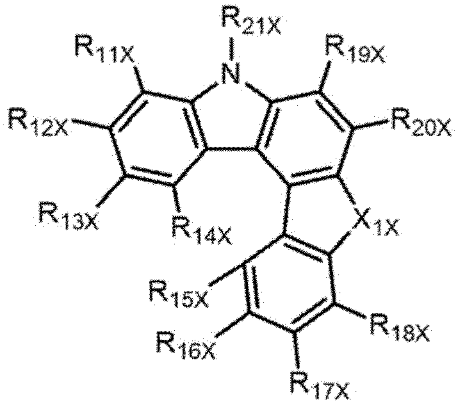

(103)

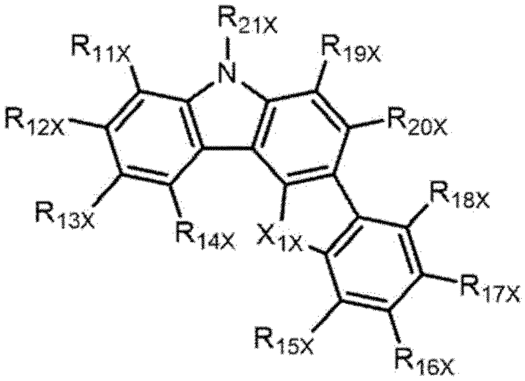

(104)

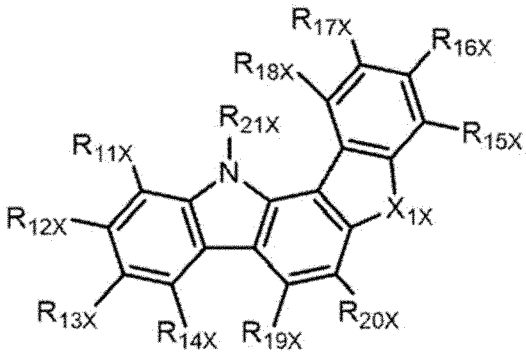

(105)

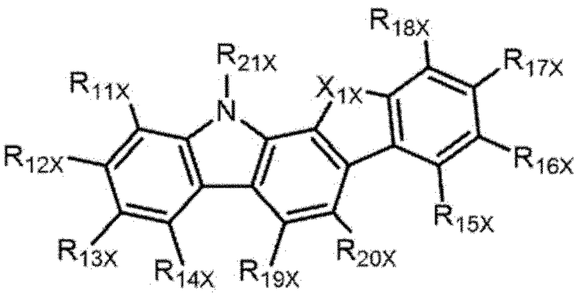

(106)

where, in the formulae (101) to (106):

$X_{1X}$ is an oxygen atom or a sulfur atom;
$R_{11X}$ to $R_{21X}$ are each independently a hydrogen atom, an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;
when $R_{101}$ is a monovalent group derived from a compound of any one of the formulae (101) to (106) and nx is 0, any one atom of: carbon atoms of a six-membered ring to which $R_{11X}$ to $R_{20X}$ are bonded; and a nitrogen atom bonded to $R_{21X}$, is bonded to any one of carbon atoms of a six-membered ring to which $R_{11}$ to $R_{18}$, $R_{22}$ to $R_{28}$, and $R_{100}$ are bonded,
when nx is 1, 2, or 3, any one atom of: carbon atoms of the six-membered ring to which $R_{11X}$ to $R_{20X}$ are bonded; and a nitrogen atom bonded to $R_{21X}$, is bonded to $L_{101}$;
when $L_{101}$ is a divalent group derived from a compound of any one of the formulae (101) to (106) and nx is 1, one of two atoms of: carbon atoms of a six-membered ring to which $R_{11X}$ to $R_{20X}$ are bonded; and a nitrogen atom bonded to $R_{21X}$, is bonded to $R_{101}$ and the other of the two atoms is bonded to any one of carbon atoms of a six-membered ring to which $R_{11}$ to $R_{18}$, $R_{22}$ to $R_{28}$, and $R_{100}$ are bonded; and

when $L_{101}$ is a divalent group derived from a compound of any one of the formulae (101) to (106) and nx is 2 or 3, one of two atoms of: carbon atoms of a six-membered ring to which $R_{11X}$ to $R_{20X}$ are bonded; and a nitrogen atom bonded to $R_{21X}$, is bonded to $R_{101}$ or $L_{101}$, and the other of the two atoms is bonded to $L_{101}$ or any one of carbon atoms of a six-membered ring to which $R_{11}$ to $R_{18}$, $R_{22}$ to $R_{28}$, and $R_{100}$ are bonded.

**17.** The organic electroluminescence device according to claim 1, wherein when n is 0, $R_{25}$ is neither a substituted or unsubstituted dibenzofuranyl group nor a substituted or unsubstituted dibenzothienyl group.

**18.** The organic electroluminescence device according to claim 17, wherein when n is 0, $R_{21}$ to $R_{28}$ are neither a substituted or unsubstituted dibenzofuranyl group nor a substituted or unsubstituted dibenzothienyl group.

**19.** The organic electroluminescence device according to claim 17 or 18, wherein when n is 0, in the formula (1), a carbon atom of a six-membered ring to which $R_{22}$ is bonded is not bonded to *.

**20.** The organic electroluminescence device according to any one of claims 1 to 19, wherein $R_{11}$ to $R_{18}$ are each a hydrogen atom in the formulae (11A), (11B), (11C), (11D), (11E), and (11F).

**21.** The organic electroluminescence device according to claim 1, wherein in the emitting layer, only the compound M3 has a larger singlet energy $S_1$ than a singlet energy $S_1(M2)$ of the delayed fluorescent compound M2.

**22.** The organic electroluminescence device according to claim 21, wherein when n is 1, $R_{100}$ is not a substituted or unsubstituted dibenzofuranyl group.

**23.** The organic electroluminescence device according to any one of claims 1 to 22, wherein

the emitting layer further comprises a fluorescent compound M1, and
a singlet energy $S_1(M1)$ of the compound M1 and a singlet energy $S_1(M2)$ of the compound M2 satisfy a relationship of a numerical formula (Numerical Formula 2) below,

$$S_1(M2) > S_1(M1) \quad \text{... (Numerical Formula 2).}$$

**24.** The organic electroluminescence device according to claim 23, wherein
the compound M1 is represented by a formula (2A) below and emits light having a maximum peak wavelength in a range from 500 nm to 560 nm,

[Formula 10]

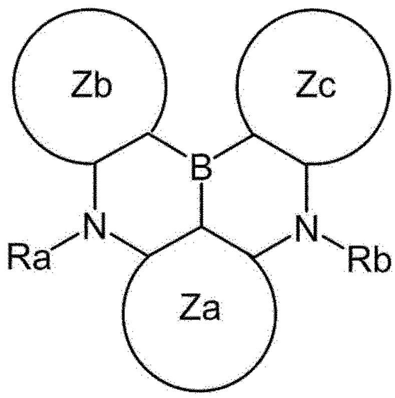

(2A)

where, in the formula (2A):

a Za ring, a Zb ring, and a Zc ring are each independently a substituted or unsubstituted aromatic hydrocarbon ring having 6 to 50 ring carbon atoms, or a substituted or unsubstituted heterocyclic ring having 5 to 50 ring atoms;
Ra is bonded to the Za ring or the Zb ring to form a substituted or unsubstituted heterocyclic ring, or not bonded

thereto to form no substituted or unsubstituted heterocyclic ring;
Rb is bonded to the Za ring or the Zc ring to form a substituted or unsubstituted heterocyclic ring, or not bonded thereto to form no substituted or unsubstituted heterocyclic ring; and
Ra and Rb not forming the substituted or unsubstituted heterocycling ring are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

**25.** The organic electroluminescence device according to any one of claims 1 to 24, wherein the emitting layer comprises no metal complex.

**26.** An electronic device comprising the organic electroluminescence device according to any one of claims 1 to 25.

**27.** A compound represented by a formula (100) below,

[Formula 11]

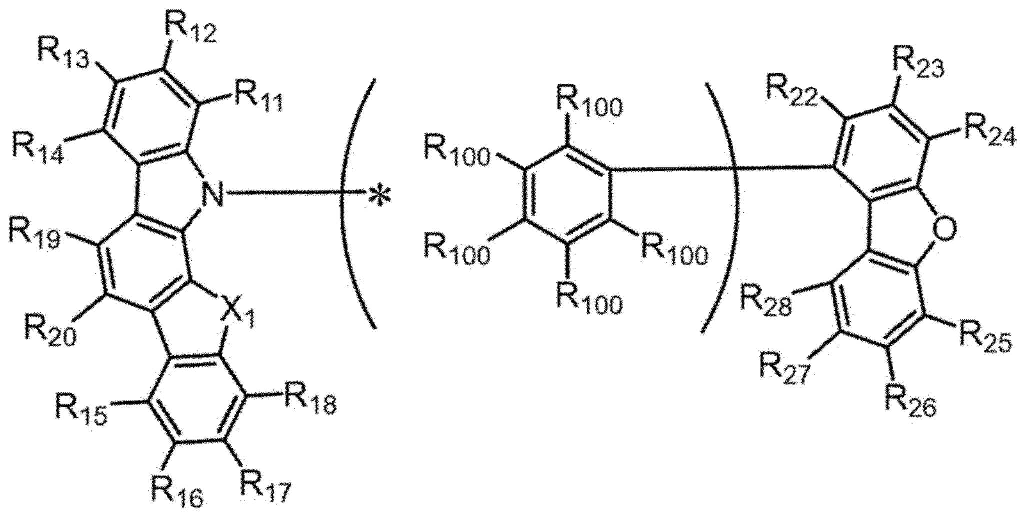

(100)

where, in the formula (100):

$X_1$ is an oxygen atom or a sulfur atom;
$R_{100}$, $R_{11}$ to $R_{20}$, and $R_{22}$ to $R_{28}$ are each independently a hydrogen atom, or a group represented by $-(L_{101})nx-R_{101}$;
nx is 0, 1, 2, or 3;
when a plurality of groups represented by $-(L_{101})nx-R_{101}$ are present, the plurality of groups represented by $-(L_{101})nx-R_{101}$ are mutually the same or different;
a plurality of $R_{100}$ are mutually the same or different;
$R_{101}$ is an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, an unsubstituted dibenzothienyl group, an unsubstituted (9-dibenzofuranyl)carbazolyl group, an unsubstituted (9-dibenzothienyl)carbazolyl group, a monovalent group derived from a compound represented by a formula (101) below, a monovalent group derived from a compound represented by a formula (102) below, a monovalent group derived from a compound represented by a formula (103) below, a monovalent group derived from a compound represented by a formula (104) below, a monovalent group derived from a compound represented by a formula (105) below, or a monovalent group derived from a compound represented by a formula (106) below;
$L_{101}$ is a substituted or unsubstituted alkylene group having 1 to 30 carbon atoms, a substituted or unsubstituted phenylene group, a substituted or unsubstituted dibenzofuranylene group, a substituted or unsubstituted dibenzothienylene group, a substituted or unsubstituted carbazolylene group, a substituted or unsubstituted (9-dibenzofuranyl)carbazolylene group, a substituted or unsubstituted (9-dibenzothienyl)carbazolylene group, a divalent group derived from a compound represented by the formula (101) below, a divalent group derived from a

compound represented by the formula (102) below, a divalent group derived from a compound represented by the formula (103) below, a divalent group derived from a compound represented by the formula (104) below, a divalent group derived from a compound represented by the formula (105) below, or a divalent group derived from a compound represented by the formula (106) below;
when $L_{101}$ is a substituted alkylene group having 1 to 30 carbon atoms, a substituted phenylene group, a substituted dibenzofranylene group, a substituted dibenzothienylene group, a substituted carbazolylene group, a substituted (9-dibenzofuranyl)carbazolylene group, or a substituted (9-dibenzothienyl)carbazolylene group, a substituent for $L_{101}$ is each independently an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;
when two or more $L_{101}$ are present, the two or more $L_{101}$ are mutually the same or different;
when two or more $R_{101}$ are present, the two or more $R_{101}$ are mutually the same or different; and
* represents a bonding position to any one of carbon atoms of a benzene ring to which $R_{100}$ is bonded,

[Formula 12]

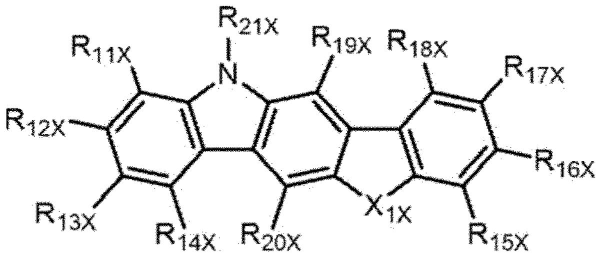

(101)

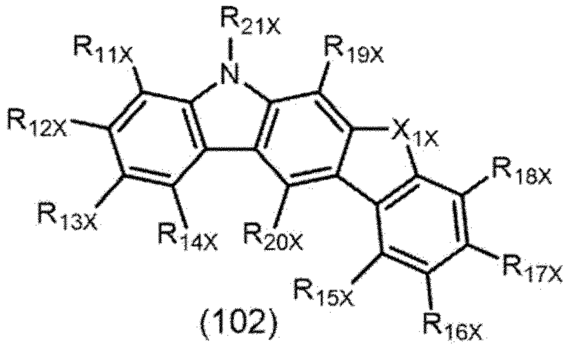

(102)

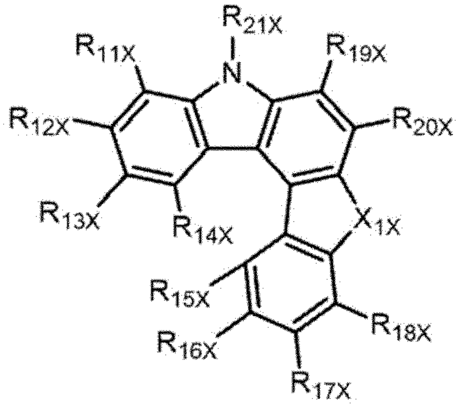

(103)

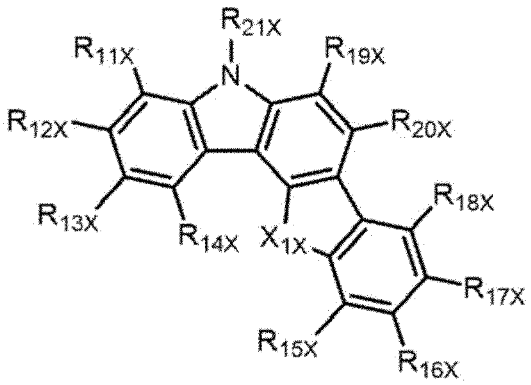

(104)

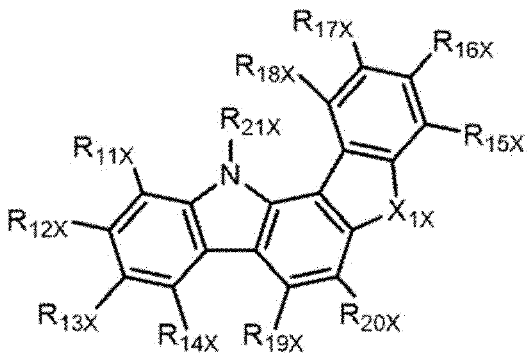

(105)

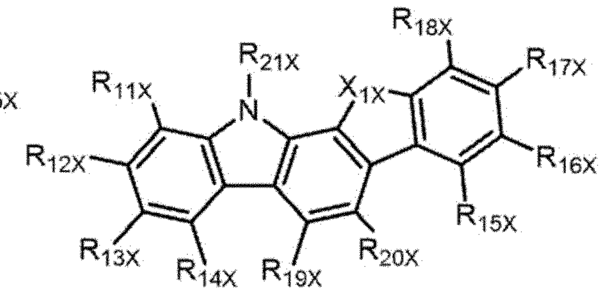

(106)

where, in the formulae (101) to (106):

$X_{1X}$ is an oxygen atom or a sulfur atom;
$R_{11X}$ to $R_{21X}$ are each independently a hydrogen atom, an unsubstituted alkyl group having 1 to 30 carbon atoms, an unsubstituted phenyl group, an unsubstituted (9-phenyl)carbazolyl group, an unsubstituted 9-carbazolyl group, an unsubstituted dibenzofuranyl group, or an unsubstituted dibenzothienyl group;
when $R_{101}$ is a monovalent group derived from a compound of any one of the formulae (101) to (106) and nx is 0, any one atom of: carbon atoms of a six-membered ring to which $R_{11X}$ to $R_{20X}$ are bonded; and a nitrogen atom bonded to $R_{21X}$, is bonded to any one of carbon atoms of a six-membered ring to which $R_{11}$ to $R_{20}$, $R_{22}$ to $R_{28}$, and $R_{100}$ are bonded,
when nx is 1, 2, or 3, any one atom of: carbon atoms of the six-membered ring to which $R_{11X}$ to $R_{20X}$ are bonded; and a nitrogen atom bonded to $R_{21X}$, is bonded to $L_{101}$;
when $L_{101}$ is a divalent group derived from a compound of any one of the formulae (101) to (106) and nx is 1, one of two atoms of: carbon atoms of a six-membered ring to which $R_{11X}$ to $R_{20X}$ are bonded; and a nitrogen atom bonded to $R_{21X}$, is bonded to $R_{101}$ and the other of the two atoms is bonded to any one of carbon atoms of a six-membered ring to which $R_{11}$ to $R_{20}$, $R_{22}$ to $R_{28}$, and $R_{100}$ are bonded; and
when $L_{101}$ is a divalent group derived from a compound of any one of the formulae (101) to (106) and nx is 2 or 3, one of two atoms of: carbon atoms of a six-membered ring to which $R_{11X}$ to $R_{20X}$ are bonded; and a nitrogen atom bonded to $R_{21X}$, is bonded to $R_{101}$ or $L_{101}$, and the other of the two atoms is bonded to $L_{101}$ or any one of carbon atoms of a six-membered ring to which $R_{11}$ to $R_{20}$, $R_{22}$ to $R_{28}$, and $R_{100}$ are bonded.

FIG.1

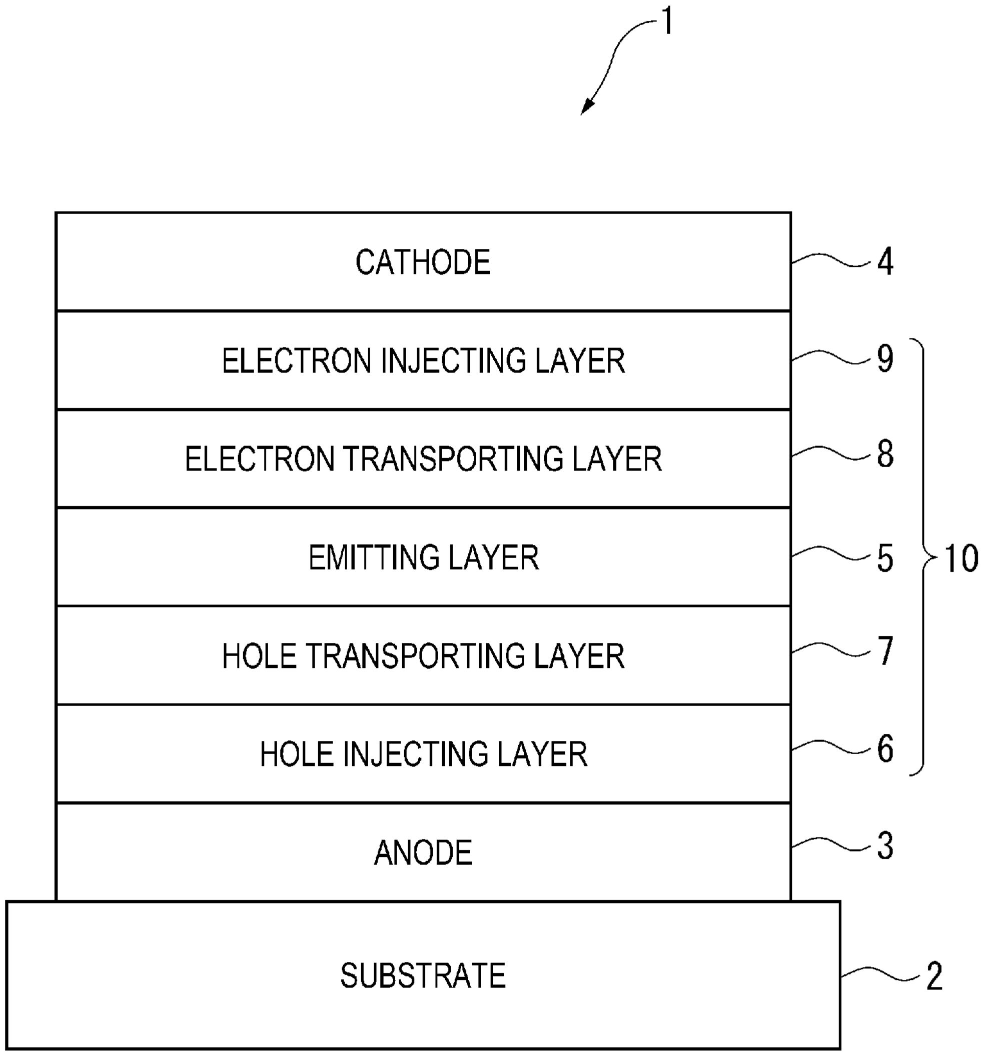
1
CATHODE
4
ELECTRON INJECTING LAYER
9
ELECTRON TRANSPORTING LAYER
8
EMITTING LAYER
5
10
HOLE TRANSPORTING LAYER
7
HOLE INJECTING LAYER
6
ANODE
3
SUBSTRATE
2

FIG.2

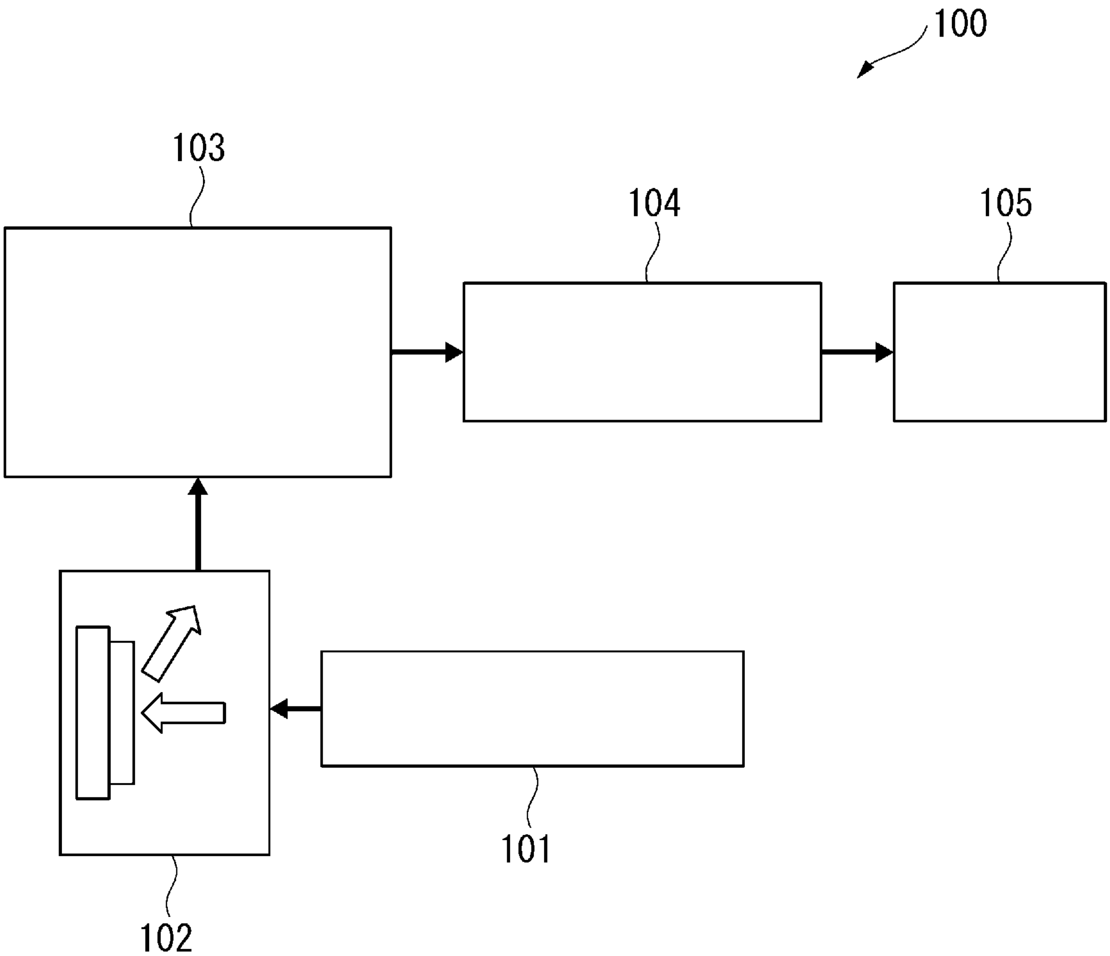

FIG.3

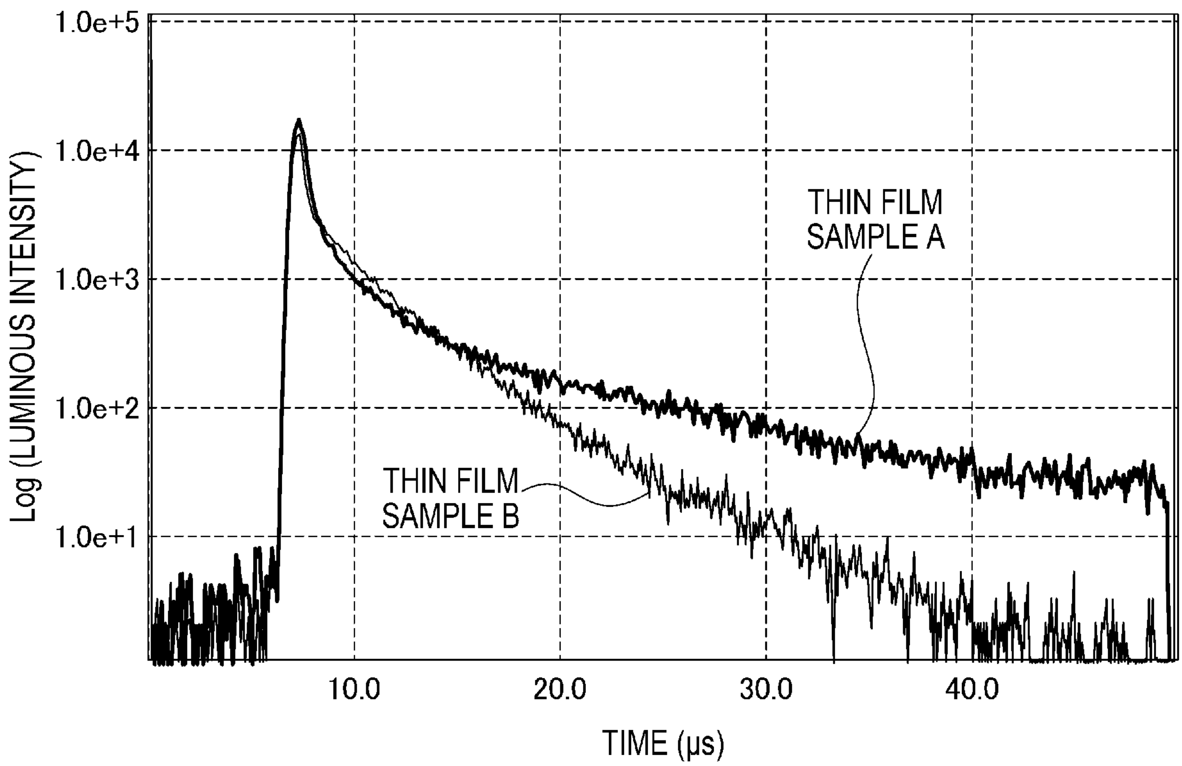
1.0e+5
1.0e+4
1.0e+3
1.0e+2
1.0e+1
Log (LUMINOUS INTENSITY)
THIN FILM SAMPLE A
THIN FILM SAMPLE B
10.0
20.0
30.0
40.0
TIME (μs)

FIG.4

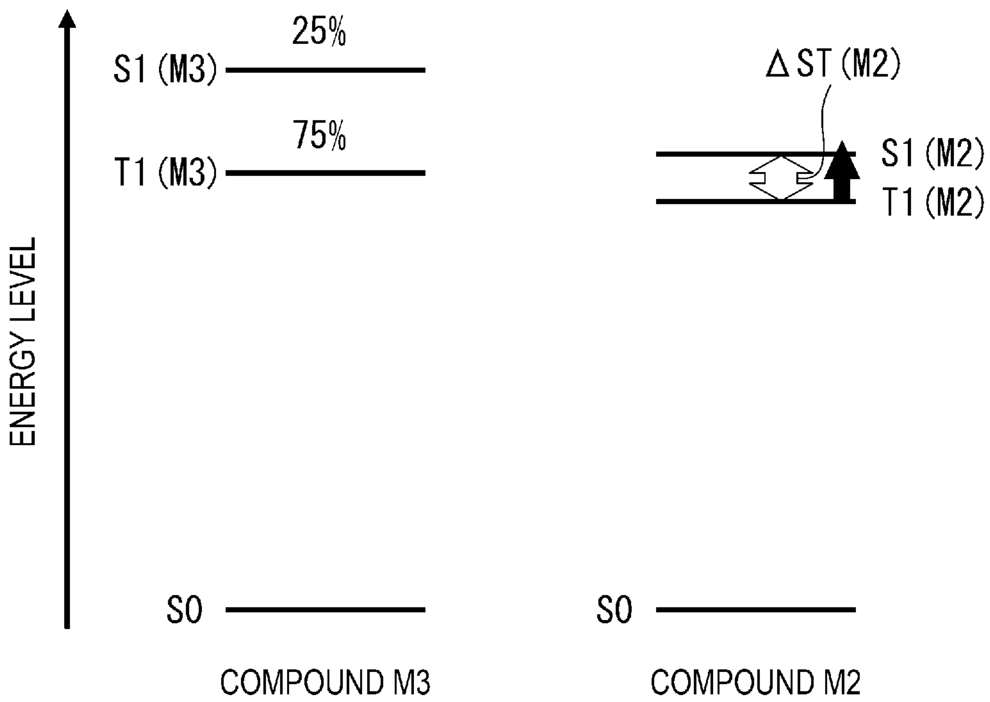
25%
S1 (M3)
75%
T1 (M3)
ENERGY LEVEL
S0
COMPOUND M3
ΔST (M2)
S1 (M2)
T1 (M2)
S0
COMPOUND M2

FIG.5

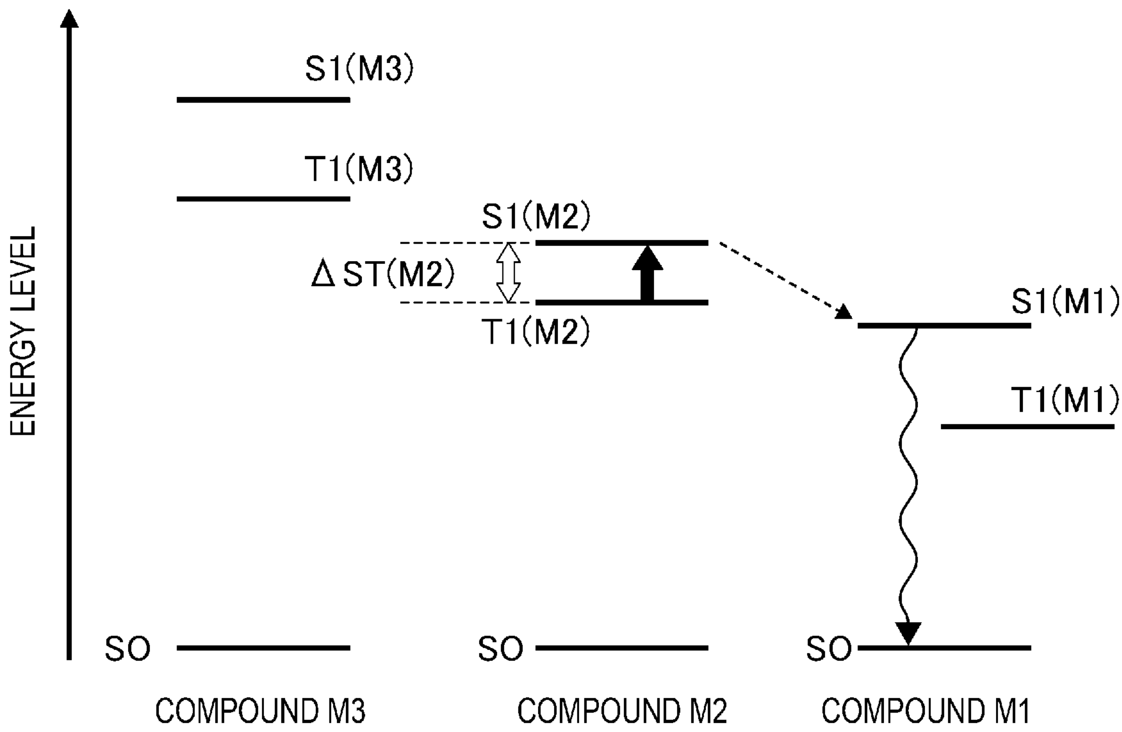
ENERGY LEVEL
S1(M3)
T1(M3)
S1(M2)
ΔST(M2)
T1(M2)
S1(M1)
T1(M1)
SO
SO
SO
COMPOUND M3
COMPOUND M2
COMPOUND M1

## INTERNATIONAL SEARCH REPORT

International application No.
**PCT/JP2022/012157**

**A. CLASSIFICATION OF SUBJECT MATTER**

***H01L 51/50***(2006.01)i; ***C07D 491/048***(2006.01)i; ***C07D 495/04***(2006.01)i; ***C09K 11/06***(2006.01)i
FI: H05B33/14 B; C09K11/06 635; C09K11/06 690; C07D495/04 103; C07D491/048 CSP

According to International Patent Classification (IPC) or to both national classification and IPC

**B. FIELDS SEARCHED**

Minimum documentation searched (classification system followed by classification symbols)

H01L51/50; C07D491/048; C07D495/04; C09K11/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | CN 111253410 A (JIANGSU SUNERA TECH. CO., LTD.) 09 June 2020 (2020-06-09) paragraphs [0020], [0028], [0131] | 1-3, 5, 8-15, 17-22, 26<br>4, 6-7, 16, 23-25, 27 |
| Y | WO 2019/176971 A1 (KYUSHU UNIV.) 19 September 2019 (2019-09-19) paragraph [0053] | 1-3, 5, 8-15, 17-22, 26 |
| A | KR 10-2019-0089625 A (SAMSUNG ELECTRONICS CO., LTD.) 31 July 2019 (2019-07-31) paragraph [0130] | 16, 27 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 May 2022** | **07 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/012157**

| Patent document cited in search report | Publication date (day/month/year) | Patent family member(s) | Publication date (day/month/year) |
|---|---|---|---|
| CN 111253410 A | 09 June 2020 | (Family: none) | |
| WO 2019/176971 A1 | 19 September 2019 | US 2020/0399246 A1<br>paragraph [0066] | |
| KR 10-2019-0089625 A | 31 July 2019 | US 2019/0229276 A1<br>paragraph [0107] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013011891 A
- WO 2020122118 A


### Non-patent literature cited in the description


- Yuki Hando-tai no Debaisu Bussei (Device Physics of Organic Semiconductors). Kodansha, 01 April 2012, 261-268
- Yuki Hando-tai no Debaisu Bussei (Device Physics of Organic Semiconductors). Kodansha, 261-268
- *Nature,* 2012, vol. 492, 234-238
- **MORRIS et al.** *J. Phys. Chem,* 1976, vol. 80, 969